# EUROPEAN PATENT SPECIFICATION

(11) **EP 1 685 113 B1**
(45) Date of publication and mention of the grant of the patent: **30.07.2008**
(21) Application number: 04818779.3
(22) Date of filing: 15.11.2004
(51) Int. Cl.: C07D 231/14, C07D 409/10, C07D 405/10, C07D 401/10, C07D 231/12, A61K 31/415, A61P 3/00

(54) **SUBSTITUTED PYRAZOLES AS PPAR AGONISTS**
SUBSTITUIERTE PYRAZOLE ALS PPAR-AGONISTEN
PYRAZOLES SUBSTITUES UTILISES EN TANT QU'AGONISTES DE PPAR

(30) Priority: 17.11.2003 GB 0326747; 19.12.2003 GB 0329462
(43) Date of publication of application: 02.08.2006
(73) Proprietor: SMITHKLINE BEECHAM CORPORATION, Philadelphia, PA 19101 (US)
(72) Inventor: Faucher, N. E., GlaxoSmithKline, Ctre de Recherche, F-91940 Les Ulis (FR); Martres, Paul GlaxoSmithKline Centre de Recherches, F-91940 Les Ulis (FR)
(74) Representative: Learoyd, Stephanie Anne
(86) International application number: PCT/EP2004/012965
(87) International publication number: WO 2005/049578

(56) References cited:
- WO-A-01/40207
- WO-A-97/31907
- US-B1- 6 528 525

## Description

### Field of the Invention

The present invention relates to certain novel compounds. In particular, the present invention relates to compounds that activate both the alpha and gamma subtypes of the human peroxisome proliferator activated receptor. The present invention also relates to methods for preparing the compounds and methods for prevention or treatment of PPAR mediated diseases or conditions.

### Background to the Invention

Several independent risk factors have been associated with cardiovascular disease. These include hypertension, increased fibrinogen levels, high levels of triglycerides, elevated LDL cholesterol, elevated total cholesterol, and low levels of HDL cholesterol. HMG CoA reductase inhibitors ("statins") are useful for treating conditions characterized by high LDL-c levels. It has been shown that lowering LDL-c is not sufficient for reducing the risk of cardiovascular disease in some patients, particularly those with normal LDL-c levels. This population pool is identified by the independent risk factor of low HDL-c. The increased risk of cardiovascular disease associated with low HDL-c levels has not yet been successfully addressed by drug therapy (i.e., currently there are no drugs on the market that are useful for raising HDL-c >40%). (Bisgaier, C. L.; Pape, M. E. Curr. Pharm. Des. 1998, 4, 53-70).

Syndrome X (including metabolic syndrome) is loosely defined as a collection of abnormalities including hyperinsuinlemia, obesity, elevated levels of trigycerides, uric acid, fibrinogen, small dense LDL-c particles, and plasminogen activator inhibitor 1 (PAI-1), and decreased levels of HDL-c.

NIDDM is described as insulin resistance which in turn causes anomalous glucose output and a decrease in glucose uptake by skeletal muscle. These factors eventually lead to impaired glucose tolerance (IGT) and hyperinsulinemia.

Peroxisome Proliferator Activated Receptors (PPARs) are orphan receptors belonging to the steroid/retinoid receptor superfamily of ligand-activated transcription factors. See, for example, Willson, T. M. and Wahli, W., Curr. Opin. Chem. Biol., (1997), Vol. 1, pp 235-241.

Three mammalian Peroxisome Proliferator-Activated Receptors have been isolated and termed PPAR-alpha, PPAR-gamma, and PPAR-delta (also known as NUC1 or PPAR-beta). These PPARs regulate expression of target genes by binding to DNA sequence elements, termed PPAR response elements (PPRE). To date, PPRE's have been identified in the enhancers of a number of genes encoding proteins that regulate lipid metabolism suggesting that PPARs play a pivotal role in the adipogenic signaling cascade and lipid homeostasis (H. Keller and W. Wahli, Trends Endocrin. Met 291-296, 4 (1993)).

Certain compounds that activate or otherwise interact with one or more of the PPARs have been implicated in the regulation of triglyceride and cholesterol levels in animal models. See, for example, WO 01/40207, WO 01/00603, WO 97/31907, WO 02/46174 (Glaxo Group Ltd et al).

### Summary of the Invention

According to a first aspect of the invention there is provided a compound of formula (I) and pharmaceutically acceptable salts, solvates and hydrolysable esters thereof: 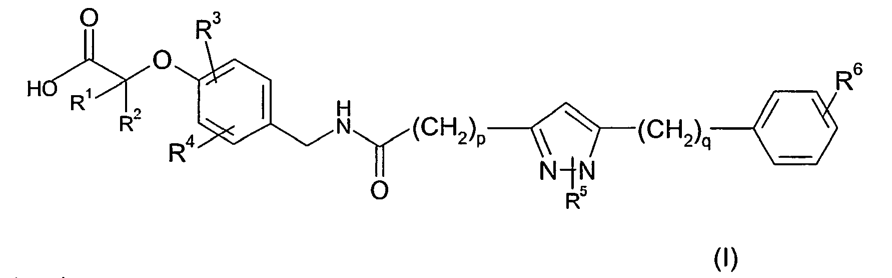 wherein:
p is O or 1;
q is O or 1;
R¹ and R² are independently H or C₁₋₃ alkyl;
R³ and R⁴ are independently H, C₁₋₆ alkyl, -OC₁₋₆ alkyl, halogen, OH, C₂₋₆ alkenyl or CF₃;
R⁵ is H, C₁₋₆ alkyl (optionally substituted by one or more halogens, -COphenyl, OC₁₋₆ alkyl, phenyl, morpholino) or C₂₋₆ alkenyl;
R⁶ is C₁₋₆ alkyl, halogen, -OCH₂ phenyl, phenyl (optionally substituted by C₁₋₃ alkyl), morpholino, pyrrolidino, piperidino, thiophenyl, furanyl, pyridinyl or -OC₂₋₆ alkenyl.

In another aspect, the present invention provides a pharmaceutical composition comprising a compound of the invention, preferably in association with a pharmaceutically acceptable diluent or carrier.

In another aspect, the present invention provides a compound of the invention for use in therapy, and in particular, in human medicine.

In another aspect, the present invention provides a compound of the invention for use in treating a human PPAR ("hPPAR") mediated disease or condition.

In another aspect, the present invention discloses a method for prevention or treatment of a hPPAR mediated disease or condition comprising administration of a compound of this invention.

In another aspect, the present invention provides the use of a compound of the invention for the manufacture of a medicament for the treatment of a hPPAR mediated disease or condition.

hPPARmediated diseases or conditions include dyslipidemia including associated diabetic dyslipidemia and mixed dyslipidemia, obesity, syndrome X (as defined in this application this embraces metabolic syndrome), heart failure, hypercholesteremia, cardiovascular disease including atherosclerosis, arteriosclerosis, and hypertriglyceridemia, type II diabetes mellitus, type I diabetes, insulin resistance, hyperlipidemia, inflammation, epithelial hyperproliferative diseases including eczema and psoriasis and conditions associated with the lining and gut and regulation of appetite and food intake in subjects suffering from disorders such as obesity, bulimia, and anorexia nervosa. In particular, the compounds of the invention are potentially useful in the treatment and prevention of cardiovascular diseases and conditions including atherosclerosis, arteriosclerosis, hypertriglyceridemia, and mixed dyslipidaemia.

### Detailed Description of the Invention

As used herein, "a compound of the invention" means a compound of formula (I) or a pharmaceutically acceptable salt, solvate, or hydrolyzable ester thereof.

While hydrolyzable esters are included in the scope of this invention, the acids are preferred because the data suggests that while the esters are useful compounds, it may actually be the acids to which they hydrolyze that are the active compounds. Esters that hydrolyze readily can produce the carboxylic acid in the assay conditions or in vivo. Generally the carboxylic acid is active in both the binding and transient transfection assays, while the ester does not usually bind well but is active in the transient transfection assay presumably due to hydrolysis. Preferred hydrolysable esters are C₁₋₆ alkyl esters wherein the alkyl group may be straight chain or branched chain. Methyl or ethyl esters are more preferred.

The compounds of the invention are modulators of PPAR alpha and PPAR gamma. In one aspect they are agonists or partial agonists of the relevant PPAR.

The compound of formula (I) is suitably a selective dual agonist of PPAR alpha and gamma. As used herein, by "agonist", or "activating compound", or "activator", or the like, is meant those compounds which have a pKi of at least 6.0 preferably at least 7.0 to the relevant PPAR, for example hPPAR alpha in the binding assay described below, and which achieve at least 50% activation of the relevant PPAR relative to the appropriate indicated positive control in the transfection assay described below at concentrations of 10⁻⁵ M or less.

Partial agonists can be defined as compounds that transactivate the relevant PPAR, for example PPAR alpha in CV1 cells with less than 50% fold activation compared to the reference PPAR alpha full agonist in the transfection assays of the type described below.

As used herein, a "selective dual hPPAR alpha/gamma agonist" is a hPPARalpha/gamma agonist whose EC₅₀ for PPARalpha and PPAR gamma is at least 10 fold lower than its EC₅₀ for PPAR delta. EC₅₀ is defined in the transfection assay described below and is the concentration at which a compound achieves 50% of its maximum activity.

In one embodiment p is 0 and q is 1. In another embodiment p is 1 and q is 0. In a further embodiment, p and q are 0.

In one embodiment R¹ and R² are independently C₁₋₃ alkyl. Preferably R¹ and R² are both C₁₋₃ alkyl, more preferably R¹ and R² are both methyl.

In one embodiment R⁴ is H.

In one embodiment R³ is C₁₋₃ alkyl or -OC₁₋₃ alkyl. Preferably R³ is methyl or -OCH₃. R³ is preferably ortho to the depicted Oxygen.

In one embodiment R⁵ is methyl.

In one embodiment R⁶ is C₁₋₆ alkyl, preferably tertiary butyl. Preferably R⁶ is in the para position on the phenyl ring.

In one embodiment the compound of formula (I) is a compound of formula (Ia) 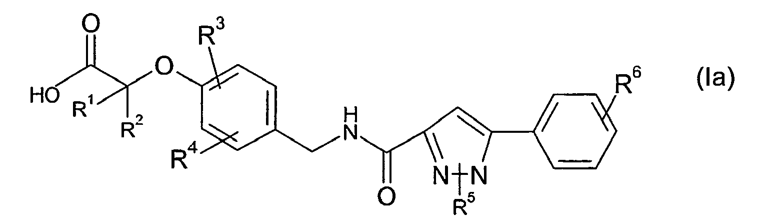 wherein
R¹ and R² are independently H or C₁₋₃ alkyl;
R³ and R⁴ are independently H, C₁₋₆ alkyl, -OC₁₋₆ alkyl, halogen, OH, C₂₋₆ alkenyl, CF₃;
R⁵ is H, C₁₋₆ alkyl or CF₃;
R⁶ is C₁₋₆ alkyl

In one embodiment, R¹ and R² in formula (la) are independently C₁₋₃ alkyl. Preferably R¹ and R² are both C₁₋₃ alkyl, more preferably R¹ and R² are both methyl.

In one embodiment R⁴ is H;

In one embodiment R³ is C₁₋₃ alkyl or -OC₁₋₃ alkyl. Preferably R³ is methyl or -OCH₃. R³ is preferably ortho to the depicted oxygen.

In one embodiment R⁵ is methyl.

In one embodiment R⁶ is C₁₋₆ alkyl, preferably tertiary butyl. Preferably R⁶ is in the para position in the phenyl ring

While embodiments for each variable have generally been listed above separately for each variable, this invention include those compounds in which several or each variable in Formula (I) is selected from the embodiments including preferred or more preferred groups for embodiments including each variable. Therefore, this invention is intended to include all combinations of embodiments including preferred, more preferred, and most preferred groups.

Suitable compounds of the invention include:
2-[(4-{[({5-[4-(1,1-dimethylethyl)phenyl]-1-methyl-1*H*-pyrazol-3-yl}carbonyl)amino]methyl}-2-methylphenyl)oxy]-2-methylpropanoic acid;
2-methyl-2-[(2-methyl-4-{[({1-methyl-3-[4-(1-methylethyl)phenyl]-1*H*-pyrazol-5-yl}carbonyl)amino]methyl}phenyl)oxy]propanoic acid;
2-{[4-{[({3-[4-(1 , 1-dimethylethyl)phenyl]-1-methyl-1 H-pyrazol-5-yl}carbonyl)amino]methyl}-2-(2-propen-1-yl)phenyl]oxy}-2-methylpropanoic acid;
2-[(4-{[({3-[4-(1,1-dimethylethyl)phenyl]-1-methyl-1H pyrazol-5-yl}carbonyl)amino]methyl}-2-propylphenyl)oxy]-2-methylpropanoic acid;
2-{[4-{[({3-[4-(1,1-dimethylethyl)phenyl]-1-ethyl-1*H*-pyrazol-5-yl}carbonyl)amino]methyl}-2-(methyloxy)phenyl]oxy}-2-methylpropanoic acid;
2-{[4-{[({5-[4-(1,1-dimethylethyl)phenyl]-1-ethyl-1*H*-pyrazol-3-yl}carbonyl)amino]methyl}-2-(methyloxy)phenyl]oxy}-2-methylpropanoic acid;
2-methyl-2-[(2-methyl-4-{[({1-methyl-5-[4-(2-methylpropyl)phenyl]-1*H*-pyrazol-3-yl}carbonyl)amino]methyl}phenyl)oxy]propanoic acid;
2-methyl-2-[(2-methyl-4-{[({1-methyl-3-[4-(2-methylpropyl)phenyl]-1*H*-pyrazol-5-yl}carbonyl)amino]methyl}phenyl)oxy]propanoic acid;
2-methyl-2-{[4-{[({1-methyl-5-[4-(-methylethyl)phenyl]-1*H*-pyrazol-3-yl}carbonyl)amino]methyl}-2-(methyloxy)phenyl]oxy}propanoic acid;
2-methyl-2-{[4-{[({1-methyl-3-[4-(1-methylethyl)phenyl]-1*H*-pyrazol-5-yl}carbonyl)amino]methyl}-2-(methyloxy)phenyl]oxy}propanoic acid;
2-[(4-{[({3-[4-(1,1-dimethylethyl)phenyl]-1-methyl-1*H*-pyrazol-5-yl}carbonyl)amino]methyl}-2-methylphenyl)oxy]-2-methylpropanoic acid;
2-{[4-1[({3-[4-(1,1 -dimethylethyl)phenyl]-1-methyl-1*H*-pyrazol-5-yl}carbonyl)amino]methyl}-2-(methyloxy)phenyl]oxy}-2-methylpropanoic acid;
2-[(4-{[({5-[4-(1,1-dimethylethyl)phenyl]-1-ethyl-1*H*-pyrazol-3-yl}carbonyl)amino]methyl}-2-methylphenyl)oxy]-2-methylpropanoic acid;
2-methyl-2-{[4-{[({1-methyl-5-[4-(2-methylpropyl)phenyl]-1*H*-pyrazol-3-yl}carbonyl)amino]methyl}-2-(methyloxy)phenyl]oxy}propanoic acid;
2-[(4-{[({3-[4-(1,1-dimethylethyl)phenyl]-1-ethyl-1*H*-pyrazol-5-yl}carbonyl)amino]methy}-2-methylphenyl)oxy]-2-methyl propanoic acid;
2-[(4-{[({3-[4-(1,1-dimethylethyl)phenyl]-1-methyl-1*H*-pyrazol-5-yl}carbonyl)amino]methyl}phenyl)oxy]-2-methylpropanoic acid;
2-[(4-{[({5-[4-(1,1-dimethylethyl)phenyl]-1-methyl-1*H*-pyrazol-3-yl}carbonyl)amino]methyl}phenyl)oxy]-2-methylpropanoic acid;
2-methyl-2-[(2-methyl-4-{[({1-methyl-5-[4-(1-methylethyl)phenyl]-1*H*-pyrazol-3-yl}carbonyl)amino]methyl}phenyl)oxy]propanoic acid;
2-methyl-2-[(2-methy)-4-{[({1-methyl-5-[4-(4-morpholinyl)phenyl]-1*H*-pyrazol-3-yl}carbonyl)amino]methyl}phenyl)oxy]propanoic acid;
2-methyl-2-[(2-methyl-4-{[({1-methyl-5-[4-(1-pyrrolidinyl)phenyl]-1*H*-pyrazol-3-yl}carbonyl)amino]methyl}phenyl)oxy]propanoic acid;
2-methyl-2-[(2-methyl-4-{[({1-methyl-5-[4-(1-piperidinyl)phenyl]-1*H*-pyrazol-3-yl}carbonyl)amino]methyl}phenyl)oxy]propanoic acid;
2-({4-[({[5-(4-biphenylyl)-1-methyl-1*H*-pyrazol-3-yl]carbonyl}amino)methyl]-2-methylphenyl}oxy)-2-methylpropanoic acid;
2-methyl-2-[(2-methyl-4-{[({1-methyl-3-[3-(1-pyrrolidinyl)phenyl]-1*H*-pyrazol 5-yl}carbonyl)amino]methyl}phenyl)oxy]propanoic acid;
2-({4-[({[3-(4-biphenylyl)-1-methyl-1*H*-pyrazol-5-yl]carbonyl}amino)methyl]-2-methylphenyl}oxy)-2-methylpropanoic acid;
2-{4-[({[5-(4-*tert*-butylphenyl)-1-methyl-1*H*-pyrazol-3-yl]carbonyl}amino) methyl]-2,6-dimethylphenoxy}-2-methylpropanoic acid;
2-{4-[({[3-(4-*tert*-butylphenyl)-1-methyl-1*H*-pyrazol-5-yl]carbonyl}amino)methyl]-2,6-dimethylphenoxy}-2-methylpropanoic acid;
2-{2-chloro-4-[({[5-(4-isobutylphenyl)-1-methyl-1*H*-pyrazol-3-yl]carbonyl}amino)methyl]-6-methylphenoxy}-2-methylpropanoic acid;
2-[4-({[(3-biphenyl-3-yl-1-methyl-1*H*-pyrazol-5-yl)carbonyl]amino}methyl)-2-methylphenoxy]-2-methylpropanoic acid;
2-({4-[({[5-(4-butylphenyl)-1-methyl-1*H*-pyrazol-3-yl]carbonyl}amino)methyl]-2-methylphenyl}oxy)-2-methylpropanoic acid;
2-({4-[({[5-(4-bromophenyl)-1-methyl-1*H*-pyrazol-3-yl]carbonyl}amino)methyl]-2-methylphenyl}oxy)-2-methylpropanoic acid;
2-methyl-2-{2-methyl-4-[({[1-methyl-5-(2'-methylbiphenyl-4-yl)-1*H*-pyrazol-3-yl]carbonyl}amino)methyl]phenoxy}propanoic acid;
2-methyl-2-[(2-methyl-4-{[({1-methyl-5-[4-(2-thienyl)phenyl]-1*H*-pyrazol-3-yl}carbonyl)amino]methyl}phenyl)oxy]propanoic acid;
2-[(4-{[({5-[4-(3-furanyl)phenyl]-1-methyl-1 *H*-pyrazol-3-yl}carbonyl)amino]methyl}-2-methylphenyl)oxy]-2-methylpropanoic acid;
2-methyl-2-[(2-methyl-4-{[({1-methyl-5-[4-(4-pyridinyl)phenyl]-1 *H*-pyrazol-3-yl}carbonyl)amino]methyl}phenyl)oxy]propanoic acid;
2-[(4-{[({5-[4-(2-furanyl)phenyl]-1-methyl-1*H*-pyrazol-3-yl}carbonyl)amino]methyl}-2-methylphenyl)oxy]-2-methylpropanoic acid;
2-methyl-2-{2-methyl-4-[({[1-methyl-3-(2'-methylbiphenyl-4-yl)-1*H*-pyrazol-5-yl]carbonyl}amino)methyl]phenoxy}propanoic acid;
2-{4-[({[5-(4-butylphenyl)-1-methyl-1*H*-pyrazol-3-yl]carbonyl}amino)methyl]-2,6-dimethylphenoxy}-2-methylpropanoic acid;
2-{[4-({2-{4-[({[5-(4-butylphenyl)-1-methyl-1*H*-pyrazol-3-yl]carbonyl}amino)methyl]-2-chloro-6-methylphenoxy}-2-methylpropanoic acid;
2-methyl-2-[(2-methyl-4-{[({1-methyl-3-[4-(4-morpholinyl)phenyl]-1*H*-pyrazol-5-yl}carbonyl)amino]methyl}phenyl)oxy]propanoic acid;
2-methyl-2-[(2-methyl-4-{[({1-methyl-5-[3-(1-piperidinyl)phenyl]-1*H*-pyrazol-3-yl}carbonyl)amino]methyl}phenyl)oxy]propanoic acid;
2-methyl-2-[(2-methyl-4-{[({1-methyl-5-[3-(1-pyrrolidinyl)phenyl]-1*H*-pyrazol-3-yl}carbonyl)amino]methyl}phenyl)oxy]propanoic acid;
2-methyl-2-{2-methyl-4-[({[1-methyl-3-(3-piperidin-1-ylphenyl)-1*H*-pyrazol-5-yl]carbonyl}amino)methyl]phenoxy}propanoic acid;
2-[(4-{[({3-[4-(1,1-dimethylethyl)phenyl]-1-methyl-1*H*-pyrazol-5-yl}acetyl)amino]methyl}-2-methylphenyl)oxy]-2-methylpropanoic acid;
2-[(4-{[({5-[4-(1,1-dimethylethyl)phenyl]-1-methyl-1*H*-pyrazol-3-yl}acetyl)amino]methyl}-2-methylphenyl)oxy]-2-methylpropanoic acid;
2-methyl-2-[(2-methyl-4-{[({1-methyl-3-[4-(1-piperidinyl)phenyl]-1*H*-pyrazol-5-yl}carbonyl)amino]methyl}phenyl)oxy]propanoic acid;
2-{[4-({[(3-{[4-(1,1-dimethylethyl)phenyl]methyl}-1-methyl-1*H*-pyrazol-5-yl)carbonyl]amino}methyl)-2-methylphenyl]oxy}-2-methylpropanoic acid;
2-({4-[({[3-[4-(1,1-dimethylethyl)phenyl]-1-(2-propen-1-yl)-1*H*-pyrazol-5-yl]carbonyl}amino)methyl]-2-methylphenyl}oxy)-2-methylpropanoic acid;
2-[(4-{[({3-[4-(1,1-dimethylethyl)phenyl]-1-[2-(methyloxy)ethyl]-1*H*-pyrazol-5-yl}carbonyl)amino]methyl}-2-methylphenyl)oxy]-2-methylpropanoic acid;
2-({4-[({[3-[4-(1,1-dimethylethyl)phenyl]-1-(2-oxo-2-phenylethyl)-1*H*-pyrazol-5-yl]carbonyl}amino)methyl]-2-methylphenyl}oxy)-2-methylpropanoic acid;
2-({4-[({[5-[4-(1,1-dimethylethyl)phenyl]-1-(phenylmethyl)-1*H*-pyrazol-3-yl]carbonyl}amino)methyl]-2-methylphenyl}oxy)-2-methylpropanoic acid;
2-({5-[({[3-(4-(1,1-dimethylethyl)phenyl]-1-(2-phenylethyl)-1*H*-pyrazol-5-yl]carbonyl}amino)methyl]-2-methylphenyl}oxy)-2-methylpropanoic acid;
2-[(4-{[({5-[4-(1,1-dimethylethyl)phenyl]-1-[2-(methyloxy)ethyl]-1*H*-pyrazol-3-yl}carbonyl)amino]methyl}-2-methylphenyl)oxy]-2-methylpropanoic acid;
2-[(4-{[({5-[4-(1,1-dimethylethyl)phenyl]-1*H*-pyrazol-3-yl}carbonyl)amino] methyl}-2-methylphenyl)oxy]-2-methylpropanoic acid;
2-[(4-{[({5-[4-(1,1-dimethylethyl)phenyl]-1-[2-(4-morpholinyl)ethyl]-1*H*-pyrazol-3-yl}carbonyl)amino]methyl}-2-methylphenyl)oxy]-2-methyl propanoic acid hydrochloride;
2-[(4-{[({3-[4-(1,1-dimethylethyl)phenyl]-1-[2-(4-morpholinyl)ethyl]-1*H*-pyrazol-5-yl}carbonyl)amino]methyl}-2-methylphenyl)oxy]-2-methylpropanoic acid;
2-methyl-2-[(2-methyl-4-{[({1-methyl-3-[4-(2-propen-1-yloxy)phenyl]-1*H*-pyrazol-5-yl}carbonyl)amino]methyl}phenyl)oxy]propanoic acid;
2-methyl-2-{[2-methyl-4-({[(1-methyl-3-{4-[(phenylmethyl)oxy]phenyl}-1*H*-pyrazol-5-yl)carbonyl]amino}methyl)phenyl]oxy}propanoic acid;
2-methyl-2-{[2-methyl-4-({[(1-methyl-5-{4-[(phenylmethyl)oxy]phenyl}-1*H*-pyrazol-3-yl)carbonyl]amino}methyl)phenyl]oxy}propanoic acid;
2-methyl-2-[(2-methyl-4-{[({1-methyl-5-[4-(2-propen-1-yloxy)phenyl]-1*H*-pyrazol-3-yl}carbonyl)amino]methyl}phenyl)oxy]propanoic acid;
2-({4-[({[3-[4-(1,1-dimethylethyl)phenyl]-1-(2-propen-1-yl)-1*H*-pyrazol-5-yl]carbonyl}amino)methyl]phenyl}oxy)-2-methylpropanoic acid;
2-({4-[({[3-[4-(1,1-dimethylethyl)phenyl]-1-(phenylmethyl)-1*H*-pyrazol-5-yl]carbonyl}amino)methyl]-2-methylphenyl}oxy)-2-methylpropanoic acid;
2-({4-[({[5-[4-(1,1-dimethylethyl)phenyl]-1-(2-propen-1-yl)-1*H*-pyrazol-3-yl]carbonyl}amino)methyl]-2-methylphenyl}oxy)-2-methylpropanoic acid.

It will be appreciated by those skilled in the art that the compounds of the present invention may also be utilized in the form of a pharmaceutically acceptable salt or solvate thereof. The physiologically acceptable salts of the compounds of formula (I) include conventional salts formed from pharmaceutically acceptable inorganic or organic acids or bases as well as quaternary ammonium acid addition salts. More specific examples of suitable acid salts include hydrochloric, hydrobromic, sulfuric, phosphoric, nitric, perchloric, fumaric, acetic, propionic, succinic, glycolic, formic, lactic, maleic, tartaric, citric, palmoic, malonic, hydroxymaleic, phenylacetic, glutamic, benzoic, salicylic, fumaric, toluenesulfonic, methanesulfonic, naphthalene-2-sulfonic, benzenesulfonic hydroxynaphthoic, hydroiodic, malic, steroic, tannic and the like. Other acids such as oxalic, while not in themselves pharmaceutically acceptable, may be useful in the preparation of salts useful as intermediates in obtaining the compounds of the invention and their pharmaceutically acceptable salts. More specific examples of suitable basic salts include sodium, lithium, potassium, magnesium, aluminium, calcium, zinc, N,N'-dibenzylethylenediamine, chloroprocaine, choline, diethanolamine, ethylenediamine, N-methylglucamine and procaine salts. References hereinafter to a compound according to the invention include both compounds of formula (I) and their pharmaceutically acceptable salts and solvates.

The compound of the invention and its pharmaceutically acceptable derivatives are conveniently administered in the form of pharmaceutical compositions. Such compositions may conveniently be presented for use in conventional manner in admixture with one or more physiologically acceptable carriers or excipients.

While it is possible that compounds of the present invention may be therapeutically administered as the raw chemical, it is preferable to present the active ingredient as a pharmaceutical composition. The carrier(s) must be "acceptable" in the sense of being compatible with the other ingredients of the formulation and not deleterious to the recipient thereof.

Accordingly, the present invention further provides for a pharmaceutical composition comprising a compound of formula (I) or a pharmaceutically acceptable salt or solvate thereof together with one or more pharmaceutically acceptable carriers therefore and, optionally, other therapeutic and/or prophylactic ingredients.

The compositions include those suitable for oral, parenteral (including subcutaneous e.g. by injection or by depot tablet, intradermal, intrathecal, intramuscular e.g. by depot and intravenous), rectal and topical (including dermal, buccal and sublingual) administration although the most suitable route may depend upon for example the condition and disorder of the recipient. The compositions may conveniently be presented in unit dosage form and may be prepared by any of the methods well known in the art of pharmacy. All methods include the step of bringing into association a compound ("active ingredient") with the carrier which constitutes one or more accessory ingredients. In general the compositions are prepared by uniformly and intimately bringing into association the active ingredient with liquid carriers or finely divided solid carriers or both and then, if necessary, shaping the product into the desired composition.

Compositions suitable for oral administration may be presented as discrete units such as capsules, cachets or tablets (e.g. chewable tablets in particular for paediatric administration) each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine the active ingredient in a free-flowing form such as a powder or granules, optionally mixed with a other conventional excipients such as binding agents, (for example, syrup, acacia, gelatin, sorbitol, tragacanth, mucilage of starch or polyvinylpyrrolidone), fillers (for example, lactose, sugar, microcrystalline cellulose, maize-starch, calcium phosphate or sorbitol), lubricants (for example, magnesium stearate, stearic acid, talc, polyethylene glycol or silica), disintegrants (for example, potato starch or sodium starch glycollate) or wetting agents, such as sodium lauryl sulfate. Moulded tablets may be made by moulding in a suitable machine a mixture of the powdered compound moistened with an inert liquid diluent. The tablets may optionally be coated or scored and may be formulated so as to provide slow or controlled release of the active ingredient therein. The tablets may be coated according to methods well-known in the art.

Alternatively, a compound of the present invention may be incorporated into oral liquid preparations such as aqueous or oily suspensions, solutions, emulsions, syrups or elixirs, for example. Moreover, compositions containing these compounds may be presented as a dry product for constitution with water or other suitable vehicle before use. Such liquid preparations may contain conventional additives such as suspending agents such as sorbitol syrup, methyl cellulose, glucose/sugar syrup, gelatin, hydroxyethylcellulose, carboxymethyl cellulose, aluminum stearate gel or hydrogenated edible fats; emulsifying agents such as lecithin, sorbitan mono-oleate or acacia; non-aqueous vehicles (which may include edible oils) such as almond oil, fractionated coconut oil, oily esters, propylene glycol or ethyl alcohol; and preservatives such as methyl or propyl p-hydroxybenzoates or sorbic acid. Such preparations may also be formulated as suppositories, e.g., containing conventional suppository bases such as cocoa butter or other glycerides.

Compositions for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents.

The compositions may be presented in unit-dose or multi-dose containers, for example sealed ampoules and vials, and may be stored in a freeze-dried (lyophilised) condition requiring only the addition of a sterile liquid carrier, for example, water-for-injection, immediately prior to use. Extemporaneous injection solutions and suspensions may be prepared from sterile powders, granules and tablets of the kind previously described.

Compositions for rectal administration may be presented as a suppository with the usual carriers such as cocoa butter, hard fat or polyethylene glycol.

Compositions for topical administration in the mouth, for example buccally or sublingually, include lozenges comprising the active ingredient in a flavoured basis such as sucrose and acacia or tragacanth, and pastilles comprising the active ingredient in a basis such as gelatin and glycerin or sucrose and acacia.

The compounds may also be formulated as depot preparations. Such long acting compositions may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

In addition to the ingredients particularly mentioned above, the compositions may include other agents conventional in the art having regard to the type of formulation in question, for example those suitable for oral administration may include flavouring agents.

It will be appreciated by those skilled in the art that reference herein to treatment extends to prophylaxis as well as the treatment of established diseases or symptoms. Moreover, it will be appreciated that the amount of a compound of the invention required for use in treatment will vary with the nature of the condition being treated and the age and the condition of the patient and will be ultimately at the discretion of the attendant physician or veterinarian. In general, however, doses employed for adult human treatment will typically be in the range of 0.02-5000 mg per day, preferably 1-1500 mg per day. The desired dose may conveniently be presented in a single dose or as divided doses administered at appropriate intervals, for example as two, three, four or more sub-doses per day. The formulations according to the invention may contain between 0.1-99% of the active ingredient, conveniently from 30-95% for tablets and capsules and 3-50% for liquid preparations.

A compound of the invention may be used in combination with other therapeutic agents for example, statins (HMG Co A reductase inhibitors) and/or other lipid lowering drugs for example MTP inhibitors and LDLR upregulators. The compounds of the invention may also be used in combination with antidiabetic agents, e.g. metformin, sulfonylureas and/or PPAR gamma agonists (for example thiazolidinediones such as e.g. Pioglitazone and Rosiglitazone). The compounds may also be used in combination with antihypertensive agents such as calcium channel antagonists and ACE inhibitors. The invention thus provides in a further aspect the use of a combination comprising a compound of formula (I) with a further therapeutic agent in the treatment of a hPPAR mediated disease.

When a compound of the invention is used in combination with other therapeutic agents, the compounds may be administered either sequentially or simultaneously by any convenient route.

The combinations referred to above may conveniently be presented for use in the form of a pharmaceutical composition and thus pharmaceutical compositions comprising a combination as defined above optimally together with a pharmaceutically acceptable carrier or excipient comprise a further aspect of the invention. The individual components of such combinations may be administered either sequentially or simultaneously in separate or combined pharmaceutical compositions.

When combined in the same composition it will be appreciated that the two compounds must be stable and compatible with each other and the other components of the composition and may be formulated for administration. When formulated separately they may be provided in any convenient composition, conveniently in such a manner as are known for such compounds in the art.

When a compound of formula (I) is used in combination with a second therapeutic agent active against the same hPPAR mediated disease, the dose of each compound may differ from that when the compound is used alone. Appropriate doses will be readily appreciated by those skilled in the art.

Compounds of this invention may be conveniently prepared by a general process wherein a moiety like (A) is coupled to an acid (B) using a peptide coupling reaction or by acylation of (A) with an ester (C). R in formula (C) is preferably C₁₋₆alkyl. Note this synthesis is preferably carried out with the acid group of moiety A protected by R. Thus while R can be H, preferably R is C₁₋₆alkyl which can be hydrolyzed off to give an acid of Formula (I), or if readily hydrolyzable, the resulting ester can be administered.

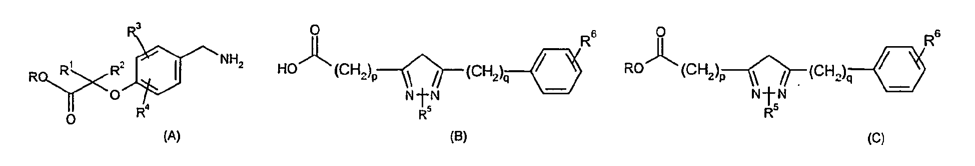

Further methods of preparing the compounds are illustrated by the schemes below. Routes of synthesis for the general structure depicted below: 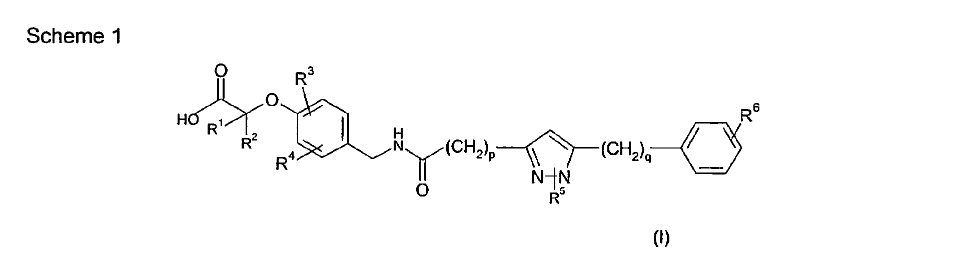 **-For R⁵=Me,** and the depicted R¹, R², R³, R⁴, R⁶, the following general route was used (see below) : 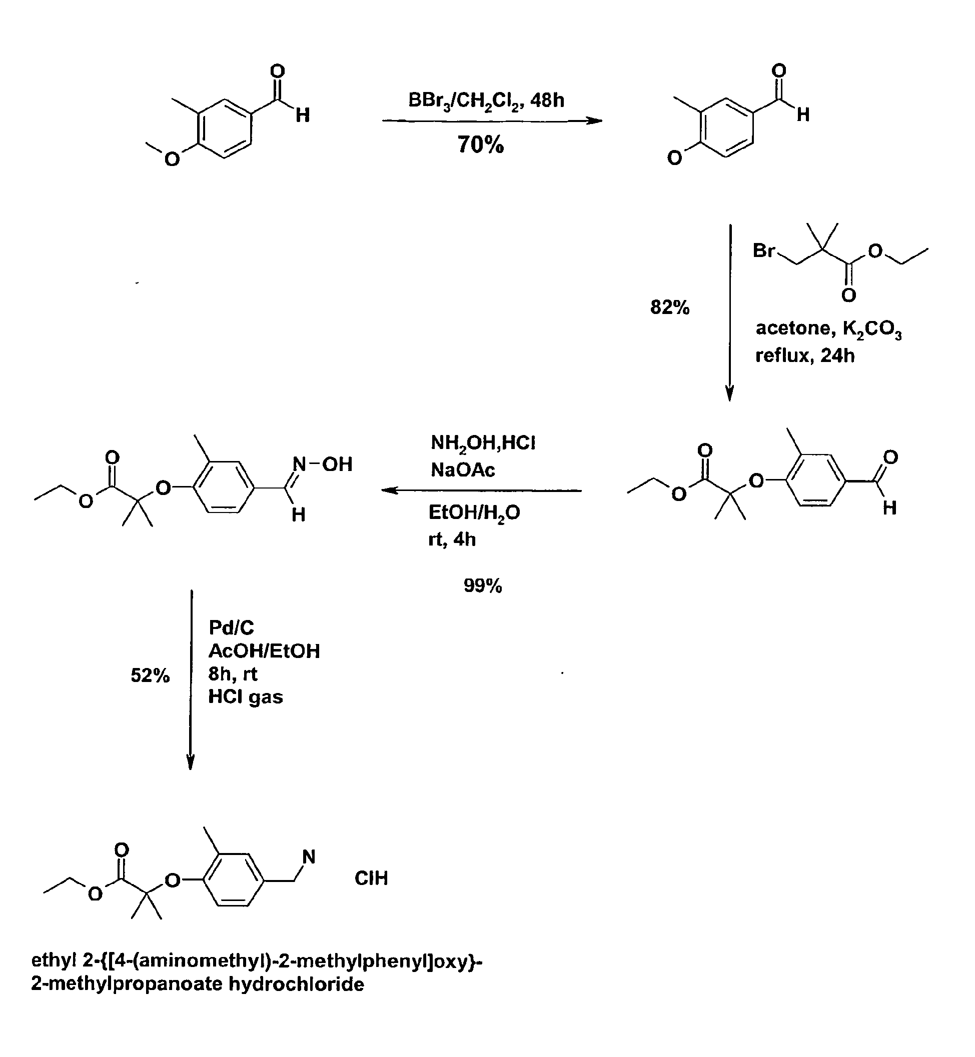 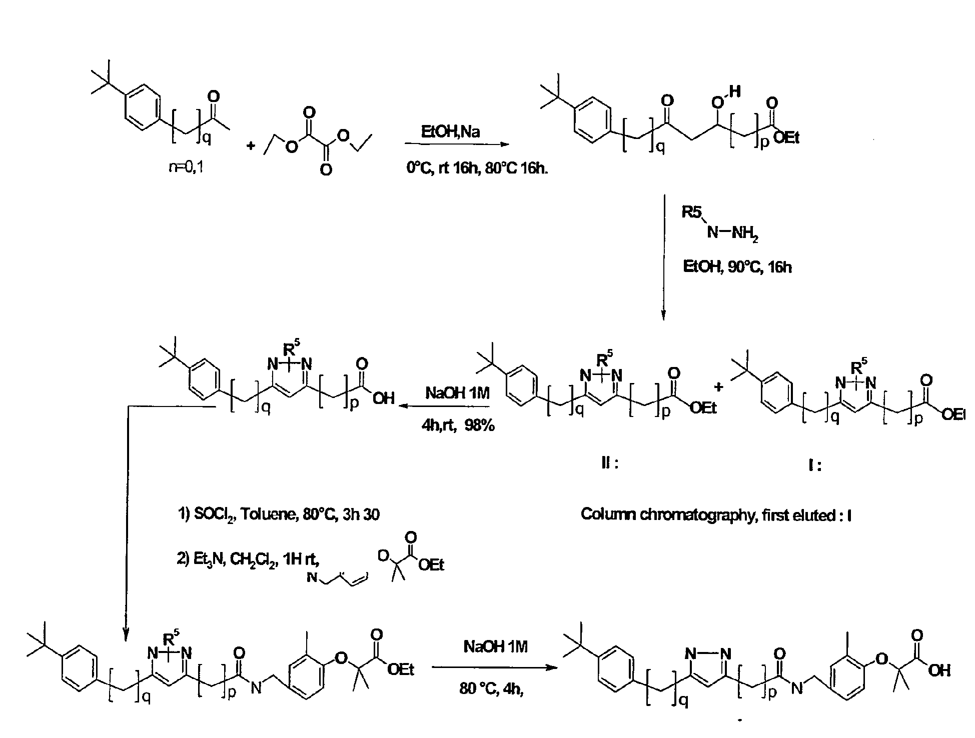 **-For R⁵=Et**, the following route was used (see below). This is illustrated where p and q are zero, R¹ and R² are both methyl: 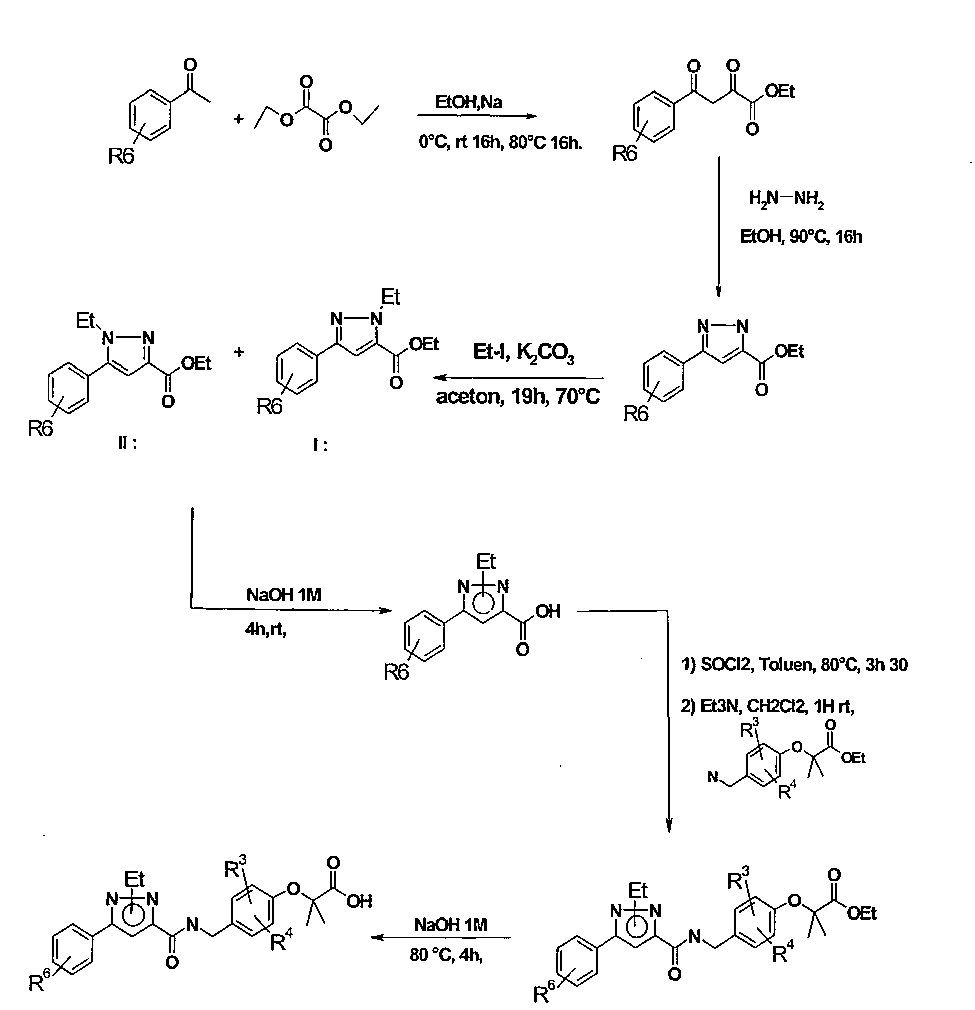 In a further process of the invention there is provided another route for the synthesis of compounds of formula (I). This is illustrated by Scheme 3 below. Scheme 3 is advantageous over Schemes 1 and 2 in that there is provided a method of achieving regio selectivity for the substituted pyrazole group as compared to Schemes 1 and 2 above which have to be resolved by chromatography.

### Scheme 3

Compounds of formula (I) having the regio selectivity depicted below: 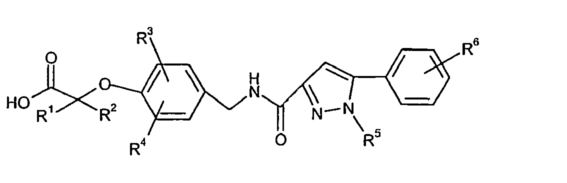 may be prepared by reacting compounds of formula (II) with compounds of formula (III): 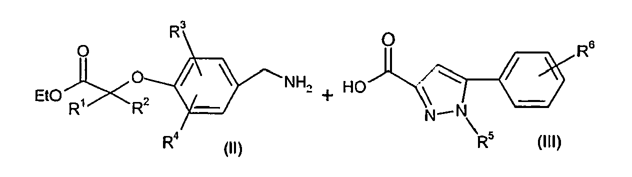 under appropriate reaction conditions - for example DCC plus NaOH.

Compounds of formula (II) may be prepared according to Scheme 3a depicted below. This is illustrated where R¹ and R² are CH₃, R³ is CH₃ and R⁴ is H. 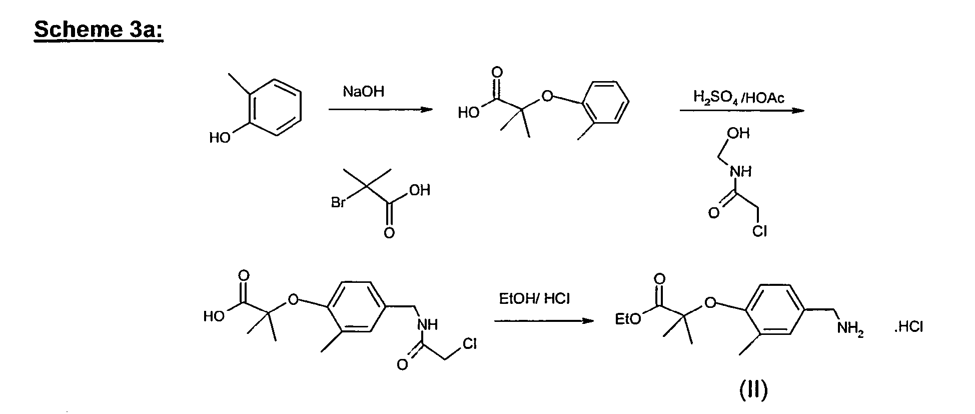 Compounds of formula (III) may be prepared accordingly to Scheme 3b depicted below. In Scheme 3b, this is illustrated for R⁶ = C(CH₃)₃ and R⁵ is CH₃. 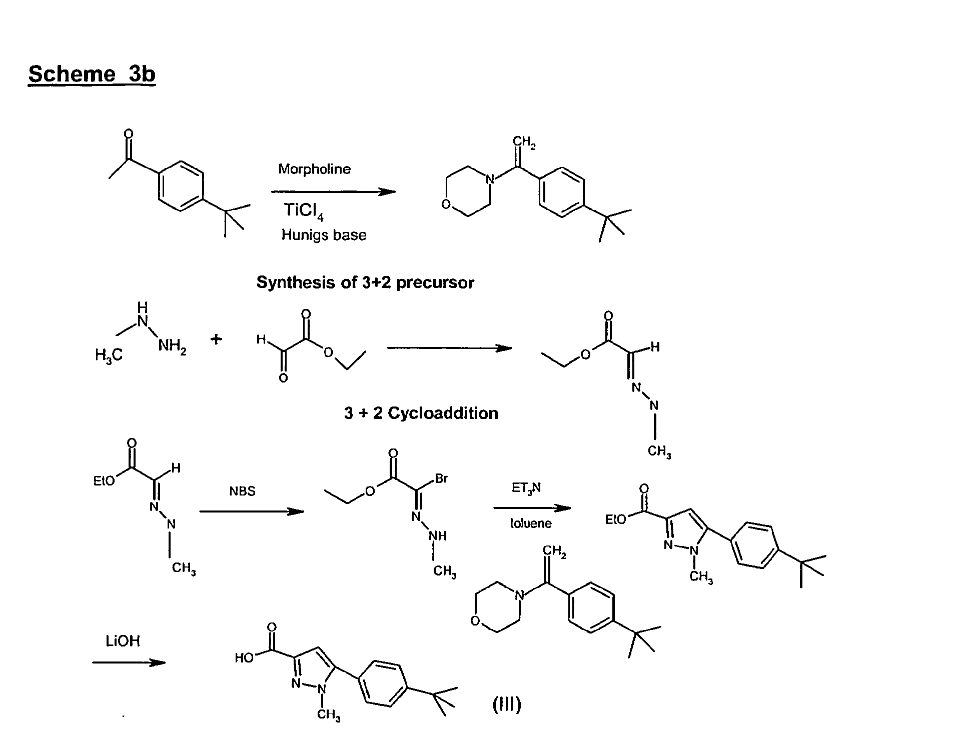 It is submitted the above schemes are illustrative and a skilled person would be able to adapt to prepare compounds with other R¹, R², R³, R⁴, R⁵ and R⁶ groups and where p and q are not zero, as appropriate, using these teachings and those in the specific examples below.

The invention will now be demonstrated by the following examples which should not be construed as constituting a limitation thereto.

### Examples

As used herein the symbols and conventions used in these processes, schemes and examples are consistent with those used in the contemporary scientific literature, for example, the Journal of the American Chemical Society or the Journal of Biological Chemistry. Unless otherwise noted, all starting materials were obtained from commercial suppliers and used without further purification. Specifically, the following abbreviations may be used in the examples and throughout the specification:
g (grams); mg (milligrams); L (liters); mL (milliliters); µL (microliters); mM (millimolar); mol (moles); mmol (millimoles); rt (room temperature); min (minutes); h (hours); MeOH (methanol); EtOH (ethanol); THF (tetrahydrofuran); AcOEt (ethyl acetate); Ac (acetyl); HOBT (1-Hydroxybenzotriazole); DMF (N,N-,dimethyl formamide); HOAT (1-Hydroxy-7-azabenzotriazole); HOBT (1-Hydroxybenzotriazole); EDCI (1-(3-Dimethylaminopropyl)-3-ethylcarbodiimide hydrochloride)); HATU 0-(7-Azabenzotriazol-1-yl)-N,N,N',N'-tetramethyluronium hexafluorophosphate; BINAP (2,2'-Bis(diphenylphosphino)-1,1'-binaphthyl.

All references to ether are to diethyl ether; brine refers to a saturated aqueous solution of NaCl. Unless otherwise indicated, all temperatures are expressed in °C (degrees Centigrade).

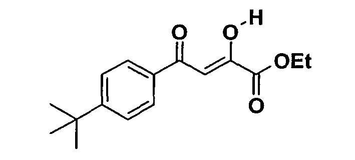

### Intermediate 1 Ethyl (2Z)-4-[4-(1,1-dimethylethyl)phenyl]-2-hydroxy-4-oxo-2-butenoate

7.91 g (0.344 mol) of Na was added to 1 L of EtOH in a 3L neck flask under nitrogen atmosphere at 0°C. A solution of 55g ( 0.312 mol) of p-tert-Butylacetophenone in 100mL of EtOH was added dropwise to the resulting solution and the reaction mixture was stirred for 45 min at 0°C then, 50.2g ( 0.344 mol) of ethyl oxalate in 100 ml of EtOH was added dropwise. The reaction mixture was stirred for 16h at rt then for 4h at 80°C. The evolution of the reaction was monitored by LC/Ms. The reaction mixture was cooled at rt and concentrated under vacuum. 1 L of ethyl acetate was added and the organic layer washed with brine, HCI 1 N x2, and brine.
The organic layer was dried with Na₂SO₄, after filtration and evaporation under vacuum, 86g of orange oil was obtained.
¹H NMR (CDCl₃): δ 8.1 (d, 1H); 7.7 ( d, 2H); 7.2 (s, 1H); 4.5 ( q , 3H) ; 1.5 ( m, 12H).

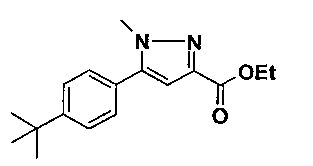

### Intermediate 2

### Ethyl 5-(4-tert-butylphenyl)-1-methyl-1H-pyrazole-3-carboxylate

24.7 ml (0.465mol) of methyl hydrazine was added to a solution of 86 g (0.31 mol) of **Intermediate 1** in 1 L of EtOH. The reaction mixture was heated at 90°C for 16h, cooled at rt and concentrated under vacuum. 1 L of ethyl acetate was added and the organic layer was washed with HCl 1 N x2 and brine.
The organic layer was dried with Na₂SO₄ after filtration and evaporation, under vacuum, the solid obtained was purified on flash silica column (Cyclohexane/AcOEt ( 95/5)) first eluted then Cyclohexane/AcOEt ( 80/20), second eluted is Intermediate 2: 19.7g.
Yield 22%
¹H NMR (CDCl₃) : δ 7.3 (d, 2H) ; 7.1 (d, 2H) ; 6.6 (s, 1 H) ; 4,2 (q, 3H) ; 3,7 (s, 3H) ; 1.2 (m, 12H).

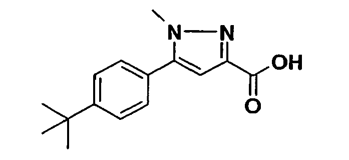

### Intermediate 3

### 5-(4-tert-butylphenyl)-1-methyl-1H-pyrazole-3-carboxylic acid

300 mL of EtOH and 687mL of NaOH 1 N was added to a solution of 19.7 g (68.7mmol) of **Intermediate 2** in 50 ml of THF. The reaction mixture was stirred 3h30 at rt, EtOH and THF were evaporated under vacuum. A solution of HCI 1 N was added dropwise to the reaction mixture to obtain precipitation of a cream solid. After filtration the solid obtained was dried under vacuum.
M = 17.4g, yield: 98%
¹H NMR (CDCl₃): δ 1.3 (s, 9H); 4 (s, 3H); 6.9 (s, 1H); 7.4 (d, 2H); 7.5 (d, 2H).

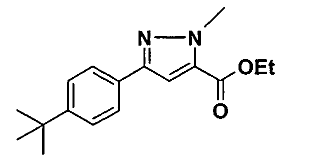

### Intermediate 4

### Ethyl 3-[4-(1,1-dimethylethyl)phenyl]-1-methyl-1H-pyrazole-5-carboxylate

24,7 mL ( 0.465 mol) of methyl hydrazine was added to a solution of 86g (0.31mol) of **Intermediate 1** in 1 L of EtOH. The reaction mixture was heated at 90°C for 16h, cooled at rt and concentrated under vacuum. 1 L of ethyl acetate was added and the organic layer was washed with brine, (HCl 1 N) x2, and brine. The organic layer was dried with Na₂SO₄. After filtration and evaporation under vacuum, the solid obtained was purified on flash silica column Cyclohexane/AcOEt ( 95/5) first eluted then Cyclohexane/AcOEt ( 80/20), first eluted is intermediate 4: 41.3g , yield 47%.
¹H NMR (CDCl₃) : δ 7.54 (d, 2H) ; 7.24 (d, 2H) ; 6.93 (s, 1 H) ; 4,2 (q, 3H) ; 4.05 (s, 3H) ; 1.22 (t, 3H) ; 1.17 (s, 9H).

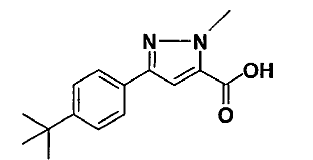

### Intermediate 5

### 3-[4-(1,1-dimethylethyl)phenyl]-1-methyl-1H-pyrazole-5-carboxylic acid

25 mL of EtOH and 42 mL of NaOH 1N was added to a solution of 4 g (14mmol) of **Intermediate 4** in 25 mL of THF. The reaction mixture was stirred 2h30 at rt EtOH and THF were evaporated under vacuum. A solution of HCl 1 N was added to the reaction mixture to obtain precipitation of a white solid. After filtration, the resulting solid was rinsed with water and dried under vacuum.
M = 3.62 g, yield: quantitative.
¹H NMR (DMSO-d6) : δ 7.75 (d, 2H) ; 7.43 (d, 2H) ; 7.24 (s, 1H) ; 4.12 (s, 3H) ; 1.3 (s, 9H)

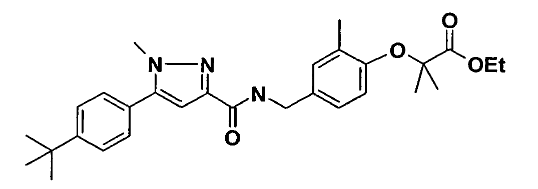

### Intermediate 6

### Ethyl 2-{4-[({[5-(4-tert-butylphenyl)-1-methyl-1H-pyrazol-3-yl]carbonyl}amino)methyl]-2-methylphenoxy}-2-methylpropanoate

21 mL (288mmol) of SOCl₂ was added dropwise under nitrogen atmosphere to a suspension of 14.85 g (57.6mmol) of **Intermediate 3** in 700 mL of anhydrous toluene. The reaction mixture was heated at 80°C for 3h30. Toluene and SOCl₂ were evaporated under vacuum and the residue was diluted in 400 mL of anhydrous CH₂CI₂ and added dropwise to a solution of 16.56g (57.6mmol) of **intermediate 10**, 24.2 mL (173mmol) of Et₃N in 800 mL of CH₂Cl₂. The reaction mixture was stirred 2h at rt, then washed twice with HCI 1 N, and with brine. The organic layer was dried with Na₂SO₄, after filtration and evaporation of solvent under vacuum the resulting oil was purified by SiO₂ flash chromatography (CH₂Cl₂/AcOEt : 95/5).
M: 32g of yellow oil yield: 96%
¹H NMR (CDCl₃) : δ 7.4 (d, 2H) ; 7.3 (d, 2H) ; 6.95-7.1 (m, 3H) ; 6.8 (s, 1 H) ; 6.5 (d, 1 H) ; 4.5 (d, 2H) ; 4.2 (q, 3H) ; 3.8 (s, 3H) ; 2.2 (s, 3H) ; 1.5 (s, 6H) ; 1.3 (s, 9H) ; 1.2 (t, 3H).

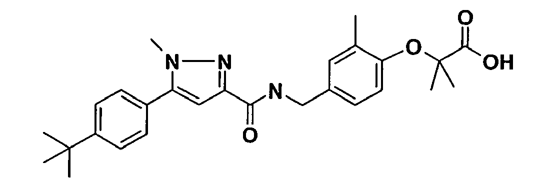

### Example 1

### 2-[(4-{[({5-[4-(1,1-dimethylethyl)phenyl]-1-methyl-1H-pyrazol-3-yl}carbonyl)amino]methyl}-2-methylphenyl)oxy]-2-methylpropanoic acid

1 L of NaOH 1 N was added dropwise to a solution of 70 g (0.142mol) of **Intermediate 6** in 250 mL of EtOH. The reaction mixture was heated at 90°C for 3 H. EtOH was evaporated under vacuum and 500ml of H₂O was added then HCl 1N until pH1 of 1 was obtained. A white precipitate appeared and the mixture was left at rt for 12 h to obtain complete precipitation. The white precipitate was filtered then solubilised in AcOEt and washed with water. The organic layer was dried on Na₂SO₄ filtered and concentrated under vacuum. The white powder obtained was re-crystallised in isopropyl acetate (1g of compound in 5 mL of isopropyl acetate) to afford 53g of white crystals.
Yield: 80%
Mp: 136-137°C
¹H-NMR (CDCl₃) : δ 7.41 (m, 3H) ; 7.26 (d, 2H) ; 7.09 (s, 1 H) ; 6.93 (d, 1H) ; 6.8 (s, 1H); 6.69 (d, 1 H) ; 4,43 (d, 2H) ; 3,79 (s, 3H) ; 2.15 (s, 3H) ; 1.55 (s, 6H) 1.29 (s, 9H).

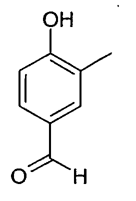

### Intermediate 7

### 4-hydroxy-3-methylbenzaldehyde

To a solution of **3-methyl-4-(methyloxy)benzaldehyde** (150g, 1mol) in CH₂Cl₂ at 0-5°C under N₂ atmosphere was added dropwise BBr₃ (1.5L, 1.5eq) in 2h. The reaction mixture was stirred at room temperature for 48h. The solvent was evaporated to give 200 mL of solution. This residue was cooled to 15°C, MeOH (500 mL) was added to give a black suspension. The mixture was refluxed for 30min and concentrated. Water (600 mL) was added and stirred for 30 min. The mixture was extracted with diethyl ether (3 x 600mL). The organic layers were dried on MgSO₄ and treated with charcoal, concentrated and purified by chromatography (Dichloromethane and Dichloromethane/Methanol 98/2) to give the title compound as a yellow solid (94.87g).
Yield=70%
¹H NMR (DMSO) : δ 10.55 (s, 1 H); 9.75 (s, 1H); 7.63 (s, 1 H); 7.60 (dd, 1 H); 6.94 (d, 1 H); 2.17 (s, 3H).

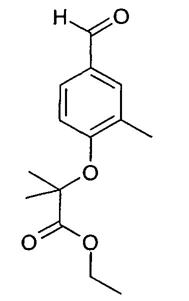

### Intermediate 8

### ethyl 2-[(4-formyl-2-methylphenyl)oxy]-2-methylpropanoate

To a solution of **intermediate 7** (94.9g, 0.70mol) in acetone (1 L) was added K₂CO₃ (145g, 1.5eq). The mixture was stirred at reflux for 16h30. Then, ethyl 2-bromoisobutyrate (155mL, 1.5eq) was added. The mixture was stirred for 24h. 0.5eq of K₂CO₃ and 0.5 eq of ethyl 2-bromoisobutyrate were added. After 20h of reaction, 0.5eq of K₂CO₃ and 0.5 eq of ethyl 2-bromoisobutyrate were added. After 60h, 0.5eq of K₂CO₃ and 0.5 eq of ethyl 2-bromoisobutyrate were added. After 4h, the mixture was filtered, cleared with acetone and concentrated. The residue was diluted in CH₂Cl₂ (800mL) and washed with NaOH (1 N), water. The organic layer was dried with MgSO₄ and concentrated to give 143.1g brown oil ( used without purification in the next step).
Yield=82%
¹H NMR (CDCl₃) : δ 9.86 (s, 1 H); 7.71 (s, 1 H); 7.60 (dd, 1 H); 6.68 (d, 1 H); 4.24 (quad, 2H); 2.30 (s, 3H); 1.69 (s, 6H); 1.23 (t, 3H).

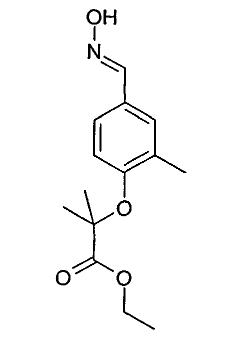

### Intermediate 9

### Ethyl 2-({4-[(E)-(hydroxyimino)methyl]-2-methylphenyl}oxy)-2-methylpropanoate

To a solution of **intermediate 8** (143.1g, 0.57mol) in EtOH (1.7L) and water (0.5L) was added hydroxylamine hydrochloride (63.6g, 1.6eq) and sodium acetate (140.86g, 3eq). The mixture was stirred at room temperature for 4h. EtOH was evaporated. The residue was extracted with ethyl acetate (3 x 500mL) and washed with water (1L). The organic layer was dried with MgSO₄ and concentrated to give brown oil (149.8g).
Yield=99%
¹H NMR (CDCl₃) : δ 8.06 (s, 1 H); 7.39 (s, 1 H); 7.24 (dd, 1 H); 6.63 (d, 1 H); 4.25 (quad, 2H); 2.26 (s, 3H); 2.15 (s, 1 H); 1.64 (s, 6H); 1.25 (t, 3H).

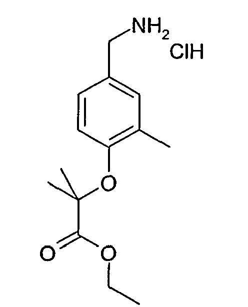

### Intermediate 10

### Ethyl2-{[4-(aminomethyl)-2-methylphenyl]oxy}-2-methylpronanoate hydrochloride

To a solution of **intermediate 9** (70g, 0.264mol) in 15 mL of AcOH and 420 mL of EtOH under nitrogen, was added 3.5 g Pd/c 10%. The reaction was put under hydrogen atmosphere (1 bar) at room temperature for 8h.
The mixture was filtered through a Whatmann filter, evaporated to dryness and diluted in H₂O (1L). The aqueous phase was washed with diethyl ether (3 x 400mL). The organic layers were washed with water (400mL layer). NaOH 35% was added to the aqueous layers until pH=14 and extracted with diethyl ether (3 x 400mL). The organic phase was dried with MgSO₄, put under HCI gas , stirred for 15min and concentrated to give a white solid (79.7g).
Yield=52%
Mp=89°C
¹H NMR (CDCl₃): δ 8.12 (m, 2H); 7.08 (s, 1 H); 6.99 (dd, 1 H), 6.38 (d, 1 H); 3.96 (quad, 2H); 3.66 (m, 2H); 1.94 (s. 3H); 1.31 (s, 6H); 0.95 (t, 3H).

Intermediate 10 may also be prepared by the following route:

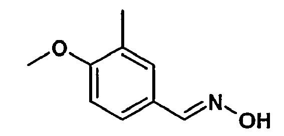

### Intermediate (i)

### 3-methyl-4-(methyloxy)benzaldehyde oxime

H₂NOH,HCl (1.6 equiv.), (3equiv.) NaOAc in 150mL H₂O is added to **4-methoxy-3-methylbenzalehyde** (1 equiv., Acros) in EtOH (150mL) at rt and the reaction is stirred for 2h. EtOH is evaporated, and the residue is extracted with CH₂Cl₂ (3 x 50mL). The combined organic layers are washed with H₂O, dried over Na₂SO₄, filtered and evaporated to dryness to afford the title compound as a white solid.

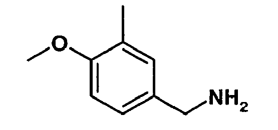

### Intermediate (ii)

### {[3-methyl-4-(methyloxy)ahenyl]methyl}amine

To Intermediate **intermediate (i)** (1 equiv.) in MeOH (200mL) at rt is added [MeCO₂]NH₄ (6 equiv.), Pd/C (0.01 equiv.) and molecular sieves The reaction is then heated to reflux for 18h. The reaction mix is filtered through celite, evaporated to dryness and treated with HCl (1 N). The aqueous layer is washed with CH₂Cl₂ , filtered, basified to pH >14 and extracted with CH₂Cl₂ (3 x 50mL). The combined organic layers are washed with H₂O, dried over Na₂SO₄, filtered and evaporated to dryness to afford the title compound as an oil.

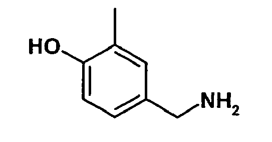

### Intermediate (iii)

### 4-(aminomethyl)-2-methylphenol

**Intermediate (il)** (1 equiv.) in excess 40% HBr/H₂O (Aldrich) is refluxed for 18h. The reaction is then evaporated to dryness to afford the title compound hydrobromide salt as a grey solid (97%).

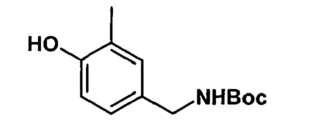

### Intermediate (iv)

### 1,1-dimethylethyl [(4-hydroxy-3-methylphenyl)methyl]carbamate

Et₃N (3 equiv.), Boc anhydride (0.95 equiv.) in CH₂Cl₂ (50mL) is added dropwise to **Intermediate (iii)** (1 equiv.) in CH₂Cl₂ (300mL) at 0°C. The reaction is allowed to warm to rt and stirring is continued for 18h. HCl (1 N) was added and the reaction extracted with CH₂Cl₂ (3 x 100mL). The organic layers are washed with H₂O, dried over Na₂SO₄, filtered and the solvent removed under vacuum to afford the title compound as a white solid (96%).

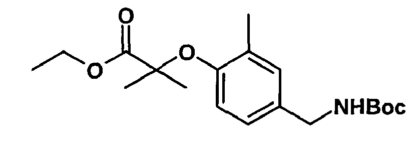

### Intermediate (v)

### Ethyl 2-({4-[({[(1,1-dimethylethyl)oxy]carbonyl}amino)methyl]-2-methylphenyl}oxy)-2-methylpropanoate

K₂CO₃ (3 equiv.) is added to **Intermediate (iv)** (1 equiv.) in DMF (150mL) and the reaction is heated to 70°C. **Ethyl 2-bromo-2-methylproprionate** (1.3 equiv.) is added dropwise and the reaction may be stirred for 72h at 70°C. The reaction is poured onto ice and extracted with CH₂Cl₂ (3 x 150mL). The combined organic layers are washed with NaOH (0.5N), then H₂O and dried over Na₂SO₄. The solution is filtered, evaporated to dryness to afford the title compound as an oil.

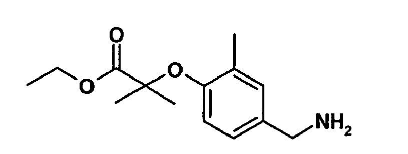

### Intermediate (10)

### Ethyl 2-{[4-(aminomethyl)-2-methylphenyl]oxy}-2-methylpropanoate

CF₃COOH (7 equiv.) is added dropwise to **Intermediate (v)** (1 equiv.) in CH₂Cl₂ (10mL) at rt and the reaction is stirred at rt for 18h. The reaction is evaporated to dryness, treated with a saturated K₂CO₃ solution and extracted with CH₂Cl₂ (3 x 150mL). The combined organic layers are dried over Na₂SO₄, filtered and evaporated to dryness to afford the title compound as an oil.

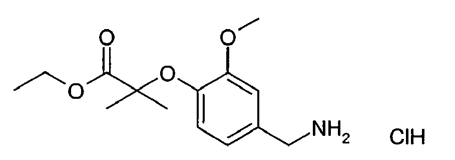

### Intermediate 11

### ethyl 2-{[4-(aminomethyl)-2-(methyloxy)phenyl]oxy}-2-methylpropanoate hydrochloride

To a solution of 9.8g (0.066mol) of **4-hydroxy-3-methoxybenzonitrile** in 100 mL of acetonitrile was added K₂CO₃ (18.16g, 0.131 mol). The mixture was stirred at 80°C for 1h and **ethyl 2-bromoisobutyrate** (19.6mL, 0.131 mol) was added dropwise. The mixture was stirred at reflux for 18h (after 1 h45 of reaction, 1 eq of K₂CO₃ and 1 eq of ethyl 2-bromoisobutyrate were added). The reaction mixture was concentrated to dryness and the residue was diluted with CH₂Cl₂ (150ml) and water (150mL), extracted with CH₂Cl₂ (150mL) and washed with NaOH 1 N, washed twice with water. The organic phase was dried with MgSO₄ and concentrated to give crystalline oil.
To a solution of this oil (16g) in 125 mL of EtOH was added concentrated HCI (20mL) and Pd/c 10% (1.6g). The reaction was put under hydrogen atmosphere (15 bars) at room temperature for 42h.

The mixture was filtered through celite , cleared with EtOH and concentrated to dryness. The residue was diluted in CH₂Cl₂ and evaporated twice to give 18.5g of the desired product.
Yield : 93%
¹H NMR (DMSO) : δ 8.48 (m, 2H); 7.27 (s, 1 H); 6.91 (s, 1 H); 6.71 (s, 1 H); 4.12 (m, 2H); 3.88 (m, 2H); 3.72 (s, 3H); 1.41 (s, 6H); 1.16 (m, 3H).

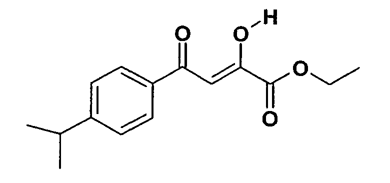

### Intermediate 12

### Ethyl 4-[4-(1-methylethyl)phenyl]-2,4-dioxobutanoate

3.4 g (0.148mol) of Na was added portionwise to 350 ml of EtOH in a 3L neck flask under nitrogen atmosphere at 0°C. A solution of 18.4g (0.114mol) of **p-iso-propylacetophenone** in 50 mL of EtOH was added dropwise and the reaction mixture was stirred for 20 min at 0°C then, 21.6 g (0.148mol) of **ethyl oxalate** in 50 mL of EtOH was added dropwise. The reaction mixture was heated for 2h at 80°C. The reaction mixture was cooled to rt and concentrated under vacuum. 500 mL of ethyl acetate were added and the organic layer was successively washed with brine, HCl (1 N) x2, and brine.
The organic layer was dried with Na₂SO₄. After filtration and evaporation under vacuum, the resulting red oil was purified using preparative chromatography providing the title compound as a red oil (5.42g).
Yield: 18%
¹H NMR (CDCl₃): δ 7.86 (d,1 H) ; 7.28 ( d, 2H) ; 6.99 (s, 1 H) ; 4.33 ( q, 3H) ; 2.91 ( m, 1H) ; 1.34 (t, 3H) ; 1.21 (d, 1 H).

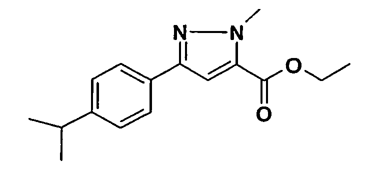

### Intermediate 13

### Ethyl 1-methyl-3-[4-(1-methylethyl)phenyl]-1H-pyrazole-5-carboxylate

1.65 mL (31 mmol) of methyl hydrazine was added to a solution of 5.4g (20.6mmol) of **Intermediate 12** in 200 mL of EtOH. The reaction mixture was heated at 90°C for 24h, cooled at rt and three quarters of the ethanol was removed under vacuum. The solution was diluted with ethyl acetate and was washed with HCl 1N, and brine. The organic layer was dried over sodium sulfate and evaporated to dryness. The resulting solid was purified on flash silica column using Cyclohexane/AcOEt (90/10) then Cyclohexane/AcOEt (80/20). The title compound was identified as the less polar isomer and recrystallized from butanol-1 at 0°C to give the title compound as 2.05 g of colorless crystals
Yield: 36%
¹H NMR (CDCl₃) : δ 7.70 (d, 2H) ; 7.28 (d, 2H) ; 7.11 (s, 1 H) ; 4,39 (q, 3H) ; 4.24 (s, 3H) ; 2.95 (hept, 1 H) ; 1.43 (t, 3H) ; 1.29 (d, 6H).

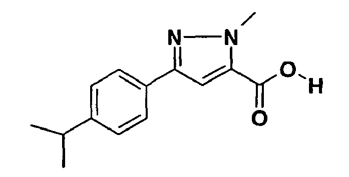

### Intermediate 14

### 1-methyl-3-[4-(1-methylethyl)phenyl]-1H-pyrazole-5-carboxylic acid.

20 mL of EtOH and 38 ml of NaOH 1 N were added to a solution of 1 g (3.7mmol) of **Intermediate 13** in 20 mL of THF. The reaction mixture was stirred 1h at rt, EtOH and THF were evaporated under vacuum. A solution of HCl 1 N was added to the reaction mixture to obtain precipitation of a white solid. After filtration, the resulting white solid was rinsed with water and dried under vacuum (m =0.71 g).
Yield: 79%
¹H NMR (CDCl3) : δ 7.65 (d, 2H) ; 7.20 (d, 2H) ; 7.15 (s, 1 H) ; 4.17 (s, 3H) ; 2.86 (hept, 1 H) ; 1.20 (d, 6H).

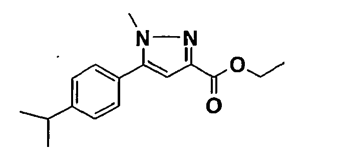

### Intermediate 15

### Ethyl 1-methyl-5-[4-(1-methylethyl)phenyl]-1H-pyrazole-3-carboxylate

1.65 mL (31 mmol) of methyl hydrazine was added to a solution of 5.4g (20.6mmol) of **Intermediate 12** in 200 mL of EtOH. The reaction mixture was heated at 90°C for 24h, cooled at rt and three quarters of the ethanol was removed under vacuum. The solution was diluted with ethyl acetate and washed with HCl 1 N, and brine. The organic layer was dried over sodium sulfate and evaporated to dryness. The resulting solid was purified on flash silica column using Cyclohexane/AcOEt (90/10) then Cyclohexane/AcOEt ( 80/20).
The title compound was eluted as the more polar isomer and recrystallized from petroleum ether to give the title compound as 1 g of colorless crystals.
Yield: 18%
¹H NMR (CDCl₃): δ 7.15 (m, 4H) ; 6.65 (s, 1 H) ; 4,25 (q, 3H) ; 3.77 (s, 3H) ; 2.79 (hept, 1H); 1.23 (t, 3H) ; 1.11 (d, 6H).

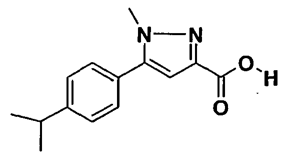

### Intermediate 16

### 1-methyl-5-[4-(1-methylethyl)phenyl]-1H-pyrazote-3-carboxylic acid

To a solution of 260 mg (0.95mmol) of **Intermediate 15** in 1 mL of THF and 3 mL of EtOH was added 6 mL of NaOH 1 N. The reaction mixture was stirred 1 h30 at rt, EtOH and THF were evaporated under vacuum. Precipitation of the carboxylic acid was achieved by acidifying with a 1 N solution of HCl. After filtration, the resulting beige powder was rinsed with water and dried under vacuum (M =219 mg).
Yield: 94%
¹H NMR (DMSO d6) : δ 7.48 (d, 2H) ; 7.38 (d, 2H) ; 6.79 (s, 1 H) ; 3.90 (s, 3H) ; 2.96 (hept, 1 H) ; 1.24 (d, 6H).

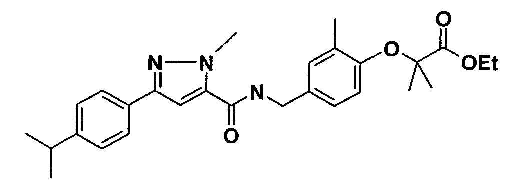

### Intermediate 17

### Ethyl 2-methyl-2-[(2-methyl-4-{[({1-methyl-3-[4-(1-methylethyl) phenyl]-1H-pyrazol-5-yl} carbonyl) amino]methyl} phenyl)oxy] propanoate

**Intermediate 14** (134 mg, 0.55 mmol) was dissolved in the minimum amount of DMF (about 5 mL), HOBT (74 mg, 0.55 mmol), EDCI (105 mg, 0.55 mmol), Et₃N (155 µL, 1.1 mmol) and **intermediate 10** (144 mg, 0.5 mmol) were successively added. The mixture was stirred at rt for 26 hours and the DMF was evaporated under reduced pressure. The residue was diluted in EtAOc and washed with a saturated NaHCO₃ solution and brine, dried over Na₂SO₄, filtered and evaporated to dryness. The product was then purified by flash chromatography on silica gel (CH₂CL₂/AcOEt = 95/5) to afford the title compound as white crystals (183mg).
Yield: 77%
¹H NMR (CDCl₃) : δ 7.60 (d, 2H) ; 7.17 (d, 2H) ; 7.06 (m, 1 H) ; 6.94 (dd, 1 H) ; 6.65 (s, 1H); 6.56 (d, 1H); 6.16 (t, 1H); 4.43 (d, 2H) ;4.18 (q, 2H) ; 4.17 (s, 3H) ; 2.85 (hept, 1H) ; 2.17 (s, 3H) ; 1.53 (s, 6H) ; 1.20 (t, 3H) ; 1.19 (d, 6H).

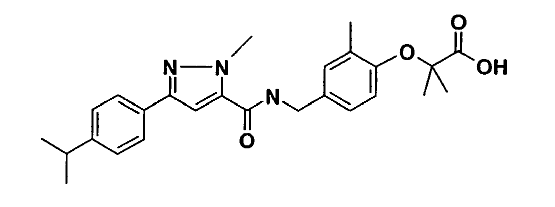

### Example 2

### 2-methyl-2-[(2-methyl-4-{[({1-methyl-3-[4-(1-methylethyl)phenyl]-1H-pyrazol-5-yl}carbonyl)amino]methyl}phenyl)oxy]propanoic acid.

**Intermediate 17,** (178 mg, 0.373 mmol) was dissolved in 50 mL of THF and 1N NaOH (3.7 mL, 3.73 mmol) was added. The solution was heated at 80°C for 1 hour and the solvents were evaporated under reduced pressure. The resulting pale yellow product was dissolved in water and acidified with 1 N HCl. The white precipitate was filtered off, washed with water and dried under vacuum to give the title compound as 136 mg of a white powder.
Yield = 81 %
¹H NMR (CDCl₃) : δ 7.60 (d, 2H) ; 7.17 (d, 2H) ; 7.09 (s, 1 H) ; 7.00 (d, 1 H) ; 6.74 (d, 1 H) ; 6.66 (s, 1 H) ; 6.25 (t, 1 H) ; 4.43 (d, 2H) ; 4.16 (s, 3H) ; 2.85 (hept, 1 H) ; 2.18 (s, 3H) ; 1.56 (s, 6H) ; 1.2 (s, 3H); 1.18 (s,3H).

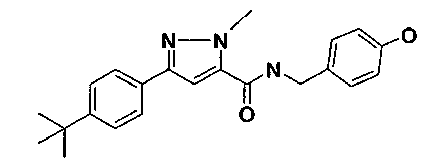

### Intermediate 18

### 3-[4-(1,1-dimethylethyl)phenyl]-N-[(4-hydroxyphenyl)methyl]-1-methyl-1H-pyrazole-5-carboxamide

3 mL (10eq) of SOCl₂ was added dropwise to a suspension of **intermediate 5** (500 mg, 1.9 mmol) in 100 mL of anhydrous toluene and the reaction mixture was heated at 90°C for 2hours. Toluene and SOCl₂ were evaporated under vacuum and the residue was diluted in 50 mL of anhydrous CH₂Cl₂ and added dropwise to a solution of **p-hydroxybenzylamine hydrobromide** (887 mg, 4.3 mmol) in 1.35 mL of Et₃N (2.5 eq.), 50 mL of CH₂Cl₂ and 5 mL DMF (required amount to get solubility). The reaction mixture was stirred 18 hours at rt, evaporated to dryness and suspended in 1 N HCl. The precipitate was filtered off, diluted in 50 mL of EtOH and heated with 1 N NaOH (10 mL) at 80°C for 1 hour to remove the phenol ester. The solvents were removed in vacuum and the phenol was precipitated using a saturated NH₄Cl solution, washed with water and dried under reduced pressure to afford the title compound as a beige powder (530 mg)
Yield: 74%
¹H NMR (DMSO d6) : δ 8.99 (m, 1 H) ; 7.66 (d, 2H) ; 7.44 (d, 2H) ; 7.26 (s, 1 H) ; 7.13 (d, 2H) ; 6.73 (d, 2H) ; 4.33 (d, 2H) ; 4.10 (s, 3H); 1.29 (s, 9H).

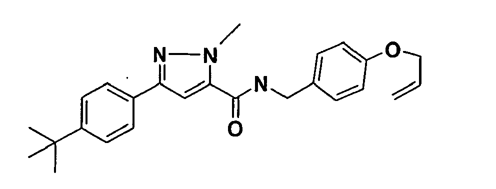

### Intermediate 19

### 3-[4-(1,1-dimethylethyl)phenyl]-1-methyl-N-{[4-(2-propen-1-yloxy)phenyl]methyl}-1H-pyrazole-5-carboxamide

**Intermediate 18** (530 mg, 1.4 mmol) was dissolved in 50 mL of acetone, K₂CO₃ (300 mg, 1.8 mmol) was added followed by addition of **allyl bromide** (200 µL, 1.4 mmol) and the resulting mixture was stirred at 70°C for 18 hours. At this stage, 3 more eq. of K₂CO₃ and 2 eq. of **allyl bromide** were added and the reaction mixture was heated for an additional 18 hours to completion. Solvents were evaporated and the residue was dissolved in ether (50 mL) and washed with water (50 mL). The organic layer was dried over sodium sulfate and evaporated to dryness to provide the title compound as a beige powder (580 mg).
Yield : 99%
¹H NMR (CDCl₃) : δ 6.61 (d, 2H) ; 7.34 (d, 2H) ; 7.21 (d, 2H) ; 6.85 (d, 2H) ; 6.65 (s, 1H) ; 6.19 (t, 1H); 5.98 (m, 1 H) ; 5.32 (dd, 2H) ; 5.22 (dd, 2H); 4.47 (m, 4H) ; 4.17 (s, 3H) ; 1.26 (s, 9H).

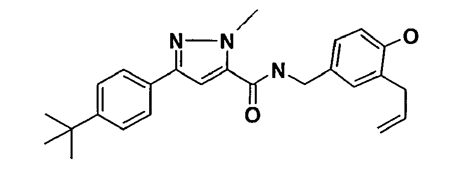

### Intermediate 20

### 3-[4-(1.1-dimethylethyl)phenyl]-N-{[4-hydroxy-3-(2-propen-1-yl)phenyl]methyl}-1-methyl-1H-pyrazole-5-carboxamide.

**Intermediate 19** (580 mg, 1.39 mmol) was heated neat at 250°C in a sealed tube for 30 minutes. After cooling down, the phenol was isolated without further purification as a beige powder (300 mg).
Yield : 52%
¹H NMR (DMSO d6) : δ 9.30 (s, 1 H) ; 8.96 (t, 1 H) ; 7.67 (d, 2H) ; 7.44 (d, 2H) ; 7.24 (m, 1 H) ; 7.00 (m, 2H) ; 6.75 (d, 1 H) ; 5.92 (m, 1 H) ; 5.01 (m, 2H) ; 4.31 (d, 2H); 4.09 (s, 3H) ; 3.30 (m, 2H) ; 1.29 (s,9H).

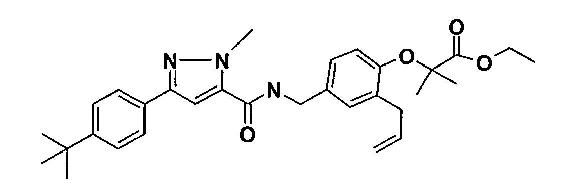

### Intermediate 21

### Ethyl 2-{[4-{[({3-[4-(1,1-dimethylethyl)phenyl]-1-methyl-1H-pyrazol-5-yl}carbonyl)amino]methyl}-2-(2-propen-1-yl)phenyl]oxy}-2-methylpropanoate.

**Intermediate 20** (300 mg, 0.72 mmol) was dissolved in 50 mL of acetone with **ethyl 2-bromoisobutyrate** (300 µL, 2.1 mmol) and K₂CO₃ (287 mg, 2.1 mmol) and the reaction mixture was heated at 70°C for 72 hours. After evaporation of the solvents, the residue was diluted in ether and washed with water. The organic layer was dried over sodium sulfate and evaporated to dryness and then purified by flash chromatography on silica gel (CH₂CL₂/MeOH = 99.5/0.5 to 98/2) to afford the title compound as a white powder recrystallized from diisopropyl oxide (220 mg).
Yield: 58%
¹H NMR (DMSO d6): δ 9.01 (t, 1 H) ; 7.67 (d, 2H) ; 7.44 (d, 2H) ; 7.26 (s, 1 H) ; 7.10 (m, 2H) ; 6.58 (d, 1 H) ; 5.05 (m, 2H) ; 4.35 (m, 2H); 4.17 (q, 2H) ; 4.09 (s, 3H) ; 3.30 (m, 2H) ; 1.51 (s, 6H) ; 1.29 (s, 9H) ; 1.17 (q, 3H).

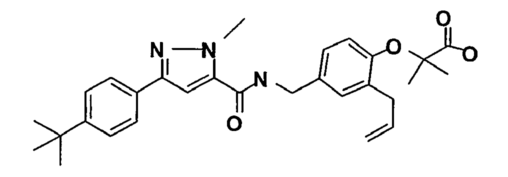

### Example 3

### 2-{[4-{[({3-[4-(1,1-dimethylethyl)phenyl]-1-methyl-1H-pyrazol-5-yl}carbonyl)amino]methyl}-2-(2-propen-1-yl)phenyl]oxy}-2-methylpropanoic acid.

**Intermediate 21** (220 mg, 0.41 mmol) was dissolved in 50 mL of ethanol and 2 mL of 1 N NaOH (2 mmol) and the solution was heated at 80°C for 18 hours. Concentration in vacuum was followed by acidification with 1 N HCl and filtration of the precipitate to give 90 mg of a white powder.
Yield : 45%
¹H NMR (CDCl₃) : δ 7.47 (d, 2H) ; 7.24 (d, 2H) ; 6.94 (m, 2H) ; 6.57 (m, 2H) ; 6.13 (m, 1H) ; 5.79 (m, 1 H) ; 4.92 (m, 2H) ; 4.35 (m, 2H) ; 4.04 (s, 3H) ; 3.23 (d, 2H) ; 1.46 (s, 6H) ; 1.15 (s, 9H).

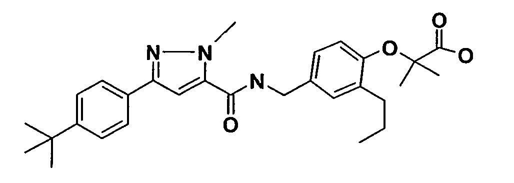

### Example 4

### 2-[(4-{[({3-[4-(1,1-dimethylethyl)phenyl]-1-methyl-1H-pyrazol-5-yl}carbonyl)amino]methyl}-2-propylphenyl)oxy]-2-methylpropanoic acid.

**Example 3** (90mg, 0.18 mmol), ammonium formate (120 mg, 1.9 mmol) and Pd/C10% (50mg) were stirred in 50 mL of ethanol for 2 hours at rt, then for 18 hours at 60°C. The reaction mixture was filtered through a celite pad and the resulting product was purified by preparative HPLC to afford the title compound as a pale pink powder (20mg).
Yield : 22%
¹H NMR (CDCl₃) : δ 7.57 (d, 2H) ; 7.31 (d, 2H) ; 7.04 (s, 1H) ; 6.92 (m, 1 H) ; 6.66 (m, 2H) ; 6.45 (m, 1 H) ; 4.41 (d, 2H) ; 4.13 (s, 3H) ; 2.49 (t, 2H) ; 1.53 (s, 6H); 1.20 (m, 11H) ; 0.87 (t, 3H).

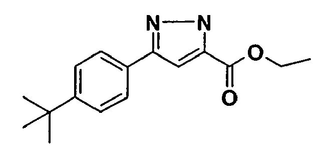

### Intermediate 22

### Ethyl 3-[4-(1,1-dimethylethyl)phenyl]-1H-pyrazole-5-carboxylate

To a solution of **intermediate 1** (76.2 g, 0.276 mol) in 750 mL of ethanol under a nitrogen atmosphere was added hydrazine hydrate (13.4 mL, 0.276 mol) and the mixture was heated at 90°C for 3 hours. The yellow crystals were separated by filtration and the red filtrate was cooled down promoting the formation of a second crop of yellow crystals which were also filtered. Ethanol was evaporated and the residue was diluted in the minimum amount of CH₂CL₂ promoting precipitation of a white solid filtered off and rinsed with CH₂CL₂ affording the title compound as a white powder (37.8 g).
Yield : 50%
¹H NMR (DMSO d6) : δ 7.76 (d, 2H) ; 7.46 (d, 2H) ; 7.18 (brs, 1 H) ; 4,31 (q, 2H) ; 1.32 (t, 3H) ; 1.30 (s, 9H).

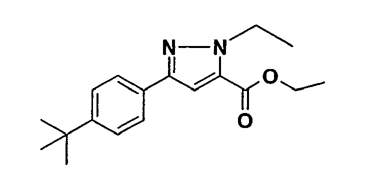

### Intermediate 23

### Ethyl 3-[4-(1,1-dimethylethyl)phenyl]-1-ethyl-1H-pyrazole-5-carboxylate.

To a solution of **intermediate 22** (18.8 g, 69 mmol) in 500 mL of acetone was added K₂CO₃ (28.7 g, 207 mmol) and ethyl iodide (27.9 mL, 346 mmol). The reaction mixture was heated at 70 °C for 19h30 under a nitrogen atmosphere and after cooling down, the inorganic salts were filtrated and the filtrate was evaporated to dryness. The resulting solid was diluted in AcOEt and water and the organic layer was separated and washed with 1 N HCl and then with brine. The organic layer was dried over sodium sulfate and evaporated to dryness and then purified by flash chromatography on silica gel (cyclohexane/EtOAc = 90/10 to 80/20) to afford the title compound as the first eluted (yellow oil which crystallizes on standing (14.4 g, yield : 70%)).
¹H NMR (CDCl₃) : δ 7.55 (d, 2H) ; 7.23 (d, 2H) ; 6.91 (s, 1H) ; 4,45 (q, 2H) ; 4,16 (q, 2H) ; 1.28 (t, 3H) ; 1.20 (t, 3H) ; 1.15 (s, 9H).

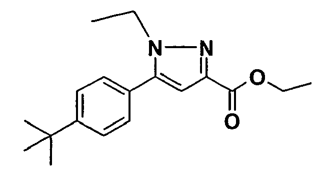

### Intermediate 24

### Ethyl 5-[4-(1,1-dimethylethyl)phenyl]-1-ethyl-1H-pyrazole-3-carboxylate.

To a solution of **intermediate 22** (18.8 g, 69 mmol) in 500 mL of acetone was added K₂CO₃ (28.7 g, 207 mmol) and ethyl iodide (27.9 mL, 346 mmol). The reaction mixture was heated at 70 °C for 19h30 under a nitrogen atmosphere and after cooling down, the inorganic salts were filtrated and the filtrate was evaporated to dryness. The resulting solid was diluted in AcOEt and water and the organic layer was separated and washed with 1 N HCI and then with brine. The organic layer was dried over sodium sulfate and evaporated to dryness and then purified by flash chromatography on silica gel (cyclohexane/EtOAc = 90/10 to 80/20) to afford the title compound as the second eluted (yellow oil (5.4 g)).
Yield: 26%
¹H NMR (CDCl₃) : δ 7.46 (d, 2H) ; 7.30 (d, 2H) ; 6.77 (s, 1H) ; 4,41 (q, 2H) ; 4,23 (q, 2H) ; 1.41 (t, 3H) ; 1.39 (t, 3H) ; 1.34 (s, 9H).

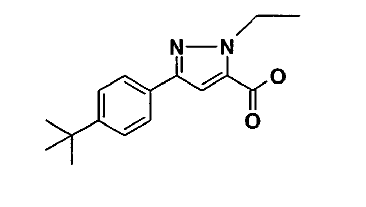

### Intermediate 25

### 3-[4-(1,1-dimethylethyl)phenyl]-1-ethyl-1H-pyrazole-5-carboxylic acid.

**Intermediate 23** (8.5 g, 28.3 mmol) was dissolved in the minimum amount of THF and 250 mL of ethanol and NaOH 1 N (283ml, 283 mmol) were added. The solution was heated to 80°C for 1 hour and concentrated under vacuum. The resulting white solid was acidified by 1 N HCl and the white precipitate was filtered off and dried to provide the title carboxylic acid as 6.7 g of a white powder.
Yield : 87%
¹H NMR (CDCl₃) : δ 7.54 (d, 2H) ; 7.43 (d, 2H) ; 7.23 (s, 1 H) ; 4,66 (q, 2H) ; 1.50 (t, 3H) ; 1.34 (s, 9H).

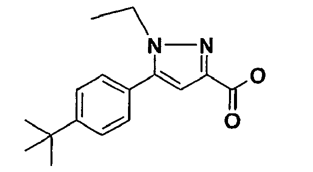

### Intermediate 26

### 5-[4-(1,1-dimethylethyl)phenyl]-1-ethyl-1H-pyrazole-3-carboxylic acid.

**Intermediate 24** (5.34g, 17.8mmol) was saponified following the procedure to make 3-[4-(1,1-dimethylethyl)phenyl]-1-ethyl-1*H*-pyrazole-5-carboxylic acid (intermediate 25) providing the title compound as a white powder (3.46g).
Yield : 71%
¹H NMR (CDCl₃) : δ 7.48 (d, 2H) ; 7.32 (d, 2H) ; 6.84 (s, 1H) ; 4,25 (q, 2H) ; 1.45 (t, 3H) ; 1.35 (s, 9H).

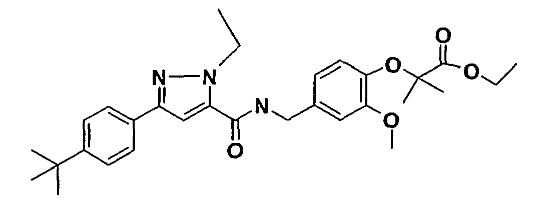

### Intermediate 27

### Ethyl 2-{[4-{[({3-[4-(1,1-dimethylethyl)phenyl]-1-ethyl-1H-pyrazol-5-yl}carbonyl)amino]methyl}-2-(methyloxy)phenyl]oxy}-2-methylpropanoate.

**Intermediate 25** (197 mg, 0.72 mmol) was dissolved in the minimum amount of DMF (about 5 mL), HOBT (98 mg, 0.72 mmol), EDCI (138 mg, 0.72 mmol), Et₃N (203 µL, 1.45 mmol) and **Intermediate 11** (200 mg, 0.66 mmol) were successively added. The mixture was stirred at rt for 26 hours and the DMF was evaporated under reduced pressure. The residue was diluted in AcOEt and washed with 1N HCI (1x), with a saturated NaHCO₃ solution (3x) and with brine (1x), dried over Na₂SO₄. This was then filtered and evaporated to dryness. The product was then purified by flash chromatography on silica gel (CH₂CL₂/AcOEt = 97/3) to afford the title compound as a yellow oil (195 mg).
Yield: 57%
¹H NMR (CDCl₃) : δ 7.69 (d, 2H) ; 7.41 (d, 2H) ; 6.83 (m, 3H) ; 6.72 (s, 1 H) ; 6.30 (t, 1H) ; 4.65 (q, 2H) ; 4.54 (d, 2H) ; 4.23 (q, 2H) ; 3.81 (s, 3H) ; 1.56 (s, 6H) ; 1.49 (t, 3H) ; 1.32 (s, 9H) ; 1.27 (t, 3H).

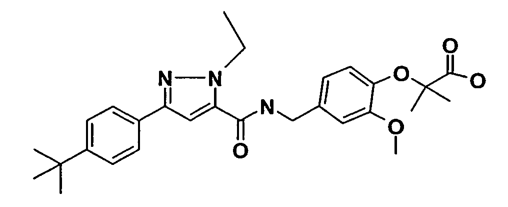

### Example 5

### 2-{[4-{[({3-[4-(1,1-dimethylethyl)phenyl]-1-ethyl-1H-pyrazol-5-yl}carbonyl)amino]methyl}-2-(methyloxy)phenyl]oxy}-2-methylpropanoic acid.

**Intermediate 27** (190 mg, 0.36 mmol) was dissolved in the minimum amount of THF and 3 mL of ethanol and 3.6 mL of 1 N NaOH (3.6 mmol) were added. The solution was heated to 80°C for 1 hour and concentrated under vacuum. The residue was diluted in water and acidified by 1 N HCl and the white precipitate was filtered off and dried to provide the title carboxylic acid as 151 mg of a white powder.
Yield : 85%
¹H NMR (CDCl₃) : δ 7.69 (d, 2H) ; 7.41 (d, 2H) ; 6.99 (d, 1 H) ; 6.94 (s, 1 H) ; 6.90 (d, 1H) ; 6.74 (s, 1 H) ; 6.37 (m, 1 H) ; 4.65 (q, 2H) ;4.58 (d, 2H) ; 3.89 (s, 3H) ; 1.51 (s, 6H) ; 1.49 (t, 3H) ; 1.32 (s, 9H).

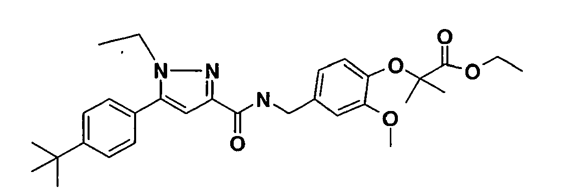

### Intermediate 28

### Ethyl 2-{[4-{[({5-[4-(1,1-dimethylethyl)phenyl]-1-ethyl-1H-pyrazol-3-yl}carbonyl)amino]methyl}-2-(methyloxy)phenyl]oxy}-2-methylpropanoate.

**Intermediate 26** (197 mg, 0.72 mmol) was dissolved in the minimum amount of DMF (about 5 mL), HOBT (98 mg, 0.72 mmol), EDCI (138 mg, 0.72 mmol), Et₃N (203 µL, 1.45 mmol) and **Intermediate 11** (200 mg, 0.66 mmol) were successively added. The mixture was stirred at rt for 26 hours and the DMF was evaporated under reduced pressure. The residue was diluted in AcOEt and washed with 1 N HCl (2x), with a saturated NaHCO₃ solution (3x) and with brine (2x), dried over Na₂SO₄, filtered and evaporated to dryness. The product was then purified by flash chromatography on silica gel (CH₂CL₂/AcOEt = 97/3) to afford the title compound as a white oil (257 mg).
Yield : 75%
¹H NMR (CDCl₃) : δ 7.47 (d, 2H) ; 7.31 (d, 2H) ; 7.20 (t, 1H) ; 6.89 (s, 1 H) ; 6.81 (brs, 3H) ; 4.56 (d, 2H) ; 4.23 (q, 2H) ; 4.13 (q, 2H) ; 3.80 (s, 3H) ; 1.55 (s, 6H) ; 1.41 (t, 3H) ; 1.35 (s, 9H) ; 1.27 (t, 3H).

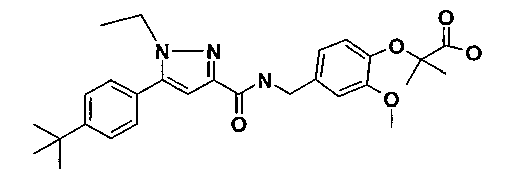

### Example 6

### 2-{[4-{[({5-[4-(1,1-dimethylethyl)phenyl]-1-ethyl-1H-pyrazol-3-yl}carbonyl)amino]methyl}-2-(methyloxy)phenyl]oxy}-2-methylpropanoic acid.

**Intermediate 28** (257 mg, 0.49 mmol) was dissolved in the minimum amount of THF and 4 mL of ethanol and 4.9 mL (4.9 mmol) of 1 N NaOH were added. The solution was stirred at rt for 2 hour and concentrated under vacuum. The residue was diluted in water and acidified by 1 N HCl and the white precipitate was filtered off and dried to provide the title carboxylic acid as 205 mg of a yellow powder.
Yield : 85%
¹H NMR (CDCl₃) : δ 7.47 (d, 2H) ; 7.30 (d, 2H) ; 7.26 (m, 1 H) ; 6.95 (m, 3H) ; 6.81 (s, 1 H) ; 4.60 (d, 2H) ; 4.14 (q, 2H) ; 3.89 (s, 3H) ; 1.50 (s, 6H) ; 1.41 (t, 3H) ; 1.35 (s, 9H).

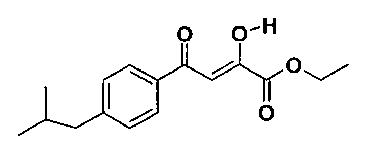

### Intermediate 29

### Ethyl 4-[4-(2-methylpropyl)phenyl]-2,4-dioxobutanoate.

**Sodium ethylate** (2.53 g, 0.11 mol) was added portionwise at 0°C in 200 mL of anhydrous ethanol under a nitrogen atmosphere. After dissolution was completed, a solution of **4'-isobutylacetophenone** (17.6 g, 0.1 mol) in 30 mL of anhydrous ethanol was added dropwise at 0°C. When the addition was over, the mixture was stirred at 0°C for 30 minutes and a solution of **ethyloxalate** (16.06 g, 0.11 mol) in 25 mL of anhydrous ethanol was added dropwise. The mixture was stirred at rt for 16 hours and evaporated under reduced pressure. The oily product was dissolved in AcOEt and washed with 1 N HCl (2x), with brine (1x), and dried over Na₂SO₄. This was then filtered and evaporated to dryness giving the title compound as an orange oil (28 g). The crude product was used without further purification (8% of starting material remaining).
Yield : quant.
¹H NMR (CDCl₃) : δ 15.31 (brs, 1H); 7.84 (d, 2H) ; 7.20 (d, 2H) ; 6.99 (s, 1H); 4.32 (q, 2H); 2.49 (m, 2H) ; 1.84 (m, 1 H) ; 1.33 (t, 3H) ; 0.84 (d, 6H).

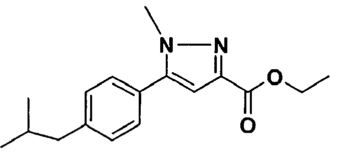

### Intermediate 30

### Ethyl 1-methyl-5-[4-(2-methylpropyl)phenyl]-1H-pyrazole-3-carboxylate.

5g (109mmol) of methyl hydrazine was added to a solution of **Intermediate 29** (10 g, 36 mmol) in 300 mL of EtOH and the reaction mixture was heated to reflux for 24 hours, cooled at rt and concentrated to a volume of approximately 70 mL. The mixture was diluted with AcOEt, washed with 1 N HCl and with brine. The organic layer was dried with Na₂SO₄, filtered and evaporated under vacuum and the resulting red oil was subjected to chromatography on silica gel (Cyclohexane/AcOEt = 90/10 to 80/20). The title compound was eluted as the second fraction (yellow oily product, 2.25 g).
Yield : 22%
¹H NMR (CDCl₃) : δ 7.30 (d, 2H) ; 7.23 (d, 2H) ; 6.82 (s, 1 H) ; 4,41 (q, 2H) ; 3.94 (s, 3H) ; 2.52 (d, 2H) ; 1.90 (hept, 1 H) ; 1.40 (t, 3H) ; 0.92 (s, 6H).

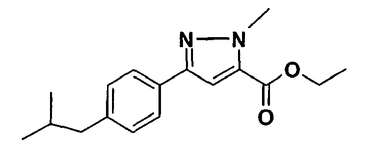

### Intermediate 31

### Ethyl 1-methyl-3-[4-(2-methylpropyl)phenyl]-1H-pyrazole-5-carboxylate.

5g of methyl hydrazine was added to a solution of **Intermediate 29** (10 g, 36 mmol) in 300 mL of EtOH was added and the reaction mixture was heated to reflux for 24 hours, cooled at rt and concentrated to a volume of approximately 70 mL. The mixture was diluted with AcOEt, washed with 1 N HCl and with brine. The organic layer was dried with Na₂SO₄, filtrated and evaporated under vacuum and the resulting red oil was subject to chromatography on silica gel (Cyclohexane/AcOEt = 90/10 to 80/20). The title compound was eluted as the first fraction (yellow oily product, 5.79 g).
Yield : 56%
¹H NMR (CDCl₃) : δ 7.68 (d, 2H) ; 7.17 (d, 2H) ; 7.09 (s, 1H) ; 4,36 (q, 2H) ; 4.21 (s, 3H) ; 2.49 (d, 2H) ; 1.87 (hept, 1 H) ; 1.39 (t, 3H) ; 0.90 (s, 6H).

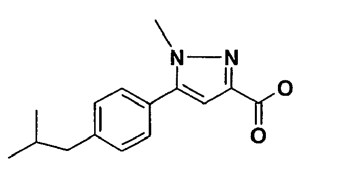

### Intermediate 32

### 1-methyl-5-[4-(2-methylpropyl)phenyl]-1H-pyrazole-3-carboxylic acid.

**Intermediate 30** (1.1 g, 3.85 mmol) was dissolved in 25 mL of THF and 25 mL of ethanol and 7.7 mL (7.7 mmol) of 1 N NaOH were added. The solution was stirred at rt for 3 hours and concentrated under vacuum. The residue was diluted in 10 mL of water and acidified with 1 N HCl and the gummy product was filtered off, triturated with pentane and dried to provide the title carboxylic acid as 980 mg of a beige powder.
Yield : quantitative
¹H NMR (CDCl₃) : δ 7.22 (d, 2H) ; 7.14 (d, 2H) ; 6.70 (s, 1 H) ; 3.83 (s, 3H) ; 2.42 (d, 2H); 1.80 (hept, 1 H) ; 0.92 (s, 6H).

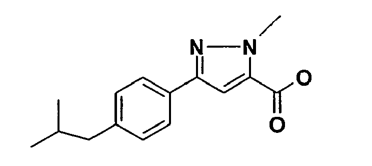

### Intermediate 33

### 1-methyl-3-[4-(2-methylpropyl)phenyl]-1H-pyrazole-5-carboxylic acid.

**Intermediate 31** (3g, 10.5mmol) was saponified following the procedure used for intermediate 32 to obtain a beige powder (2.52g).
Yield : 93%
¹H NMR (CDCl₃) : δ 7.70 (d, 2H) ; 7.22 (s, 1H) ; 7.19 (d, 2H) ; 4.24 (s, 3H) ; 2.49 (d, 2H) ; 1.88 (hept, 1 H) ; 0.91 (s, 6H).

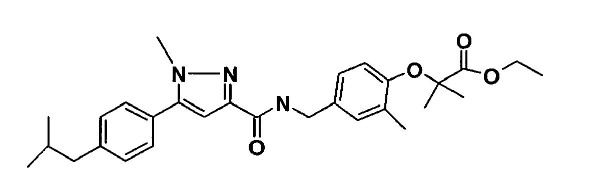

### Intermediate 34

### Ethyl 2-methyl-2-[(2-methyl-4-{[({1-methyl-5-[4-(2-methylpropyl) phenyl]-1H-pyrazol-3-yl} carbonyl) amino] methyl} phenyl) oxy] propanoate

**Intermediate 32** (142 mg, 0.55 mmol) was dissolved in 3 mL of DMF, HOBT (75 mg, 0.55 mmol), EDCI (105 mg, 0.55 mmol), Et₃N (155 µL, 1.1 mmol) and **Intermediate 10** (144 mg, 0.5 mmol) were successively added. The mixture was stirred at rt for 24 hours and was diluted with EtAOc and washed with a saturated NaHCO₃ solution and brine, dried over Na₂SO₄, filtered and evaporated to dryness. The product was then purified by flash chromatography on silica gel (CH₂CL₂/AcOEt = 95/5) to afford 140 mg of the title compound.
Yield: 57%
¹H NMR (CDCl₃) : δ 7.30 (d, 2H) ; 7.23 (d, 2H) ; 7.01-7.13 (m, 3H) ; 6.84 (s, 1 H) ; 6.61 (d, 1 H) ; 4.51 (d, 2H) ; 4.23 (q, 2H) ; 3.85 (s, 3H) ; 2.52 (d, 2H) ; 2.21 (s, 3H) ; 1.90 (hept, 1H); 1.57 (s, 6H) ; 1.25 (t, 3H) ; 0.93 (d, 6H).

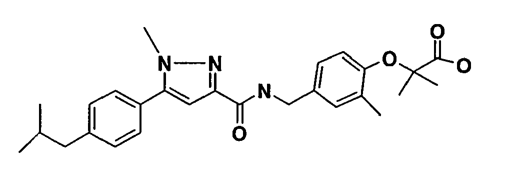

### Example 7

### 2-methyl-2-[(2-methyl-4-{[({1-methyl-5-[4-(2-methylpropyl) phenyl]-1H-pyrazol-3-yl} carbonyl) amino] methyl} phenyl) oxy]propanoic acid.

**Intermediate 34** (130 mg, 0.26 mmol) was dissolved in 2 mL of THF and 5 mL of ethanol and 2.6 mL of 1 N NaOH (2.6 mmol) were added. The solution was heated to 80°C for 24 hours and concentrated under vacuum. The residue was acidified with 1 N HCl and the white solid was filtered off and dried under reduced pressure at 50°C to provide the title carboxylic acid as 116 mg of a white powder.
Yield : 96%
¹H NMR (CDCl₃) : δ 7.30 (d, 2H) ; 7.22 (d, 2H) ; 7.17 (s, 1 H) ; 7.06 (dd, 1 H) ; 6.85 (s, 1 H) ; 6.78 (d, 1 H) ; 4.52 (d, 2H) ; 3.85 (s, 3H) ; 2.51 (d, 2H) ; 2.23 (s, 3H) ; 1.90 (hept, 1 H) ; 1.60 (s, 6H) ; 0.93 (d, 6H).

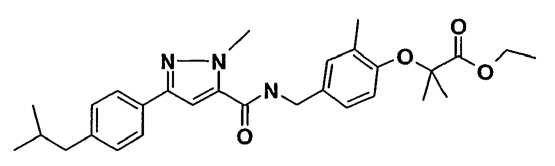

### Intermediate 35

### Ethyl 2-methyl-2-[(2-methyl-4-{[({1-methyl-3-[4-(2-methylpropyl) phenyl]-1H-pyrazol-5-yl}carbonyl) amino]methyl} phenyl) oxy] propanoate.

**Intermediate 33** (142 mg, 0.55 mmol) was dissolved in 3 mL of DMF, HOBT (75 mg, 0.55 mmol), EDCI (105 mg, 0.55 mmol), Et₃N (155 µL, 1.1 mmol) and **intermediate 10** (144 mg, 0.5 mmol) were successively added. The mixture was stirred at rt for 24 hours and was diluted with AcOEt and washed with a saturated NaHCO₃ solution and brine, dried over Na₂SO₄, filtered and evaporated to dryness. The product was then purified by flash chromatography on silica gel (CH₂CL₂/AcOEt = 95/5) to afford 205 mg of the title compound.
Yield: 83%
¹H NMR (CDCl₃) :δ 7.63 (d, 2H) ; 7.14 (d, 2H) ; 7.11 (m, 1 H) ; 7.01 (dd, 1H); 6.72 (s, 1H); 6.62 (d, 1 H) ; 6.31 (t, 1 H) ; 4.47 (d, 2H) ; 4.23 (q, 2H) ; 4.21 (s, 3H) ; 2.47 (d, 2H) ; 2.22 (s, 3H) ; 1.86 (hept, 1 H) ; 1.58 (s, 6H) ; 1.25 (t, 3H) ; 0.89 (d, 6H).

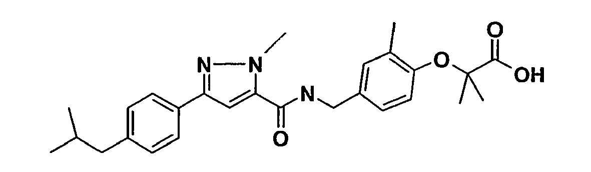

### Example 8

### 2-methyl-2-[(2-methyl-4-{[({1-methyl-3-[4-(2-methylpropyl) phenyl]-1H-pyrazol-5-yl} carbonyl) amino] methyl} phenyl) oxy]propanoic acid.

**Intermediate 35** (202 mg, 0.41 mmol) was dissolved in 2 mL of THF and 15 mL of ethanol and 4.1 mL (4.1 mmol) of 1 N NaOH were added. The solution was heated to 80°C for 2.5 hours and concentrated under vacuum. The residue was diluted in 10 mL of water and acidified with 1 N HCl and the white solid was filtered off and dried under reduced pressure at 50°C to provide the title carboxylic acid as 185 mg of a white powder.
Yield : 97%
¹H NMR (CDCl₃) : δ 7.63 (d, 2H) ; 7.15 (m, 3H) ; 7.05 (m, 1 H) ; 6.78 (m, 1 H) ; 6.72 (s, 1 H); 6.30 (t, 1 H) ; 4.49 (d, 2H) ; 4.21 (s, 3H) ; 2.47 (d, 2H) ; 2.24 (s, 3H) ; 1.86 (hept, 1 H); 1.62 (s, 6H) ; 0.89 (d, 6H).

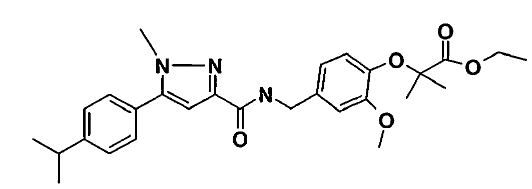

### Intermediate 36

### Ethyl 2-methyl-2-{[4-{[({1-methyl-5-[4-(1-methylethyl)phenyl]-1H-pyrazol-3-yl}carbonyl)amino]methyl}-2-(methyloxy)phenyl]oxy}propanoate.

**Intermediate 16** (105 mg, 0.43 mmol) was dissolved in 3 mL of DMF, HOBT (58 mg, 0.43 mmol), EDCI (82 mg, 0.43 mmol), Et₃N (120 µL, 0.86 mmol) and **intermediate 11** (118 mg, 0.39 mmol) were successively added. The mixture was stirred at rt for 24 hours and was diluted with AcOEt and washed with a saturated NaHCO₃ solution and brine, dried over Na₂SO₄, filtered and evaporated to dryness. The product was then purified by flash chromatography on silica gel (CH₂CL₂/AcOEt = 90/10) to afford 151 mg of the title compound.
Yield: 78%
¹H NMR (CDCI₃) : δ 7.63 (d, 2H) ; 7.14 (d, 2H) ; 7.11 (m, 1 H) ; 7.01 (dd, 1 H) ; 6.72 (s, 1H) ; 6.62 (d, 1 H) ; 6.31 (t, 1 H) ; 4.47 (d, 2H) ; 4.23 (q, 2H) ; 4.21 (s, 3H) ; 2.47 (d, 2H) ; 2.22 (s, 3H) ; 1.86 (hept, 1 H) ; 1.58 (s, 6H) ; 1.25 (t, 3H) ; 0.89 (d, 6H).

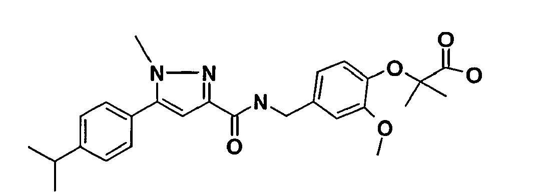

### Example 9

### 2-methyl-2-{[4-{[({1-methyl-5-[4-(1-methylethyl)phenyl]-1H-pyrazol-3-yl} carbonyl)amino]methyl}-2-(methyloxy) phenyl]oxy} propanoic acid.

To a solution of 145 mg (0.29 mmol) of **intermediate 36** in 1mL of THF and 5 mL of EtOH was added 2.9 mL (2.9mmol) of NaOH (1 N) and heated at 80°C for 2h.
After evaporation of the solvent, the residue was dissolved in water and the solution was acidified with HCl (1N). The white solid formed was collected by filtration (95mg).
Yield= 70%
¹H NMR (CDCl₃) : δ 7.25 (s, 4H); 7.15 (m,1H); 6.93-6.80 (m; 3H); 6.78 (s, 1H); 4.53 (d; 2H); 3.81 (s, 3H); 3.80 (s, 3H); 2.89 (spt, 1 H); 1.43 (s, 6H); 1.21 (d, 6H).

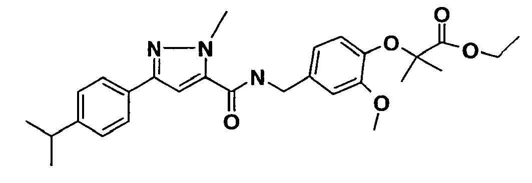

### Intermediate 37

### Ethyl 2-methyl-2-{[4-{[({1-methyl-3-[4-(1-methylethyl)phenyl]-1H-pyrazol-5-yl}carbonyl)amino]methyl}-2-(methyloxy) phenyl]oxy} propanoate.

To a solution of 134 mg (0.55mmol) of **intermediate 14** in 5 mL of DMF was added 74 mg (0.55mmol) of HOBT, 105 mg (0.55mmol) of EDCI, 155µL of Et₃N (1.1mmol) and then 152 mg (0.5mmol) of **intermediate 11**.
After 70h, the solvent was evaporated and the residue dissolved in ethylacetate. The organic layer was washed successively by K₂CO₃, HCl (1 N) and brine.

The solid obtained was purified by chromatography ( Dichloromethane/ Ethylacetate 90/10) to give 153mg of the desired product.
Yield= 62%
¹H NMR (CDCl₃) : δ 7.55 (d, 2H) ; 7.12 (d, 2H) ; 6.91 (m, 1 H) ; 6.80 (s, 1 H) ; 6.72-6.60 (m, 3H) ; 4.40 (d, 2H); 4.18-4.07( q, 2H; s, 3H) ; 3.62 (s, 3H); 2.80 (m, 1H); 1.45( s, 6H) ; 1.21-1.11 (t, 3H; d, 6H)

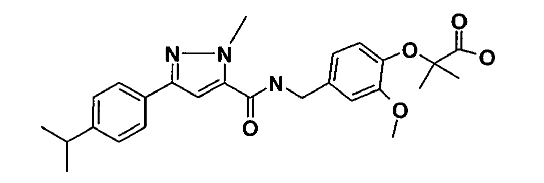

### Example 10

### 2-methyl-2-{[4-{[({1-methyl-3-[4-(1-methylethyl)phenyl]-1H-pyrazol-5-yl}carbonyl)amino]methyl}-2-(methyloxy)phenyl]oxy}propanoic acid

To a solution of 64 mg (0.130mmol) of **intermediate 37** in THF, was added 2 mL of EtOH and 1.3 mL of NaOH (1N). After 50 min at 80°C, the solvents were evaporated. The yellow solid obtained was dissolved in water, acidified with HCl (1 N) until formation of a white solid. The solid was filtered and purified by chromatography (Dichloromethane/ Methanol; from 95/5 to 80/20). The residue was then, dissolved in ethylacetate, washed with HCl (1 N) and brine; dried on Na₂SO₄ and evaporated to give a white powder (32mg).
Yield: 53%
¹H NMR (CDCl₃): δ 7.60 (d, 2H) ; 7.18 (d, 2H) ; 6.90 (m, 3H) ; 6.69 (s, 1 H) ; 6.31 (t, 1 H) ; 4.52 (d, 2H) ; 4.17 (s, 3H ) ; 3.84 (s, 3H) ; 2.85 (hept, 1 H) ; 1.45 (s, 6H) ; 1.18 (d, 6H)

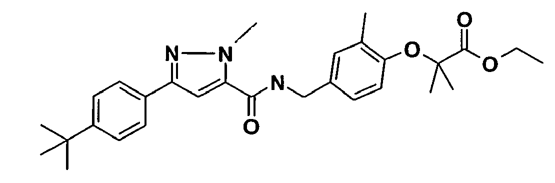

### Intermediate 38

### Ethyl 2-[(4-{[({3-[4-(1,1-dimethylethyl)phenyl]-1-methyl-1H-pyrazol-5-yl}carbonyl)amino]methyl}-2-methylphenyl)oxy]-2-methylpropanoate.

To a solution of 3g (11.6mmol) **intermediate 5** in 5mL of DMF, was added successively, 1.57g (11.6mmol) of HOBT, 2.22g (11.6mmol) of EDCI, 3.25 mL (23.2mmol) of Et₃N and then 3.12g (10.5mmol) of **intermediate 10.** After stirring the solution for 24h, the organic layer was extracted with ethylacetate, washed with brine, NaOH (0.5 N) and brine.
The solid obtained was purified by chromatography (Dichloromethane/Ethylacetate 95/5) to give a white solid (3.8g).
Yield= 73%
¹H NMR (CDCl₃): δ 7.81 (d, 2H) ; 7.55 (d,2H) ; 7.27 (s,1H); 7.16 (d, 1 H) ; 6.87 (s, 1 H) ; 6.78 (d, 1 H) ; 6.41 (m, 1 H) ; 4.64 (d, 2H) ; 4.40 (quad, 2H) ; 4.37 (s, 3H) ; 2.38 (s, 3H) ; 1.74 (s, 6H) ; 1.47 (s,9H) ; 1.41 (t, 3H).

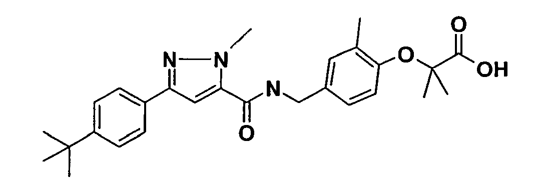

### Example 11

### 2-[(4-{[({3-[4-(1,1-dimethylethyl)phenyl]-1-methyl-1H-pyrazol-5-yl}carbonyl)amino]methyl}-2-methylphenyl)oxy]-2-methylpropanoic acid .

To a solution of 3.69g (7.5mmol) of **intermediate 38** in 50mL of THF and 50mL of EtOH was added 73 mL of NaOH (1N). The reaction mixture was heated at 80°C for 1h30 and concentrated under vacuum. 30mL of water was added and the solution was acidified by a dropwise addition of HCI (1N). The white solid was collected by filtration and dried at 50°C under vacuum (3.52g).
Yield= quantitative
¹H NMR (CDCl₃): d 7.58 (d, 2H); 7.31 (d, 2H); 7.08 (s,1 H); 6.98 (d, 1 H); 6.71 (d, 1 H); 6.94 (s, 1 H); 6.25 (m, 1 H); 4.42 (d, 2H); 4.14 (s, 3H); 2.17 (s, 3H); 1.55 (s, 6H); 1.25 (s, 9H)

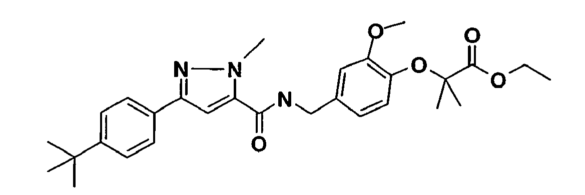

### Intermediate 39

### Ethyl 2-{[4-{[({3-[4-(1,1-dimethylethyl)phenyl]-1-methyl-1H-pyrazol-5-yl}carbonyl)amino]methyl}-2-(methyloxy)phenyl]oxy}-2-methylpropanoate.

To a solution of 220 mg (0.85mmol) **intermediate 5** in toluene was added 310 µL (4.25mmol) of SOCl₂.
The reaction mixture was heated at 100°C for 3h and evaporated to dryness. The residue was diluted in toluene and evaporated again.
The yellow oil obtained was diluted in CH₂Cl₂ anhydrous then added to a stirred solution of 284 mg (0.93mmol) of **intermediate 11** in CH₂Cl₂ with 370µL (2.55mmol) of Et₃N under nitrogen.
The reaction mixture was stirred at room temperature overnight, then washed twice with HCl (1N) and once with NaHCO₃. The organic layer was dried on Na₂SO₄ and filtered to give after evaporation to dryness the title compound as a pale oil (435mg).
Yield= quantitative
¹H NMR (CDCl₃): d 7.60 (d, 2H); 7.33 (d, 2H); 6.79-6.67 (m, 4H); 6.48 (m, 1H); 4.44 (d, 2H); 4.16 (q, 2H); 4.15(s, 3H) ; 3.70 (s, 3H); 1.48 (s, 6H) ; 1.25 ( s, 9H) ; 1.20 (t, 3H)

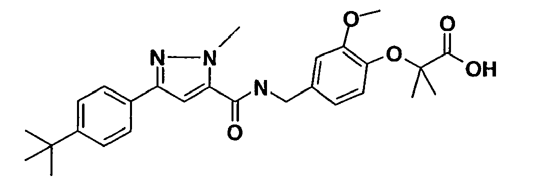

### Example 12

### 2-{[4-{[({3-[4-(1,1-dimethylethyl)phenyl]-1-methyl-1H-pyrazol-5-yl}carbonyl)amino]methyl}-2-(methyloxy)phenyl]oxy}-2-methyl propanoic acid.

To a solution of 435 mg (0.85mmol) of **intermediate 39** in EtOH was added 1.3 mL of NaOH(1N).

After 3h at 80°C, the reaction was not complete; so 1.3 mL of NaOH (1 N) was added, then the solution was heated for 1h at 85°C; the reaction was still not complete so 1.3 mL of NaOH (1 N) was added and the reaction was heated at the same temperature for 1 h.
The reaction mixture was evaporated, and HCl (1 N) was added. The white off precipitate was triturated and filtered (408mg).
Yield= quantitative
Mp=102-105°C; gummy
¹H NMR (DMSO): δ 9.02 (m, 1 H); 7.67 (d, 2H); 7.45 (d, 2H); 7.29 (s, 1 H), 6.99 (s, 1 H); 6.80 (s, 2H); 4.40 (d, 2H), 4.11 (s, 3H); 3.74 (s, 3H); 1.44 (s, 6H); 1.30 (s, 9H)

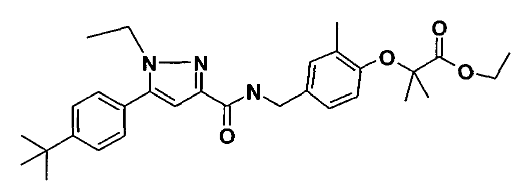

### Intermediate 40

### Ethyl 2-[(4-{[({5-[4-(1,1-dimethylethyl)phenyl]-1-ethyl-1H-pyrazol-3-yl}carbonyl)amino]methyl}-2-methylphenyl)oxy]-2-methylpropanoate

To a solution of 208 mg (0.765 mmol) of **intermediate 26** in DMF was added successively 103 mg (0.765mmol) of HOBT, 146 mg (0.765mmol)of EDCI, 214 µL (1.53mmol) of Et₃N and 200 mg (0.70mmol) of **intermediate 10**. After stirring for 5h, the mixture was concentrated, diluted in ethylacetate and washed twice with NaHCO₃, once with HCl (1 N) and brine. The organic layer was dried on Na₂SO₄, filtered and evaporated to dryness. The residue was purified by chromatography (Dichloromethane/Ethylacetate 99/1) to give the title compound as a slightly white oil (263mg).
Yield=74%
¹H NMR (CDCl₃) : δ 7.40 (d, 2H); 7.24 (d, 2H); 7.09 (m, 2H); 6.97 (d, 1H); 6.74 (s, 1H); 6.56 (d , 1 H) ; 4.46 (d, 2H) ; 4.17 (q, 2H) ; 4.07 (q, 2H) ; 2.15 (s, 3H) ; 1.51 (s, 6H) ; 1.34 (t, 3H) ; 1.29 (s, 9H) ; 1.19 (t, 3H)

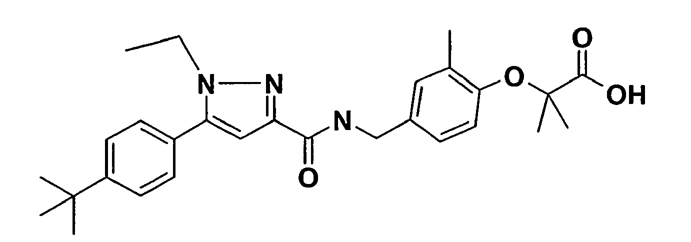

### Example 13

### 2-[(4-{[({5-[4-(1,1-dimethylethyl)phenyl]-1-ethyl-1H-pyrazol-3-yl}carbonyl)amino]methyl}-2-methylphenyl)oxy]-2-methylpropanoic acid

To a solution of 250 mg (0.49 mmol) of **intermediate 40** in THF was added EtOH then 4.9 mL of NaOH (1N). After stirring the mixture at 80°C for 1 h30, solvent was removed by vacuum to obtain a yellow solid which was added to water and acidified with HCl (1 N) to give a white solid which was filtered and dried (120mg).
Yield=51%
1H NMR (CDCl₃): 7.26 (d, 2H); 7.95 (d, 3H); 6.97 (s, 1H); 6.82 (d, 1H); 6.65-6.52 (m, 2H); 4.31 (d, 2H), 4.93 (q, 2H); 2.02 (s, 3H); 1.40 (s, 6H); 1.23-1.10 (t, 3H; s, 9H)

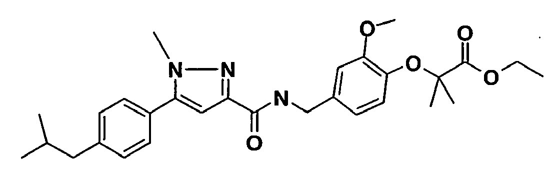

### Intermediate 41

### Ethyl 2-methyl-2-{[4-{[({1-methyl-5-[4-(2-methylpropyl)phenyl]-1H-pyrazol-3-yl}carbonyl)amino]methyl}-2-(methyloxy) phenyl] oxy}propanoate.

To a solution of 142 mg (0.55mmol) of **intermediate 32** in 1mL of DMF was added successively 75 mg (0.55mmol) of HOBT, 105 mg (0.55mmol) of EDCI, 155µL (1.1mmol) of Et₃N and 152 mg ( 0.5 mmol) of **intermediate 11.** After stirring for 24h, the organic layer was extracted with ethylacetate, washed with brine, dried on Na₂SO₄, filtered and evaporated to dryness. The residue was purified by chromatography (Dichloromethane/Ethylacetate 90/10) to give the title compound as a thick oil (135mg).
Yield=53%
¹H NMR (CDCI₃) : δ 7.25 (d, 2H) ; 7.15 (d, 2H) ; 7.1 ( m, 1 H) ; 6.8 (s, 1 H) ; 6.78 (s, 1 H); 6.75 (s, 2H) ; 4.45 (d, 2H) ; 4.15 (q, 2H) ; 3.8 (s, 3H) ; 3.7 (s, 3H) ; 2.55 (d, 2H) ; 1.8 (m, 1 H) ; 1.5, (s, 6H) ; 1.2 (t, 3H) ; 0.9 (d, 6H)

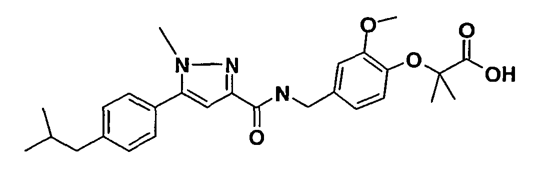

### Example 14

### 2-methyl-2-{[4-{[({1-methyl-5-[4-(2-methylpropyl)phenyl]-1H-pyrazol-3-yl} carbonyl)amino]methyl}-2-(methyloxy) phenyl] oxy}propanoic acid.

To a solution of 135 mg (0.266 mmol) of **intermediate 41** in 2 mL of THF, was added 5 mL of EtOH and 2.6 mL of NaOH (1 N). After stirring at 80°C for 24h the mixture was concentrated, acidified with HCl (1 N) to give after filtration a white solid which was washed with water and dried under vacuum (119mg).
Yield=93%
¹H NMR (CDCl₃): δ 7.23 (d, 2H); 7.15 (d, 2H); 6.92-6.80 (m, 3H); 6.78 (s, 1H); 4.52 (d, 2H); 3.81 (s, 3H); 3.80 (s, 3H); 2.45 (d, 2H); 1.83 (m, 1 H); 1.43 (s, 6H); 0.86 (d, 6H).

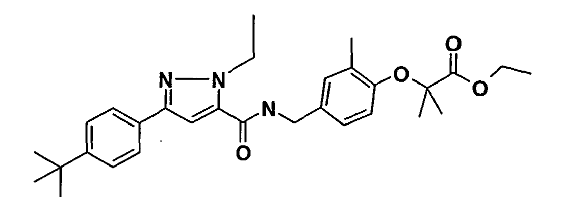

### Intermediate 42

### Ethyl 2-[(4-{[({3-[4-(1,1-dimethylethyl)phenyl]-1-ethyl-1H-pyrazol-5-yl}carbonyl)amino]methyl}-2-methylphenyl)oxy]-2-methyl propanoate.

To a solution of 208 mg (0.765 mmol) of **intermediate 25** in DMF was added successively 103 mg (0.765mmol) of HOBT, 146 mg (0.765mmol) of EDCI, 214µL (1.53mmol) of Et₃N and 200mg (0.70mmol) of **intermediate 10**. After stirring for 5h, the mixture was concentrated, dissolved in ethylacetate, washed twice with NaHCO₃, once with HCI (1 N) and brine, dried on Na₂SO₄, filtered and concentrated to dryness. The residue was purified by chromatography (Dichloromethane/Ethylacetate 99/1) to give the title compound as white crystals (291 mg).
Yield= 82%
¹H NMR (CDCl₃) : δ 7.6 (d, 2H) ; 7.35 (d, 2H) ; 7.08 (s, 1 H) ; 6.98 (d, 1 H) ; 6.62 (s, 1H) ; 6.56 (d, 1 H) ; 6.2 (m, 1H) ; 4.6 (q, 2H) ; 4.43 (d, 2H) ; 4.2 (q, 2H) ; 2.19 (s, 3H) ; 1.5 (s, 6H) ; 1.41 (t, 3H) ; 1.26 (s, 9H) ; 1.2 (t, 3H)

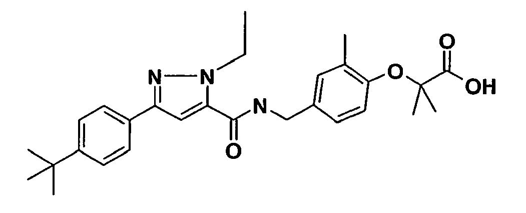

### Example 15

### 2-[(4-{[({3-[4-(1,1-dimethylethyl)phenyl]-1-ethyl-1H-pyrazol-5-yl}carbonyl)amino]methyl}-2-methylphenyl)oxy]-2-methyl propanoic acid.

To a solution of 279 mg (0.55 mmol) of **intermediate 42** in THF was added 5 mL of absolute ethanol and 5.5 mL of NaOH (1 N). After stirring at 80°C for 1 h20, the mixture was concentrated to give a yellow solid which was dissolved in water and acidified with HCl (1 N) to give a pale yellow solid after filtration (210mg).
Yield=80%
¹H NMR (CDCl₃): δ 7.47 (d, 2H); 7.20 (d, 2H); 6.96 (s, 1 H); 6.87 (d, 1 H); 6.61 (d, 1 H); 6.50 (s, 1 H); 6.11 (m, 1 H); 4.44 (q, 2H); 4.30 (d, 2H); 2.05 (s, 3H); 1.42 (s, 6H); 1.29 (t, 3H); 1.12 (s,9H)

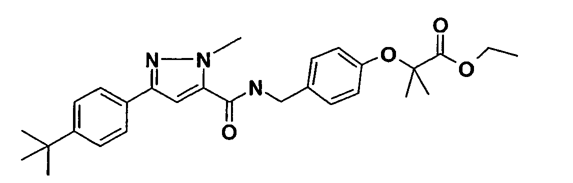

### Intermediate 43

### Ethyl 2-[(4-{[({3-[4-(1,1-dimethylethyl)phenyl]-1-methyl-1H-pyrazol-5-yl}arbonyl)amino]methyl}phenyl)oxy]-2-methyl propanoate.

To a solution of 6.52g (0.025 mol) of **intermediate 5** in 165 mL of CH₂CI₂ and 165 mL of DMF was added 3.41 g (1.2eq) of HOBT, 4.84 g (1.2eq) of EDCI, 3.6 mL (1.2eq) of Et₃N and was stirred for 15 minutes. A solution of 5 g (0.021 mol) of **intermediate 45** dissolved in 10 mL of CH₂Cl₂ was added dropwise to the mixture and stirred at room temperature for 3 days.
Then water was added ; the organic layer was extracted twice with CH₂Cl₂, and washed with Na₂CO₃, water, HCl (1 N), water and brine. After evaporation to dryness, the residue was purified by chromatography (CH₂Cl₂/MeOH 98/2) to give the title compound as a yellow oil (5.2g).
Yield=52%
¹H NMR (CDCl₃) : δ 7.61 (d, 2H) ; 7.34 (d, 2H) ; 7.16 (d, 2H) ; 6.77 (d, 2H) ; 6.66 (s, 1 H) ; 6.18 (m, 1 H) ; 4.47 (d, 2H) ; 4.16 [(s, 3H) + (q, 2H)] ; 1.53 (s, 6H) ; 1.26 (s, 9H) ; 1.19 (t, 3H)

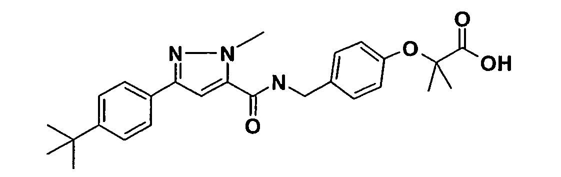

### Example 16

### 2-[(4-{[({3-[4-(1,1-dimethylethyl)phenyl]-1-methyl-1H-pyrazol-5-yl}carbonyl)amino]methyl}phenyl)oxy]-2-methylpropanoic acid.

To a solution of 5.2 g (11 mmol) of **intermediate 43** in 100mL of EtOH was added 21.8 mL of NaOH (1N). After stirring at 70°C for 3h, the mixture was concentrated, dissolved in water and washed with ethylacetate. The aqueous layer was acidified with a 1 N HCl solution until pH=5 and extracted with 150 mL of CH₂Cl₂. The white precipitate formed in the organic layer was filtered, washed with water and pentane. The organic layer was concentrated to dryness, triturated in water with a little of Et₂O, filtered, and washed with water and pentane to give a white solid.
Both white solids were combined to give a single batch of the title compound (4.19g).
Yield=85%
Mp=154.6°C
¹H NMR (DMSO): δ 9.02 (m, 1H); 7.68 (d, 2H); 7.45 (d,2H); 7.28 (s,1H); 7.24 (d, 2H); 6.81 (d, 2H); 4.39 (d, 2H); 4.11 (s, 3H); 1.50 (s, 6H); 1.30 (s, 9H)

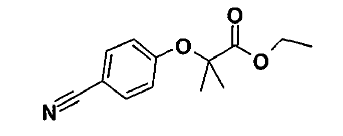

### Intermediate 44

### Ethyl 2-[(4-cyanophenyl)oxy]-2-methylpropanoate.

To a solution of 25.05g (0.210 mol) of **4-cyanophenol** in 250 mL of acetonitrile was added 56.66g (0.42mol) of K₂CO₃, then the mixture was refluxed for 1 h. At this temperature 61.74 mL (0.42mol) of **ethyl-2-bromo-isobutyrate** was added dropwise. After two days at reflux, 0.5eq of **ethyl-2-bromo-isobutyrate** was added dropwise, followed by 0.5 eq of K₂CO₃. After 24h at reflux, the mixture was filtered, then evaporated to dryness. 100 mL of NaOH (1 N) was added to the residue, and the organic layer was extracted with 150 mL of Et₂O and twice with 75 mL of Et₂O, then washed with 100 mL and 50 mL of NaOH (1N) and 100 mL of brine. The organic layer was then dried on MgSO₄, filtered and evaporated to dryness. The oil obtained was diluted in 200 mL of iPr₂O and filtered to give the title compound as a yellow oil (63.1g).
Yield= quantitative.
¹H NMR (CDCl₃): δ 7.47 (d, 2H); 6.78 (d, 2H); 4.15 (q, 2H); 1.58 (s, 6H); 1.14 (t, 3H)

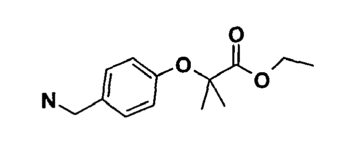

### Intermediate 45

### Ethyl 2-{[4-(aminomethyl)phenyl]oxy}-2-methylpropanoate.

To a solution of 63.1g (0.27 mol) of **intermediate 44** in 80 mL of AcOH and 160 mL of EtOH under nitrogen, was added 4.73 g (7.5% w/w) Pd/c 10% under 3 Bars.
After 6h under hydrogen, the reaction was not complete; the mixture was filtered through a Whatmann filter, evaporated to dryness and dissolved in the same volume of solvent and same quantity of Pd/c but under 3 Bars at 50°C. After 6h under hydrogen, the reaction was complete.
The mixture was filtered through a Whatmann filter and evaporated to dryness, diluted in 400 mL H₂O and 400 mL AcOEt, stirred for 5 minutes, extracted with 260 mL H₂O, acidified with a concentrated HCl solution, washed with 260 mL of AcOEt.
At 10°C, NaOH (37%) was added to the aqueous solution until pH=8. 400mL of CH₂Cl₂ were added to the solution which was stirred for 5 minutes, then extracted twice with 200mL of CH₂Cl₂, dried on MgSO₄, filtered and evaporated to dryness to give the title compound as a yellow oil (9.8g).
Yield= 15%
¹H NMR (CDCl₃): δ 7.18 (d, 2H); 6.82 (d, 2H); 4.24 (q, 2H); 2.08 (s, 1 H); 1.93 (s, 1 H); 1.59( s, 6H); 1.26 (t, 3H)

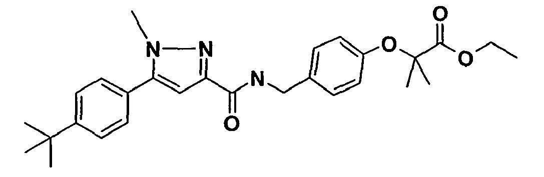

### Intermediate 46

### Ethyl 2-[(4-{[({5-[4-(1,1-dimethylethyl)phenyl]-1-methyl-1H-pyrazol-3-yl}carbonyl)amino]methyl}phenyl)oxy]-2-methyl propanoate.

To a solution of 5.11g (20mmol) of intermediate 3 in 165 mL of CH₂Cl₂ and 165 mL of DMF was added 2.676 g (20mmol) of HOBT, 3.79 g (20mmol) of EDCI and 2.8 mL (20mmol) of Et₃N. The mixture was stirred at room temperature for 15 minutes, then 3.92 g (16.5mmol) of intermediate 45 diluted in 8 mL of CH₂Cl₂ was added dropwise.
After 3 days at 22°C, 170 mL of water was added to the reaction mixture. The organic layer was extracted twice with 90 mL of CH₂Cl₂, then washed successively with 170 mL of Na₂CO₃, 170 mL of water, 170 mL of HCl (1 N), 170 mL of water, 170 mL of brine, then dried on MgSO₄, filtered and evaporated to dryness.
The mixture was purified by chromatography (CH₂Cl₂/MeOH 99/1 to 98/2), to give the title compound (1.4g).
Yield=18%
¹H NMR (CDCl₃): δ 7.51 (d, 2H); 7.37 (d, 2H); 7.29 (d, 2H); 6.84 (d, 2H); 4.59 (d, 2H); 4.25 (q, 2H); 3.89 (s, 3H); 1.61 (s, 6H); 1.38 (s, 9H); 1.28 (t, 3H)

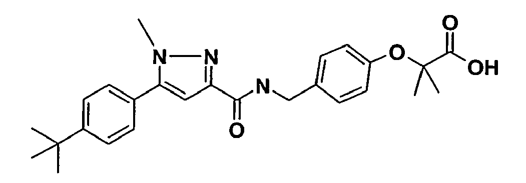

### Example 17

### 2-[(4-{[({5-[4-(1,1-dimethylethyl)phenyl]-1-methyl-1H-pyrazol-3-yl}carbonyl)amino]methyl}phenyl)oxy]-2-methylpropanoic acid.

To a solution of 3.2 g (6.7 mmol) of **intermediate 46** in 64 mL of EtOH was added 13.4 mL of NaOH (1N).
After 1h10 at 70°C, the mixture was allowed to go to room temperature, then 50 mL of water was added and the organic layer extracted twice with 25 mL of AcOEt. The aqueous layer was acidified with HCI (1 N) and the organic layer extracted with 30 mL of CH₂Cl₂, dried on MgSO₄, filtered and evaporated to dryness to give the title compound as a white solid (2.64g).
Yield=88%
Mp= 70°C (gummy)
¹H NMR (CDCl₃): δ 7.41 (m, 2H); 7.27 (d, 3H); 7.19 (s, 1H); 6.82 (m, 3H); 4.50 (m , 2H); 3.80 (s, 3H); 1.52 (s, 6H); 1.29 (s, 9H)

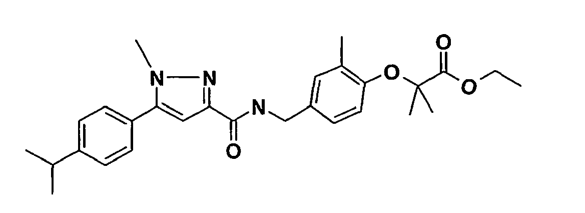

### Intermediate 47

### ethyl-2-methyl-2-[(2-methyl-4-{[({1-methyl-5-[4-(1-methylethyl)phenyl]-1H-pyrazol-3-yl}carbonyl)amino]methyl}phenyl)oxy] propanoate

To a solution of 105 mg (0.43 mmol) of **intermediate 16** in 3 mL of DMF was added successively 58 mg (0.43 mmol) of HOBT, 82 mg (0.43 mmol) of EDCI, 120 µL (0.86 mmol) of Et₃N and 113 mg (0.39 mmol) of **intermediate 10.** The reaction mixture was stirred for 24h at room temperature, extracted with ethylacetate, washed with brine, dried on Na₂SO₄ and evaporated to dryness. The residue was purified by chromatography (Dichloromethane/ethylacetate 95/5) to give the desired product (145mg).
Yield=78%
¹H NMR (CDCl₃) : δ 7.25 (s, 4H) ; 7.07 (s, 1 H); 7.04 (m, 1 H); 6.96 (dd, 1 H) ; 6.77 (s, 1H); 6.55 (d, 1 H) ; 4.45 (d, 2H) ; 4.17 (quad, 2H), 3.78 (s, 3H) ; 2.89 (m, 1 H) ; 2.14 ( s, 3H) ; 1.51 (s, 6H) ; 1.21 (d, 6H) ; 1.19 (t, 3H)

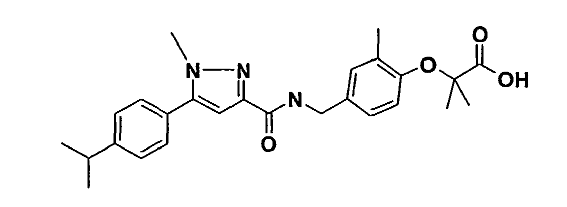

### Example 18

### 2-methyl-2-[(2-methyl-4-{[({1-methyl-5-[4-(1-methylethyl)phenyl]-1H-pyrazol-3-yl}carbonyl)amino]methyl}phenyl)oxy]propanoic acid

To a solution of 140 mg (0.29 mmol) of **intermediate 47** in 1mL of THF and 5 mL of EtOH was added 2.9 mL of NaOH (1N). The reaction mixture was heated at 80°C for 2h ; then evaporated to dryness, dissolved in water, acidified with HCl (1 N) ; a white precipitate appeared which was filtered (115mg).
Yield= 88%
¹H NMR (CDCl₃): δ 7.15 (m, 1 H); 7.12 (s, 4H) ; 6.96 (s, 1H); 6.82 (dd, 1 H) ; 6.65 (s, 1 H) ; 6.57 (d, 1 H) ; 4.31 (d, 2H) ; 3.65 (s, 3H) ; 2.76 (m, 1 H) ; 2.02 (s, 3H) ; 1.41 (s, 6H) ; 1.08 (d, 6H)

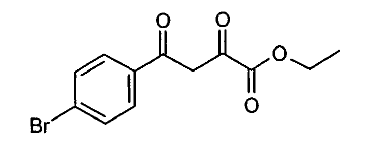

### Intermediate 48

### ethyl 4-(4-bromophenyl)-2,4-dioxobutanoate

To a solution of 400mL of anhydrous ethanol under nitrogen at 0°C was added carefully sodium (2.53g, 0.11 mol); when it was completely dissolved, 4-bromoacetophenone (19.9g, 0.1 mol) in 50 mL of ethanol was added dropwise. The reaction mixture was stirred at 10°C for 1 h, then ethyloxalate (16.06g, 0.11 mol) in 50mL of ethanol was added dropwise, then stirred for 1h30, when a precipitate appeared. The resulting mixture was concentrated, dissolved in ethylacetate, washed twice with HCI (1 N), brine, dried on Na₂SO₄, concentrated to dryness, to give an orange oil which crystallized at low temperature (31.4g).
Yield = quantitative.
¹H NMR (CDCl₃) δ: 7.67 (d, 2H); 7.46 (d, 2H); 6.84 (s, 1 H); 4.21 (quad, 2H); 1.22 (t, 3H)

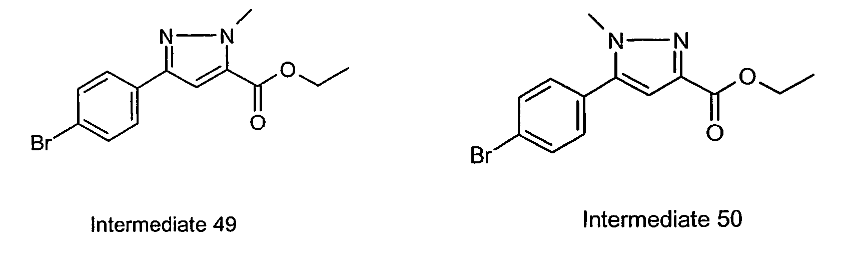

### Intermediate 49

### ethyl 3-(4-bromophenyl)-1-methyl-1H-pyrazole-5-carboxylate

### Intermediate 50

### ethyl 5-(4-bromophenyl)-1-methyl-1H-pyrazole-3-carboxylate

To a hot (70°C) solution of **intermediate 48** (31.4 g, 0.105 mol) in 500 mL of ethanol was added two volumes of methylhydrazine (5.85 mL, 0.11mol), then it was stirred at 90°C for 2h. The resulting mixture was concentrated to dryness, dissolved in ethylacetate, washed with HCl (1N), brine, dried on Na₂SO₄ and evaporated to dryness, then purified by flash chromatography (Cyclohexane/ethylacetate 90/10 to 70/30) to obtain two products, intermediate 49 first eluted and intermediate 50 :
*The first was triturated in heptane to give crystals : 12.9g of - **Intermediate 49**.
   Yield : 40%
   ¹H NMR (CDCl₃) δ: 7.49 (d, 2H); 7.34 (d, 2H); 6.91 (s, 1H); 4.19 (quad, 2H); 4.03 (s, 3H); 1.22 (t, 3H)
*The second (12.1g): - **Intermediate 50.**
   Yield : 37%
   ¹H NMR (CDCl₃) δ: 7.55 (d, 2H); 7.22 (d, 2H); 6.78 (s, 1H); 4.36 (quad, 2H); 3.87 (s, 3H); 1.34 (t, 3H)

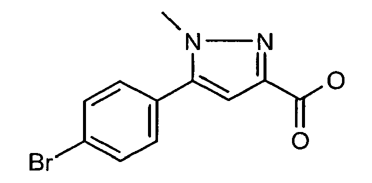

### Intermediate 51

### 5-(4-bromophenyl)-1-methyl-1H-pyrazole-3-carboxylic acid

To a solution of **intermediate 50** (5g, 16 mmol) in 10 mL of THF was added 50 mL of ethanol and 80 mL of NaOH (1N), then the reaction mixture was stirred for 1h at room temperature. The resulting mixture was evaporated to dryness, treated with 150 mL of water, acidified by a dropwise addition of 100 mL of HCl (1N). The precipitate obtained was filtered off and dried under vacuum to give a white solid (4.46g).
Yield=99%
¹H NMR (CDCl₃) δ: 7.56 (d, 2H); 7.23 (d, 2H); 6.83 (s, 1H); 3.89 (s, 3H)

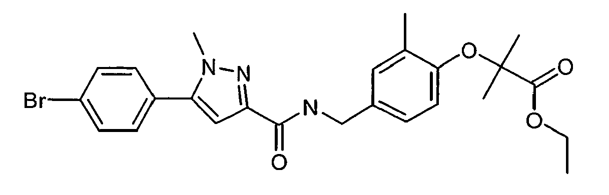

### Intermediate 52

### ethyl 2-({4-[({[5-(4-bromophenyl)-1-methyl-1H-pyrazol-3-yl]carbonyl}amino)methyl]-2-methylphenyl}oxy)-2-methylpropanoate

To a suspension of **intermediate 51** (4.4 g, 15.7 mmol) in 100 mL of toluene, was added SOCl₂ (5.7 mL, 78.5mmol) and the resulting mixture was heated at 110°C for 3h. Then the reaction mixture was evaporated twice with toluene to eliminate the excess of SOCl₂ to give the intermediate **B**.
To a solution of **intermediate 153** (4.51 g, 15.7mmol) and Et₃N (6.6 mL, 47mmol) in 100 mL of CH₂Cl₂ anhydrous at 0°C, was added the intermediate **B** diluted in 50 mL of CH₂Cl₂. The resulting mixture was stirred for 2h, then it was washed with NaHCO₃ sat, HCl (1 N) and brine, then dried on Na₂SO₄ and evaporated to dryness.
The solid obtained was triturated in heptane to give a white solid as the title compound (3.6g).
Yield = 89%
¹H NMR (CDCl₃) δ: 7.54 (d, 2H); 7.21 (d, 2H); 7.07 (s, 1 H); 7 (m, NH); 6.96 (dd, 1 H); 6.79 (s, 1 H); 6.55 (d, 1 H); 4.45 (d, 2H); 4.18 (quad, 2H); 3.78 (s, 3H); 2.15 (s, 3H); 1.51 (s, 6H); 1.19 (t, 3H)

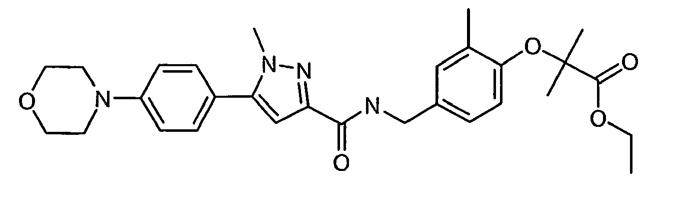

### Intermediate 53

### ethyl 2-methyl-2-[(2-methyl-4-{[({1-methyl-5-[4-(4-morpholinyl)phenyl]-1H-pyrazol-3-yl}carbonyl)amino]methyl}phenyl)oxy]propanoate

In a tube was added successively BINAP (47 mg, 0.075mmol), Pd₂(dba)₃ (23 mg, 0.025mmol),Cs₂CO₃ (228 mg, 0.7mmol), **intermediate 52** (257 mg, 0.5 mmol), 2 mL of DME and **morpholine** (65 mg, 0.75mmol). The reaction mixture was stirred under nitrogen at 100°C for 16h, then filtered through celite with CH₂Cl₂, evaporated to dryness and purified by flash chromatography (CH₂Cl₂/Ethylacetate 90/10 to 80/20) to give an amorphous solid (220mg).
Yield = 84%
¹H NMR (CDCl₃) δ: 7.25 (d, 2H); 7.07 (s, 1 H); 7.04 (m, NH); 6.96 (d, 1 H); 6.90 (d, 2H); 6.74 (s, 1H); 6.55 (d, 1H); 4.45 (d, 2H); 4.18 (quad, 2H); 3.81 (m, 4H); 3.78 (s, 3H); 3.16 (m, 4H); 2.15 (s, 3H); 1.51 (s, 6H); 1.19 (t, 3H)

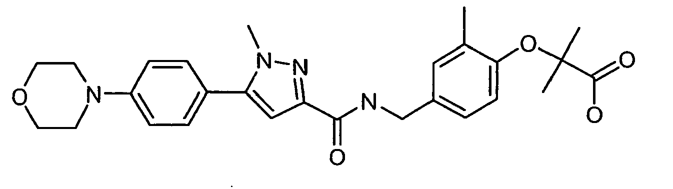

### Example 19

### 2-methyl-2-[(2-methyl-4-{[({1-methyl-5-[4-(4-morpholinyl)phenyl-1H-pyrazol-3-yl}carbonyl)amino]methyl}phenyl)oxy]propanoic acid

To a solution of **intermediate 53** (200 mg, 0.38 mmol) in 5 mL of THF and 20 mL of EtOH was added 20 mL of NaOH (1 N) and the reaction mixture was stirred at 90°C for 2h. The resulting mixture was evaporated to dryness, treated with water (10 mL) and 7.6 mL of HCl (1 N) to give after filtration a cream solid which was dried under vacuum (145mg).
Yield=77%
¹H NMR (CDCl₃) δ: 7.28 (m, NH); 7.23 (d, 2H); 7.08 (s, 1 H); 6.92 (m, 3H); 6.74 (s, 1 H); 6.68 (d, 1H); 4.42 (d, 2H); 3.81 (m, 4H); 3.76 (s, 3H); 3.16 (m, 4H); 2.14 (s, 3H); 1.53 (s, 6H)

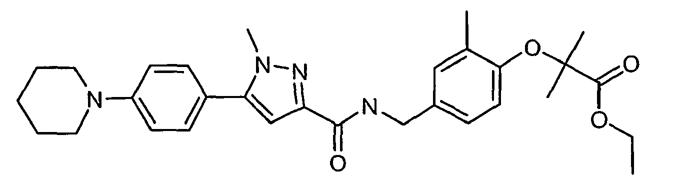

### Intermediate 54

### Ethyl 2-methyl-2-[(2-methyl-4-{[({1-methyl-5-[4-(1-piperidinyl)phenyl]-1H-pyrazol-3-yl}carbonyl)amino]methyl}phenyl)oxy]propanoate

In a tube was added successively BINAP (47 mg, 0.075mmol), Pd₂(dba)₃ (23 mg, 0.025mmol),Cs₂CO₃ (228 mg, 0.7mmol), **intermediate 52** (257 mg, 0.5 mmol), 2 mL of DME and piperidine (1.5- 3 eq). The reaction mixture was stirred under nitrogen at 100°C for 16h, then filtered through celite with CH₂Cl₂, evaporated to dryness and purified by flash chromatography (CH₂Cl₂/Ethylacetate 95/5) to give the product (195mg).
Yield = 75%
¹H NMR (CDCl₃) δ: 7.19 (s, 4H); 7.07 (s, 1 H); 7.03 (m, 1 H); 6.96 (dd, 1 H); 6.73 (s, 1 H); 6.55 (d, 1 H); 4.45 (d, 2H); 4.18 (quad, 2H); 3.78 (s, 3H); 3.18 (m, 4H); 2.15 (s, 3H); 1.51 (s, 12H); 1.20 (t, 3H)

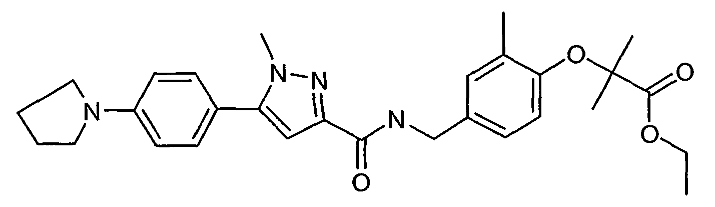

### Intermediate 55

### Ethyl 2-methyl-2-[(2-methyl-4-{[({1-methyl-5-[4-(1-pyrrolidinyl)phenyl]-1H-pyrazol-3-yl}carbonyl)amino]methyl}phenyl)oxy]propanoate

In a tube was added successively BINAP (47 mg, 0.075mmol), Pd₂(dba)₃ (23 mg, 0.025mmol),Cs₂CO₃ (228 mg, 0.7mmol), **intermediate 52** (257 mg, 0.5 mmol), 2 mL of DME and pyrrolidine (1.5- 3 eq). The reaction mixture was stirred under nitrogen at 100°C for 16h, then filtered through celite with CH₂Cl₂, evaporated to dryness and purified by flash chromatography (CH₂Cl₂/Ethylacetate 95/5) to give the product (163mg).
Yield = 65%
¹H NMR (CDCl₃) δ: 7.19 (d, 2H); 7.07 (s, 1 H); 7.03 (m, NH); 6.97 (dd, 1 H); 6.70 (s, 1 H); 6.55 (d, 3H); 4.45 (d, 2H); 4.17 (quad, 2H); 3.77 (s, 3H); 3.26 (m, 4H); 2.15 (s, 3H); 1.96 (m, 4H); 1.51 (s, 6H); 1.19 (t, 3H)

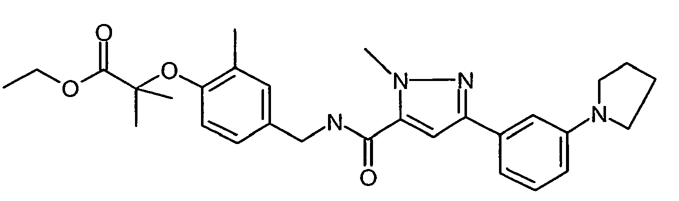

### Intermediate 56

### Ethyl 2-methyl-2-{2-methyl-4-[({[1-methyl-3-(3-pyrrolidin-1-ylphenyl)-1H-pyrazol-5-yl]carbonyl}amino)methyl]phenoxy}propanoate

In a tube was added successively BINAP (47 mg, 0.075mmol), Pd₂(dba)₃ (23 mg, 0.025mmol),Cs₂CO₃ (228 mg, 0.7mmol), **intermediate 62** (257 mg, 0.5 mmol), 2 mL of DME and piperidine (1.5- 3 eq). The reaction mixture was stirred under nitrogen at 100°C for 16h, then filtered through celite with CH₂Cl₂, evaporated to dryness and purified by flash chromatography (CH₂Cl₂/Ethylacetate 93/7) to give the product (150mg).
Yield = 59%
¹H NMR (CDCl₃) d: 7.22-7.13 (m, 3H); 7.06 (s, 1 H); 6.95 (d, 2H); 6.67 (s, 1 H); 6.57 (d, 1 H); 6.16 (m, 1H); 4.42 (d, 2H); 4.18 (t, 2H); 4.17 (s, 3H); 3.28 (m, 4H); 2.17 (s, 3H); 1.96 (m, 4H); 1.53 (s, 6H); 1.19 (t, 3H).

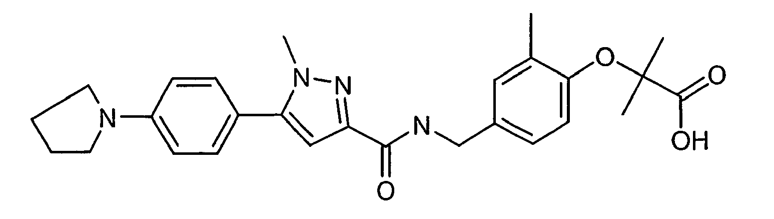

### Example 20

### 2-methyl-2-[(2-methyl-4-{[({1-methyl-5-[4-(1-pyrrolidinyl)phenyl]-1H-pyrazol-3-yl}carbonyl)amino]methyl}phenyl)oxy]propanoic acid

To a solution of intermediate 55 (164 mg, 0.32 mmol) in 10 mL of methanol, was added 1.6 mL of NaOH (1 N) and was stirred at 90°C for 2h30. Then 1.6 mL of HCI (1 N) was added, and the organic layer extracted with ethylacetate, washed with water, dried on Na₂SO₄ and purified by flash chromatography (CH₂Cl₂/MeOH 95/5) to give an off-white powder (100mg).
Yield=65%
¹H NMR (CDCI₃) δ: 7.29 (m, 1H); 7.17 (d, 2H); 7.10 (s, 1H); 6.95 (dd, 1H); 6.69 (s, 1H; d, 1 H); 6.54 (d, 2H); 4.43 (d, 2H); 3.76 (s, 3H); 3.25 (m, 4H); 2.14 (s, 3H); 1.96 (m, 4H); 1.53 (s, 6H)

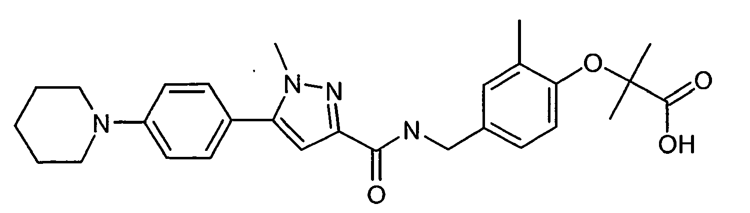

### Example 21

### 2-methyl-2-[(2-methyl-4-{[({1-methyl-5-[4-(1-piperidinyl)phenyl]-1H-pyrazol-3-yl}carbonyl)amino]methyl}phenyl)oxy]propanoic acid

To a solution of intermediate 54 (1.52g, 2.93 mmol) in 15 mL of EtOH was added 5.86 mL of NaOH (1N), and was stirred at 80°C for 2h. The resulting mixture was evaporated and treated with 15 mL of water, 7.5 mL of HCI (1N). The organic layer was extracted with ethylacetate and washed with water, dried with MgSO₄, evaporated to give the title compound as a cream solid (1.25g).
Yield = 87%
¹H NMR (DMSO) δ : 8.53 (m, 1H); 7.35 (d, 2H); 7.10 (s, 1H); 7.02 (d, 3H); 6.65 (m, 2H); 4.31 (d, 2H) ; 3.87 (s, 3H) ; 3.33 (m, 4H) ; 2.13 (s, 3H) ; 1.59 (m, 6H) ;1.49 (s, 6H)

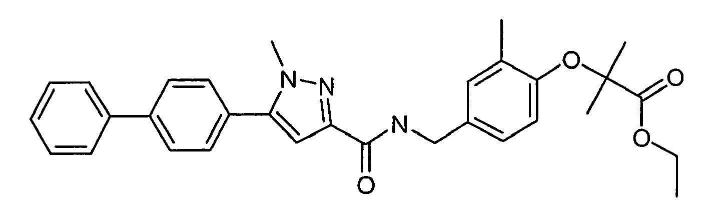

### Intermediate 57

### Ethyl2-({4-[({[-(4-biphenylyl)-1-methyl-1H-pyrazol-3-yl]carbonyl}amino) methyl]-2-methylphenyl}oxy)-2-methylpropanoate

To a solution of **intermediate 52** (257 mg, 0.5 mmol) in 6 mL of toluene was added Pd(PPh₃)₄ (29 mg, 2.5mmol), and was stirred for 10 min. Then, Na₂CO₃ 2M (0.65 mL, 1.3mmol) and phenylboronic acid (67 mg, 0.55mmol) in 2-3 mL of EtOH was added, then the reaction mixture was stirred at reflux overnight.
3 mL of water was added to the reaction mixture, it was filtered through celite, washed with ethylacetate and water and evaporated to dryness. The residue was treated with ethylacetate and washed with NaHCO₃, water, evaporated to dryness and purified by flash chromatography (CH₂Cl₂/ethylacetate 95/5) to give the desired product (186mg).
Yield=73%
¹H NMR (CDCl₃) δ: 7.63 (d, 2H); 7.56 (d, 2H); 7.41 (m, 4H); 7.32 (m, NH); 7.09 (m, 2H); 6.97 (m, 1H); 6.85 (s, 1H); 6.56 (d, 1H); 4.47 (d, 2H); 4.18 (quad, 2H); 3.85 (s, 3H); 2.16 (s, 3H); 1.52 (s, 6H); 1.20 (t, 3H)

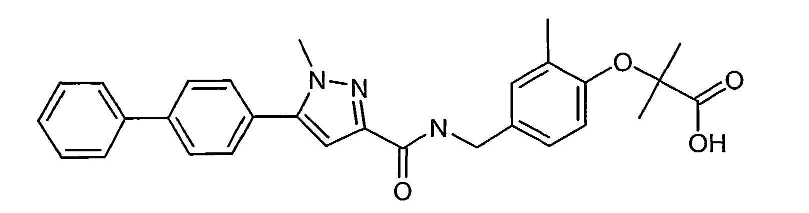

### Example 22

### 2-({4-[({[5-(4-biphenylyl)-1-methyl-1H-pyrazol-3-yl]carbonyl}amino)methyl]-2-methylphenyl}oxyl)-2-methylpropanoic acid

To a solution of **intermediate 57** (186 mg, 0.36 mmol) in 4 mL of ethanol was added 4 mL of NaOH (2N) and was stirred at room temperature for 3-4h.
The resulting mixture was evaporated, treated with water and HCl (5N). The organic layer was extracted with ethylacetate, washed with water and evaporated to dryness.
The residue was diluted in NaOH (1N), then acidified with HCl (1 N) to give a precipitate which was filtered off and washed with water to give a white solid as the title compound (125mg).
Yield = 71%
¹H NMR (CDCI₃) δ: 7.62 (d, 2H); 7.55 (d, 2H); 7.40 (m, 4H); 7.32 (m, 2H); 7.09 (s, 1H); 6.96 (d, 1 H); 6.86 (s, 1 H); 6.70 (d, 1 H); 4.44 (d, 2H); 3.83 (s, 3H); 2.16 (s, 3H); 1.55 (s, 6H)

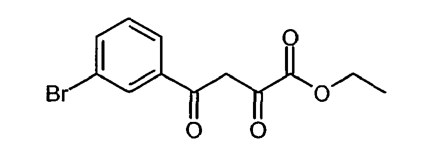

### Intermediate 58

### Ethyl 4-(3-bromophenyl)-2,4-dioxobutanoate

To 200mL of ethanol anhydrous under nitrogen at 0°C was added carefully sodium (2.53g, 0.11 mol) ; when it was completely dissolved, **3-bromoacetophenone** (19.9g, 0.1 mol) in 50 mL of ethanol was added dropwise at 10°C. The reaction mixture was stirred at 10°C for 2h, then **ethyloxalate** (16.06g, 0.11 mol) in 50 mL of ethanol was added dropwise at rt, then stirred for 1 h30 when a yellow precipitate appeared. To the resulting mixture was added 500 mL of ethylacetate. It was then washed with HCl (1 N), brine, dried on Na₂SO₄, concentrated to dryness, to give a yellow oil (30g).
Yield = quantitative.
¹H NMR (CDCl₃) d: 7.98 (s, 1H); 7.77 (d, 1H); 7.59 (d, 1H); 7.25 (t, 1H); 6.88 (s, 1H); 4.27 (quad, 2H); 1.28 (t, 3H)

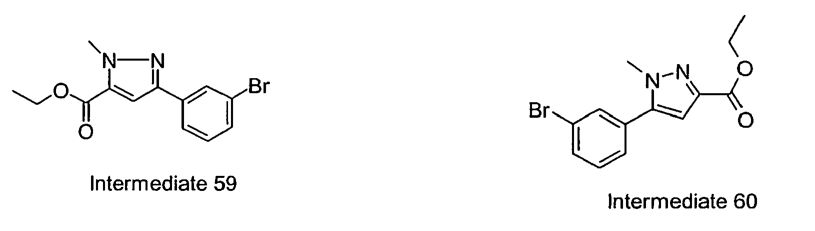

### Intermediate 59

### Ethyl 3-(3-bromophenyl)-1-methyl-1H-pyrazole-5-carboxylate

### Intermediate 60

### Ethyl 5-(3-bromophenyl)-1-methyl-1H-pyrazole-3-carboxylate

To a solution of **intermediate 58** (30 g, 0.1 mol) in 500 mL of ethanol at 60°C was added 5.85 mL (0.11 mol) of methylhydrazine. The reaction mixture was stirred at 90°C for 2h30, then evaporated to dryness, treated with ethylacetate, washed with HCI (1 N), brine, dried on Na₂SO₄ and purified by flash chromatography (AcOEt/Cyclohexane : 2/8) to obtain intermediate 59 (first eluted) and intermediate 60.
To give:
Intermediate 59 : m= 6.5g of (white crystals recristallyzed in heptane)
Yield=21 %
¹H NMR (CDCl₃) δ: 7.98 (s, 1 H); 7.74 (d, 1 H); 7.46 (d, 1 H); 7.28 (t, 1 H); 7.13 (s, 1 H); 4.39 (quad, 2H); 4.24 (s, 3H); 1.43 (t, 3H)
intermediate 60: m=11g
Yield=35%
¹H NMR (CDCl₃) δ: 7.45 (m, 2H); 7.23 (m, 2H); 6.73 (s, 1H); 4.29 (quad, 2H); 3.83 (s, 3H); 1.28 (t, 3H)

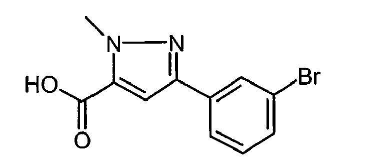

### Intermediate 61

### 3-(3-bromophenyl)-1-methyl-1H-pyrazole-5-carboxylic acid

To a solution of **intermediate 59** (5g, 16 mmol) in 5 mL of THF and 50 mL of ethanol was added 80 mL of NaOH (1 N) and was stirred at rt for 3h.
The resulting mixture was evaporated, treated with 100 mL of water, by a dropwise addition of 100 mL of HCl (1 N), stirred for 1h, filtered and dried under vacuum (4.3g).
Yield = 96 %
¹H NMR (CDCl₃) δ: 7.91 (s, 1 H); 7.65 (d, 1 H); 7.40 (d, 1 H); 7.23 (d, 1 H); 7.19 (d, 1 H); 4.19 (s, 3H)

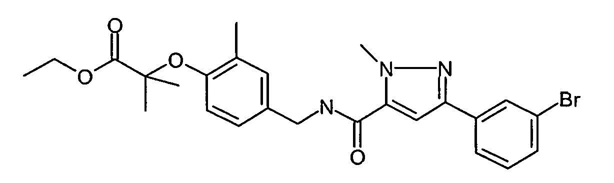

### Intermediate 62

### Ethyl 2-({4-[({[3-(3-bromophenyl)-1-methyl-1H-pyrazol-5-yl]carbonyl}amino)methyl]-2-methylphenyl}oxy)-2-methylpropanoate

To a solution of **intermediate 61** (2.2 g, 7.8 mmol) in 100 mL of toluene was added 5 mL (68mmol) of SOCl₂, and was stirred at 120°C for 3h.
Then the reaction mixture was evaporated twice with toluene to eliminate the excess of SOCl₂ to give the intermediate **C**.
To a solution of **intermediate 10** (2.24 g, 7.8mmol) with 3.27 mL (23.4mmol) of Et₃N in 150 mL of CH₂Cl₂ anhydrous was added intermediate **C** (diluted in 100 mL of CH₂Cl₂), then it was stirred at rt for 16h.

The resulting mixture was treated with 150 mL of HCI (1 N), the organic layer was extracted and washed with 150 mL of NaOH (1 N), brine, dried on Na₂SO₄ and under vacuum to give an amorphous solid (3.5g).
Yield = 87%
¹H NMR (CDCl₃) δ: 7.83 (s, 1 H); 7.59 (d, 1 H); 7.35 (d, 1 H); 7.17 (d, 1 H); 7.05 (s, 1 H); 6.94 (d, 1 H); 6.68 (s, 1 H); 6.56 (d, 1 H); 6.25 (m, NH); 4.41 (d, 2H); 4.22-4.11 (quad, 2H; s, 3H); 2.15 (s, 3H); 1.52 (s, 6H); 1.19 (t, 3H)

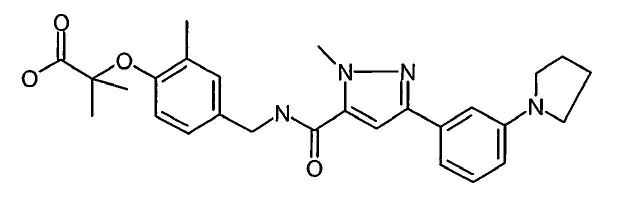

### Example 23

### 2-methyl-2-[(2-methyl-4-{[({1-methyl-3-[3-(1-pyrrolidinyl)phenyl]-1H-pyrazol 5-yl}carbonyl)amino]methyl}phenyl)oxy]propanoic acid

To a solution of **intermediate 56** (150 mg, 0.298 mmol) in 10 mL of methanol was added 3 mL of NaOH (1N). It was then stirred at 90°C for 3h. 3 mL of HCl (1 N) was added to the resulting mixture, then the organic layer was extracted with ethylacetate, washed with water and brine, dried on Na₂SO₄ and evaporated to dryness. The residue was crystallised in toluene and washed with hexane to give the title compound as a white powder (100mg).
Yield=70%
¹H NMR (CDCl₃) δ: 6.94 (s, 2H); 6.83 (dd, 2H); 6.57 (s, 2H); 6.41 (m, 1 H); 6.20 (m, 1 H); 4.27 (d, 2H); 4.01 (s, 3H); 3.16 (m, 4H); 2.02 (s, 3H); 1.82 (m, 4H); 1.40 (s, 6H)

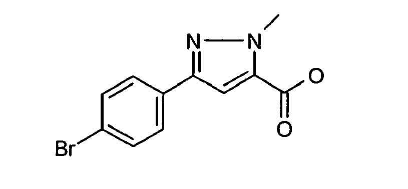

### Intermediate 63

### 3-(4-bromophenyl)-1-methyl-1H-pyrazole-5-carboxylic acid

To a solution of **intermediate 49** (5 g, 16 mmol) in 10 mL of THF and 150 mL of ethanol was added 80 mL of NaOH (1 N). The reaction mixture was stirred at rt for 3h, evaporated to dryness, then treated with water, and acidified with HCl (1N) to give a white precipitate which was filtered off (4.5g).
Quantitative yield.
1 H NMR (DMSO) δ: 6.60 (d, 2H); 7.40 (d, 2H); 7.13 (s, 1 H); 3.93 (s, 3H).

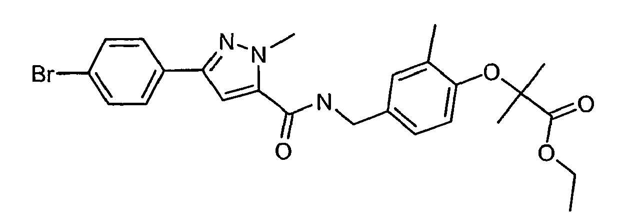

### Intermediate 64

### Ethyl-2-({4-[({[3-(4-bromophenyl)-1-methyl-1H-pyrazol-5-yl]carbonyl}amino) methyl]-2-methylphenyl}oxy)-2-methylpropanoate

To a solution of **intermediate 63** (2.2g, 7.8 mmol) in 100 mL of toluene was added 2.86 mL (40mmol) of SOCl₂, and was stirred at 110°C for 3h. Then the reaction mixture was evaporated twice with toluene to eliminate the excess of SOCl₂ to give the intermediate **D**. To a solution of **intermediate 153** (2.25 g, 7.8mmol) and 3.3 mL (23.5mmol) of Et₃N in 100 mL of CH₂Cl₂ anhydrous was added dropwise, intermediate **D** diluted in 50 mL of CH₂Cl₂. The resulting mixture was stirred at rt for 2h, washed with HCl (1 N), NaOH (1 N) and brine, then it was dried on Na₂SO₄ and evaporated to give a white-off solid (3.6g).
Yield = 89%
¹H NMR (CDCl₃) δ: 7.49 (d, 2H); 7.37 (d, 2H); 6.99 (s, 1 H); 6.88 (d, 1 H); 6.60 (s, 1 H); 6.50 (d, 1 H); 6.14 (m, NH); 4.35 (d, 2H); 4.18-4.06 (quad, 2H; s, 3H); 2.10 (s, 3H); 1.46 (s, 6H); 1.13 (t, 3H)

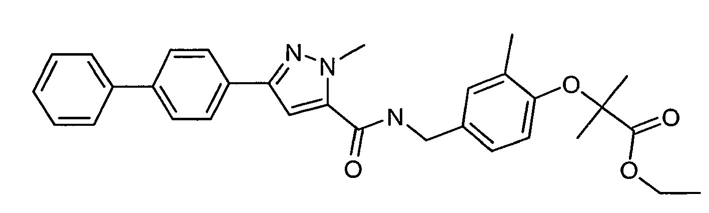

### Intermediate 65

### Ethyl-2-({4-[({[3-(4-biphenylyl)-1-methyl-1H-pyrazol-5-yl]carbonyl}amino) methyl]-2-methylphenyl}oxy)-2-methylpropanoate

To a solution of **intermediate 64** (257 mg, 0.5 mmol) in 6 mL of toluene was added Pd(PPh₃)₄ (29 mg, 0.025mmol) and was stirred for 10 min. Then Na₂CO₃, 2M (0.65 mL, 1.3mmol) and phenylboronic acid (91 mg, 0.75mmol) in 3 mL of ethanol were added to the reaction mixture and it was stirred at reflux for 2 days. 3 mL of water was added to the resulting mixture, which was filtered through celite, washed with water and ethylacetate, evaporated to dryness then treated with ethylacetate, washed with NaHCO₃, water, dried on Na₂SO₄, evaporated to dryness and purified by flash chromatography (CH₂Cl₂/ethylacetate 95/5) to give the desired product (146mg).
Yield = 60%
¹H NMR (CDCl₃) δ: 7.78 (d, 2H); 7.60-7.54 (m, 4H); 7.38 (t, 2H); 7.28 (d, 1 H); 7.07 (s, 1 H); 6.97 (d, 1 H); 6.73 (s, 1 H); 6.58 (d, 1 H); 6.21 (m, NH); 4.44 (d, 2H); 4.24-4.13 (s, 3H; quad, 2H); 2.18 (s, 3H); 1.53 (s, 6H); 1.20 (t, 3H)

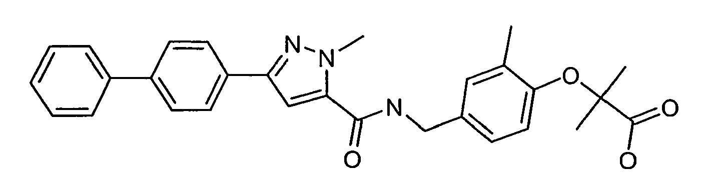

### Example 24

### 2-({4-[({[3-(4-biphenylyl)-1-methyl-1H-pyrazol-5-yl]carbonyl}amino)methyl]-2-methylphenyl}oxy)-2-methylpropanoic acid

To a solution of **intermediate 65** (146 mg, 0.28 mmol) in 4 mL of ethanol was added 4 mL of NaOH (2N), and was stirred at rt overnight. The resulting mixture was evaporated, then treated with water, acidified with HCl (5N) and stirred for 5 min. It was then filtered off, washed with water and hexane to give the title compound as a white powder (102mg).
Yield = 74%
Mp= 90-95 °C (becomes gummy)
¹H NMR (CDCl₃) δ: 7.76 (d, 2H); 7.59-7.52 (m, 4H); 7.37 (t, 2H), 7.29 (d, 1 H); 7.10 (s, 1 H); 7.02 (d, 1H); 6.73 [(d, 1H) + (s, 1H)]; 6.26 (m, NH); 4.45 (d, 2H); 4.18 (s, 3H); 2.19 (s, 3H); 1.56 (s, 6H).

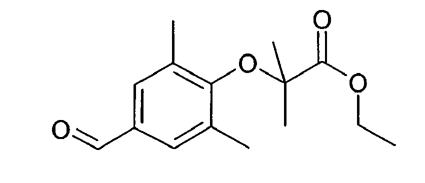

### Intermediate 66

### ethyl 2-(4-formyl-2,6-dimethylphenoxy)-2-methylpropanoate

A solution of 8.16g (0.053 mol) of 4-hydroxy-3,5-dimethylbenzaldehyde and 14.72g (0.106mol) of K₂CO₃ in 80 mL of acetone was refluxed for 30 min. Then, 12 mL ( 0.08 mol) of ethyl 2-bromo-2-methylpropanoate was added to the reaction mixture, which was stirred at reflux for 65h. 3 eq of K₂CO₃ and 3.5 eq of bromo derivative were added progressively during reflux. The resulting mixture was filtered off , washed with acetone and evaporated to dryness. The residue was diluted in CH₂Cl₂, washed with NaOH (1N); an emulsion appeared that was filtered through celite, washed with water, filtered again through celite, dried on MgSO₄ and evaporated to dryness to a dark oil, which was purified by flash chromatography (CH₂Cl₂ 100%) to give a dark oil (5.3g).
Yield: 37%
¹H NMR (DMSO= 2.51) δ: 9.87 (s, CHO); 7.60 (s, 2H); 4.20 (quad, 2H); 2.21 (s, 6H); 1.42 (s, 6H); 1.26 (t, 3H)

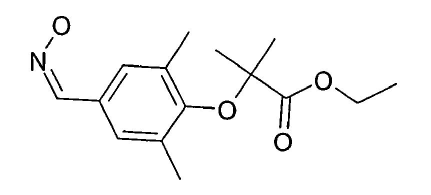

### Intermediate 67

### Ethyl-2-{4-[(Z)-(hydroxyimino)methyl]-2,6-dimethylphenoxy}-2-methyl propanoate

To a solution of 5.31g (0.02 mol) of **intermediate 66** in 50 mL of ethanol and 16 mL of water was added 2.3g (0.032mol) of hydroxylamine hydrochloride and 4.94g (0.06mol) of sodium acetate. The reaction mixture was stirred at rt for 18h and evaporated. The residue was diluted in ethyl acetate and water, extracted twice with ethyl acetate, washed with water and brine, dried on MgSO₄, and evaporated to dryness to give an orange oil (5.56g).
Yield: 99%
¹H NMR (DMSO): 11.07 (s, OH); 8.00 (s, 1H); 7.24 (s, 2H); 4.18 (quad, 2H); 2.14 (s, 6H); 1.39 (s, 6H); 1.25 (t, 3H)

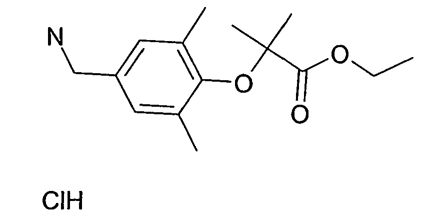

### Intermediate 68

### Ethyl-2-[4-(aminomethyl)-2,6-dimethylphenoxy]-2-methylpropanoate hydrochloride

To a solution of 5.55g (0.02mol) of intermediate 67 in 70 mL of ethanol under nitrogen was added 1.14 mL of acetic acid (1eq) and 555 mg of Pd/C 10% (10% w/w) , then it was purged under hydrogen. The reaction was maintained at 15 bar of hydrogen pressure at rt for 3 days.
The resulting mixture was filtered through celite, washed with ethanol and evaporated to dryness. The residue was diluted in 75 mL of water, washed twice with 30 mL of diethyl ether. The organic layers were washed with 30 mL of water. The aqueous layers were mixed and NaOH (30%) was added until pH=10. It was then extracted three times with 30 mL of diethyl ether, dried on MgSO₄, and filtered.
The resulting solution was put under HCl atmosphere for a a few minutes. The solution was evaporated to dryness and purified with flash chromatography (CH₂Cl₂ 100% to CH₂Cl₂/MeOH 90/10) to give a sticky cream solid (800mg).
Yield: 13%
¹H NMR (DMSO): 8.37 (s, NH₂); 7.15 (s, 2H); 4.19 (quad, 2H); 3.88 (s, 2H); 2.13 (s, 6H); 1.38 (s, 6H); 1.26 (t, 3H)

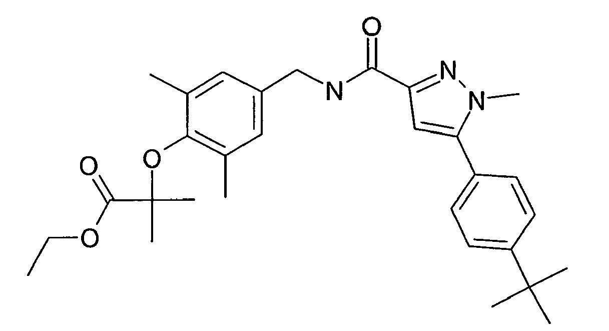

### Intermediate 69

### Ethyl-2-{4-[({[5-(4-tert-butylphenyl)-1-methyl-1H-pyrazol-3-yl]carbonyl}amino)methyl]-2,6-dimethylphenoxy}-2-methylpropanoate

A solution of 43 mg (0.165 mmol) of **intermediate 3** in 30 mL of toluene and 60 µL (0.825mmol) of SOCl₂ was heated at 110°C for 4h. The resulting mixture was evaporated, diluted in toluene and evaporated again to give the corresponding acid chloride. To a solution of 50 mg (0.165mmol) of **intermediate 68** in 20 mL of CH₂Cl₂ and 71 µL (0.49mmol) of Et₃N was added the acid chloride (diluted in 10 MI of CH₂Cl₂). The reaction mixture was stirred at rt for 16h.
The resulting mixture was diluted in CH₂Cl₂, washed twice with HCl (1 N), NaHCO₃, and once with brine and evaporated to dryness to give a pale oil (70mg).
Yield: 84%
¹H NMR (CDCl₃) δ: 7.41 (d, 2H); 7.3 (d, 2H); 7.1 (m, NH); 6.9 (s, 2H); 6.8 (s, 1H); 4.45 (d, 2H); 4.22 (quad, 2H); 3.8 (s, 3H); 2.1 (s, 6H); 1.4 (s, 6H); 1.39 (s, 9H); 1.38 (t, 3H)

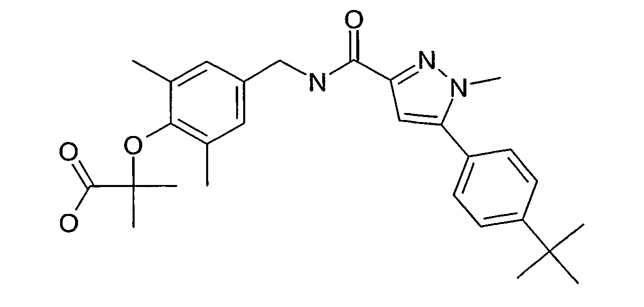

### Example 25:

### 2-{4-[({[5-(4-tert-butylphenyl)-1-methyl-1H-pyrazol-3-yl]carbonyl}amino) methyl]-2,6-dimethylphenoxy}-2-methylpropanoic acid

A solution of 70 mg (0.138mmol) of **intermediate 69** in ethanol and 1.4 mL of NaOH (1 N) was heated at 80°C for 4h. The resulting mixture was evaporated and acidified with cold (0°C) HCl (1 N). The resulting precipitate was filtered off and washed with water and pentane, then dried under vacuum to give a white solid (70mg).
Quantitative yield
Mp: >102°C, becomes gummy
LC-TOF MNa+ calc= 500, 2526 MNa+ exp= 500.2511 δ = 3ppm
¹H NMR (CDCl₃) δ: 7.43 (d, 2H); 7.28 (d, 2H); 7.22 (m, NH); 6.93 (s, 2H); 6.81 (s, 1 H); 4.46 (d, 2H); 3.83 (s, 3H); 2.15 (s, 6H); 1.43 (s, 6H); 1.29 (s, 9H)

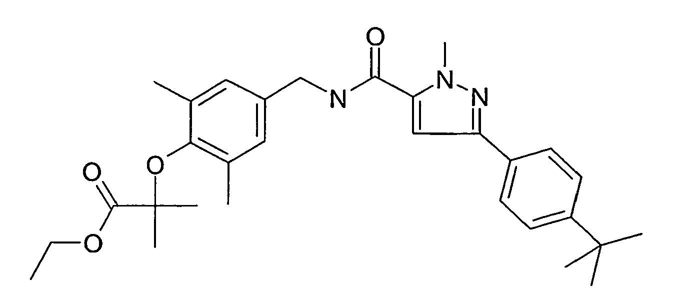

### Intermediate 70

### Ethyl-2-{4-[({[3-(4-tert-butytphenyl)-1-methyl-1H-pyrazol-5-yl]carbonyl} amino)methyl]-2,6-dimethylphenoxy}-2-methylpropanoate

A solution of 43 mg (0.165 mmol) of **intermediate 5** in 30 mL of toluene and 60 µL (0.825mmol) of SOCl₂ was heated at 110°C for 4h. The resulting mixture was evaporated, diluted in toluene and evaporated again to give the corresponding acid chloride. To a solution of 50 mg (0.165mmol) of **intermediate 68** in 20 mL of CH₂Cl₂ and 71 µL (0.49mmol) of Et₃N was added the acid chloride (diluted in 10 MI of CH₂Cl₂). The reaction mixture was stirred at rt for 16h.
The resulting mixture was diluted in CH₂Cl₂, washed twice with HCl (1 N), NaHCO₃, and once with brine and evaporated to dryness to give a pale yellow oil (50mg).
Yield: 60%
¹H NMR (CDCl₃) δ: 7.6 (d, 2H); 7.3 (d, 2H); 6.88 (s, 2H); 6.7 (s, 1H); 6.3 (m, NH); 4.4 (d, 2H); 4.2 (quad, 2H); 4.15 (s, 3H); 2.1 (s, 6H); 1.4 (s, 6H); 1.26 (t, 3H); 1.25 (s, 9H)

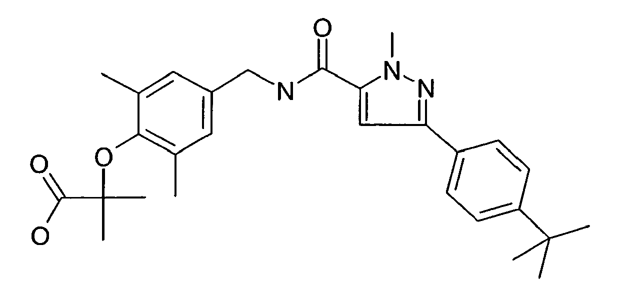

### Example 26

### 2-{4-[({[3-(4-tert-butylphenyl)-1-methyl-1H-pyrazol-5-yl]carbonyl}amino)methyl]-2,6-dimethylphenoxy}-2-methylpropanoic acid

A solution of 50 mg (0.1mol) of **intermediate 70** in ethanol and 1 mL of NaOH (1 N) was heated at 80°C for 4h. The resulting mixture was evaporated, acidified with cold (0°C) HCl (1N). The resulting precipitate was filtered off and washed with water and pentane, then dried under vacuum to give a white powder (50mg).
Quantitative yield
Mp: >122°C, becomes gummy
LC-TOF MH+ calc= 478.2706 MH+ exp= 478.2704 δ = 0.4ppm
1H NMR (CDCI₃) δ: 7.49 (d, 2H); 7.22 (d, 2H); 6.79 (s, 2H); 6.55 (s, 1H); 6.10 (m, NH); 4.31 (d, 2H); 4.05 (s, 3H); 2.05 (s, 6H); 1.33 (s, 6H); 1.14 (s; 9H)

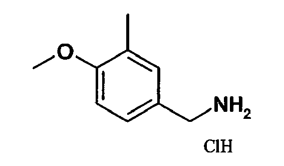

### Intermediate 71

### {(3-methyl-4-(methyloxy)phenyl]methyl}amine hydrochloride

4-methoxy-3-methylbenzaldehyde (10g, 66.6mmol), hydroxylamine hydrochloride (7.87g, 111.2mmol) and Et₃N (63ml, 440 mmol) in CH₂Cl₂ (250ml) were stirred at rt for 18h. The mixture was quenched with water (150ml), the aqueous phase extracted with CH₂Cl₂ (150ml) and ethylacetate (150ml). The organic layers were evaporated to give the product A.
To a solution of 10.6 g (280mmol) of NaBH₄ in 150 mL of anhydrous DME under argon at 0°C was added dropwise TiCI₄ (15.3ml, 140mmol) then the product A diluted in 50 mL of DME was added dropwise maintaining temperature at 0°C.
The resulting mixture was stirred at rt overnight.
At 0°C, the mixture was quenched with a progressive addition of 200 mL of water, then basified with NaOH (25%) A dark blue precipitate formed. The organic layer was extracted three times with ethyl acetate, filtered and evaporated. The resulting yellow oil was diluted in anhydrous diethyl ether and, at 0°C, 50 mL of HCl (2N in diethyl ether) was added to give a white precipitate which was filtered off (10.9g).
Yield: 87%
¹H NMR (DMSO) δ: 7.33-7.24 (m, 2H); 6.95 (d, 1H); 3.88 (m, 2H); 3.78 (s, 3H); 2.14 (s, 3H)

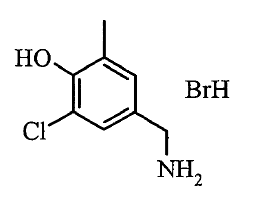

### Intermediate 72

### 4-(aminomethyl)-2-chloro-6-methylphenol hydrobromide

To a solution of intermediate 71 (2g, 10.8mmol) in 20 mL of glacial acetic acid at T<30°C was added dropwise 2.57 mL (3eq) of SO₂Cl₂. The reaction mixture was stirred at rt for 2h. Then 100 mL of HBr (48%) was added and the resulting mixture was heated at 90°C for 18h. The mixture was evaporated, diluted in toluene and evaporated again to give a brown solid.
Yield: the product was directly used in the next step.
¹H NMR (DMSO) δ: 7.39 (s, 1 H); 7.24 (s, 1 H); 3.95 (m, 2H); 2.26 (s, 3H)

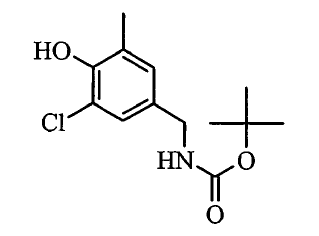

### Intermediate 73

### Tert-butyl (3-chloro-4-hydroxy-5-methylbenzyl)carbamate

To a solution of intermediate 72 in CH₂Cl₂, THF and 4.77 mL of Et₃N was added 6.19 mL of Boc₂O, and it was stirred at rt for 18h. 3 mL of Boc₂O was added and stirred for 4h.
The resulting mixture was evaporated, diluted in CH₂Cl₂, treated with water/NaOH (1 N) and separated. The aqueous layer was neutralised with HCl (1N)/H₂O. The resulting solution was evaporated, the precipitate formed was filtered and washed with pentane to give a cream powder (1.64g).
Yield: 55.8 %
¹H NMR (CDCl₃) δ: 7.01 (s, 1 H); 6.88 (s, 1 H); 5.51 (m, 1 H); 4.72 (m, 1 H); 4.10 (d, 2H); 2.19 (s, 3H); 1.39 (s, 9H)

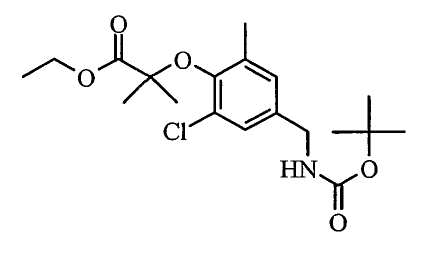

### Intermediate 74

### Ethyl 2-(4-{[(tert-butoxycarbonyl)amino]methyl}-2-chloro-6-methylphenoxy)-2-methylpropanoate

To a solution of 1.64 g (6.03mmol) of **intermediate 73** in acetone, was added 2.5 g (3eq) of K₂CO₃and 2.68 mL (3eq) of **ethyl 2-bromo-2-methylpropanoate**. The reaction mixture was refluxed for 120h. 3 eq of bromo derivative were added at 24h, 72h and 96h. The resulting mixture was evaporated, diluted in diethyl ether and washed with water. The organic layer was evaporated and purified by flash chromatography (cyclohexane/CH₂Cl₂ 80/20 to CH₂Cl₂ 100%) and automated chromatography CH₂Cl₂ 100% to give a colourless oil (1.3g).
Yield: 59%
¹H NMR (CDCl₃) δ: 7.02 (d, 1 H); 6.88 (d, 1 H); 4.21 (quad, 2H); 4.13 (d, 2H); 2.14 (s, 3H); 1.44 (s, 6H); 1.39 (s, 9H); 1.28 (t, 3H)

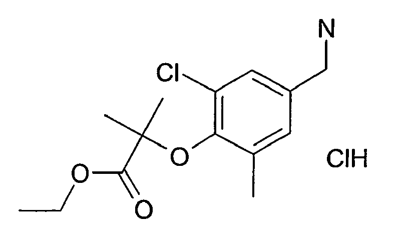

### Intermediate 75

### Ethyl 2-[4-(aminomethyl)-2-chloro-6-methylphenoxy]-2-methylpropanoate hydrochloride

To a solution of 1.3 g (3.38mmol) of **intermediate 74** in CH₂Cl₂, cooled by water/ice bath was added 1.04 mL (4eq) of Trifluoroacetic acid, and was stirred at rt for 18h. The resulting mixture was evaporated to dryness and diluted in diethyl ether. A precipitate was formed which was filtered to give a gummy residue. This was put in water and NaOH (1 N) was added until the mixture was pH=8-9. The organic layer was extracted with diethyl ether, dried on Na₂SO₄ and evaporated. The residue was diluted in a small amount of diethyl ether, then diethyl ether saturated in HCl was added to give a precipitate which was filtered and dried in a dessicator to afford a cream solid (600mg).
Yield: 55%
¹H NMR (DMSO) δ: 7.44(s, 1 H); 7.28 (s, 1 H); 4.14 (quad, 2H); 3.90 (m, 2H); 2.15 (s, 3H); 1.39 (s, 6H); 1.21 (t, 3H)

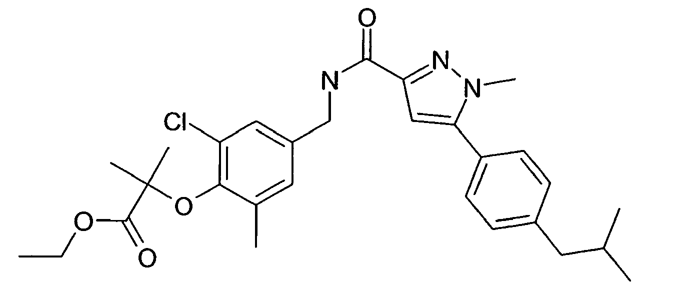

### Intermediate 76

### Ethyl-2-{2-chloro-4-[({[5-(4-isobutylphenyl)-1-methyl-1H-pyrazol-3-yl]carbonyl}amino)methyl]-6-methylphenoxy}-2-methylpropanoate

A solution of 200mg (0.77mmol) **intermediate 32** of toluene with 280µL (3.85mmol) of SOCl₂ was heated at 110 °C for 4h.The resulting mixture was evaporated.
The residue (diluted in 10 mL of CH₂Cl₂ anhydrous) was added dropwise to a solution of 273mg (0.85mmol) of **intermediate 75** in 50 mL of CH₂Cl₂ and 245µL (1.7mmol) of Et₃N.
After stirring at rt for 4h, the resulting mixture was diluted in 50mL of CH₂Cl₂, washed with HCl (0.5N), NaHCO₃ and water, dried on MgSO₄ , evaporated and purified by flash chromatography (CH₂Cl₂ to CH₂Cl₂/MeOH 99/1) to give a colourless oil (210mg).
Yield: 52 %
¹H NMR (CDCl₃) δ: 7.25 (d, 2H); 7.17 (d, 2H); 7.11 (d, 1 H); 6.98 (d, 1 H); 6.79 (s, 1 H); 4.46 (d, 2H); 4.22 (quad, 2H); 3.81 (s, 3H); 2.46 (d, 2H); 2.15 (s, 3H); 1.83 (m, 1 H); 1.45 (s, 6H); 1.28 (t, 3H); 0.87 (d, 6H)

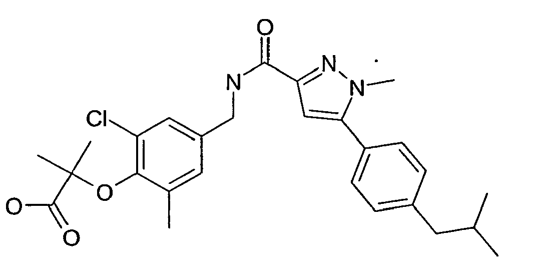

### Example 27

### 2-{2-chloro-4-[({[5-(4-isobutylphenyl)-1-methyl-1H-pyrazol-3-yl]carbonyl}amino)methyl]-6-methylphenoxy}-2-methylpropanoic acid

A solution of 210 mg (0.4mmol) of **intermediate 76** in 50 mL of ethanol and 2 mL of NaOH (1 N) was heated at 60°C for 5h. The resulting mixture was evaporated, diluted in 5 mL of water, acidified dropwise with HCl (1N) until pH=4. The resulting solid was filtered, diluted in CH₂Cl₂, dried on Na₂SO₄, and evaporated under vacuum to give a white foam (184mg).
Yield: 87%
Mp: 75-80°C, becomes gummy
LC-TOF MH+ calc= 498.2159MH+ exp= 498.2126 δ =6.7ppm
¹H NMR (CDCl₃) δ: 7.35-7.21 (m, 3H); 7.16 (m, 2H); 7.04 (s, 1 H); 6.80 (s, 1 H); 4.48 (d, 2H); 3.82 (s, 3H); 2.46 (d, 2H); 2.21 (s, 3H); 1.84 (m, 1 H); 1.51 (s, 6H); 0.87 (d; 6H)

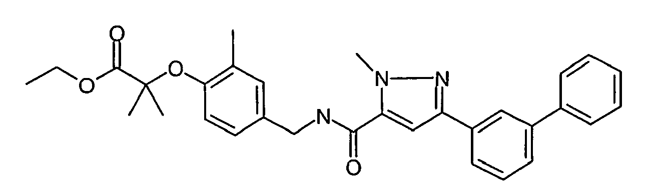

### Intermediate 77

### Ethyl 2-[4-({[(3-biphenyl-3-yl-1-methyl-1H-pyrazol-5-yl)carbonyl]amino}methyl)-2-methylphenoxy]-2-methylpropanoate

To a solution of 270 mg (0.52mmol) of **intermediate 62** in 6.3 mL of toluene was added 30 mg (0.026mmol) of Pd(PPh₃)₄, stirred for 10 min. Then 0.7 mL of aqueous solution of Na₂CO₃ (2N) and 96 mg (0.78mmol) of p-phenylboronic acid in 2-3 mL of ethanol were added to the reaction mixture. The resulting mixture was refluxed overnight, then 3mL of water were added, stirred for 10 min, filtered through celite, washed with water and ethyl acetate and evaporated. The residue was diluted in ethyl acetate, washed with NaHCO₃ and water, dried on Na₂SO₄, evaporated and purified on silica gel (CH₂Cl₂/AcOEt 95/5).
Yield: the product was directly used in the next step.
¹H NMR (CDCl₃) δ: 7.92 (s, 1 H); 7.67 (d, 1 H); 7.57 (d, 2H); 7.48 (d, 1 H); 7.43-7.33 (m, 3H); 7.29 (d, 1 H); 7.07 (s, 1 H); 6.97 (dd, 1 H); 6.74 (s, 1H); 6.58 (d, 1 H); 6.17 (m, NH); 4.43 (d, 2H); 4.22-4.13 (s, 3H; quad, 2H); 2.17 (s, 3H); 1.53 (s, 6H); 1.20 (t, 3H)

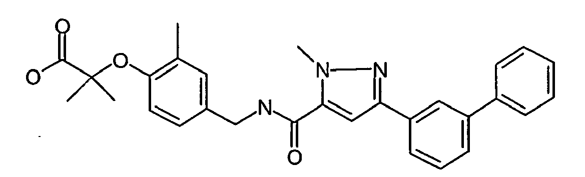

### Example 28

### 2-[4-({[(3-biphenyl-3-yl-1-methyl-1H-pyrazol-5-yl)carbonyl]amino}methyl)-2-methylphenoxy]-2-methylpropanoic acid

A solution of **intermediate 77** in 3-4mL of ethanol and 4 mL of NaOH (2N) was stirred at rt overnight. The resulting mixture was evaporated, then diluted in water, acidified with HCl (5N) until pH=1, stirred for 30 min. The precipitate was filtered, washed with water and dried to give a white solid (153mg).
Yield: 63 % from intermediate 62
Mp: 88°C, becomes gummy
LC-TOF MH+ exp= 484.2194 MH+calc=484.2236 δ=8.4 ppm
¹H NMR (CDCl₃) δ: 7.91 (s, 1H); 7.65 (d, 1H); 7.56 (d, 2H); 7.47 (d, 1H); 7.42-7.33 (m, 3H); 7.29 (d, 1 H); 7.08 (s, 1 H); 6.99 (d, 1 H); 6.75 (s, 1 H); 6.71 (d, 1 H); 6.30 (m, NH); 4.43 (d, 2H); 4.18 (s, 3H); 2.17 (s, 3H); 1.55 (s, 6H)

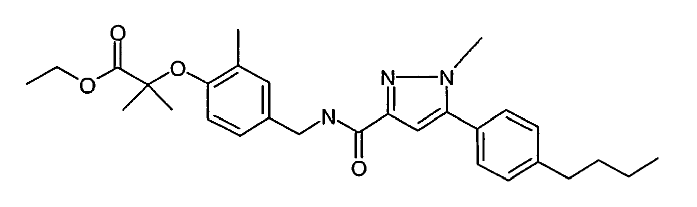

### Intermediate 78

### Ethyl 2-({4-[({[5-(4-butylphenyl)-1-methyl-1H-pyrazol-3-yl]carbonyl}amino)methyl]-2-methylphenyl}oxy)-2-methylpropanoate

To a solution of 257 mg (0.5mmol) of **intermediate 52** in 6mL of toluene was added 0.029g (0.025mmol) of Pd(PPh₃)₄, stirred for 10 min. Then 0.65 mL (1.3mmol) of aqueous solution of Na₂CO₃ (2N) and 76 mg (0.75mmol) of buthylboronic acid dissolved in 2-3 mL of ethanol were added. The reaction mixture was refluxed overnight. Then 3 mL of water was added, the solution was filtered through celite, washed with water and ethyl acetate and evaporated. The residue was diluted in ethyl acetate, washed with NaHCO₃ and water, dried on Na₂SO₄, evaporated and purified on silica gel (CH₂Cl2/AcOEt 95/5).
Yield: the product was directly used in the next step.
¹H NMR (CDCl₃) δ: 7.28-7.20 (m, 4H); 7.08 (s, 1 H); 6.97 (d, 1 H); 6.78 (s, 1 H); 6.55 (d, 1 H); 4.46 (d, 2H); 4.18 (quad, 2H); 3.79 (s, 3H); 2.59 (t, 2H); 2.15 (s, 3H); 1.57 (m, 2H); 1.51 (s, 6H); 1.32 (m, 2H); 1.20 (t, 3H); 0.88 (t, 3H)

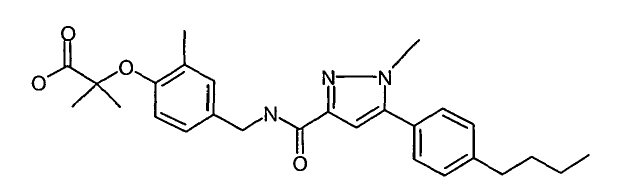

### Example 29

### 2-({4-[({[5-(4-butylphenyl)-1-methyl-1H-pyrazol-3-yl]carbonyl}amino)methyl]-2-methylphenyl}oxy)-2-methylpropanoic acid

A solution of **intermediate 78** in ethanol and NaOH (2N) was stirred at rt overnight. The resulting mixture was evaporated, acidified with HCI (5N) until pH=1 and stirred for 30 min. Then the precipitate was filtered, washed with water and dried under vacuum. The residue was not purified (24mg).
Yield: 10 % from intermediate 78
Mp: 112°C
RMN (CDCl₃) δ: 7.27-7.20 (m, 4H); 7.12 (s, 1 H); 7.00 (d, 1 H); 6.78 (s, 1 H); 6.72 (d, 1 H); 4.46 (d, 2H); 3.79 (s, 3H); 2.59 (t, 2H); 2.16 (s, 3H); 1.57 (m, 2H); 1.54 (s, 6H); 1.32 (m, 2H); 0.88 (t, 3H)

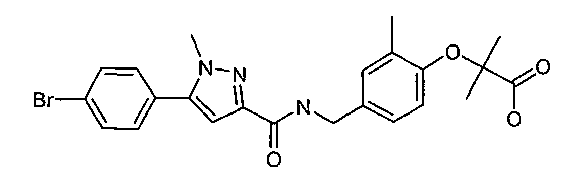

### Example 30

### 2-({4-[({[5-(4-bromophenyl)-1-methyl-1H-pyrazol-3-yl]carbonyl}amino)methyl]-2-methylphenyl}oxy)-2-methylpropanoic acid

A solution of 30 mg (0.06mmol) of **intermediate 52** in 3 mL of ethanol and 3 mL of NaoH (2N) was stirred at rt overnight, then ethanol was evaporated. The aqueous solution was acidified with HCl (5N) until pH=1 and stirred for 30 min. The precipitate was filtered, washed with water and dried under vacuum to give a white powder (15mg).
Yield: 53%
Mp: 84-92°C, becomes gummy
LC-TOF MH+=486.1045 MH+calc=486.1028 δ=3.5 ppm
¹H NMR (CDCl3) d: 7.54 (d, 2H); 7.22 (s, 2H); 7.11 (s, 1 H); 7.99 (d, 1 H); 6.80 (s, 1 H); 6.72 (d, 1 H); 4.45 (d, 2H); 3.78 (s, 3H); 2.16 (s, 3H); 1.53 (s, 6H)

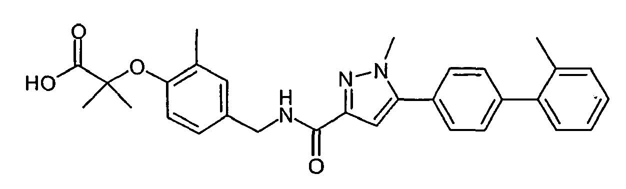

### Example 31

### 2-methyl-2-{2-methyl-4-[({[1-methyl-5-(2'-methylbiphenyl-4-yl)-1H-pyrazol-3-yl]carbonyl}amino)methyl]phenoxy}propanoic acid

To a solution of 150 mg (0.3mmol) of **intermediate 51** in 3.5 mL of toluene was added 17 mg of Pd(PPh₃)₄ (0.0145mmol), and it was stirred for 10 min. Then 0.4 mL (0.78mmol) of aqueous solution of Na₂CO₃ (2M) and 50 mg (0.37mmol) of 2-methylphenyl boronic acid in 2 mL of ethanol were added and the reaction mixture was refluxed overnight.
Then 3 mL of water was added, the resulting solution was filtered through celite, washed with water and ethyl acetate and evaporated. The residue was diluted in ethyl acetate, washed with NaHCO₃, water, dried, evaporated and purified by flash chromatography (CH₂CI₂ 100%) to give the desired product.
A solution of 51 mg of this product in 2 mL of ethanol and 2 mL of NaOH (2N) was heated at 50°C for 4h.
The resulting mixture was evaporated, diluted in water, acidified with HCI 5N until pH=1. The precipitate was filtered dried under vacuum to give a white powder (29mg).
Yield: 20%
Mp: 90-96°C, becomes gummy
¹H NMR (DMSO) d: 8.70 (m, NH); 7.72 (d, 2H); 7.56 (d, 2H); 7.36 (m, 4H); 7.18 (s, 1 H); 7.10 (d, 1 H); 6.91 (s, 1H); 6.72 (d, 1 H); 4.39 (d, 2H); 4.03 ( s, 3H); 3.39 (d, 2H); 2.35 (s, 3H); 2.20 (s, 3H); 1.56 (s, 6H)

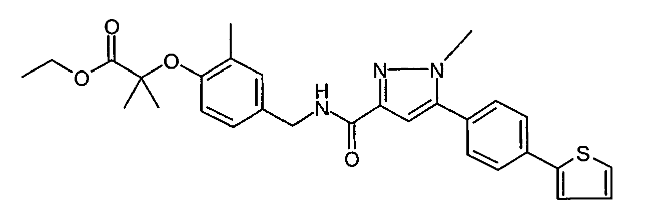

### Intermediate 79

### Ethyl 2-methyl-2-[(2-methyl-4-{[({1-methyl-5-[4-(2-thienyl)phenyl]-1H-pyrazol-3-yl}carbonyl)amino]methyl}phenyl)oxy]propanoate

To a solution of 150 mg (0.3mmol) of intermediate 51 in 3.5 mL of toluene was added 17 mg of Pd(PPh₃)₄ (0.0145mmol) and it was stirred for 10 min. Then 0.4 mL of aqueous solution of Na₂CO₃ (2M) and 46 mg (0.36mmol) of 2-methylphenyl boronic acid in 2 mL of ethanol was added and the reaction mixture was refluxed overnight but the reaction was not completed. 17 mg of Pd catalyst, 0.4 mL of Na₂CO₃aq. and 45 mg of boronic derivative were added, and the resulting mixture was refluxed overnight.
Then 3 mL of water was added, the resulting solution was filtered through celite, washed with water and ethyl acetate and evaporated. The residue was diluted in ethyl acetate, washed with NaHCO₃, water, dried, evaporated, purified by flash chromatography (CH₂Cl₂ 100% to CH₂Cl₂/ethyl acetate 80/20) and re-crystallised in methanol to give the desired product (116mg).
Yield:37%
¹H NMR (CDCl₃) δ: 7.64 (d, 2H); 7.35 (d, 2H); 7.30 (d, 1 H); 7.26 (d, 1 H); 7.06 (m, 3H); 6.96 (d, 1H); 6.80 (s, 1H); 6.55 (d, 1H); 4.48 (d, 2H); 4.15 (quad, 2H); 3.81 (s, 3H); 2.15 (s, 3H); 1.50 (s, 6H); 1.20 (t, 3H)

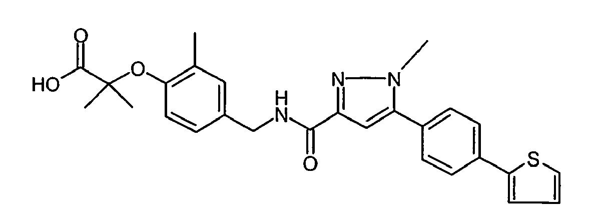

### Example 32

### 2-methyl-2-[(2-methyl-4-{[({1-methyl-5-[4-(2-thienyl)phenyl]-1H-pyrazol-3-yl}carbonyl)amino]methyl}phenyl)oxy]propanoic acid

A solution of 57 mg (0.11mmol) of **intermediate 79** in ethanol and 2.5 mL NaOH (2N) was heated at 78°C for 3h. Then ethanol was evaporated. The residue was diluted in 5 mL of water, acidified with HCI (1 N), and the generated precipitate was filtered, washed with water and dried under vacuum to give a white powder (46mg).
Yield: 85%
Mp: 92°C, becomes gummy
LC-TOF M+22exp= 512.1594 δ=5.2 ppm
¹H NMR (CDCl₃) δ: 7.65 (d, 2H); 7.37 (d, 2H); 7.34 (s, 1 H); 7.32 (d, 1 H); 7.27 (d, 1 H); 7.12 (m, 1 H); 7.07-6.97 (m; 2H); 6.84 (s, 1 H); 6.73 (1 H); 4.47 (d, 2H); 3.83 (s, 3H); 2.17 (s, 3H); 1.54 (6H)

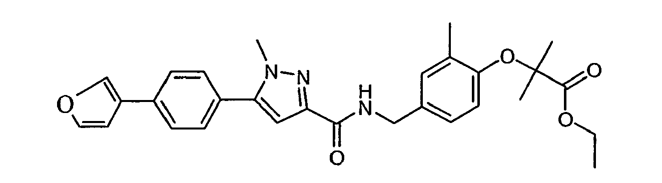

### Intermediate 80

### Ethyl-2-[(4-{[({5-[4-(3-furanyl)phenyl]-1-methyl-1H-pyrazol-3-yl}carbonyl)amino]methyl}-2-methylphenyl)oxy]-2-methylpropanoate

To a solution of 250 mg (0.486mmol) of **intermediate 52** in 6 mL of toluene was added 28 mg of Pd(PPh₃)₄ (0.024mmol) and it was stirred for 10 min. Then 0.65 mL of an aqueous solution of Na₂CO₃ (2M), 109 mg (1 mmol) of 3-furanylboronic acid diluted in 3 mL of ethanol were added, then it was refluxed overnight. To the resulting mixture was added 6 mL of water, then it was filtered through celite, washed with water and ethyl acetate, evaporated, diluted in ethyl acetate, washed with NaHCO₃ and water, dried on Na₂SO₄ and evaporated to dryness.
The residue was re-crystallised in methanol (153mg).
Yield: 59%
¹H NMR (CDCl₃) δ: 7.73 (s, 1 H); 7.52 (d, 2H); 7.44 (m, 1 H); 7.36 (d, 2H); 7.09-7.00 (m, 2H); 6.97 (d, 1 H); 6.82 (s, 1 H); 6.97 (s, 1 H); 6.55 (d, 1 H); 4.46(d, 2H); 4.18 (quad, 2H); 3.82 (s, 3H); 2.15 (s, 3H); 1.51 (s, 6H); 1.20 (t, 3H)

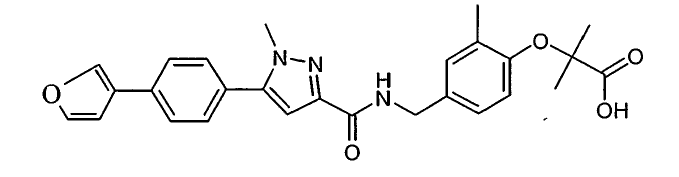

### Example 33

### 2-[(4-{[({5-[4-(3-furanyl)phenyl]-1-methyl-1H-pyrazol-3-yl}carbonyl)amino]methyl}-2-methylphenyl)oxy]-2-methylpropanoic acid

A solution of 153 mg (0.3mmol) of intermediate 80 in 5 mL of ethanol and 5 mL NaOH (2N) was stirred overnight. Then ethanol was evaporated. The residue was acidified with HCl (5N), extracted with ethyl acetate, dried on Na₂SO₄ and evaporated. Then it was purified by LC-Preparative to give a light yellow powder (5mg).
Yield: 3.46%
Mp: 102°C, becomes gummy
LC-TOF MH+exp= 474.1971 MH+calc=474.2029 δ=12.2 ppm
¹H NMR (DMSO) δ: 8.44 (m, 1 H); 8.13 (s, 1 H); 7.61 (m, 1 H); 7.60 (d, 2H); 7.42 (d, 2H); 6.92 (s, 1 H); 6.88 (s, 1 H); 6.83 (d, 1 H); 6.64 (s, 1 H); 6.49 (d, 1 H); 4.14 (d, 2H); 3.76 (s, 1 H); 1.96 (s, 3H); 1.31 (s, 6H).

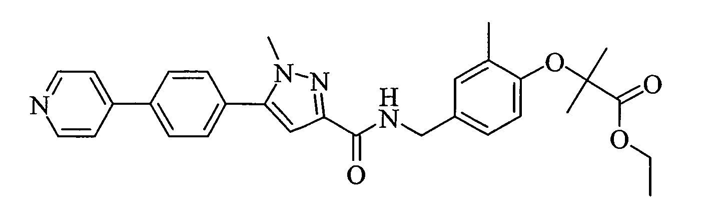

### Intermediate 81

### Ethyl-2-methyl-2-[(2-methyl-4-{[({1-methyl-5-[4-(4-pyridinyl)phenyl]-1H-pyrazol-3-yl}carbonyl)amino]methyl}phenyl)oxy]propanoate

To a solution of 250 mg (0.486mmol) of **intermediate 52** in 6 mL of toluene was added 28 mg of Pd(PPh₃)₄ (0.024mmol) and it was stirred for 10 min. Then 0.65 mL of an aqueous solution of Na₂CO₃ (2M), 120 mg (1mmol) of **4-pyridineboronic acid** diluted in 3 mL of ethanol were added, then it was refluxed overnight. To the resulting mixture was added 6 mL of water, then it was filtered through celite, washed with water and ethyl acetate, evaporated, diluted in ethyl acetate, washed with NaHCO₃ and water, dried on Na₂SO₄ and evaporated to dryness.
The residue was purified by flash chromatography (CH₂Cl₂ 100% to CH₂Cl₂/ethyl acetate 90/10) to give the desired product (175mg).
Yield: 70%
¹H NMR (CDCl₃) δ: 8.65 (d, 2H); 7.69 (d, 2H); 4.48 (m, 4H); 7.09-7.03 (m, 2H); 6.98 (d, 1 H); 6.87 (s, 1 H); 6.56 (d, 1 H); 4.47 (d, 2H); 4.18 (quad; 2H); 3.86 (s, 3H); 2.16 (s, 3H); 1.52 (s, 6H); 1.20 (t, 3H)

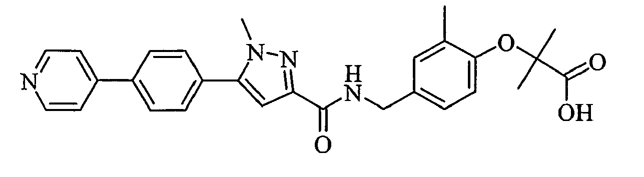

### Example 34

### 2-methyl-2-[(2-methyl-4-{[({1-methyl-5-[4-(4-pyridinyl)phenyl]-1H-pyrazol-3-yl}carbonyl)amino]methyl}phenyl)oxy]propanoic acid

A solution of 189 mg (0.37mmol) of **intermediate 81** in 6 mL of ethanol and 6 mL of NaOH (2N) was stirred at rt overnight. Then ethanol was evaporated, water was added to the resulting solution. HCl 5N was added to precipitate the acid which was filtered and dried to give a white powder (111 mg).
Yield: 62%
Mp: 264°C, decomposes
LC-TOF MH+exp= 485.2197 MH+calc= 485.2189 δ=1.6 ppm
¹H NMR (DMSO) δ: 8.75 (m, 3H); 8.04 (d, 2H); 7.87 (d, 2H); 7.82 (d, 2H); 7.19 (d, 1H); 7.09 (d, 1 H); 6.95 (s, 1 H); 6.72 (d, 1 H); 4.40 (d, 2H); 4.04 (s, 3H); 2.21 (s, 3H); 1.56 (s, 6H)

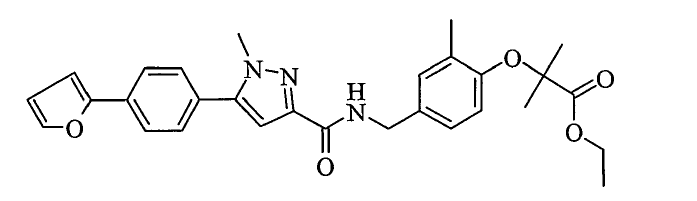

### Intermediate 82

### Ethyl-2-[(4-{[({5-[4-(2-furanyl)phenyl]-1-methyl-1H-pyrazol-3-yl}carbonyl)amino]methyl}-2-methylphenyl)oxy]-2-methylpropanoate

To a solution of 250 mg (0.486mmol) of **intermediate 52** in 6 mL of toluene was added 28 mg of Pd(PPh₃)₄ (0.024mmol) and it was stirred for 10 min. Then 0.65 mL of an aqueous solution of Na₂CO₃ (2M), 109 mg (1mmol) of 2-furanylboronic acid diluted in 3 mL of ethanol were added, then it was refluxed overnight. To the resulting mixture was added 6 mL of water, then it was filtered through celite, washed with water and ethyl acetate, evaporated, diluted in ethyl acetate, washed with NaHCO₃ and water, dried on Na₂SO₄ and evaporated to dryness.
The residue was purified by flash chromatography (CH₂Cl₂ 100% to CH₂Cl₂/ethyl acetate 90/10) to give the desired product (175mg).
Yield: 72%
¹H NMR (CDCl₃) δ: 7.70 (d, 2H); 7.44 (s, 1 H); 7.37 (d, 2H); 7.09-7.01 (m, 2H); 6.97 (d, 1H); 6.83 (s, 1 H); 6.68 (d, 1 H); 6.56 (d, 1H); 6.44 (m, 1 H); 4.46 (d, 2H); 4.18 (quad, 2H); 3.82 (s, 3H); 2.16 (s, 3H); 1.51 (s, 6H); 1.20 (t, 3H)

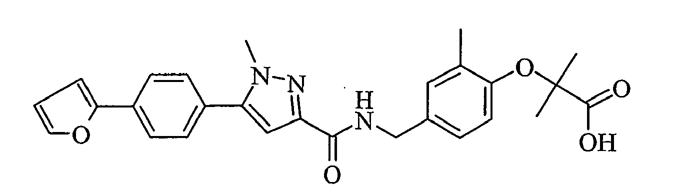

### Example 35

### 2-[(4-{[({5-[4-(2-furanyl)phenyl]-1-methyl-1H-pyrazol-3-yl}carbonyl)amino]methyl}-2-methylphenyl)oxy]-2-methylpropanoic acid

A solution of 175 mg (0.35mmol) of **intermediate 82** in 6 mL of ethanol and 6 mL of NaOH (2N) was stirred at rt overnight. Then ethanol was evaporated, water was added to the resulting solution. HCl 5N was added to precipitate the acid which was filtered, washed with water and dried to give a white powder (142mg).
Yield: 86%
Mp: 96°C, becomes gummy
LC-TOF MH+exp= 474.2057 MH+calc= 474.2029 δ=5.9 ppm
¹H NMR (CDCl₃) d: 7.69 (d, 2H); 7.45 (s, 1 H); 7.36 (d, 2H); 7.26 (m, 1 H); 7.10 (d, 1 H); 6.96 (d, 1 H); 6.84 (s, 1 H); 6.70 (d, 1 H); 6.67 (d, 1 H); 6.44 (m, 1 H); 4.45 (d, 2H); 3.82 (s, 3H); 2.16 (s, 3H); 1.55 (s, 6H)

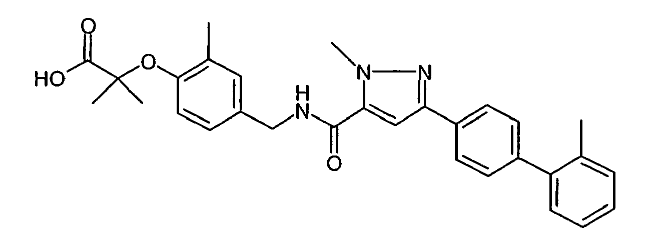

### Example 36

### 2-methyl-2-{2-methyl-4-[({[1-methyl-3-(2'-methylbiphenyl-4-yl)-1H-pyrazol-5-yl]carbonyl}amino)methyl]phenoxy}propanoic acid

To a solution of 200 mg (0.39mmol) of intermediate 64 in 5 mL of toluene was added 22.4 mg of Pd(PPh₃)₄ (0.02mmol) and it was stirred for 10 min. Then an aqueous solution of Na₂CO₃ (2M) and 106 mg (0.8mmol) of 2-methyl phenyl boronic acid diluted in 2.5 mL of ethanol and it was refluxed overnight. Then water was added, it was filtered through celite, washed with water and ethyl acetate and evaporated. The residue was diluted in ethyl acetate, washed with NaHCO₃, water, then dried on Na₂SO₄, evaporated and purified by flash chromatography (CH₂Cl₂ to CH₂Cl₂/ethyl acetate 95/5). A solution of this product in 4 mL of ethanol and 4 mL of NaOH (2N) was stirred at rt overnight. This was filtered, ethanol was evaporated and the resulting solution was diluted in water. Then the aqueous solution was acidified with HCl 5N until pH=1 to form a precipitate which was filtered, washed with water and dried to give a white powder (181 mg).
Yield: 93%
Mp: 90-100°C, becomes gummy
LC-TOF MH+exp= 498.2384 MH+calc= 498.2393 δ=1.8 ppm
¹H NMR (DMSO) δ: 8.88 (m, NH); 7.68 (d, 2H); 7.28 (d, 2H); 7.23 (s, 1H); 7.18-7.09 (m, 4H); 7.01 (s,1H); 6.94 (d, 1 H); 6.54 (d, 1 H); 4.23 (d, 2H); 4.00 (s, 1 H); 2.13 (s, 3H); 2.02 (s, 3H); 1.37 (s, 6H)

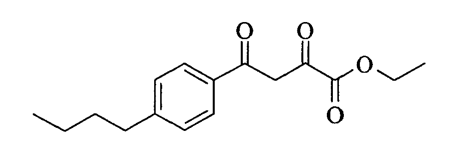

### Intermediate 83

### ethyl 4-(4-butylphenyl)-2,4-dioxobutanoate

1.44 g (62.6mmol) of sodium were dissolved in a solution of 115 mL of ethanol under nitrogen. The solution was cooled to 0-5°C.
Then 10g (56.7mmol) of **1-(4-butylphenyl)ethanone** diluted in 20 mL of ethanol was added dropwise and it was stirred 30 min. A 8.5 mL (62.6mmol) of diethyl ethanedioate diluted in 15 mL of ethanol was added dropwise and the reaction mixture was stirred at rt for 20h30. Then it was evaporated, diluted in ethyl acetate, washed twice with HCl 1N, water and once with brine, dried on MgSO₄ and evaporated to give orange oil (14.37g).
Yield: 92%
The spectrum is keto-enol equilibrium
¹H NMR (DMSO= 2.50) δ: 8.02-7.86 (2d, 2H); 7.24-7.35 (2d, 2H); 7.11 (s, 0.7 H enol form); 4.36-4.18 (2 quad, 2H); 2.68 (t, 2H); 2.55 (s, 0.2H ketone form); 1.58 (m, 2H); 1.34 (m, 5H); 0.9 (t, 3H)

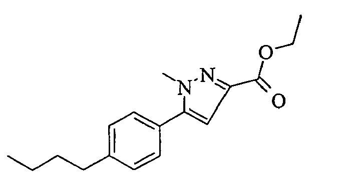

### Intermediate 84

### ethyl 5-(4-butylphenyl)-1-methyl-1H-pyrazole-3-carboxylate

To a solution of 14.37 g (52mmol) of **intermediate 83** in 150 mL of ethanol was added 8.3 mL (156mmol) of methyl hydrazine and it was refluxed for 19h.
The resulting mixture was evaporated, diluted in ethyl acetate and water, washed with HCl 1 N, water and brine, dried on MgSO₄, evaporated and purified by flash chromatography (cyclohexane/ethyl acetate 80/20 to 70/30) to give the desired product as the second fraction collected (yellow oil 3.3g).
Yield: 22%
¹H NMR (DMSO=2.50) δ: 7.48 (d, 2H); 7.34 (d, 2H); 6.85 (s, 1H); 4.28 (quad, 2H); 3.91 (s, 3H); 2.64 (t, 2H); 1.59 (m, 2H); 1.40-1.28 (t+m, 5H); 0.92 (t, 3H)

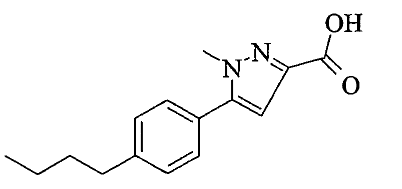

### Intermediate 85

### 5-(4-butylphenyl)-1-methyl-1H-pyrazole-3-carboxylic acid

A solution of 3.3g (11.5mmol) of **intermediate 84** in 50 mL of ethanol and 23 mL of NaOH 1 N was heated at 80°C for 2h15. Then it was evaporated, diluted in water, acidified with HCI 1 N until pH=1, a chewing-gum like residue was separated from the aqueous layer, dissolved in CH₂Cl₂, dried on MgSO₄ and evaporated to give a cream solid (2.80g).
Yield : 94%
¹H NMR (DMSO) δ : 7.47 (d, 2H) ; 7.33 (d, 2H) ; 6.76 (s, 1 H) ; 3.88 (s, 3H) ; 2.64 (t, 2H) ; 1.59 (m, 2H) ; 1.33 (m, 2H) ; 0.90 (t, 3H)

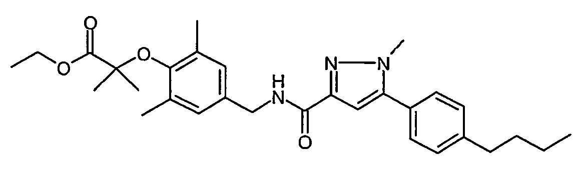

### Intermediate 86

### ethyl 2-{4-[({[5-(4-butylphenyl)-1-methyl-1H-pyrazol-3-yl]carbonyl}amino)methyl]-2,6-dimethylphenoxy}-2-methylpropanoate

A solution of 43 mg (0.166mmol) of **intermediate 85** in 3 mL of toluene with 60µL (0.83mmol) of SOCl₂ was heated at 110°C for 3h. Then 60µL of SOCl₂ were added, it was heated for 1h, but no further evolution was observed. The resulting mixture was evaporated, diluted in 10 mL of toluene and evaporated twice.
The residue was diluted in 1 mL of CH₂Cl₂ was added at 0° under nitrogen to a solution of 50 mg (0.166mmol) of **intermediate 68** in 2 mL of CH₂Cl₂ and 62 µL (0.45mmol) of Et₃N. The reaction mixture was stirred at this temperature for 2h. It was washed with NaHCO₃, HCI 1 N and brine. The organic layer was dried on Na₂SO₄ and evaporated (63mg).
Yield: 75%
¹H NMR (CDCl₃) δ: 7.25 (d, 2H); 7.21 (d, 2H); 7.07 (m, NH); 6.89 (s, 2H); 6.79 (s, 1 H); 4.45 (d, 2H); 4.22 (quad, 2H); 3.80 (s, 3H); 2.59 (t, 2H); 2.12 (s, 6H); 1.57 (m, 2H); 1.39 (s, 6H); 1.35-1.25 (m, 5H); 0.88 (t, 3H)

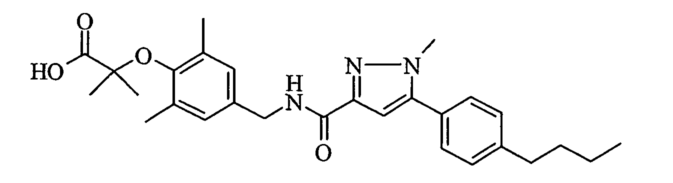

### Example 37

### 2-{4-[({[5-(4-butylphenyl)-1-methyl-1H-pyrazol-3-yl]carbonyl}amino)methyl]-2,6-dimethylphenoxy}-2-methylpropanoic acid

A solution of 63 mg (0.12mmol) of **intermediate 86** in 4 mL of ethanol and 4 mL of NaOH 2N was stirred at rt overnight. It was then evaporated, diluted in water and acidified with HCI 1 N until pH=1. The precipitate formed was filtered and dried under vacuum to give a light brown powder (41 mg).
Yield: 69%
Mp: 72°C, becomes gummy
LC-TOF MH+exp= 478.2708 MH+calc=478.2706 δ=0.4 ppm
¹H NMR (CDCl₃) δ: 7.25 (d, 2H); 7.21 (d, 2H); 7.14 (m, NH); 6.94 (s, 2H); 6.79 (s, 1 H); 4.46 (d, 2H); 3.81 (s, 3H); 2.60 (t, 2H); 2.15 (s, 6H); 1.57 (m, 2H); 1.44 (s, 6H); 1.32 (m, 2H); 0.88 (t, 3H)

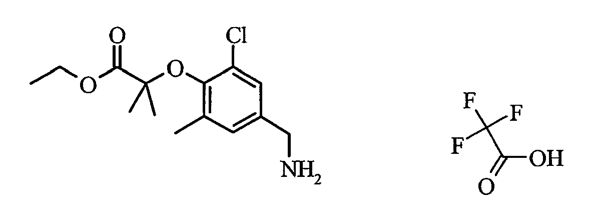

### Intermediate 87

### ethyl 2-[4-(aminomethyl)-2-chloro-6-methylphenoxy]-2-methylpropanoate trifluoroacetic salt

To a solution of 4.43 g (11.48mmol) of intermediate 74 in 45 mL of CH₂Cl₂ at 0°C was added 3.54 mL (46mmol) of trifluoroacetic acid and the reaction mixture was stirred at rt for 28h. Then 2eq of trifluoroacetic acid was added and it was stirred for 24h.
The resulting mixture was evaporated to dryness, diluted and evaporated with toluene three times to give white cottony solid (4.08g).
Yield: 89%
¹H NMR (DMSO) δ: 7.43 (s, 1H); 7.28 (s, 1H); 4.18 (quad, 2H); 3.97 (s, 2H); 2.20 (s, 3H); 1.44 (s, 6H); 1.25 (t, 3H)

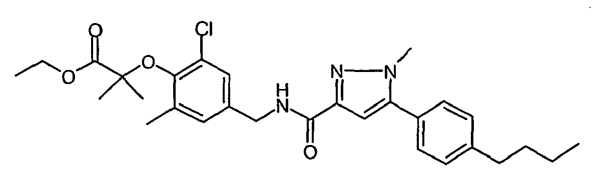

### Intermediate 88

### ethyl 2-{4-[({[5-(4-butylphenyl)-1-methyl-1H-pyrazol-3-yl]carbonyl}amino)methyl]-2-chloro-6-methylphenoxy}-2-methylpropanoate

A solution of 50 mg (0.193mmol) of **intermediate 85** in 3.5 mL of toluene with 70µL (0.965mmol) of SOCl₂ was heated at 110°C for 3h. The solution was co-evaporated with toluene three times. The residue diluted in 1.5 mL of CH₂CI₂ was added at 0°C under nitrogen to a solution of 78 mg (0.193mmol) of **intermediate 87** with 70µL (0.521mmol) of Et₃N. The resulting mixture was stirred for 2h, washed with NaHCO₃, HCI (1 N), brine, dried on Na₂SO₄ and evaporated (92mg).
Yield: 90%
¹H NMR (CDCl₃) δ: 7.26 (d, 2H); 7.21 (d, 2H); 7.13 (m, NH); 7.11 (s, 1H); 6.98 (s, 1H); 6.79 (s, 1 H); 4.46 (d, 2H); 4.22 (quad, 2H); 3.81 (s, 3H); 2.60 (t, 2H); 2.16 (s, 3H); 1.57 (m, 2H); 1.45 (s, 6H); 1.24-1.37 (m, 2H; t, 3H); 0.88 (t, 3H)

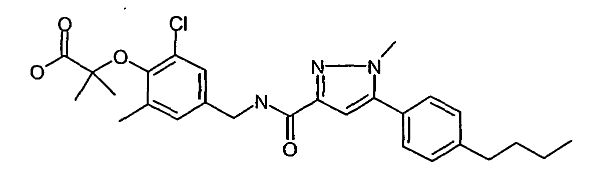

### Example 38

### 2-{4-[({[5-(4-butylphenyl)-1-methyl-1H-pyrazol-3-yl]carbonyl}amino)methyl]-2-chloro-6-methylphenoxy}-2-methylpropanoic acid

A solution of 92 mg (0.17mmol) of intermediate 88 in 5 mL of ethanol and 8 mL of NaOH (2N) was stirred at rt overnight then heated at 80°C for 2h30.Then it was evaporated, diluted in water, acidified with HCl 1 N until pH=1, extracted with ethyl acetate and evaporated (22mg). The residue was purified by flash chromatography (CH₂Cl₂/MeOH 95/5).
Yield: 25%
Mp: 74°C, becomes gummy
LC-TOF MH+exp= 498.2162 MH+calc= 498.2159 δ=0.6 ppm
¹H NMR (CDCl₃) δ: 7.28-7.19 (m, 4H); 7.16 (s, 1H); 7.04 (s, 1H); 6.79 (s, 1H); 4.48 (d, 2H); 3.82 (s, 3H); 2.60 (t, 2H); 2.21 (s, 3H); 1.56 (m, 2H); 1.51 (s, 6H); 1.31 (m, 2H); 0.88 (t, 3H)

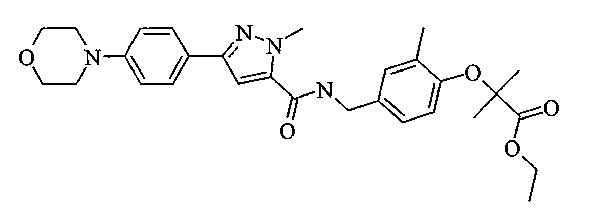

### Intermediate 89

### Ethyl 2-methyl-2-[(2-methyl-4-{[({1-methyl-3-[4-(4-morpholinyl)phenyl]-1H-pyrazol-5-yl}carbonyl)amino]methyl}phenyl)oxy]propanoate

In a tube was added successively BINAP (47 mg, 0.075mmol), Pd₂(dba)₃ (23 mg, 0.025mmol), Cs₂CO₃ (228 mg, 0.7mmol), **intermediate 64** (257 mg, 0.5 mmol), 2 mL of DME and morpholine (1.5- 3 eq). The reaction mixture was stirred under nitrogen at 100°C for 16h, then filtered through celite with CH₂Cl₂ and evaporated to dryness (80mg).
Yield = 30%
¹H NMR (CDCl₃) d: 7.60 (d, 2H); 7.06 (s, 1H); 6.95 (d, 1H); 6.86 (d, 2H); 6.60 (s, 1H); 6.57 (d, 1 H); 6.15 (m, NH); 4.42 (d, 2H); 4.23-4.13 (quad, 2H; s, 3H); 3.80 (m, 4H); 3.12 (m, 4H); 2.17 (s, 3H); 1.53 (s, 6H); 1.20 (t, 3H)

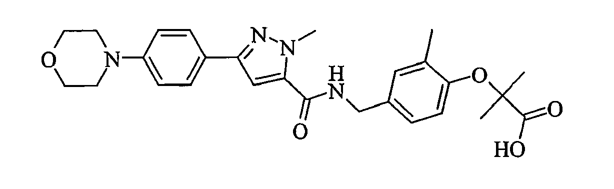

### Example 39

### 2-methyl-2-[(2-methyl-4-{[({1-methyl-3-[4-(4-morpholinyl)phenyl]-1H-pyrazol-5-yl}carbonyl)amino]methyl}phenyl)oxy]propanoic acid

A solution of 50 mg (0.15mmol) of **intermediate 89** in 10 mL of methanol and 770 µL of NaOH (1 N) was heated at 90°C for 2h30. After cooling at rt, the resulting mixture was acidified with 1.6 mL of HCl (1N), then it was extracted with ethyl acetate, washed with water, dried on Na₂SO₄, evaporated to dryness and purified by flash chromatography (CH₂CI₂/methanol 95/5) to give an off-white powder (60mg).
Yield: 81%
¹H NMR (CDCI₃) δ: 7.59 (d, 2H); 7.08 (s, 1 H); 6.99 (d, 1 H); 6.87 (d, 2H); 6.71 (d, 1 H); 6.62 (s, 1 H); 6.28 (m, NH); 4.43 (d, 2H); 4.14 (s, 3H); 3.81 (m, 4H); 3.13 (m, 4H); 2.17 (s, 3H); 1.55 (s, 6H)

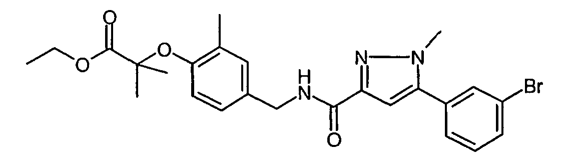

### Intermediate 90

### Ethyl-2-({4-[({[5-(3-bromophenyl)-1-methyl-1H-pyrazol-3-yl]carbonyl}amino) methyl]-2-methylphenyl}oxy)-2-methylpropanoate

To a suspension of 2.2 g (7.8mmol) of **intermediate 157** in 100 mL of toluene and 5 mL (68mmol) of SOCl₂ was heated at 120 °C for 3h. SOCl₂ was co evaporated twice with toluene. The residue was dissolved in 50 mL of CH₂Cl₂ and added dropwise to a solution of 2.24 g (7.8mmol) of **intermediate 10** in 150 mL of CH₂Cl₂ anhydrous and 3.27 mL (23.4mmol) of Et₃N. The reaction mixture was stirred at rt for 16h, then the solution washed with HCl (1N), NaOH (1 N), brine and dried on MgSO₄ to give a brown oil (4.02g).
Quantitative yield
¹H NMR (CDCl₃=) δ: 7.59 (d, 2H); 7.37 (d, 2H); 7.17 (s, 1H); 7.12 (m, NH); 7.05 (d, 1H); 6.89 (s, 1 H); 6.64 (d, 1 H); 4.54 (d, 2H); 4.27 (quad, 2); 3.88 (s, 3H); 2.24 (s, 3H); 1.60 (s, 6H); 1.28 (t, 3H)

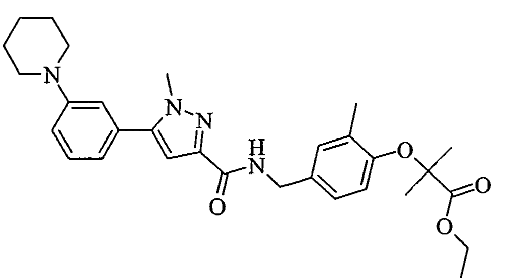

### Intermediate 91

### Ethyl-2-methyl-2-[(2-methyl-4-{[({1-methyl-5-[3-(1-piperidinyl)phenyl]-1H-pyrazol-3-yl}carbonyl)amino]methyl}phenyl)oxy]propanoate

In a tube was added successively BINAP (47 mg, 0.075mmol), Pd₂(dba)₃ (23 mg, 0.025mmol), Cs₂CO₃ (228 mg, 0.7mmol), the **intermediate 90** (257 mg, 0.5 mmol), 2 mL of DME and piperidine (1.5- 3 eq). The reaction mixture was stirred under nitrogen at 100°C for 16h, then filtered through celite with CH₂CI₂ , evaporated to dryness and purified by flash chromatography (CH₂Cl₂/ethyl acetate 95/5) to give 110mg of yellow solid.
Yield = 42%
¹H NMR (CDCl₃) δ: 7.24 (t, 1 H); 7.08 (s, 1 H); 7.03 (m, 1 H); 6.97 (d, 1 H); 6.91 (d, 1 H); 6.85 (m, 1 H); 6.78 (s, 1 H); 6.74 (d, 1 H); 6.55 (d, 1 H); 4.45 (d, 2H); 4.47 (quad, 2H); 3.78 (s, 3H); 3.13 (m, 4H); 2.15 (s, 3H); 1.64 (m, 4H); 1.51 (s, 6H); 1.51 (m, 2H); 1.19 (t, 3H)

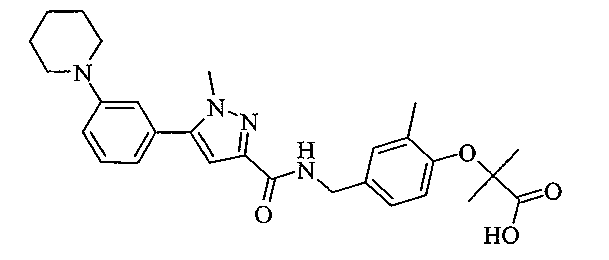

### Example 40

### 2-methyl-2-[(2-methyl-4-{[({1-methyl-5-[3-(1-piperidinyl)phenyl]-1H-pyrazol-3-yl}carbonyl)amino]methyl}phenyl)oxy]propanoic acid

A solution of 110 mg (0.21 mmol) of **intermediate 91** in 5 mL of methanol and 2.1 mL of NaOH (1N) was heated at 90°C for 2h30. After cooling at rt, the resulting mixture was acidified with 2.1 mL of HCl (1 N), then it was extracted with 50 mL of ethyl acetate, washed with brine, dried on Na2SO4, evaporated to dryness and purified by flash chromatography (CH₂Cl₂/methanol 95/5) to give after recrystallisation in toluene a white powder (60mg).
Yield: 58%
¹H NMR (CDCl₃) δ: 7.27 (m, 2H); 7.10 (m, 2H); 6.93 (d, 2H); 6.80 (s, 1H); 6.70 (d, 1H); 4.44 (d, 2H); 3.78 (s, 3H); 3.14 (m, 4H); 2.16 (s, 3H); 1.65 (m, 4H); 1.54 (s, 8H)

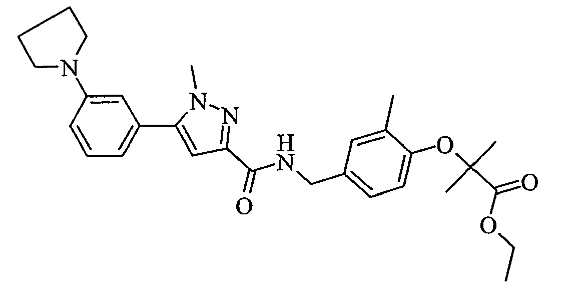

### Intermediate 92

### Ethyl-2-methyl-2-[(2-methyl-4-{[({1-methyl-5-[3-(1-pyrrolidinyl)phenyl]-1H-pyrazol-3-yl}carbonyl)amino]methyl}phenyl)oxy]propanoate

In a tube was added successively BINAP (47 mg, 0.075mmol), Pd₂(dba)₃ (23 mg, 0.025mmol), Cs₂CO₃ (228 mg, 0.7mmol), the **intermediate 90** (257 mg, 0.5 mmol), 2 mL of DME and pyrrolidine (1.5- 3 eq). The reaction mixture was stirred under nitrogen at 100°C for 16h, then filtered through celite with CH₂Cl₂, evaporated to dryness and purified by flash chromatography (CH₂Cl₂/ethyl acetate 95/5) (210mg).
Yield = 83%
1 H NMR (CDCl3) □: 7.20 (d, 1 H); 7.10-7.01 (m, 2H); 6.97 (d, 1 H); 6.79 (s, 1 H); 6.61-6.50 (m, 3H); 6.48 (s, 1 H); 4.45 (d, 2H); 4.17 (quad, 2H); 3.80 (s, 3H); 3.22 (m, 4H); 2.15 (s, 3H); 1.95 (m, 4H); 1.51 (s, 6H); 1.19 (t, 3H)

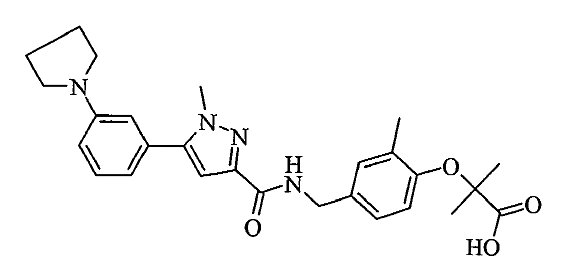

### Example 41

### 2-methyl-2-[(2-methyl-4-{[({1-methyl-5-[3-(1-pyrrolidinyl)phenyl]-1H-pyrazol-3-yl}carbonyl)amino]methyl}phenyl)oxy]propanoic acid

A solution of 210 mg (0.41mmol) of **intermediate 92** in 10 mL of methanol and 4.1 mL of NaOH (1 N) was heated at 90°C for 2h30. After cooling at rt, the resulting mixture was acidified with 4.1 mL of HCl (1 N), then it was extracted with ethyl acetate, washed with brine, dried on Na₂SO₄, evaporated to dryness and purified by flash chromatography (CH₂Cl₂/methanol 95/5). The resulting solid was re-crystallised in toluene/hexane to give a white powder (50mg).
Yield: 26%
¹H NMR (CDCl₃) δ: 720-7.12 (m, 2H); 7.04 (s, 2H); 6.91 (d, 1H); 6.72 (s, 1H); 6.64 (d, 1 H); 6.60-6.46 (m, 2H); 4.38 (d, 2H); 3.73 (s, 3H); 3.18 (m, 4H); 2.08 (s, 3H); 1.90 (m, 4H); 1.46 (s, 6H)

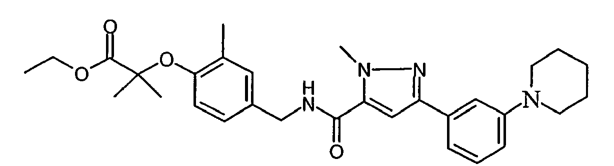

### Intermediate 93

### ethyl 2-methyl-2-{2-methyl-4-[({[1-methyl-3-(3-piperidin-1-ylphenyl)-1H-pyrazol-5-yl]carbonyl}amino)methyl]phenoxy}propanoate

In a tube was added successively BINAP (47 mg, 0.075mmol), Pd₂(dba)₃ (23 mg, 0. 025mmol), Cs₂CO₃ (228 mg, 0.7mmol), the **intermediate 62** (257 mg, 0.5 mmol), 2 mL of DME and piperidine (1.5- 3 eq). The reaction mixture was stirred under nitrogen at 100°C for 16h, then filtered through celite with CH₂Cl₂, evaporated to dryness and purified by flash chromatography (CH₂Cl₂/ethyl acetate 90/10) (75mg).
Yield = 29%
¹H NMR (CDCl₃) δ: 7.29 (s, 1 H); 7.16 (d, 1 H); 7.09 (d, 1 H); 7.06 (s, 1 H); 6.96 (d, 1 H); 6.83 (d, 1 H); 6.66 (s, 1 H); 6.57 (d, 1 H); 6.20 (s, NH); 4.41 (d, 2H); 4.22-4.13 (quad, 2H; s, 3H); 3.13 (m, 4H); 2.16 (s, 3H); 1.64 (m, 4H); 1.52 (s, 6H); 1.50 (m, 2H); 1.19 (t, 3H)

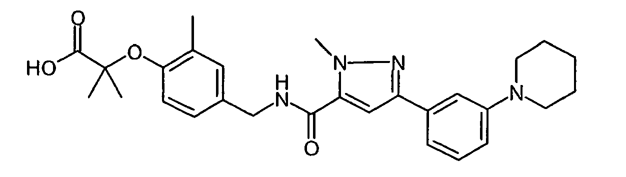

### Example 42

### 2-methyl-2-{2-methyl-4-[({[1-methyl-3-(3-piperidin-1-ylphenyl)-1H-pyrazol-5-yl]carbonyl}amino)methyl]phenoxy}propanoic acid

A solution of 70 mg (0.135mmol) of **intermediate 93** in 5 mL of methanol and 1.35 mL of NaOH (1 N) was heated at 90°C for 3h. After cooling at rt, the resulting mixture was acidified with 1.35 mL of HCl (1N), then it was extracted with ethyl acetate, washed with brine, dried on Na₂SO₄ and evaporated. The resulting solid was re-crystallised in toluene and washed with hexane to give a cream solid (35mg).
Yield: 53%
LCTOF MH+theo=491.2658 MH+ exp= 491.2620 δ=-7.8ppm
¹H NMR (CDCI₃) δ: 7.53 (s, 1 H); 7.28-7.20 (m, 2H); 7.03 (s, 1 H); 6.95 (d, 1 H); 6.86 (d, 1H); 6.78 (s, 1H); 6.62 (d, 1H); 4.35 (d, 2H); 4.14 (s, 3H); 3.17 (m, 4H); 2.13 (s, 3H); 1.74 (m, 4H); 1.53 (s, 6H); 1.53 (m, 2H)

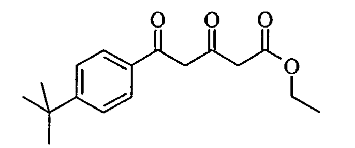

### Intermediate 94

### ethyl 5-[4-(1,1-dimethylethyl)phenyl]-3.5-dioxopentanoate

To a suspension of 2.2 g (0.055mol) of NaH (previously washed twice with petroleum ether) in anhydrous THF was added dropwise at 0°C 6.4 mL (0.05mol) of ethyl 3-oxobutanoate, and the resulting mixture was stirred at this temperature for 20 min. Then 34.4 mL (0.055mol) of BuLi (1.6M in hexanes) were added dropwise at 0°C and it was stirred for 10 min. After that, a solution of 11 g (0.05mol) of 4-(1,1-dimethylethyl)-N-methyl-N-(methyloxy)benzamide in 30 mL of anhydrous THF was added dropwise. The resulting solution was stirred at rt for 2h, quenched with HCl (1 N) until pH was acid, extracted with ethyl acetate, washed with brine, dried on Na₂SO₄ and concentrated to dryness to give an orange oil (14.85g).
Quantitative yield
The spectrum is keto-enol equilibrium
¹H NMR (CDCl₃) δ: 7.75 (d, 2H); 7.4(d, 2H); 6.2 (s, 1H); 4.16 (quad, 2H); 3.40 (s, 2H); 1.25 (s, 9H); 1.22 (t, 3H)

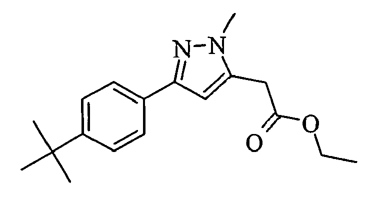

### Intermediate 95

### ethyl {3-[4-(1,1-dimethylethyl)phenyl]-1-methyl-1H-pyrazol-5-yl}acetate

To a solution of 14.85 g (50mmol) of **intermediate 94** in 150 mL of ethanol was added 3.2 mL (60mmol) of methyl hydrazine, and it was stirred at 70°C for 2h. The resulting mixture was concentrated, diluted in ethyl acetate, washed twice with HCl (1 N) and once with brine, dried on Na₂SO₄ and concentrated. The residue was purified by flash chromatography
(CH₂Cl₂ 100%) (6g).
Yield: 40%
¹H NMR (CDCl₃) δ: 7.71 (d, 2H); 7.42 (d, 2H); 6.48 (s, 1H); 4.22 (quad, 2H); 3.89 (s, 3H); 3.72 (s, 2H); 1.35 (s, 9H); 1.30 (t, 3H)

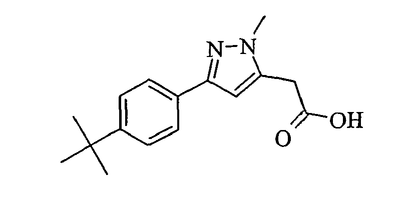

### Intermediate 96

### {3-[4-(1,1-dimethylethyl)phenyl]-1-methyl-1H-pyrazol-5-yl}acetic acid

A solution of 6 g (20mmol) of **intermediate 95** in 100 mL of methanol and 100 mL of NaOH (1 N) was heated at 70°C for 4h. The resulting mixture was evaporated, acidified with HCl (1 N), extracted with ethyl acetate, washed with brine, dried on Na₂SO₄, evaporated to dryness to give an off-white powder (5g).
Yield : 92%
¹H NMR (CDCl₃) δ: 7.45 (d, 2H); 7.18 (d, 2H); 6.27 (s, 1H); 3.66 (s, 3H); 3.53 (s, 2H); 1.10 (s, 9H)

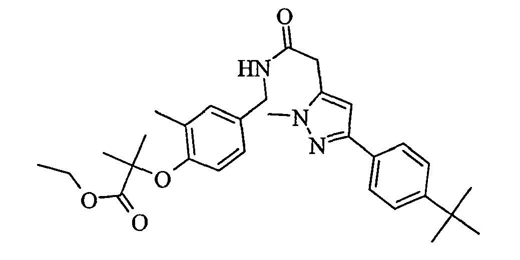

### Intermediate 97

### Ethyl-2-[(4-{[({3-[4-(1,1-dimethylethyl)phenyl]-1-methyl-1H-pyrazol-5-yl}acetyl)amino]methyl}-2-methylphenyl)oxy]-2-methylpropanoate

To a solution of 299 mg (1.1mmol) of **intermediate 96** in 5 mL of DMF was added 149 mg (1.1mmol) HOBT, 210 mg (1.1mmol) of EDCl, 288 mg (1mmol) of **intermediate 10** and 280µL (2mmol) of Et₃N and it was stirred at rt for 16h.

Then DMF was evaporated, the residue was diluted in ethyl acetate, washed with HCI (1 N), NaHCO₃, brine, dried on Na₂SO₄, concentrated to dryness and purified by flash chromatography (CH₂Cl₂/AcOEt 80/20) to give a colourless oil (380mg).
Yield: 75%
¹H NMR (CDCl₃) δ: 7.59 (d, 2H); 7.33 (d, 2H); 6.91 (s, 1 H); 6.81 (dd, 1 H); 6.50 (d, 1 H); 6.36 (s, 1 H); 5.78 (m, NH); 4.23 (d, 2H); 4.15 (quad, 2H); 3.73 (s, 3H); 3.58 (s, 2H); 2.11 (s, 3H); 1.49 (s, 6H); 1.26 (s, 9H); 1.16 (t, 3H);

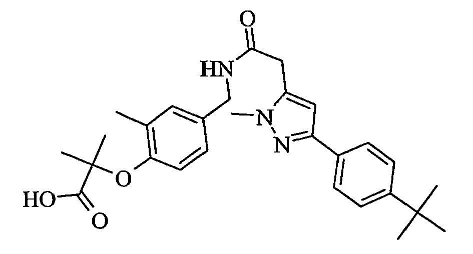

### Example 43

### 2-[(4-{[({3-[4-(1,1-dimethylethyl)phenyl]-1-methyl-1H-pyrazol-5-yl}acethyl)amino]methyl}-2-methylphenyl)oxy]-2-methylpropanoic acid

A solution of 370 mg (0.73mmol) of **intermediate 97** in 10 mL of ethanol and 7.3 mL of NaOH (1N) was stirred at 70°C for 2h. The resulting mixture was evaporated, diluted in 20 mL of water, acidified with a dropwise addition of HCl (1 N) until pH was acid to give a white precipitate which was filtered and dried under vaccum (285mg).
Yield: 82%
¹H NMR (CDCl₃) δ: 7.57 (d, 2H); 7.32 (d, 2H); 6.92 (s, 1H); 6.78 (d, 1H); 6.61 (d, 1H); 6.35 (s, 1 H); 6.09 (m, NH); 4.21 (d, 2H); 3.66 (s, 3H); 3.58 (s, 2H); 2.11 (s, 3H); 1.52 (s, 6H); 1.25(s,9H)

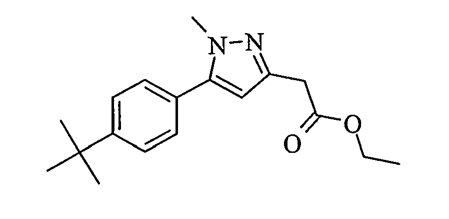

### Intermediate 98

### ethyl {5-[4-(1.1-dimethylethyl)phenyl]-1-methyl-1H-pyrazol-3-yl}acetate

To a solution of 14.85 g (50mmol) of **intermediate 94** in 150 mL of ethanol was added 3.2 mL (60mmol) of methyl hydrazine, and it was stirred at 70°C for 2h. The resulting mixture was concentrated, diluted in ethyl acetate, washed twice with HCI (1 N) and once with brine, dried on Na₂SO₄ and concentrated. The residue was purified by flash chromatography (CH₂Cl₂ 100% to CH₂Cl₂/AcOEt 95/5) to give the title compound as the second fraction (1.1g).
Yield: 7%
¹H NMR (CDCl₃) δ: 7.40 (d, 2H); 7.28 (d, 2H); 6.19 (s, 1H); 4.13 (quad, 2H); 3.78 (s, 3H); 3.62 (s, 2H); 1.29 (s, 9H); 1.21 (t, 3H)

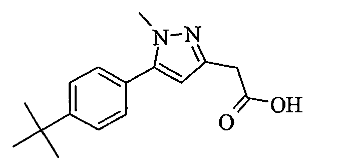

### Intermediate 99

### {5-[4-(1.1-dimethylethyl)phenyl]-1-methyl-1H-pyrazol-3-yl}acetic acid

A solution of 1 g (3.3mmol) of **intermediate 98** in 100 mL of methanol and 33 mL of NaOH (1N) was heated at 70°C for 4h. The resulting mixture was evaporated, acidified with HCl (1N), extracted with ethyl acetate, washed with brine, dried on Na₂SO₄, evaporated to dryness and triturated in heptane to give an off-white powder (0.85g).
Yield : 95%
¹H NMR (CDCl₃) δ: 7.41 (d, 2H); 7.27 (d, 2H); 6.18 (s, 1H); 3.80 (s, 3H); 3.71 (s, 2H); 1.29 (s, 9H)

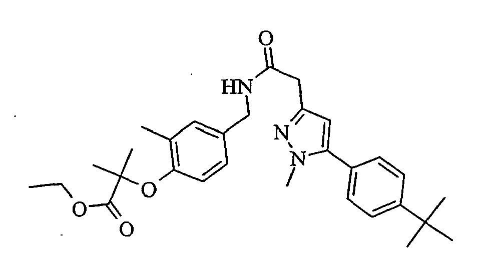

### Intermediate 100

### Ethyl-2-[(4-{[({5-[4-(1,1-dimethylethyl)phenyl]-1-methyl-1H-pyrazol-3-yl}acetyl)amino]methyl}-2-methylphenyl)oxy]-2-methylpropanoate

To a solution of 299 mg (1.1 mmol) of **intermediate 99** in 5 mL of DMF was added 148.5 mg (1.1mmol) HOBT, 210 mg (1.1mmol) of EDCI, 288 mg (1mmol) of **intermediate 10** and 280µL (2mmol) of Et3N and it was stirred at rt for 16h.
Then DMF was evaporated, the residue was diluted in ethyl acetate, washed with HCl (1 N), NaHCO₃, brine, dried on Na₂SO₄, concentrated to dryness and purified by flash chromatography (CH₂Cl₂/AcOEt 65/35) to give colourless oil (310mg).
Yield: 61 %
¹H NMR (CDCl₃) δ: 7.40 (d, 2H); 7.26 (d, 2H); 6.96 (s, 1 H); 6.86 (dd, 1 H); 6.71 (m, NH); 6.52 (d, 1 H); 6.13 (s, 1 H); 4.30 (d, 2H); 4.16 (quad, 2H); 3.76 (s, 3H); 3.59 (s, 2H); 2.13 (s, 3H); 1.50 (s, 6H); 1.29 (s, 9H); 1.18 (t, 3H)

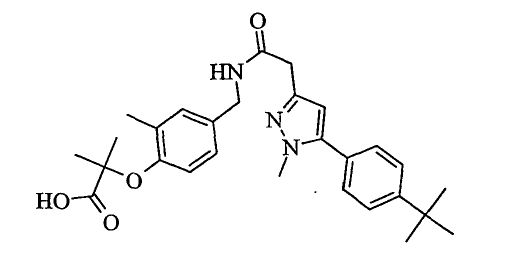

### Example 44

### 2-[(4-{[({5-[4-(1,1-dimethylethyl)phenyl]-1-methyl-1H-pyrazol-3-yl}acetyl)amino]methyl}-2-methylphenyl)oxy]-2-methylpropanoic acid

A solution of 300 mg (0.59mmol) of **intermediate 100** in 10 mL of ethanol and 5.9 mL of NaOH (1 N) was stirred at 70°C for 2h. The resulting mixture was evaporated, diluted in 20 mL of water, acidified with a dropwise addition of HCl (1 N) until pH was acid to give a white precipitate which was filtered and dried under vacuum (m=240mg).
Yield: 85%
¹H NMR (CDCI₃) δ: 7.25 (d, 2H); 7.09 (d, 2H); 6.93 (m, 1 H); 6.80 (s, 1 H); 6.63 (d, 1 H); 6.45 (d, 1 H); 6.05 (s, 1 H); 4.09 (d, 2H); 3.58 (s, 3H); 3.41 (s, 2H); 1.96 (s, 3H); 1.35 (s, 6H); 1.13 (s, 9H)

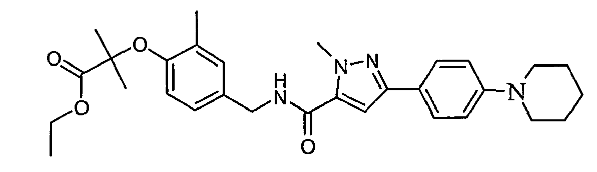

### Intermediate 101

### Ethyl-2-methyl-2-[(2-methyl-4-{[({1-methyl-3-[4-(1-piperidinyl)phenyl]-1H-pyrazol-5-yl}carbonyl)amino]methyl}phenyl)oxy]propanoate

In a tube was added successively BINAP (47 mg, 0.075mmol), Pd₂(dba)₃ (23 mg, 0.025mmol),Cs₂CO₃ (228 mg, 0.7mmol), **intermediate 64** (257 mg, 0.5 mmol), 2 mL of DME and piperidine (2eq). The reaction mixture was stirred under nitrogen at 100°C for 16h, then filtered through celite, extracted with ethyl acetate, washed with brine, dried on Na₂SO₄ and purified on silica gel-a light degradation was observed (m=90mg).
Yield = 35%
¹H NMR (CDCl₃) δ: 7.56 (d, 2H); 7.05 (s, 1 H); 6.98-6.78 (m, 3H); 6.58 (s, 1 H); 6.56 (d, 1 H); 6.14 (m, NH); 4.41 (d, 2H); 4.17 (quad, 2H); 4.14 (s, 3H); 3.12 (m, 4H); 2.16 (s, 3H); 1.64 (m, 4H); 1.52 (s, 6H); 1.49 (m, 2H); 1.19 (t, 3H)

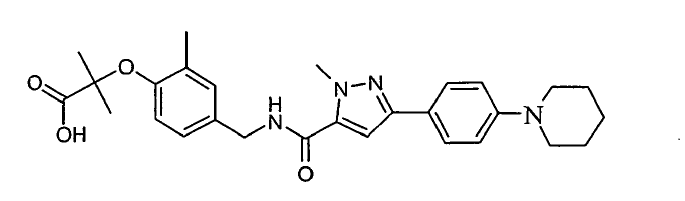

### Example 45

### 2-methyl-2-[(2-methyl-4-{[({1-methyl-3-[4-(1-piperidinyl)phenyl]-1H-pyrazol-5-yl}carbonyl)amino]methyl}phenyl)oxy]propanoic acid

A solution of 85 mg (0.164mmol) of **intermediate 101** in 3 mL of methanol and 1.65 mL of NaOH (1 N) was heated at 70°C for 3h. The methanol was evaporated and it was diluted in 2 mL of water and 1.65 mL of HCl (1N). The resulting precipitate was filtered, dried under vacuum and purified by flash chromatography (CH₂Cl₂/AcOEt 95/5) to give a light pink solid (19mg).
Yield: 24%
LC TOF MH+theo=491.2658 MH+exp=491.2586 δ= -14.7 ppm
¹H NMR (CDCl₃) δ: 7.55 (d, 2H); 7.05 (d, 1H); 6.98-6.91 (m, 3H); 6.97 (s, 1H); 6.65 (d, 1H); 6.55 (m, 1 H); 4.39 (d, 2H); 4.11 (s, 3H); 3.13 (m, 4H); 2.14 (s, 3H); 1.69 (m, 4H); 1.54 (s, 6H +2H)

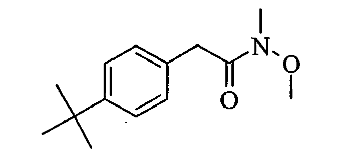

### Intermediate 102

### 2-[4-(1,1-dimethylethyl)phenyl]-N-methyl-N-(methyloxy)acetamide

A solution of 5 g (26mmol) of [4-(1,1-dimethylethyl)phenyl]acetic acid in 40 mL of toluene and 9.5 mL (130mmol) of SOCl₂ was heated at 90°C for 2h. The solution was co-evaporated with toluene. The residue, diluted in 30 mL of CH₂Cl₂, was added dropwise to a solution of 2.79 g (28.6mmol) of N,O-dimethylhydroxylamine hydrochloride and 10.9 mL (78mmol) of Et₃N in CH₂Cl₂. The resulting mixture was stirred at rt for 24h. Then HCI (1 N) was added and the layers were separated. The organic layer was washed with brine, dried on Na₂SO₄, evaporated and purified by flash chromatography (Cyclohexane/AcOEt 75/25) (m=2.95g).
Yield: 48%
¹H NMR (CDCl₃= 7:14) δ: 7.22 (d, 2H); 7.11 (d, 2H); 3.62 (s, 2H); 3.51 (s, 3H); 3.07 (s, 3H); 1.18 (s, 9H)

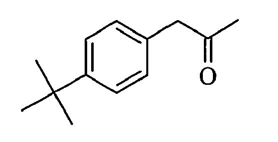

### Intermediate 103

### 1-[4-(1,1-dimethylethyl)phenyl]-2-propanone

To a solution of 2.94 g (12.5mmol) of **intermediate 102** in anhydrous THF at 0°C was added dropwise 19 mL (57mmol) of methyl magnesium bromide (3M in Et₂O), and the solution was stirred at this temperature for 1 h30. The reaction was quenched with a dropwise addition of methanol, then addition of HCl (1N). The organic layer was extracted with ethyl acetate, washed with brine, dried on Na₂SO₄ and evaporated to dryness to give a yellow oil (2.4g).
Quantitative yield
¹H NMR (CDCl₃=7.18) δ: 7.28 (d ,2H); 7.07 (d, 2H); 3.59 (s, 2H); 2.08 (s, 3H); 1.24 (s, 9H)

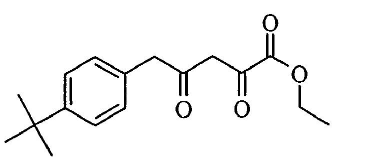

### Intermediate 104

### Ethyl 5-[4-(1,1-dimethylethyl)phenyl]-2,4-dioxopentanoate

At 0°C, 320 mg (13.9mmol) of sodium was added to 30 mL of ethanol and it was stirred until complete solubility of Na. At 0°C, 2.4g (12.6mmol) of **intermediate 103** in 5 mL of ethanol was added via a syringe and the solution was stirred for 1h. At 0°C, 1.89 mL (13.9mmol) of ethyl oxalate in 5 mL of methanol were added.
The resulting mixture was stirred at rt for 16h; then it was quenched with HCI (1 N), extracted with ethyl acetate, washed with HCl (1 N), brine, dried on Na₂SO₄, evaporated and purified by flash chromatography (Cyclohexane/ethyl acetate 90/10) to a yellow oil (1.55g).
Yield: 42%
¹H NMR (CDCl₃=7.19) δ: 7.30 (d, 2H); 7.11 (d, 2H); 4.26 (quad, 2H); 3.68 (s, 2H); 2.09 (s, 2H); 1.28 (t, 3H); 1.24 (s, 9H)

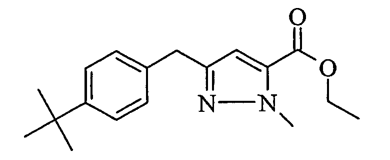

### Intermediate 105

### Ethyl-3-{[4-(1,1-dimethylethyl)phenyl]methyl}-1-methyl-1 H-pyrazole-5-carboxylate

To a solution of 1.52 g (5.24mmol) of **intermediate 104** in 20 mL of ethanol was added 420 µL (7.9mmol) of methyl hydrazine, then it was heated at 90°C for 3h.
The solution was extracted with ethyl acetate, washed with HCl (1 N), brine, dried on Na₂SO₄, evaporated and purified by flash chromatography (cyclohexane/ ethyl acetate 90/10 to 70/30) to give the desired isomer as the first fraction collected (990mg).
Yield: 50%
¹H NMR (CDCl₃= 7.17) of the desired clean product δ: 7.24 (d, 2H); 7.11 (d, 2H); 6.51 (s, 1 H); 4.21 (quad, 2H); 4.05 (s, 3H); 3.85 (s, 2H); 1.25 (t, 3H); 1.22 (s, 9H)

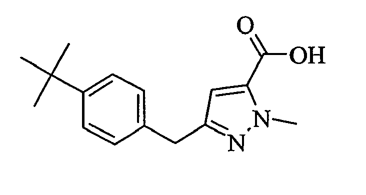

### Intermediate 106

### 3-{[4-(1,1-dimethylethyl)phenyl]methyl}-1-methyl-1H-pyrazote-5-carboxylic acid

A solution of 940 mg (3.1 mmol) of **intermediate 105** in 20 mL of ethanol and 31 mL of NaOH (1 N) was heated at 90°C for 4h. The solution was washed with 5 mL of ethyl acetate. The aqueous layer was concentrated until the volume was reduced to 30 mL, then 10 mL of water was added followed by HCl (1 N) to give a white precipitate which was filtered and dried under vacuum to give a white powder (640mg).
Yield: 76%
¹H NMR (CDCl₃=7.19) δ: 7.25 (d, 2H); 7.12 (d, 2H); 6.62 (s, 1 H); 4.07 (s, 3H); 3.88 (s, 2H); 1.23 (s, 9H)

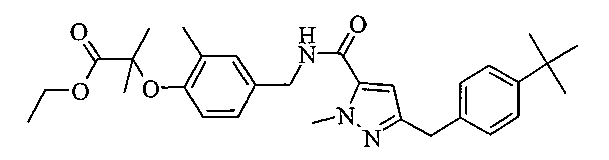

### Intermediate 107

### Ethyl 2-{[4-({[(3-{[4-(1,1-dimethylethyl)phenyl]methyl}-1-methyl-1H-pyrazol-5-yl)carbonyl]amino}methyl)-2-methylphenyl]oxy}-2-methylpropanoate

To a solution of 150 mg (0.55mmol) of **intermediate 106** in 5 mL in DMF, was added 75 mg (0.55mmol) of HOBT, 105 mg (0.55mmol) of EDCI, 155 µL (1.1mmol) of Et₃N and 144 mg (0.5mmol) of **intermediate 10.** The reaction mixture was stirred at rt for 24h. Then the product was extracted with ethyl acetate, washed with HCl (1 N), NaOH (1 N), brine, dried on Na₂SO₄ and evaporated to give a viscous colorless oil which was purified by flash
chromatography (CH₂Cl₂/AcOEt 95/5) (m=120mg).
Yield: 47%
¹H NMR (CDCl₃= 7.19 ) δ: 7.24 (d, 2H); 7.09 (d, 2H); 6.99 (m, 1H); 6.89 (d, 1H); 6.53 (d, 1H); 6.10 (s, 1H); 6.02 (m, NH); 4.35 (d, 2H); 4.17 (quad, 2H); 4.08 (s, 3H); 3.84 (s, 2H); 2.14 (s, 3H); 1.51 (s, 6H); 1.22 (s, 9H) 1.18 (t, 3H)

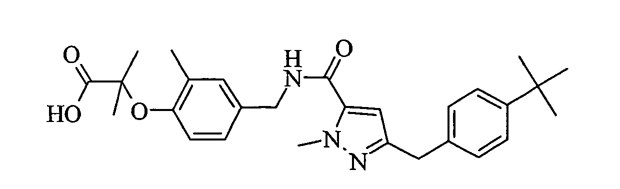

### Example 46

### 2-{[4-({[(3-{[4-(1,1-dimethylethyl)phenyl]methyl}-1-methyl-1H-pyrazol-5-yl)carbonyl]amino}methyl)-2-methylphenyl]oxy}-2-methylpropanoic acid

A solution of 120 mg (0.24mmol) of **intermediate 107** in 5mL of methanol and 2.4 mL of NaOH (1 N) was heated at 90°C for 3h. Then, 5mL of water was added, followed by HCl (1 N) until the pH was acid to form a gum. The product was extracted with ethyl acetate, dried on Na₂SO₄, concentrated, diluted in NaOH (1 N), slowly acidified with HCl(1N) to give a white precipitate which was filtered and dried under vacuum at 50°C (m=100g).
Yield:87%
¹H NMR (CDCl₃= 7.19) δ: 7.23 (d, 2H); 7.09 (d, 2H); 7.02 (s, 1 H); 6.91 (d, 1 H); 6.67 (d, 1 H); 6.13 (s, 1 H + NH); 4.34 (m, 2H); 4.08 (s, 3H); 3.84 (s, 2H); 2.15 (s, 3H); 1.54 (s, 6H); 1.21 (s, 9H)

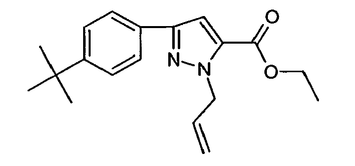

### Intermediate 108

### Ethyl-3-[4-(1,1-dimethylethyl)phenyl]-1-(2-propen-1-yl)-1H-pyrazole-5-carboxylate

To a solution of 500 mg (1.8mmol) of **intermediate 22** in acetone was added 760 mg (3eq) of K₂CO₃ and 320 µL (2eq) of 3-bromo-1-propene. The reaction mixture was heated at 70°C for 18h. The mixture was purified by Flash Master (Cyclohexane/ethyl acetate 70/30 and CH₂Cl₂) to give the desired isomer as the first fraction collected (off-white solid, m=450mg). Yield: 80%
¹H NMR (CDCl₃ = 7.12) δ: 7.64 (d, 2H, J = 8.5 Hz); 7.31 (d, 2H, J = 8.5 Hz); 7.02 (s, 1H); 6.02-5.87 (m, 1 H); 5.11 (m, 2H); 5.08-4.94 (m, 2H), 4.23 (quad, 2H); 1.26 (t, 3H); 1.22 (s, 9H).

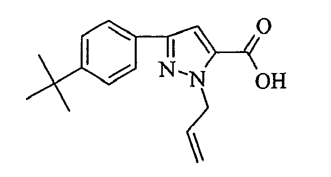

### Intermediate 109

### 3-[4-(1,1-dimethylethyl)phenyl]-1-(2-propen-1-yl)-1H-pyrazole-5-carboxylic acid

A solution of 450 mg (1.44mmol) of **intermediate 108** in ethanol and an excess (5eq) of NaOH (1 N) was heated at 50°C for 4h. The resulting mixture was evaporated, diluted in water and acidified with HCl(1N). The precipitate formed was filtered, washed with water and pentane to give a white solid (440mg).
Quantitative yield
¹H NMR (CDCl₃= 7.13 ) δ: 7.63 (d, 2H, *J* = 8.5 Hz); 7.32 (d, 2H, *J* = 8.5 Hz); 7.12 (s, 1 H); 6.01-5.86 (m, 1 H); 5.11 (m, 2H); 5.09-4.95 (m, 2H); 1.22 (s, 9H)

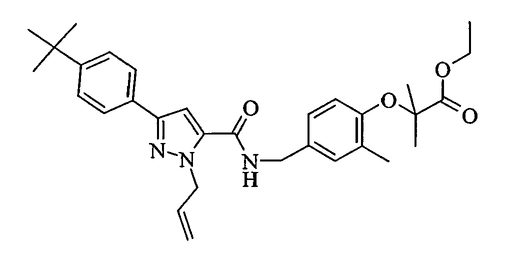

### Intermediate 110

### Ethyl 2-({4-[({[3-[4-(1.1-dimethylethyl)phenyl]-1-(2-propen-1-yl)-1H-pyrazol-5-yl]carbonyl}amino)methyl]-2-methylphenyl}oxy)-2-methylpropanoate

A solution of 220 mg (0.77mmol) of **intermediate 109**, 340 mg (1.5eq) of **intermediate 10**, 440 mg (1.5eq) of HATU and 300 µL (3eq) of Et₃N in CH₂Cl₂ was stirred at rt for 18h. The resulting mixture was washed with HCI (1 N) and water, dried , evaporated and purified by flash master (CH₂Cl₂+ 0.5% of MeOH) to give a white solid (50mg).
Yield:80%
¹H NMR (CDCl₃= 7.18 ) δ: 7.60 (d, 2H, *J* = 8.5 Hz); 7.32 (d, 2H, *J* = 8.5 Hz); 7.02 (d, 1 H, *J* = 1.9 Hz); 6.91 (dd, 1H, *J* = 8.3 Hz, 2.1 Hz); 6.70 (s, 1 H); 6.54 (d, 1H, *J* = 8.3 Hz); 6.37 (m, NH); 5.99 (m, 1H); 5.15 (d, 2H); 5.10-4.97 (m, 2H); 4.38 (d, 2H); 4.16 (quad, 2H); 2.14 (s, 3H); 1.51 (s, 6H); 1.24 (s, 9H); 1.18 (t, 3H)

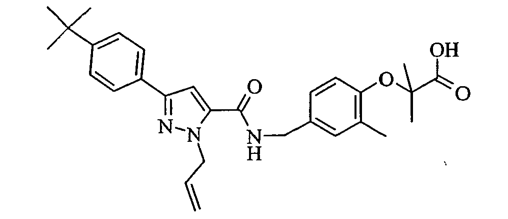

### Example 47

### 2-({4-[({[3-[4-(1,1-dimethylethyl)phenyl]-1-(2-propen-1-yl)-1H-pyrazol-5-yl]carbonyl}amino)methyl]-2-methylphenyl}oxy)-2-methylpropanoic acid

A solution of 340 mg (0.67mmol) of **intermediate 110** in ethanol and an excess (5eq) of NaOH (1 N) was heated at 50°C for 4h. The resulting mixture was evaporated, diluted in water, acidified with HCI (1N). The precipitate formed was filtered, washed with water and pentane and re-crystallized in toluene to give a white solid (130mg).
Yield: 40%
¹H NMR (CDCl₃=7.19) δ: 7.62(d, 2H, J = 8.3 Hz); 7.33 (d, 2H, J = 8.5 Hz); 7.07 (d, 1H, J = 1.7 Hz); 6.98 (dd, 1H, J = 8.5 Hz, 1.9 Hz); 6.72 (d, 1 H, J = 8.5 Hz); 6.69 (s, 1 H); 6.29 (m, NH); 6.00 (m, 1 H); 5.17 (d, 2H); 5.12-5.00 (m, 2H); 4.42 (d, 2H); 2.17 (s, 3H); 1.55 (s, 6H); 1.25 (s, 9H)
Mp=170°C

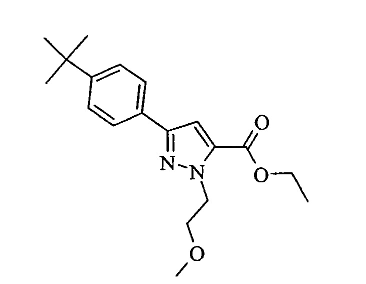

### Intermediate 111

### Ethyl 3-[4-(1,1-dimethylethyl)phenyl]-1-[2-(methyloxy)ethyl]-1H-pyrazole-5-carboxylate

To a solution of 500 mg (1.8mmol) of intermediate 22 in acetone was added 770 mg (3eq) of K₂CO₃ and 350 µL (2eq) of 2-bromoethyl methyl ether. The reaction mixture was heated at 70°C for 18h. The reaction mixture was purified by Flash Master (Cyclohexane/ethyl acetate 70/30 and CH₂Cl₂) to give the desired isomer as the first fraction collected (oil, m=210mg).
Yield: 35%
¹H NMR (CDCl₃= 7.15) δ: 7.66 (d, 2H, *J* = 8.6 Hz); 7.34 (d, 2H, *J* = 8.7 Hz); 7.01 (s, 1H); 4.71 (t, 2H); 4.27 (quad, 2H); 3.72 (t, 2H); 3.23 (s, 3H); 1.30 (t, 3H); 1.25 (s, 9H)

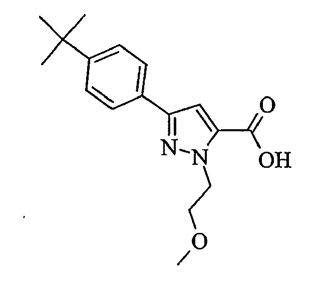

### Intermediate 112

### 3-[4-(1,1-dimethylethyl)phenyl]-1-[2-(methyloxy)ethyl]-1H-pyrazole-5-carboxylic acid

A solution of 210 mg (0.63mmol) of **intermediate 111** in ethanol and an excess (5eq) of NaOH (1 N) was heated at 50°C for 4h. The resulting mixture was evaporated, diluted in water and acidified with HCl (1 N). The precipitate formed was filtered, washed with water and pentane to give a white solid (190mg).
Quantitative yield
¹H NMR (CDCl₃=7.19) δ: 7.67 (d, 2H, J = 8.5 Hz); 7.37 (d, 2H, J = 8.5 Hz); 7.15 (s, 1 H); 4.76 (t, 2H); 3.79 (t, 2H); 3.29 (s, 3H); 1.27 (s, 9H)

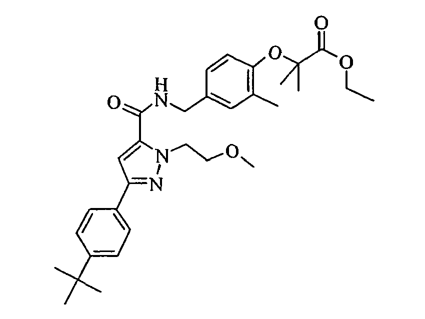

### Intermediate 113

### Ethyl-2-[(4-{[({3-[4-(1,1-dimethylethyl)phenyl]-1-[2-(methyloxy)ethyl]-1H-pyrazol-5-yl}carbonyl)amino]methyl}-2-methylphenyl)oxy]-2-methyl propanoate

A solution of 290 mg (0.96mmol) of **intermediate 112,** 330 mg (1.2eq) of **intermediate 10,** 550 mg (1.5eq) of HATU and 400 µL (3eq) of Et₃N in CH₂Cl₂ was stirred at rt for 18h. The resulting mixture was washed with HCl (1 N) and water, dried, evaporated and purified by flash master (CH₂Cl₂+ 0.5% of MeOH) (m=280mg).
Yield:54%
¹H NMR (CDCl₃=7.10 ) δ: 7.53 (d, 2H, *J* = 8.3 Hz); 7.24 (d, 2H, *J* = 8.3 Hz); 6.96 (m, 1H); 6.90-6.74 (m,1H); 6.67 (s, 1 H); 6.47 (d, 1 H, J = 8.3 Hz); 4.51 (t, 2H); 4.31 (d, 2H); 4.08 (quad, 2H); 3.65 (t, 2H); 3.05 (s, 3H); 2.06 (s, 3H); 1.42 (s, 6H); 1.16 (s, 9H); 1.10 (t, 3H)

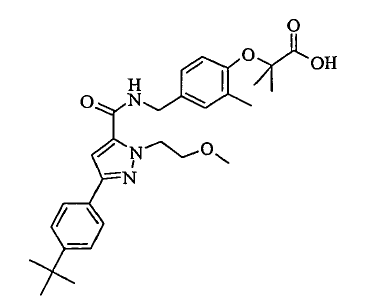

### Example 48

### 2-[(4-{[({3-[4-(1,1-dimethylethyl)phenyl]-1-[2-(methyloxy)ethyl]-1H-pyrazol-5-yl}carbonyl)amino]methyl}-2-methylphenyl)oxy]-2-methylpropanoic acid

A solution of 280 mg (0.52mmol) of **intermediate 113** in ethanol and an excess (5eq) of NaOH (1 N) was heated at 50°C for 4h. The resulting mixture was evaporated, diluted in water, acidified with HCl (1N). The precipitate formed was filtered, washed with water and pentane and re-crystallized in toluene to give a white powder (170mg).
Yield: 64%
¹H NMR (CDCl₃= 7.16 ) δ: 7.61 (d, 2H, J = 8.3 Hz); 7.32 (d, 2H, J = 8.3 Hz); 7.11 (m, NH); 7.06 (s, 1 H); 6.97 (d, 1 H, J = 8.1 Hz); 6.74 (s, 1 H); 6.70 (d, 1 H, J = 8.1 Hz); 4.58 (t, 2H); 4.40 (d, 2H); 3.73 (t, 2H); 3.12 (s, 3H); 2.15 (s, 3H); 1.52 (s, 6H); 1.23 (s, 9H)
Mp=112°C
LC-TOF MH+calc=508.2811 MH+=exp=508.2878 δ=13.2ppm

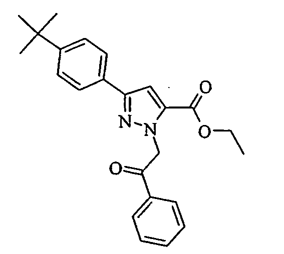

### Intermediate 114

### Ethyl 3-[4-(1,1-dimethylethyl)phenyl]-1-(2-oxo-2-phenylethyl)-1H-pyrazole-5-carboxylate

To a solution of 500 mg (1.8mmol) of **intermediate 22** in acetone was added 380 mg (1.5eq) of K₂CO₃ and 390 mg (1.1eq) of 2-bromo-1-phenylethanone. The reaction mixture was heated at 70°C for 18h. The reaction mixture was purified by Flash Master (Cyclohexane/ethyl acetate 70/30 and CH₂Cl₂) to give the desired isomer as the first fraction collected (m=460mg).
Yield: 65%
¹H NMR (CDCl₃) δ: 7.87 (d, 2H); 7.67 (d, 2H); 7.48 (m, 1 H); 7.40-7.30 (m, 4H); 7.13 (s, 1 H); 5.93 (s, 2H); 4.46 (quad, 2H); 1.24 (s, 9H); 1.19 (t, 3H)

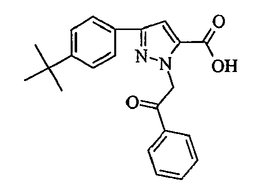

### Intermediate 115

### 3-[4-(1,1-dimethylethyl)phenyl]-1-(2-oxo-2-phenylethyl)-1H-pyrazole-5-carboxylic acid

A solution of 460 mg (1.18mmol) of **intermediate 114** in ethanol and an excess (5eq) of NaOH (1N) was heated at 50°C for 4h. the resulting mixture was evaporated, diluted in water, acidified with HCl (1N). The precipitate formed was filtered, washed with water and pentane to give a yellow solid (390mg).
Yield: 91 %
¹ H NMR (CDCl₃= 7.17) δ: 7.87 (d, 2H, J = 7.9 Hz); 7.63 (d, 2H, J = 8.3 Hz); 7.51 (t, 1 H, J = 7.5 Hz); 7.42-7.32 (m, 4H); 7.15 (s, 1 H); 5.95 (s, 2H); 1.25 (s, 9H)

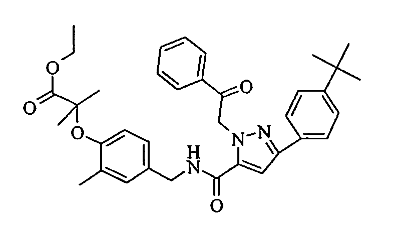

### Intermediate 116

### Ethyl-2-({4-[({[3-[4-(1,1-dimethylethyl)phenyl]-1-(2-oxo-2-phenylethyl)-1H-pyrazol-5-yl]carbonyl}amino)methyl]-2-methylphenyl}oxy)-2-methylpropanoate

A solution of 200 mg (0.55mmol) of **intermediate 115,** 240 mg (1.5eq) of **intermediate 10,** 320 mg (1.5eq) of HATU and 230 µL (3eq) of Et₃N in CH₂Cl₂ was stirred at rt for 18h. The resulting mixture was washed with HCl (1 N) and water, dried, evaporated and purified by flash master (CH₂Cl₂+ 0.5% of MeOH) to give a white solid (340mg).
Quantitative yield
1 H NMR (CDCl₃= 7.18) δ: 7.93 (d, 2H, *J* = 7.2 Hz); 7.59 (d, 2H, *J* = 8.5 Hz); 7.53 (d, 1H, J = 7.4 Hz); 7.42 (t, 2H); 7.31 (d, 2H, J = 8.5 Hz); 6.97 (d, 1H, *J* = 1.9 Hz); 6.86 (dd, 1H, *J* = 2.1 Hz, 8.3 Hz); 6.82 (s, 1 H); 6.50-6.55 (m, NH; d, 1 H); 6.07 (s, 2H); 4.24 (d, 2H); 4.15 (quad, 2H); 2.11 (s, 3H); 1.49 (s, 6H); 1.24 (s, 9H); 1.17 (t, 3H).

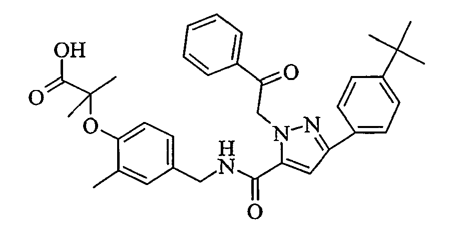

### Example 49

### 2-({4-[({[3-[4-(1.1-dimethylethyl)phenyl]-1-(2-oxo-2-phenylethyl)-1H-pyrazol-5-yl]carbonyl}amino)methyl]-2-methylphenyl}oxy)-2-methylpropanoic acid

A solution of 340 mg (0.6mmol) of **intermediate 116** in ethanol and an excess (5eq) of NaOH (1 N) was heated at 50°C for 4h. The resulting mixture was evaporated, diluted in water, acidified with HCI (1N). The precipitate formed was filtered, washed with water and pentane and re-crystallized in toluene to give an off-white solid (130mg).
Yield: 38%
¹H NMR (CDCl₃= 7.11) δ: 7.84 (d, 2H, *J* = 7.2 Hz); 7.52 (d, 2H, *J* = 8.5 Hz); 7.45 (d, 1 H, *J* = 7.3 Hz); 7.34 (t, 2H, J = 7.8 Hz, 7.3 Hz); 7.24 (d, 1 H, J = 8.5 Hz); 6.94 (d, 1 H, J = 1.9 Hz); 6.83 (dd, 1 H, J = 8.3 Hz, 2.1 Hz); 6.71 (s, 1 H); 6.58 (d, 1 H, J = 8.3 Hz); 6.37 (m, NH); 5.99 (s, 2H); 4.23 (d, 2H); 2.05 (s, 3H); 1.44 (s, 6H); 1.17 (s, 9H).
Mp=116°C
LC-TOF MH+calc=568.2811 MH+=exp=568.2840 δ=5.6ppm

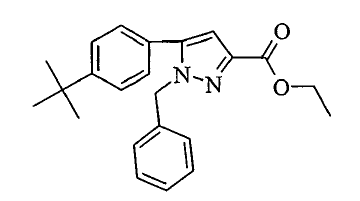

### Intermediate 117

### Ethyl-5-[4-(1,1-dimethylethyl)phenyl]-1-(phenylmethyl)-1H-pyrazole-3-carboxylate

To a solution of 500 mg (1.8mmol) of **intermediate 22** in acetone was added 380 mg (1.5eq) of K₂CO₃ and 250 µL (1.1eq) of (bromomethyl)benzene. The reaction mixture was heated at 70°C for 18h.
The reaction mixture was purified by Flash Master (Cyclohexane/ethyl acetate 70/30 and CH₂Cl₂) to give the desired isomer as the second fraction collected (oil, m=40mg).
Yield: 6%
NMR (CDCl₃) δ: 7.31 (d, 2H, *J* = 8.1 Hz); 7.19-7.15 (m,H); 7.12 (s, 1 H); 6.95 (dd, 2H, *J* = 7.6 Hz, 2.3 Hz); 6.80 (s, 1 H); 5.35 (s, 2H); 4.36 (quad, 2H); 1.33 (t, 3H); 1.25 (s, 9H).

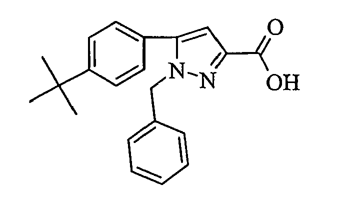

### Intermediate 118

### 5-[4-(1,1-dimethylethyl)phenyl]-1-(phenylmethyl)-1H-pyrazole-3-carboxylic acid

A solution of 40 mg (0.11 mmol) of **intermediate 117** in ethanol and an excess (5eq) of NaOH (1 N) was heated at 50°C for 4h. The resulting mixture was evaporated, diluted in water, acidified with HCl(1N). The precipitate formed was filtered, washed with water and pentane to give a cream solid (30mg).
Yield: 81 %
¹H NMR (CDCl₃) δ: 7.35 (d, 2H, J = 8.3 Hz); 7.22-7.14 (m, 5H); 6.98 (d, 2H, J = 7.5 Hz); 6.86 (s, 1 H); 5.37 (s, 2H); 1.27 (s, 9H).

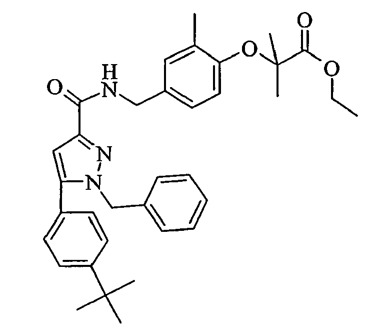

### Intermediate 119

### Ethyl 2-({4-[({[5-[4-(1,1-dimethylethyl)phenyl]-1-(phenylmethyl)-1H-pyrazol-3-yl]carbonyl}amino)methyl]-2-methylphenyl}oxy)-2-methylpropanoate

A solution of 30 mg (0.09mmol) of **intermediate 118**, 40 mg (1.5eq) of **intermediate 10**, 52 mg (1.5eq) of HATU and 40 µL (3eq) of Et₃N in CH₂Cl₂ was stirred at rt for 18h. The resulting mixture was washed with HCl (1 N) and water, dried , evaporated and purified by flash master (CH₂Cl₂+ 0.5% of MeOH) to give a colorless oil (70mg).
Quantitative yield
¹H NMR (CDCl₃= 7.18 ) δ: 7.33 (d, 2H, *J* = 8.5 Hz); 7.21 (d, 2H, *J* = 7.6 Hz); 7.17 (d, 2H, *J* = 8.3 Hz); 7.13-7.05 (m, 2H); 6.98-6.90 (m, 3H); 6.84 (s, 1 H); 6.54 (d, 1H, J = 8.4 Hz); 5.25 (s, 2H); 4.44 (d, 2H); 4.16 (quad, 2H); 2.14 (s, 3H); 1.50 (s, 6H); 1.25 (s, 9H);1.18 (t, 3H)

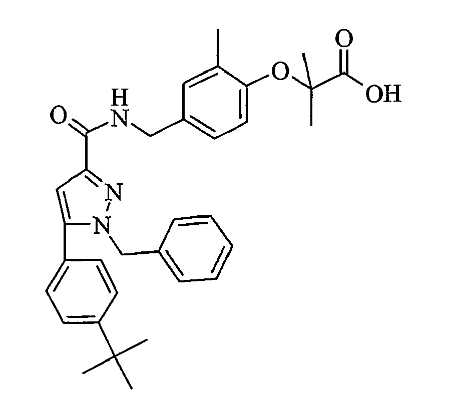

### Example 50

### 2-({4-[({[5-[4-(1,1-dimethylethyl)phenyl]-1-(phenylmethyl)-1H-pyrazol-3-yl]carbonyl}amino)methyl]-2-methylphenyl}oxy)-2-methylpropanoic acid

A solution of 70 mg (0.12mmol) of **intermediate 119** in ethanol and an excess (5eq) of NaOH (1 N) was heated at 50°C for 4h. The resulting mixture was evaporated, diluted in water and acidified with HCI (1 N). The precipitate formed was filtered, washed with water and pentane and re-crystallized in toluene to give a white solid (60mg).
Yield: 88%
¹H NMR (CDCl₃= 7.19) δ: 7.34 (d, 2H, *J* = 8.5 Hz) 7.23 (d, 4H, *J* = 7.2 Hz); 7.16 (s, 1H); 7.11 (m, 1 H); 7.01-6.92 (m, 3H); 6.85 (s, 1 H); 6.71 (d, 1H, *J* = 8.3 Hz); 5.27 (s, 2H); 4.45 (d, 2H); 2.16 (s, 3H); 1.52 (s, 6H); 1.26 (s, 9H)
Mp=88°C

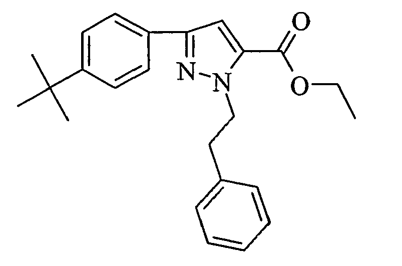

### Intermediate 120

### Ethyl-3-[4-(1,1-dimethylethyl)phenyl]-1-(2-phenylethyl)-1H-pyrazole-5-carboxylate

To a solution of 500 mg ( 1.8mmol) of **intermediate 22** in acetone was added 70 mg ( 1.5eq) of NaH and 250 µL (1.1eq) of (2-bromoethyl)benzene. The reaction mixture was heated at 70°C for 18h. The reaction mixture was purified by Flash Master (Cyclohexane/ethyl acetate 70/30 and CH₂Cl₂) to give the desired isomer as the first fraction collected (colorless oil, 220mg).
Yield: 32%
¹H NMR (CDCl₃) δ: 7.67 (d, 2H, *J* = 8.5 Hz); 7.37 (d, 2H, *J* = 8.3 Hz); 7.24-7.11 (m, 5H); 6.99 (s, 1 H); 4.74 (m, 2H); 4.23 (quad, 2H); 3.09 (t, 2H); 1.29 (t, 3H); 1.27 (s, 9H).

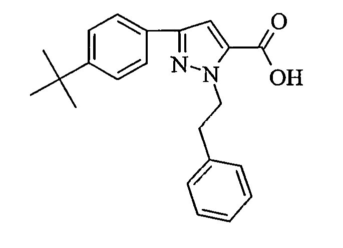

### Intermediate 121

### 3-[4-(1,1-dimethylethyl)phenyl]-1-(2-phenylethyl)-1H-pyrazole-5-carboxylic acid

A solution of 220 mg (0.6mmol) of **intermediate 120** in ethanol and an excess (5eq) of NaOH (1 N) was heated at 50°C for 4h. The resulting mixture was evaporated, diluted in water, acidified with HCl (1N). The precipitate formed was filtered, washed with water and pentane to give a white solid (180mg).
Yield: 88%
¹H NMR (CDCl₃=7.15) δ: 7.69 (d, 2H, *J* = 8.5 Hz); 7.39 (d, 2H, *J* = 8.5 Hz); 7.25-7.10 (m, 6H); 4.78 (t, 2H); 3.13 (t, 2H); 1.28 (s, 9H)

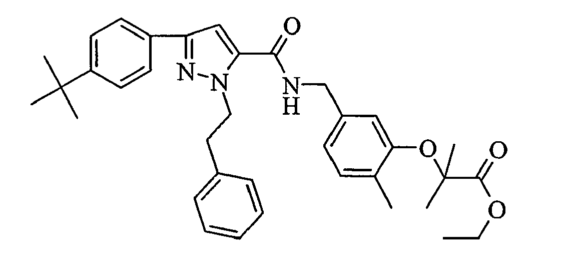

### Intermediate 122

### Ethyl-2-({5-[({[3-[4-(1,1-dimethylethyl)phenyl]-1-(2-phenylethyl)-1H-pyrazol-5-yl]carbonyl}amino)methyl]-2-methylphenyl}oxy)-2-methylpropanoate

A solution of 180 mg (0.51mmol) of **intermediate 121**, 225 mg (1.5eq) of **intermediate 10**, 300 mg (1.5eq) of HATU and 220 µL (3eq) of Et₃N in CH₂Cl₂ was stirred at rt for 18h. The resulting mixture was washed with HCl (1 N) and water, dried , evaporated and purified by flash master (CH₂Cl₂+ 0.5% of MeOH) to give a white solid (240mg).
Yield: 85%

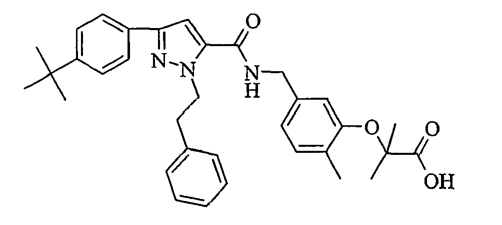

### Example 51

### 2-({5-[({[3-[4-(1,1-dimethylethyl)phenyl]-1-(2-phenylethyl)-1H-pyrazol-5-yl]carbonyl}amino)methyl]-2-methylphenyl}oxy)-2-methylpropanoic acid

A solution of 240 mg (0.43mmol) of **intermediate 122** in ethanol and an excess (5eq) of NaOH (1N) was heated at 50°C for 4h. The resulting mixture was evaporated, diluted in water, acidified with HCl (1N). The precipitate formed was filtered, washed with water and pentane and re-crystallized in toluene to give a white powder (100mg).
Yield: 44%
¹H NMR (CDCl₃=7.39) δ: 7.82 (d,2H, *J* = 8.5 Hz); 7.55 (d, 2H, *J* = 8.5 Hz); 7.40-7.25 (m, 6H); 7.16 (dd, 1 H, *J* = 8.6 Hz, 2.2 Hz); 6.93 (d, 1 H, *J* = 8.3 Hz); 6.77 (s, 1 H); 6.12 (m, NH); 4.95 (t, 2H); 4.53(d, 2H); 3.31 (t, 2H); 2.38 (s, 3H); 1.75 (s, 6H); 1.46 (s, 9H)
Mp=213°C

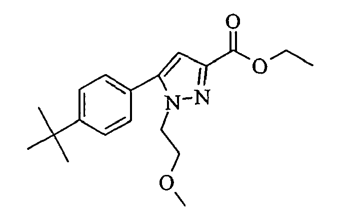

### Intermediate 123

### Ethyl 5-[4-(1,1-dimethylethyl)phenyl]-1-[2-(methyloxy)ethyl]-1H-pyrazole-3-carboxylate

To a solution of 500 mg (1.8mmol) of **intermediate 22** in acetone was added 770 mg (3eq) of K₂CO₃ and 350 µL (2eq) of 2-bromoethyl methyl ether. The reaction mixture was heated at 70°C for 18h. The reaction mixture was purified by Flash Master (Cyclohexane/ethyl acetate 70/30 and CH₂Cl₂) to give the desired isomer as the second fraction collected (oil, 80mg).
Yield: 13%
¹H NMR (CDCl₃=7.19) δ: 7.38 (dd; 4H, *J* = 8.7 Hz, 7.1 Hz); 6.72 (s, 1H); 4.36 (quad, 2H); 4.25 (t, 2H); 3.79 (t, 2H); 3.20 (s, 3H); 1.33 (t, 3H); 1.29 (s, 9H)

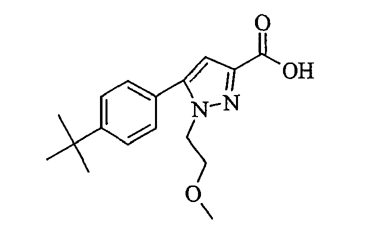

### Intermediate 124

### 5-[4-(1,1-dimethylethyl)phenyl]-1-[2-(methyloxy)ethyl]-1H-pyrazole-3-carboxylic acid

A solution of 80 mg (0.24mmol) of intermediate 123 in ethanol and an excess (5eq) of NaOH (1 N) was heated at 50°C for 4h. The resulting mixture was evaporated, diluted in water, acidified with HCl(1N). The precipitate formed was filtered, washed with water and pentane (50mg).
Yield: 68%

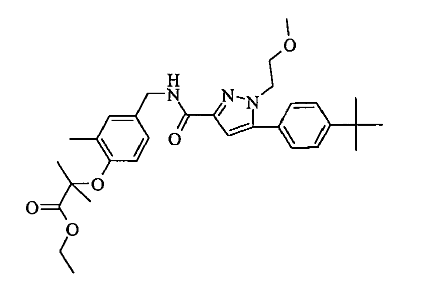

### Intermediate 125

### Ethyl-2-[(4-{[({5-[4-(1,1-dimethylethyl)phenyl]-1-[2-(methyloxy)ethyl]-1H-pyrazol-3-yl}carbonyl)amino]methyl}-2-methylphenyl)oxy]-2-methylpropanoate

A solution of 50 mg (0.16mmol) of **intermediate 124**, 60 mg (1.2eq) of **intermediate 10**, 100 mg (1.5eq) of HATU and 100 µL (3eq) of Et₃N in CH₂CI₂ was stirred at rt for 18h. The resulting mixture was washed with HCl (1 N) and water, dried , evaporated and purified by flash master (CH₂CI₂+ 0.5% of MeOH) (m=90mg).
Quantitative yield
¹H NMR (CDCl₃=7.20) δ: 7.41 (d, 2H); 7.33 (d, 2H); 7.13 (m, 1H); 7.08 (d, 1H); 6.97 (dd, 1 H); 6.73 (s, 1 H); 6.55 (d, 1 H); 4.44 (d, 2H); 4.16 (quad, 2H; t, 2H); 3.71 (t, 2H); 3.20 (s, 3H); 2.15 (s, 3H); 1.51 (s, 6H); 1.28 (s, 9H); 1.19 (t, 3H).

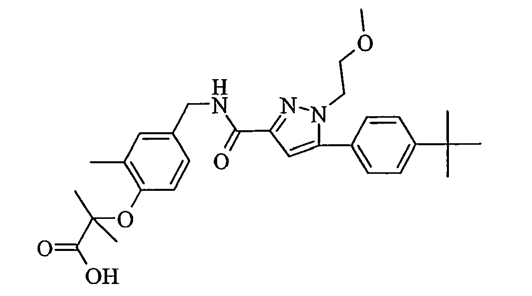

### Example 52

### 2-[(4-{[({5-[4-(1.1-dimethylethyl)phenyl]-1-[2-(methyloxy)ethyl]-1H-pyrazol-3-yl}carbonyl)amino]methyl}-2-methylphenyl)oxy]-2-methylpropanoic acid

A solution of 50 mg (0.1 mmol) of **intermediate 125** in ethanol and an excess (5eq) of NaOH (1 N) was heated at 50°C for 4h. The resulting mixture was evaporated, diluted in water, acidified with HCl (1N). The precipitate formed was filtered, washed with water and pentane to give a white powder (70mg).
Quantitative yield.
¹H NMR (CDCl₃=7.19) δ: 7.41 (d, 2H, J = 8.3 Hz); 7.34 (d, 2H, J = 8.5 Hz); 7.12 (d, 1 H, J = 1.5 Hz); 6.98 (dd, 1 H, J = 8.1 Hz, 1.5 Hz); 6.76 (s, 1 H); 6.72 (d, 1 H, J = 8.3 Hz); 4.46 (d, 2H); 4.17 (t, 2H); 3.71 (t, 2H); 3.20 (s, 3H); 2.17 (s, 3H); 1.54 (s, 6H); 1.29 (s, 9H)
Mp=65°C became gummy
LC-TOF MH+calc=508.2811 MH+=exp=508.2796 δ= -2.9ppm

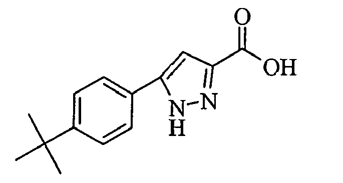

### Intermediate 126

### 5-[4-(1,1-dimethylethyl)phenyl]-1H-pyrazole-3-carboxylic acid

A solution of 1.5g (5.4mmol) of **intermediate 22** in ethanol and an excess (5eq) of NaOH (1 N) was heated at 50°C for 4h. The resulting mixture was evaporated, diluted in water, acidified with HCl(1N). The precipitate formed was filtered, washed with water and pentane to give a white solid (1.30g).
Yield: 96%
¹H NMR (DMSO=2.55 ) δ: 7.80 (d, 2H, *J* = 8.3 Hz); 7.51 (d, 2H, *J* = 8.3 Hz); 7.19 (s, 1 H); 1.35 (s, 9H).

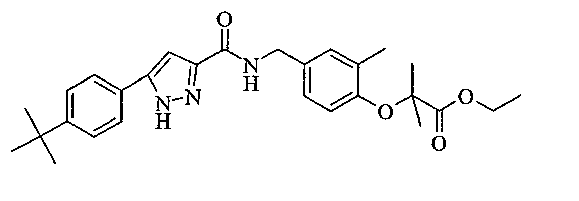

### Intermediate 127

### Ethyl-2-[(4-{[({5-[4-(1,1-dimethylethyl)phenyl]-1H-pyrazol-3-yl}carbonyl)amino]methyl}-2-methylphenyl)oxy]-2-methylpropanoate

A solution of 500mg (2mmol) of **intermediate 126** in toluene and 750µL (5eq) of SOCl₂ was heated at 80°C for 3h. The resulting solution was evaporated, diluted in toluene and evaporated again. The residue, diluted in CH₂Cl₂, was added to a solution of 650mg (1.1eq) of **intermediate 10**, 900µL (3eq) of Et₃N and CH₂Cl₂, and stirred at rt for 2h. The resulting mixture was washed with HCl (1 N), NaOH (1 N), water, dried on Na₂SO₄ and evaporated (m=1g).
Quantitative yield.
¹H NMR (CDCl₃=7.19) δ: 7.47 (d, 2H, *J =* 8.4 Hz); 7.37 (d, 2H, *J =* 8.5 Hz); 7.05 (s, 1 H); 6.98 (s, 1 H); 6.94 (d, 1H, *J* = 8.3 Hz); 6.53 (d, 1 H); 4.45 (d, 2H); 4.16 (quad, 2H); 2.11 (s, 3H); 1.50 (s, 6H); 1.25 (s, 9H); 1.17 (t, 3H).

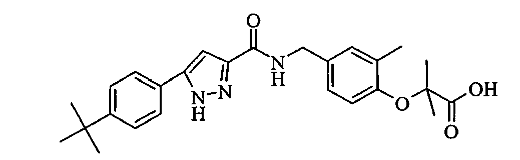

### Example 53

### 2-[(4-{[({5-[4-(1,1-dimethylethyl)phenyl]-1H-pyrazol-3-yl}carbonyl)amino] methyl}-2-methylphenyl)oxy]-2-methylpropanoic acid

A solution of 80 mg (0.17mmol) of **intermediate 127** in ethanol and an excess (5eq) of NaOH (1 N) was heated at 50°C for 4h. The resulting mixture was evaporated, diluted in water, acidified with HCl (1 N). The precipitate formed was filtered, washed with water and pentane to give a white powder (60mg).
Yield: 80%
¹H NMR (CDCl₃=7.49) δ: 7.81 (d, 2H, *J* = 8.5 Hz); 7.69 (d, 2H, *J* = 8.5 Hz); 7.37 (s, 1 H); 7.31 (s, 1 H); 7.23 (d, 1H, *J* = 6.6 Hz); 6.92 (d, 1 H, *J* = 8.5 Hz); 4.73 (d, 2H); 2.42 (s, 3H); 1.83 (s, 6H); 1.56 (s, 9H)
Mp=100°C becomes gummy
LC-TOF MH+calc=450.2911 MH+=exp=450.2393 δ= -10.2ppm

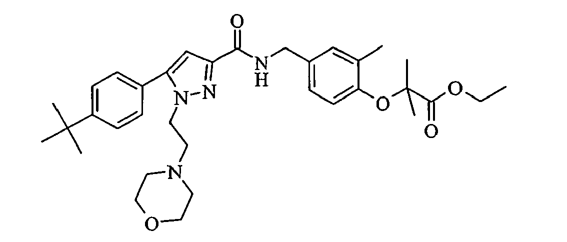

### Intermediate 128

### ethyl 2-[(4-{[({5-[4-(1,1-dimethylethyl)phenyl]-1-[2-(4-morpholinyl)ethyl]-1H-pyrazol-3-yl}carbonyl)amino]methyl}-2-methylphenyl)oxy]-2-methylpropanoate

To a solution of 250 mg (0.52mmol) of **intermediate 127** in acetone was added 180 mg (2.5eq) of K₂CO₃ and 150mg (1.5eq) of 4-(2-chloroethyl)morpholine hydrochloride. The reaction mixture was heated at 70°C for 18h.
The reaction mixture was purified by Flash Master (Cyclohexane/ethyl acetate 70/30 and CH₂Cl₂) to give the desired isomer as the first fraction collected (60mg).
Yield: 20%
1H NMR (CDCl₃= 7.19) d: 7.40 (d, 2H, *J* = 8.2 Hz); 7.30 (d, 2H, *J* = 8.5 Hz); 7.08 (m, 2H); 6.97 (dd, 1 H, J = 8.5 Hz, 2.3 Hz); 6.73 (s, 1 H); 6.55 (d, 1 H, J = 8.3 Hz); 4.45 (d, 2H); 4.16 (t, 2H); 4.15 (quad, 2H); 3.49 (t, 4H); 2.68 (t, 2H); 2.23 (t, 4H); 2.15 (s, 3H); 1.51 (s, 6H); 1.28 (s, 9H)

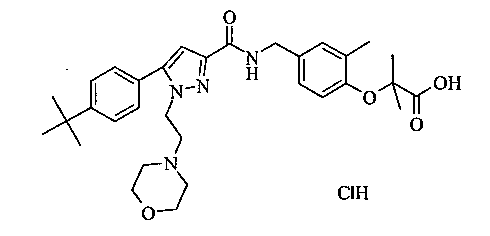

### Example 54

### 2-[(4-{[({5-[4-(1,1-dimethylethyl)phenyl]-1-[2-(4-morpholinyl)ethyl]-1H-pyrazol-3-yl}carbonyl)amino]methyl}-2-methylphenyl)oxy]-2-methyl propanoic acid hydrochloride

A solution of 60 mg (0.11mmol) of **intermediate 128** in ethanol and an excess (5eq) of NaOH (1 N) was heated at 50°C for 4h. The resulting mixture was evaporated, diluted in water, acidified with HCl (1 N). The precipitate formed was filtered, washed with water and pentane and re-crystallized in toluene to give a grey solid (130mg).
Quantitative yield.
¹H NMR (DMSO=2.38) δ: 8.57 (m, 1H); 7.45 (d, 2H, *J* = 8.6 Hz); 7.38 (d, 2H, *J* = 8.7 Hz); 7.00 (d, 1 H, *J* = 1.9 Hz); 6.92 (dd, 1 H, *J* = 8.3 Hz, 1.9 Hz); 6.70 (s, 1 H); 6.53 (d, 1H, *J* = 8.5 Hz); 4.48 (m, 2H); 4.24 (d, 2H); 3.83 (m, 2H); 3.60 (m, 4H); 3.30 (m, 4H); 2.02 (s, 3H); 1.37 (s, 6H); 1.21 (s, 9H)
Mp=150°C

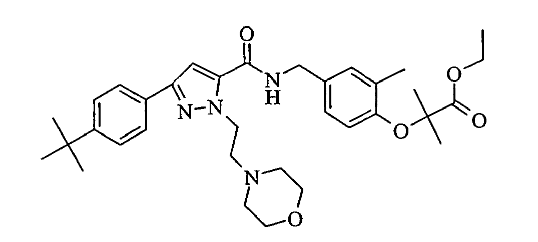

### Intermediate 129

### Ethyl-2-[(4-{[({3-[4-(1,1-dimethylethyl)phenyl]-1-[2-(4-morpholinyl)ethyl]-1H-pyrazol-5-yl}carbonyl)amino]methyl}-2-methylphenyl)oxy]-2-methyl propanoate

To a solution of 250 mg (0.52mmol) of **intermediate 127** in acetone was added 180 mg (2.5eq) of K₂CO₃ and 150mg (1.5eq) of 4-(2-chloroethyl)morpholine hydrochloride. The reaction mixture was heated at 70°C for 18h. The reaction mixture was purified by Flash Master (Cyclohexane/ethyl acetate 70/30 and CH₂Cl₂) to give the desired isomer as the second fraction collected (50mg)
Yield: 20%
¹H NMR (CDCl₃=7.10) δ: 7.53 (d, 2H, *J* = 8.5 Hz); 7.25 (d, 2H, *J* = 8.3 Hz); 7.20 (m, 1 H); 6.97 (d, 1 H, *J* = 1.4 Hz); 6.86 (dd, 1 H, *J* = 8.4 Hz, 2 Hz); 6.64 (s, 1 H); 6.48 (d, 1 H, *J* = 8.2 Hz); 4.50 (t, 2H); 4.34 (d, 2H); 4.09 (quad, 2H); 3.38 (t, 4H); 2.68 (t, 2H); 2.23 (t, 4H); 2.07 (s, 3H); 1.43 (s, 6H); 1.17 (s, 9H); 1.10 (t, 3H)

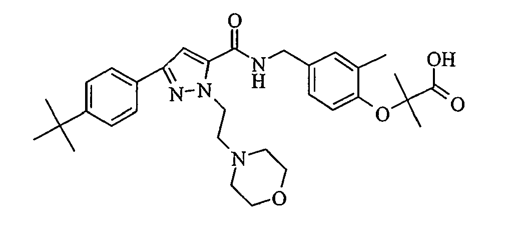

### Example 55

### 2-[(4-{[({3-[4-(1,1-dimethylethyl)phenyl]-1-[2-(4-morpholinyl)ethyl]-1H-pyrazol-5-yl}carbonyl)amino]methyl}-2-methylphenyl)oxy]-2-methyl propanoic acid

A solution of 50 mg (0.09mmol) of **intermediate 129** in ethanol and an excess (5eq) of NaOH (1 N) was heated at 50°C for 4h. The resulting mixture was evaporated, diluted in water, acidified with HCl (1 N). The precipitate formed was filtered, washed with water and pentane and re-crystallized in toluene to give a grey powder (40mg).
Quantitative yield.
¹H NMR (DMSO=2.63) δ: 9.34 (m, 1H); 7.83 (d, 2H, *J* = 8.5 Hz); 7.60 (d, 2H, *J* = 8.5 Hz); 7.57 (s, 1 H); 7.27 (d, 1 H, *J* = 1.9 Hz); 7.20 (dd, 1H, *J* = 8.5 Hz, 2.1 Hz); 6.79 (d, 1 H, *J* = 8.2 Hz); 5.09 (m, 2H); 4.50 (d, 2H); 4.10 (m, 2H); 4.00-3.55 (m, 6H); 3.30 (m, 2H); 2.28 (s, 3H); 1.63 (s, 6H); 1.43 (s, 9H);
Mp=140°C

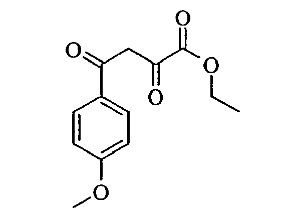

### Intermediate 130

### ethyl 4-[4-(methyloxy)phenyl]-2,4-dioxobutanoate

1.7 g (1.1eq) of sodium were dissolved in ethanol at room temperature. Then 10 g (66.6mmol) of 1-[4-(methyloxy)phenyl]ethanone were dropwise added and the solution was stirred for 30 min. 10 mL (1.1eq) of diethyl ethanedioate diluted in ethanol were dropwise added, and it was stirred at rt for 1h then heated at 80°C for 2h, followed by a night at rt. The resulting mixture was evaporated, acidified with HCl (1 N) until pH was acid, then it was extracted with diethyl ether, washed with HCl (1 N) and water, dried on Na₂SO₄ and evaporated to give an orange oil (16.7g).
Quantitative yield
¹H NMR (CDCl₃=7.10) δ: 7.83 (d, 2H); 6.87 (s, 1H); 6.82 (d, 2H); 4.23 (quad, 2H); 3.73 (s, 2H); 1.25 (t, 3H). The product was enolate derivative in solution.

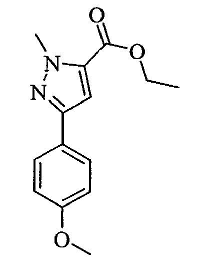

### Intermediate 131

### ethyl 1-methyl-3-[4-(methyloxy)phenyl]-1H-pyrazole-5-carboxylate

To a solution of 16.7g (70mmol) of **intermediate 130** in ethanol was added 4 mL (1.05eq) of methyl hydrazine. The reaction solution was heated at 80°C for 2h. Then it was evaporated, extracted with diethyl ether and washed with HCl (1N), dried on Na₂SO₄, evaporated and purified by flash chromatography (Cyclohexane/dichloromethane 70/30 to dichloromethane 100%) to give the desired isomer as the first fraction collected (9.9g).
Yield: 57% (white solid).
¹H NMR (CDCl₃= 7.31) δ: 7.78 (d, 2H, *J* = 8.7 Hz); 7.10 (s, 1 H); 6.99 (d, 2H, *J* = 8.8 Hz); 4.42 (quad, 2H); 4.26 (s, 3H); 3.89 (s, 3H); 1.45 (t, 3H);

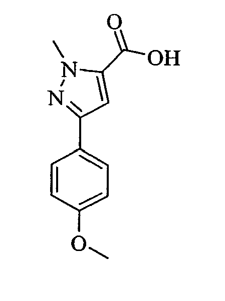

### Intermediate 132

### 1-methyl-3-[4-(methyloxy)phenyl]-1H-pyrazole-5-carboxylic acid

A solution of 500mg (1.8mmol) of **intermediate 131** in ethanol and an excess (5eq) of NaOH (1 N) was heated at 50°C for 4h. the resulting mixture was evaporated, diluted in water, acidified with HCl (1N). The precipitate formed was filtered, washed with water and pentane to give a white solid (0.5g).
Quantitative yield
¹H NMR (CDCl₃=7.21) δ: 7.64 (d, 2H, *J* = 8.7 Hz); 7.01 (s, 1 H); 6.87 (d, 2H, *J* = 8.7 Hz); 4.14 (s, 3H); 3.75 (s, 3H).

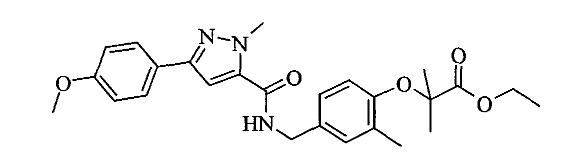

### Intermediate 133

### Ethyl-2-methyl-2-[(2-methyl-4-{[({1-methyl-3-[4-(methyloxy)phenyl]-1H-pyrazol-5-yl}carbonyl)amino]methyl}phenyl)oxy]propanoate

A solution of 500mg (2.1mmol) of **intermediate 132** in toluene and 770µL (5eq) of SOCl₂ was heated at 80°C for 3h. The resulting solution was evaporated, diluted in toluene and evaporated again. The residue, diluted in CH₂Cl₂, was added to a solution of 750mg (1.2eq) of **intermediate 10** and 900µL (3eq) of Et₃N in CH₂Cl₂, and it was stirred at rt for2h. The resulting mixture was washed with HCl (1N), NaOH (1N), water, dried on Na₂SO₄ and evaporated (m=830mg).
Yield: 85%
¹H NMR (CDCl₃=7.19) δ: 7.59 (d, 2H, *J* = 8.6 Hz); 7.04 (d, 1H, *J =* 1.8 Hz); 6.93 (dd, 1 H, *J* = 8.3 Hz, 2.3 Hz); 6.84 (d, 2H, *J =* 8.6 Hz); 6.62 (s, 1 H); 6.55 (d, 1H, *J =* 8.3 Hz); 6.32 (m, 1 H); 4.40 (d, 2H); 4.17 (quad, 2H); 4.13 (s, 3H); 3.74 (s, 3H); 2.15 (s, 3H); 1.52 (s, 6H); 1.19 (t, 3H).

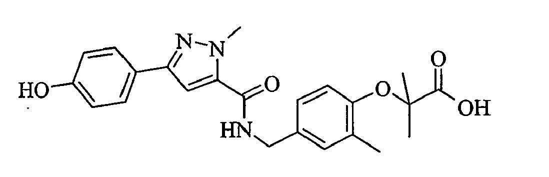

### Intermediate 134

### 2-({4-[({[3-(4-hydroxyphenyl)-1-methyl-1H-pyrazol-5-yl]carbonyl}amino)methyl]-2-methylphenyl}oxy)-2-methylpropanoic acid

To a solution of 700mg (1.5mmol) of **intermediate 133** in CH₂Cl₂ was added BBr₃ (1 M in CH₂Cl₂). The solution was stirred for 18h. Then HCl (1 N) and diethyl ether were added and the resulting mixture was stirred for 15 min. The layers were separated and the organic layer was dried on Na₂SO₄, evaporated and added to CH₂Cl₂ to form a precipitate which was filtered and washed with CH₂Cl₂.
Yield: 63% (m=400mg, grey solid).
¹H NMR (DMSO=2.70) δ: 7.75 (d, 2H, *J* = 8.7 Hz); 7.35 (s, 1 H); 7.32 (d, 1H, *J* = 1.5 Hz); 7.25 (dd, 1 H, *J* = 8.4 Hz, 1.9 Hz); 7.00 (d, 2H, *J* = 8.6 Hz); 6.86 (d, 1 H, *J* = 8.5 Hz); 4.54 (d, 2H); 4.27 (s, 3H); 2.35 (s, 3H); 1.69 (s, 6H).

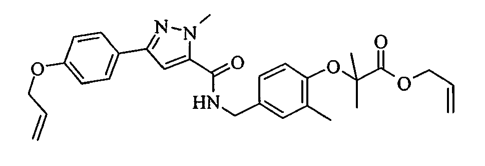

### Intermediate 135

### 2-propen-1-yl-2-methyl-2-[(2-methyl-4-{[({1-methyl-3-[4-(2-propen-1-yloxy)phenyl]-1H-pyrazol-5-yl}carbonyl)amino]methyl}phenyl)oxy]propanoate

To a solution of 100 mg (0.24mmol) of **intermediate 134** in acetone was added 170 mg (5eq) of K₂CO₃ and 55µL (2.5eq) of 3-bromo-1-propene. The reaction mixture was heated at 70°C for 18h. The reaction mixture was purified by Flash Master (Cyclohexane/CH₂Cl₂ 70/30 and CH₂CI₂) to give the desired product (m=70mg).
Yield: 59%
¹H NMR (CDCl₃= 7.19) δ: 7.59 (d, 2H, *J* = 8.9 Hz); 7.04 (s, 1 H); 6.94 (dd, 1H, *J* = 8.5 Hz, 2.1 Hz); 6.86 (d, 2H, *J* = 8.7 Hz); 6.61 (s, 1 H); 6.56 (d, 1 H, *J* = 8.3 Hz); 6.23 (m, 1 H); 5.99 (m, 1 H); 5.82 (m, 1 H); 5.35 (d, 1 H); 5.28-5.14 (m, 3H); 4.61 (d, 2H); 4.48 (d, 2H); 4.41 (d, 2H); 4.14 (s, 3H); 2.16 (s, 3H); 1.54 (s, 6H)

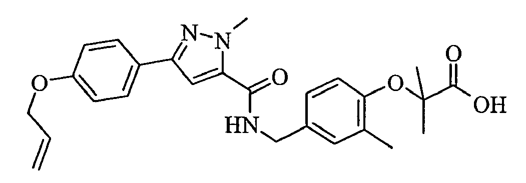

### Example 56

### 2-methyl-2-[(2-methyl-4-{[({1-methyl-3-[4-(2-propen-1-yloxy)phenyl]-1H-pyrazol-5-yl}carbonyl)amino]methyl}phenyl)oxy]propanoic acid

A solution of 70 mg (0.14mmol) of **intermediate 135** in ethanol and an excess (5eq) of NaOH (1 N) was heated at 50°C for 4h. The resulting mixture was evaporated, diluted in water, acidified with HCl (1 N). The precipitate formed was filtered, washed with water and pentane (m=40mg).
Yield: 62%
¹H NMR (CDCl₃=7.19) δ: 7.60 (d, 2H); 7.09 (s, 1H);6.99 (d, 1H); 6.86 (d, 2H); 6.72 (d, 1H); 6.62 (s, 1 H); 6.30 (m, 1 H); 5.99 (m, 1 H); 5.35(d, 1 H); 5.23 (d, 1 H); 4.48 (d, 2H); 4.43 (d, 2H); 4.15 (s, 3H); 2.18 (s, 3H); 1.56 (s, 6H).
Mp=70°C becomes gummy
LC-TOF MH+cal=464.2174 MH+exp=464.2185 δ= -3ppm

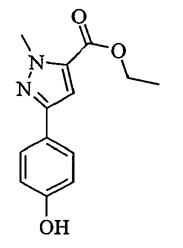

### Intermediate 136

### ethyl 3-(4-hydroxyphenyl)-1-methyl-1H-pyrazole-5-carboxylate

To a solution of 2.1g (7.8mmol) of **intermediate 131** in CH₂Cl₂ was added two volumes 15mL (2eq) of BBr₃ (1M in CH₂Cl₂), and it was stirred at rt for 18h. Then HCl (1N) and diethyl ether were added and the resulting mixture was stirred for 15 min. The layers were separated and the organic layer was dried on Na₂SO₄, evaporated, added to CH₂Cl₂ to form a precipitate which was filtered and washed with CH₂Cl₂ (m=2g).
Quantitative yield
¹H NMR (DMSO=2.51) δ: 9.58 (m, 1 H); 7.66 (d, 2H); 7.18 (s, 1 H); 6.80 (d, 2H); 4.33 (quad, 2H); 4.11 (s, 3H); 1.33 (t, 3H)

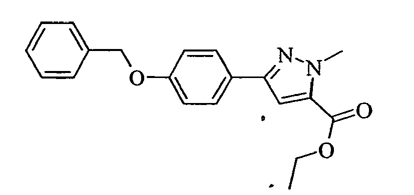

### Intermediate 137

### Ethyl 1-methyl-3-{4-[(phenylmethyl)oxy]phenyl}-1H-pyrazole-5-carboxylate

To a solution of 300 mg (1.2mmol) of **intermediate 136** in acetone was added 510 mg (3eq) of K₂CO₃ and 160µL (1.1eq) of benzyl bromide. The reaction mixture was heated at 70°C for 18h. The reaction mixture was purified by flash chromatography (CH₂Cl₂ + 0.5 % MeOH) to give a cream solid (380mg).
Yield: 93%
1 H NMR (CDCl₃= 7.18) δ: 7.65 (d, 2H); 7.40-7.24 (m, 5H); 6.97 (s, 1 H); 6.94 (d, 2H); 5.02 (s, 2H); 4.29 (quad, 2H); 4.13 (s, 3H); 1.32 (t, 3H)

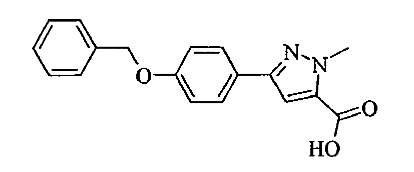

### Intermediate 138

### 1-methyl-3-{4-[(phenylmethyl)oxy]phenyl}-1H-pyrazole-5-carboxylic acid

A solution of 380 mg (1.13mmol) of **intermediate 137** in ethanol and an excess (5eq) of NaOH (1 N) was heated at 50°C for 4h. The resulting mixture was evaporated, diluted in water, acidified with HCl (1N). The precipitate formed was filtered, washed with water and pentane (m=220mg).
Yield: 55%
¹H NMR (DMSO=2.29) δ: 7.56 (d, 2H); 7.28-7.10 (m, 5H); 6.99 (s, 1H); 6.84 (d, 2H); 4.93 (s, 2H); 3.89 (s, 3H)

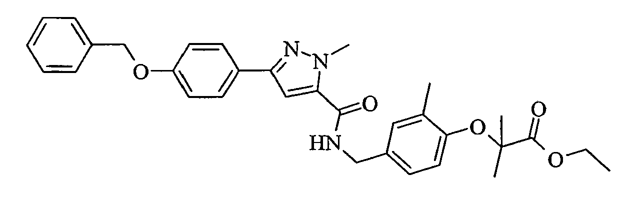

### Intermediate 139

### Ethyl 2-methyl-2-{[2-methyl-4-({[(1-methyl-3-{4-[(phenylmethyl)oxy]phenyl}-1H-pyrazol-5-yl)carbonyl]amino}methyl)phenyl]oxy}propanoate

A solution of 220 mg (0.65mmol) of **intermediate 138**, 226 mg (1.2eq) of **intermediate 10**, 375 mg (1.5eq) of HATU and 300 µL (3eq) of Et₃N in CH₂Cl₂ was stirred at rt for 18h. The resulting mixture was washed with HCl (1 N) and water, dried and evaporated (m=350mg).
Yield: 99%
¹H NMR (CDCl₃= 7.25) δ: 7.67 (d, 2H); 7.47-7.37 (m, 5H); 7.12 (s, 1H); 7.01 (d, 1H); 6.99 (d, 2H); 6.74 (s, 1H); 6.66 (m, NH); 6.64 (d, 1H); 5.05 (s, 2H); 4.46 (d, 2H); 4.26 (quad, 2H); 4.21 (s, 3H); 2.24 (s, 3H); 1.61 (s, 6H); 1.28 (t, 3H)

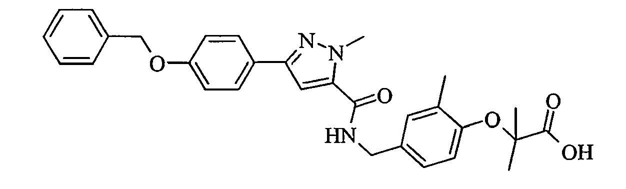

### Example 57

### 2-methyl-2-{[2-methyl-4-({[(1-methyl-3-{4-[(phenylmethyl)oxy]phenyl}-1H-pyrazol-5-yl)carbonyl]amino}methyl)phenyl]oxy}propanoic acid

A solution of 350 mg (0.65mmol) of **intermediate 139** in ethanol and an excess (5eq) of NaOH (1 N) was heated at 50°C for 4h. The resulting mixture was evaporated, diluted in water, acidified with HCl (1 N). The precipitate formed was filtered, washed with water and pentane (m=200mg).
Yield: 60%
¹H NMR (CDCl₃= 7.28) δ: 7.68 (d, 2H); 7.50-7.31 (m, 5H); 7.17 (s, 1 H); 7.06 (d, 1 H); 7.00 (d, 2H); 6.79 (d, 1 H); 6.70 (s, 1 H); 6.39 (m, NH); 5.09 (s, 2H); 4.50 (d, 2H); 4.22 (s, 3H); 2.26 (s, 3H); 1.64 (s, 6H)
Mp=72°C
LC-TOF MH+cal=514.2342 MH+exp=514.2315 δ= -5.2ppm

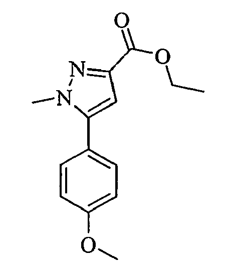

### Intermediate 140

### Ethyl 1-methyl-5-[4-(methyloxy)phenyl]-1H-pyrazole-3-carboxylate

To a solution of 16.7g (70mmol) of intermediate 130 in ethanol was added 4 mL (1.05eq) of methyl hydrazine. The reaction solution was heated at 80°C for 2h. Then it was evaporated, extracted with diethyl ether and HCI (1 N), dried on Na₂SO₄, evaporated and purified by flash chromatography (Cyclohexane/dichloromethane 70/30 to dichloromethane 100%) to give the desired isomer as the second fraction collected (yellow solid, m=4.25g).
Yield: 24%
¹H NMR (CDCl₃= 7.19) δ: 7.27 (d, 2H); 6.93 (d, 2H); 6.73 (s, 1H); 4.36 (quad, 2H); 3.86 (s, 3H); 3.80 (s, 3H); 1.34 (t, 3H)

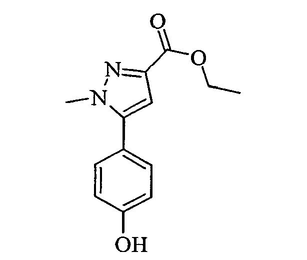

### Intermediate 141

### Ethyl 5-(4-hydroxyphenyl)-1-methyl-1H-pyrazole-3-carboxylate

To a solution of 4.1g (15.7mmol) of **intermediate 140** in CH₂Cl₂ was added 2eq of BBr₃ (1M in CH₂Cl₂). The reaction mixture was stirred at rt for 18h. The reaction mixture was dissolved in diethyl ether and ethanol and was washed with water, dried on Na₂SO₄ and evaporated. The residue was put in ethanol under HCl saturated atmosphere to give the esters of the acid starting material and the desired product, then it was purified by flash chromatography to give a brown solid (600mg).
Yield: 15%
¹H NMR (CDCl₃) δ: 7.65 (m, 1 H); 7.19 (d, 2H); 6.94 (d, 2H); 6.73 (s, 1 H); 4.33 (quad, 2H); 3.84 (s, 3H); 1.30 (t, 3H)

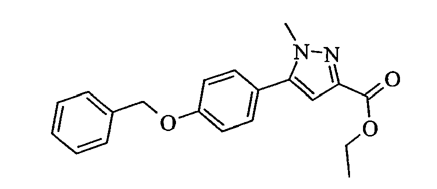

### Intermediate 142

### Ethyl 1-methyl-5-{4-[(phenylmethyl)oxy]phenyl}-1H-pyrazole-3-carboxylate

To a solution of 200 mg (0.81 mmol) of **intermediate 141** in acetone was added 340mg (3eq) of K₂CO₃ and 110µL (1.1 eq) of benzyl bromide. The reaction mixture was heated at 70°C for 18h. The reaction mixture was purified by flash chromatography (cyclohexane/CH₂Cl₂ 70/30 then CH₂Cl₂ ) (m=250mg).
Yield: 91%
¹H NMR (CDCl₃=7.18) δ: 7.40-7.16 (m, 7H); 6.96 (d, 2H); 6.70 (s, 1H); 5.00 (s, 2H); 4.31 (quad, 2H); 3.81 (s, 3H); 1.30 (t, 3H)

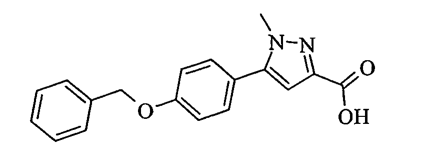

### Intermediate 143

### 1-methyl-5-{4-[(phenylmethyl)oxy]phenyl}-1H-pyrazole-3-carboxylic acid

A solution of 250 mg (0.74mmol) of **intermediate 142** in ethanol and an excess (5eq) of NaOH (1 N) was heated at 50°C for 4h. The resulting mixture was evaporated, diluted in water, acidified with HCl (1 N). The precipitate formed was filtered, washed with water and pentane (m=230mg).
Quantitative yield
¹H NMR (CDCl₃= 7.19) δ: 7.41-7.25 (m, 7H); 7.01 (d, 2H); 6.79 (s, 1H); 5.06 (s, 2H); 3.88 (s, 3H)

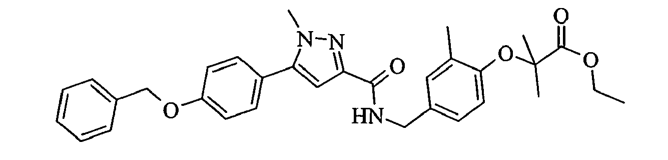

### Intermediate 144

### Ethyl 2-methyl-2-{[2-methyl-4-({[(1-methyl-5-{4-[(phenylmethyl)oxy]phenyl}-1H-pyrazol-3-yl)carbonyl]amino}methyl)phenyl]oxy}propanoate

A solution of 230mg (0.75mmol) of **intermediate 143** in toluene and 272µL (5eq) of SOCI₂ was heated at 80°C for 3h. The resulting solution was evaporated, diluted in toluene and evaporated again. The residue, diluted in CH₂Cl₂, was added to a solution of 260mg (1.2eq) of **intermediate 10** and 320µL (3eq) of Et₃N in CH₂Cl₂, and it was stirred at rt for 2h. The resulting mixture was washed with HCl (1N), NaOH (1N), water, dried on Na2SO4 and evaporated (m=200mg).
Yield: 49%
¹H NMR (CDCl₃= 7.10) δ: 7.29-7.08 (m, 8H); 7.01 (m, NH); 6.97 (s, 1H); 6.85 (d, 2H); 6.64 (s, 1H); 6.45 (d, 1H); 4.92 (s, 2H); 4.34 (d, 2H); 4.06 (quad, 2H); 3.64 (s, 3H); 2.04 (s, 3H); 1.41 (s, 6H); 1.08 (t, 3H)

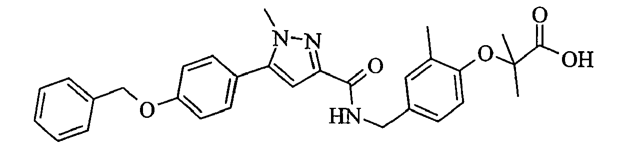

### Example 58

### 2-methyl-2-{[2-methyl-4-({[(1-methyl-5-{4-[(phenylmethyl)oxy]phenyl}-1H-pyrazol-3-yl)carbonyl]amino}methyl)phenyl]oxy}propanoic acid

A solution of 200 mg (0.37mmol) of **intermediate 144** in ethanol and an excess (5eq) of NaOH (1 N) was heated at 50°C for 4h. The resulting mixture was evaporated, diluted in water, acidified with HCl (1N). The precipitate formed was filtered, washed with water and pentane to give a white powder (240mg).
Quantitative yield.
¹H NMR (CDCl₃= 7.10) d: 7.32-7.14 (m, 8H); 7.03-7.01 (m, 2H); 6.90 (d, 2H); 6.67 (s, 1 H); 6.62 (d, 1 H); 4.96 (s, 2H); 4.36 (d, 2H); 3.68 (s, 3H); 2.07 (s, 3H); 1.45 (s, 6H)
Mp=120°C

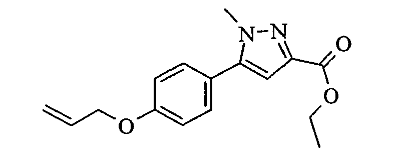

### Intermediate 145

### ethyl 1-methyl-5-[4-(2-propen-1-yloxy)phenyl]-1H-pyrazole-3-carboxylate

To a solution of 200 mg (0.81 mmol) of **intermediate 141** in acetone was added 340mg (3eq) of K₂CO₃ and 175µL (2.5eq) of 3-bromo-1-propene. The reaction mixture was heated at 70°C for 18h. The reaction mixture was purified by flash chromatography (cyclohexane/CH₂Cl₂ 70/30 then CH₂Cl₂ ) (m=160mg).
Yield: 65%
¹H NMR (CDCl₃=7.20) δ: 7.25 (d, 2H); 6.92 (d, 2H); 6.71 (s, 1H); 5.99 (m, 1H); 5.35 (d, 1H); 5.22 (d, 1 H); 4.50 (d, 2H); 4.33 (quad, 2H); 3.85 (s, 3H); 1.32 (t, 3H)

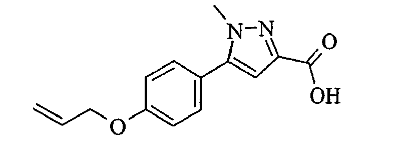

### Intermediate 146

### 1-methyl-5-[4-(2-propen-1-yloxy)phenyl]-1H-pyrazole-3-carboxylic acid

A solution of 160 mg (0.53mmol) of **intermediate 145** in ethanol and an excess (5eq) of NaOH (1 N) was heated at 50°C for 4h. The resulting mixture was evaporated, diluted in water, acidified with HCl (1 N). The precipitate formed was filtered, washed with water and pentane to give a white solid (140mg).
Yield: 97%
¹H NMR (CDCl₃=7.19) δ: 7.28 (d, 2H); 6.95 (d, 2H); 6.80 (s, 1 H); 6.01 (m, 1 H); 5.40 (d, 1 H); 5.28 (d, 1 H); 4.53 (d, 2H); 3.89 (s, 3H)

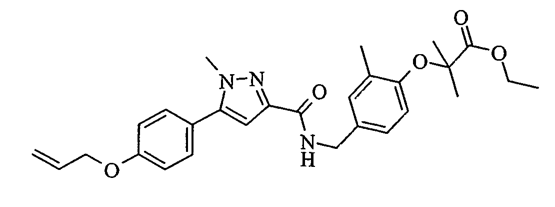

### Intermediate 147

### Ethyl-2-methyl-2-[(2-methyl-4-{[({1-methyl-5-[4-(2-propen-1-yloxy)phenyl]-1H-pyrazol-3-yl}carbonyl)amino]methyl}phenyl)oxy]propanoate

A solution of 140mg (0.51mmol) of **intermediate 146** in toluene and 200pL (5eq) of SOCl₂ was heated at 80°C for 3h. The resulting solution was evaporated, diluted in toluene and evaporated again. The residue diluted in CH₂Cl₂ was added to a solution of 180mg (1.2eq) of **intermediate 10** and 220µL (3eq) of Et₃N in CH₂CI₂, and it was stirred at rt for 2h. The resulting mixture was washed with HCI (1 N), NaOH (1 N), water, dried on Na₂SO₄ and evaporated (m=210mg).
Yield: 85%
1H NMR (CDCl₃=7.19) δ: 7.25 (d, 2H); 7.09-6.89 (m, 4H+ NH); 6.74 (s, 1 H); 6.54 (d, 1 H); 5.99 (m, 1 H); 5.37 (d, 1 H); 5.25 (d, 1 H); 4.51 (d, 2H); 4.45 (d, 2H); 4.17 (quad, 2H); 3.76 (s, 3H); 2.14 (s, 3H); 1.50(s, 6H); 1.19 (t, 3H);

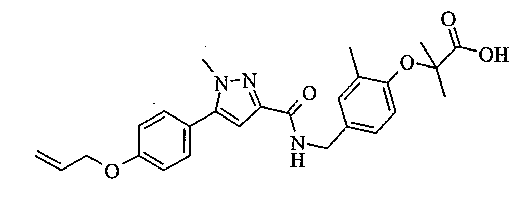

### Example 59

### 2-methyl-2-[(2-methyl-4-{[({1-methyl-5-[4-(2-propen-1-yloxy)phenyl]-1H-pyrazol-3-yl}carbonyl)amino]methyl}phenyl)oxy]propanoic acid

A solution of 210 mg (0.43mmol) of **intermediate 147** in ethanol and an excess (5eq) of NaOH (1 N) was heated at 50°C for 4h. The resulting mixture was evaporated, diluted in water, acidified with HCI (1 N). The precipitate formed was filtered, washed with water and pentane to give a white solid (120mg).
Yield: 61%
¹H NMR (CDCl₃=7.13) δ: 7.18 (d, 3H); 7.04 (s, 1 H); 6.92-6.83 (m, 3H); 6.69 (s, 1 H); 6.64 (d, 1 H); 5.94 (m, 1 H); 5.31 (d, 1 H); 5.19 (d, 1 H); 4.45 (d, 2H); 4.38 (d, 2H); 3.70 (s, 3H); 2.09 (s, 3H); 1.47 (s, 6H)
Mp=138°C

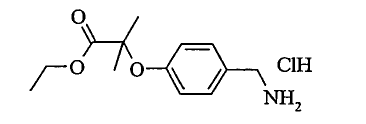

### Intermediate 148

### Ethyl 2-{[4-(aminomethyl)phenyl]oxy}-2-methylpropanoate hydrochloride

To a solution of 14.125g (61 mmol) of **intermediate 44** in 100mL of ethanol and 19.5mL of concentrated HCl under nitrogen, was added 1.41g of Pd/C 10% (10% w/w). The reaction was maintained at 15 bars pressure of hydrogen for 2 days. The reaction mixture was filtered through celite, washed with ethanol, evaporated to dryness and co-evaporated with CH₂CI₂ and dried under vacuum. The residue was co-evaporated again with CH₂CI₂ and dried under vacuum to give a dark solid (17.05g).
Quantitative yield
1 H NMR (DMSO=2.49) δ: 8.29 (s, NH2); 7.37 (d, 2H); 6.79 (d, 2H); 4.15 (d, 2H); 3.91 (s, 2H); 1.51 (s, 6H); 1.15 (t, 3H)

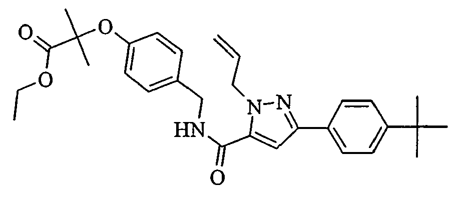

### Intermediate 149

### Ethyl-2-({4-[({[3-[4-(1,1-dimethylethyl)phenyl]-1-(2-propen-1-yl)-1H-pyrazol-5-yl]carbonyl}amino)methyl]phenyl}oxy)-2-methylpropanoate

To a solution of 2g (7.03mmol) of **intermediate 109** in 15 mL of DMF was added 1.05g (7.71mmol) of HOAT, 1.5g (7.8mmol) of EDCI, 3.1mL (22.2mmol) of Et3N and 3.9g (14.3mmol) of **intermediate 148** diluted in 15mL of DMF. The reaction mixture was stirred at rt for 18h30. The resulting mixture was evaporated, diluted in ethyl acetate, washed twice with water, once with brine, dried on MgSO4, evaporated and purified by flash chromatography (cyclohexane/ethyl acetate 80/20) to give a cream solid (1.82g).
Yield: 51%
1H NMR (DMSO=2.51) d: 9.05 (m, NH); 7.68 (d, 2H); 7.45 (d, 2H); 7.30 (s, 1H); 7.23 (d, 2H); 6.77 (d, 2H); 6.01 (m, 1H); 5.18 (d, 2H); 5.12 (d, 1H); 4.99 (d, 1H); 4.38 (d, 2H); 4.16 (quad, 2H); 1.51 (s, 6H); 1.30 (s, 9H); 1.25 (t, 3H)

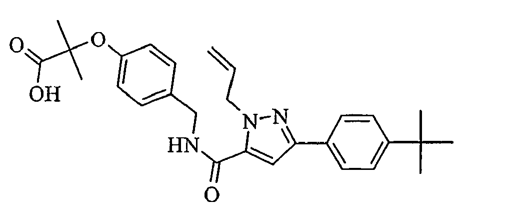

### Example 60

### 2-({4-[({[3-[4-(1,1-dimethylethyl)phenyl]-1-(2-propen-1-yl)-1H-pyrazol-5-yl]carbonyl}amino)methyl]phenyl}oxy)-2-methylpropanoic acid

A suspension of 1.82g (3.61 mmol) of **intermediate 149** in 20mL of ethanol and 7.3mL of NaOH (1N) was heated at 80°C for 1h15. The resulting solution was evaporated, diluted in water, acidified with HCl(1N) until pH=1. The precipitate formed was filtered, washed with water and dried under vacuum in presence of P₂O₅ (m=1.71g).
Quantitative yield
¹H NMR (DMSO=2.50) δ: 9.04 (m, NH); 7.68 (d, 2H); 7.45 (d, 2H); 7.30 (s, 1H); 7.22 (d, 2H); 6.78 (d, 2H); 6.00 (m, 1H); 5.19-4.95 (m, 4H); 4.38 (d, 2H); 1.49 (s, 6H); 1.30 (s, 9H)

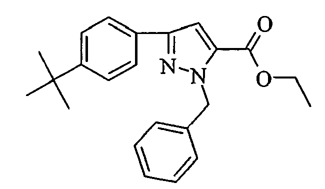

### Intermediate 150

### Ethyl-3-[4-(1,1-dimhylethyl)phenyl]-1-(phenylmethyl)-1H-pyrazole-5-carboxylate

To a solution of 3g (11.02mmol) of **intermediate 22** in acetone was added 3.05g (22.06mmol) of K₂CO₃. This was then stirred for 15 min. Then 2.62mL (22.04mmol) of (bromomethyl)benzene was added dropwise. The reaction mixture was refluxed overnight. The resulting mixture was filtered, washed with acetone, evaporated,purified by Flash chromatography (Cyclohexane/ethyl acetate 95/5) to give the desired isomer as the first fraction collected (3.59g).
Yield: 90%
¹H NMR (DMSO=2.50) δ: 7.81 (d, 2H); 7.44 (d, 2H); 7.39 (s, 1H); 7.36-7.27 (m, 3H); 7.18 (d, 2H); 5.77 (s, 2H); 4.30 (quad, 2H); 1.30 [(s, 9H) + (t, 3H)]

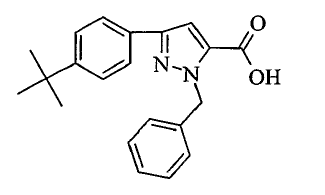

### Intermediate 151

### 3-[4-(1,1-dimethylethyl)phenyl]-1-(phenylmethyl)-1H-pyrazole-5-carboxylic acid

A solution of 3.59g (9.90mmol) of **intermediate 150** in 40mL of ethanol and 20mL of NaOH (1 N) was heated at 80°C for 1 h30. The resulting solution was evaporated, diluted in water, acidified with HCl(1N) until pH=1. The precipitate formed was filtered, washed with water and dried under vacuum in presence of P₂O₅ (m=3.05g).
Yield: 92%
¹H NMR (DMSO=2.51) δ: 7.77 (d, 2H); 7.43 (d, 2H); 7.35-7.26 (m, 4H); 7.17 (d, 2H); 5.78 (s, 2H); 1.29 (s, 9H)

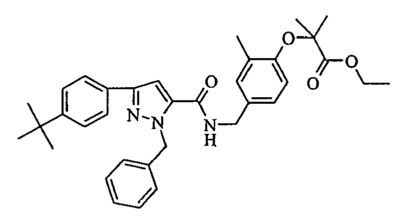

### Intermediate 152

### Ethyl-2-({4-[({[3-[4-(1,1-dimethylethyl)phenyl]-1-(phenylmethyl)-1H-pyrazol-5-yl]carbonyl}amino)methyl]-2-methylphenyl}oxy)-2-methylpropanoate

To a solution of 4.3g (13.8mmol) of **intermediate 10** in 20 mL of DMF was added 1.37g (10.1mmol) of HOAT, 1.94g (10.1mmol) of EDCI, 4mL (28.7mmol) of Et₃N and 3.05g (9.12mmol) of **intermediate 152** diluted in 20mL of DMF. The reaction mixture was stirred at rt for 67h30. The resulting mixture was evaporated, diluted in ethyl acetate and water. The layers were separated, the aqueous one was washed with ethyl acetate. The organic layers were joined together, washed with water, brine, dried on MgSO₄, evaporated and purified by flash chromatography (cyclohexane/ethyl acetate 80/20) (pale yellow solid, m=2.86g).
Yield: 41%
¹H NMR (DMSO=2.50) δ: 9.02 (m, 1H); 7.72 (d, 2H); 7.69 (d, 2H); 7.49-7.17 (m, 7H); 7.09 (d, 1 H); 6.95 (d, 1 H); 5.78 (s, 2H); 4.33 (d, 2H); 4.18 (quad, 2H); 2.13 (s, 3H); 1.51 (s, 6H); 1.30 (s, 9H); 1.18 (t, 3H).

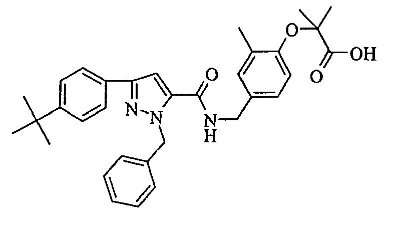

### Example 61

### 2-({4-[({[3-[4-(1,1-dimethylethyl)phenyl]-1-(phenylmethyl)-1H-pyrazol-5-yl]carbonyl}amino)methyl]-2-methylphenyl}oxy)-2-methylpropanoic acid

A suspension of 1.585g (2.79mmol) of **intermediate 152** in 30mL of ethanol and 5.6mL of NaOH (1 N) was heated at 80°C for 1 h30. The resulting solution was evaporated, diluted in water, acidified with HCl(1N) until pH=1. The precipitate formed was filtered, washed with water and dried under vacuum in presence of P₂O₅ (white solid, m=1.34g).
Yield: 89%
¹H NMR (DMSO= 2.50) δ: 9.03 (m, NH); 7.69 (d, 2H); 7.45 (d, 2H); 7.03-7.25 (m, 6H); 7.07 (s, 1 H); 6.92 (d, 1 H); 6.64 (d, 1 H); 5.78 (s, 2H); 4.33 (d, 2H); 2.12 (s, 3H); 1.49 (s, 6H); 1.30 (s, 9H)

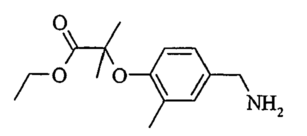

### Intermediate 153

### Ethyl 2-{[4-(aminomethyl)-2-methylphenyl]oxy}-2-methylpropanoate acetate

To a solution of 22.76g (85.84mmol) of **intermediate 9** in 85mL of ethanol and 4.95mL of acetic acid under nitrogen was added 2.3g of Pd/c (10%). The reaction was maintained at 15 bars of hydrogen pressure for 64h30. The resulting mixture was filtered through a celite filter, washed with ethanol, concentrated to dryness and purified by flash chromatography (dichloromethane/methanol 80/20) to give a yellow oil (18.93g).
Yield: 71%
¹H NMR (DMSO= 2.50) δ: 7.15 (s, 1H); 7.04 (d, 1H); 6.56 (d, 1H); 4.18 (quad, 2H); 3.65 (s, 2H); 2.15 (s, 3H); 1.50 (s, 6H); 1.18 (t, 3H)

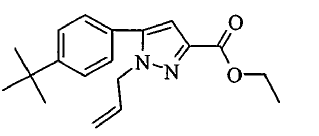

### Intermediate 154

### Ethyl-5-[4-(1,1-dimethylethyl)phenyl]-1-(2-propen-1-yl)-1H-pyrazole-3-carboxylate

To a solution of 25.79g (94.8mmol) of **intermediate 22** in 210mL of acetone was added 26.2g (0.19mol) of K₂CO₃ and it was stirred for 20 min. Then 16.5mL (0.19mol) of 3-bromo-1-propene was added and the reaction mixture was refluxed for 20h. The resulting mixture was filtered, washed with acetone, evaporated and purified by Flash chromatography (Cyclohexane/ethyl acetate 90/10 to 80/20) to give the desired isomer as the second fraction collected (yellow oil, 5.9g).
Yield: 20%
¹H NMR (DMSO=2.50) δ: 7.70 (m, NH); 7.53 (d, 2H); 7.45 (d, 2H); 6.88 (s, 1H); 6.01 (m, 1 H); 5.21 (d, 1 H); 4.92 (d, 1 H); 4.84 (d, 2H); 4.29 (quad, 2H); 1.33 [(t, 3H) + (s, 9H)]

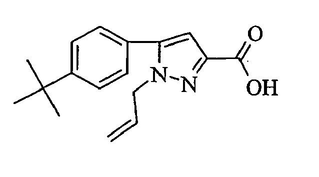

### Intermediate 155

### 5-[4-(1,1-dimethylethyl)phenyl]-1-(2-propen-1-yl)-1H-pyrazole-3-carboxylic acid

A solution of 5.9 g (18.9mmol) of **intermediate 154** in 80 mL of ethanol and 38 mL of NaOH (1 N) was refluxed for 1 h30. The resulting mixture was evaporated, diluted in water and acidified with HCl(1N). The precipitate formed was filtered, washed with water and dried under vacuum in presence of P₂O₅ to give a cream solid (4.67g).
Yield: 87%
¹H NMR (DMSO=2.50) δ: 7.53 (d, 2H); 7.44 (d, 2H); 6.82 (s, 1 H); 6.02 (m, 1 H); 5.19 (d, 1 H); 4.90 (d, 1 H); 4.82 (d, 2H); 1.32 (s, 9H)

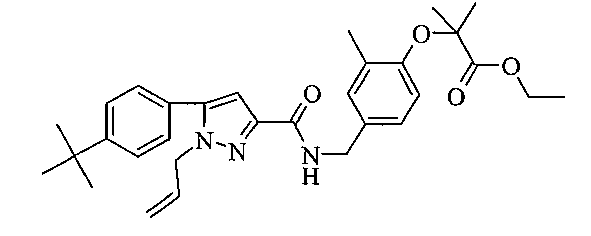

### Intermediate 156

### Ethyl 2-({4-[({[5-[4-(1,1-dimethylethyl)phenyl]-1-(2-propen-1-yl)-1H-pyrazol-3-yl]carbonyl}amino)methyl]-2-methylphenyl}oxy)-2-methylpropanoate

To a solution of 2.5g (8.8mmol) of **intermediate 155** in 20 mL of DMF was added 1.32g (9.7mmol) of HOAT, 1.86g (9.7mmol) of EDCI, 3.8mL (27.3mmol) of Et₃N and 4.11g (13.2mmol) of **intermediate 153** diluted in 20mL of DMF. The reaction mixture was stirred at rt overnight. The resulting mixture was evaporated, diluted in ethyl acetate and water. The layers were separated and the aqueous layer was washed with ethyl acetate. The organic layers were joined together, washed three times with water, once with brine, dried on MgSO₄, evaporated and purified by flash chromatography (cyclohexane/ethyl acetate 70/30) to give a yellow oil (2.34g).
Yield: 51 %
¹H NMR (DMSO=2.5) δ: 7.53 (d, 2H); 7.43 (d, 2H); 7.12 (s, 1 H); 7.02 (d, 1 H); 6.77 (s, 1 H); 6.56 (d, 1 H); 6.0 (m, 1 H); 5.19 (d, 1 H); 4.89 (d, 1 H); 4.81 (d, 2H); 4.32 (d, 2H); 4.15 (quad, 2H); 2.15 (s, 3H); 1.5 (s, 6H); 1.33 (s, 9H); 1.18 (t, 3H)

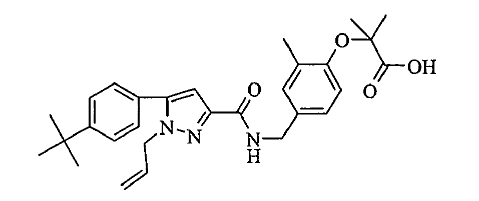

### Example 62

### 2-({4-[({[5-[4-(1,1-dimethylethyl)phenyl]-1-(2-propen-1-yl)-1H-pyrazol-3-yl]carbonyl}amino)methyl]-2-methylphenyl}oxy)-2-methylpropanoic acid

A solution of 2.34 g (4.52mmol) of **intermediate 156** in 30 mL of ethanol and 9 mL of NaOH (1 N) was refluxed for 2h. The resulting solution was evaporated, diluted in water, acidified with HCI(1N) until pH1 was obtained. The gummy residue formed was filtered, diluted in CH₂Cl₂, dried on MgSO₄ and evaporated to give a yellow solid (1.75g).
Yield: 79%
¹H NMR (DMSO=2.5) δ: 8.58 (m, NH); 7.53 (d, 2H); 7.44 (d, 2H); 7.12 (s, 1 H); 7.03 (d, 1 H); 6.78 (s, 1 H); 6.65 (d, 1 H); 6.05 (m, 1 H); 5.2 (d, 1 H); 4.90 (d, 1 H); 4.8 (d, 2H); 3.34 (d, 2H); 2.15 (s, 3H); 1.5 (s, 6H); 1.2 (s, 9H)

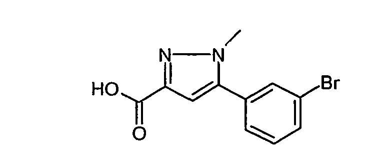

### Intermediate 157

### Ethyl-2-({4-[({[5-(3-bromophenyl)-1-methyl-1H-pyrazol-3-yl]carbonyl}amino)methyl]-2-methylphenyl}oxy)-2-methylpropanoate

A solution of 5 g (16mmol) of **intermediate 60** in 5 mL of THF, 50 mL of ethanol and 80 mL of NaOH (1 N) was stirred at rt for 3h. The resulting solution was evaporated, dissolved in 100 mL of water and acidified with a dropwise addition of HCI (1 N) to give white precipitate.

The resulting mixture was stirred at rt for 1h, filtered, to give after drying under vacuum a white solid (4.62g).
Quantitative yield
¹H NMR (CDCl₃=7.16) δ: 7.86-7.81 (m, 2H); 7.62-7.58 (m, 2H); 7.49 (s, 1H); 4.21 (s, 3H)

### Example 1

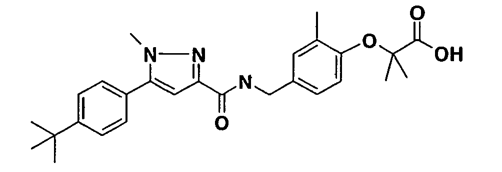

### 2-[(4-{[({5-[4-(1,1-dimethylethyl)phenyl]-1-methyl-1H-pyrazol-3-yl}carbonyl)amino]methyl}-2-methylphenyl)oxy]-2-methylpropanoic acid

This compound was also prepared by the method disclosed in Scheme 3 as follows.

Preparation of the Intermediate of formula III:

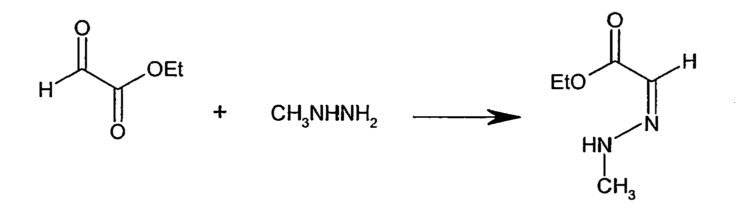

### Intermediate a

### Methylhydrazonol acetic acid ethyl ester

Methyl hydrazine (50 mL, 0.94 mol, 1.0 eq) was dissolved in toluene and cooled to 10°C. Ethyl glyoxylate (50% solution in toluene, 200.5 g of the solution, 1.03 mol, 1.1 eq) was added dropwise maintaining the temperature below 25°C. THF (50 mL) was used to rinse the addition funnel and was added to the reaction mixture. The reaction was heated to 50°C for 5 h. At temperature, THF and any volatiles (i.e. methyl hydrazine or toluene) was distilled off and heptanes was added to the reaction mixture. The reaction was cooled to 5-10°C during which time, solids began to precipitate out. After 1 h held at 0°C, the reaction was filtered and the cake washed with tbme/heptanes. Product was a pink to purple solid (isolated 93 g, 77% yield, 96% HPLC purity).

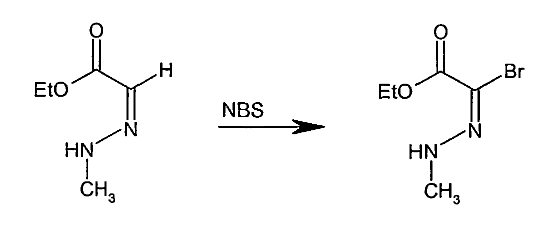

### Intermediate b

### Bromo[methylhydrazono] acetic acid ethyl ester

N-Bromosuccinimide (34.2 g, 0.19 mol, 1.0 eq.) was added as a solid in portions to a slurry of Intermediate a (25g, 0.19 mol, 1.0 eq.)in ethyl acetate (125 mL) so as to maintain the temperature at less than 25°C. The reaction was stirred at 5-10°C for 1 h. Once the reaction was deemed complete, it is filtered and the cake was washed with ethyl acetate (75 mL) which was added to the filtrate. The combined filtrate and wash were bottled and stored at 5-10°C for use in the next step. Yield was > 95% solution yield via HPLC.

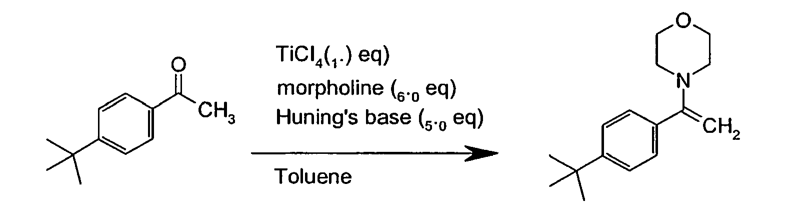

### Intermediate c

### 4-[1-(4- tert-butyl-phenyl)-vinyl]-morpholine

Morpholine was added to slurry of toluene (60 mL) and sodium sulfate (50 g, which served as a mechanical abrasive and flocculent) at 5°C. Titanium tetrachloride (30 mL, 0.34 mol, 6.0 eq was added dropwise to form a light green slurry (titanium tetrachloride - morpholine complex). Hunig's base (48 mL, 0.28 mol, 5.0 eq) was added followed by t-butyl acetophenone (10.0 g, 0.056 mol, 1.0 eq). The ice bath was removed and the reaction was heated to 70°C for 3 h. Once the reaction was deemed complete, it was cooled to 20°C and filtered. The cake was washed with toluene which was added to the filtrate. The filtrate (containing Intermediate c) was drummed out for use in the next step. The cake was quenched with water and discarded. The aqueous filtrate was also discarded. Solution yield was > 95% via GC.

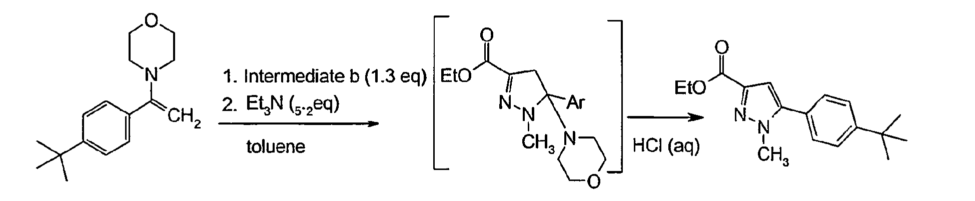

### Intermediate d

### 1-Methyl-5-(4-tert-butylphenyl)-2H-pyrazole-3-(carboxylic acid ethyl ester)

The toluene solution of t-butyl acetophenone enamine Intermediate c (0.14 mol, 34.8 g, 1.0 eq) from the previous step was distilled under reduced pressure until the morpholine and Hunig's base content was less than < *10 mol% each with respect to* Intermediate c. To the resulting concentrated solution of Intermediate c was added ethyl acetate (210 mL) and triethylamine (115 mL, 0.82 mol, 5.8 eq) at ambient temperature. The mixture was heated to 40°C at which time an ethyl acetate (210 mL) solution of Intermediate b (43.0 g, 0.21 mmol, 1.45 eq) was added dropwise over half an hour. The reaction was held at 40 °C for an additional 3.5 h. After the reaction was deemed complete, the reaction was cooled to 0-5°C and 4N HCl (284 mL, 1.1 mol, 8.0 eq) was added dropwise so as to maintain the temperature below 30°C. After acidification was complete, the mixture stirred for an additional hour at 20°C. The phases were allowed to separate and aqueous layer discarded. The organic layer was washed with fresh water (150 mL) and then separated via phase cut. The organic layer was distilled under reduced pressure to remove ethyl acetate in preparation for the hydrolysis step. Solution yield is approximately 80-85%. Alternatively, Intermediate d maybe isolated from the organic phase by precipitation from toluene/ethyl acetate/heptanes.

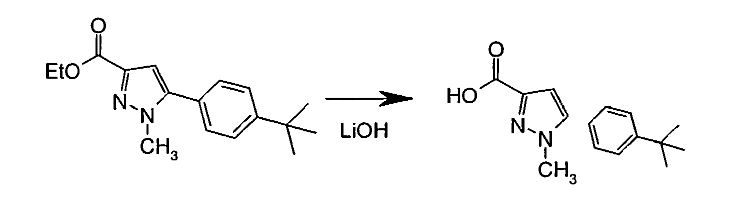

### Intermediate e

### 1-Methyl-5-(4-tert-butylphenyl)-2H-pyrazole-3-(carboxylic acid)

To a solution of pyrazole ester Intermediate d in toluene was added THF (150 mL). LiOH (17.9 g, 0.43 mol, 4 eq.) in 180 mL water was added and the reaction was heated to 60°C for 4-6 h. After the reaction was deemed complete, the THF (150-200 mL) was distilled off. TbMe (150mL) was added and water (100 mL) was added and stirred and the phases cut. The aqueous layer (containing the lithium salt of Intermediate e) was washed with fresh tbMe (120 mL, to remove any remaining organic impurities) and then phase cut. Isopropanol (30 mL) was added to the aqueous layer which was acidified to pH = 1 with 6N HCI (85 mL). During the acidification, white/yellow solids precipitate out. After filtration, the cake was washed with water (100 mL) followed by a wash with tBME/heptanes (10:90). Isolated yield was 21.0 g, ~81% from Intermediate d.

### Intermediate f

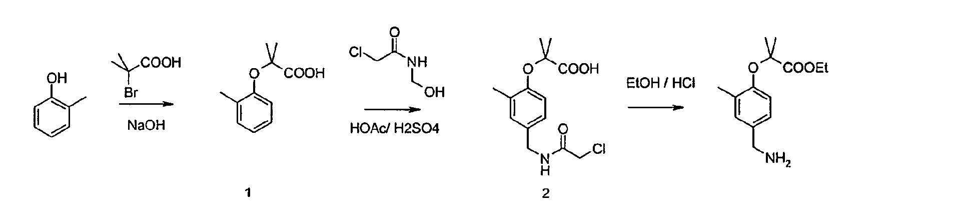

Cresol was reacted with α-bromoisobutyric acid in the presence of NaOH to give acid 1, which was then amidomethylated using N-hydroxymethyl chloroacetamide in HOAc/H₂SO₄ to afford acid-amide 2. The amide was alcoholyzed in ethanol using HCl gas with concomitant ester formation to give the desired product Intermediate f.

Intermediate f and Intermediate e were coupled together to form a compound of Example 1 in a manner similar to that described for the alternative route to Example 1 above.

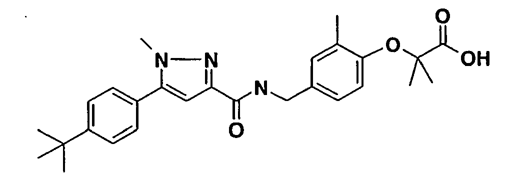

### BINDING AND TRANSFECTION ASSAYS

### Binding Assay:

Compounds were tested for their ability to bind to hPPAR gamma hPPARalpha or PPARdelta using a Scintillation Proximity Assay (SPA). The PPAR ligand binding domain (LBD) was expressed in E. coli as polyHis tagged fusion proteins and purified. The LBD was then labelled with biotin and immobilised on streptavidin-modified scintillation proximity beads. The beads were then incubated with a constant amount of the appropriate radioligand (5-{4-[2-(Methyl-pyridin-2-yl-amino)-ethoxy]-benzyl}-thiazolidine-2,4-dione *(*J.Med.Chem. 1994, 37(23), 3977), for PPARgamma), and labelled GW 2433 (see Brown, P. J et al . Chem. Biol., 4, 909-918 (1997*),* for the structure and synthesis of this ligand) for PPAR alpha and PPAR delta) and variable concentrations of test compound, and after equilibration the radioactivity bound to the beads was measured by a scintillation counter. The amount of nonspecific binding, as assessed by control wells containing 50 µM of the corresponding unlabeled ligand, was subtracted from each data point. For each compound tested, plots of ligand concentration vs. CPM of radioligand bound were constructed and apparent KI values were estimated from nonlinear least squares fit of the data assuming simple competitive binding. The details of this assay have been reported elsewhere (see, Blanchard, S. G. et. al. Development of a Scintillation Proximity Assay for Peroxisome Proliferator-Activated Receptor gamma Ligand Binding Domain. Anal. Biochem., 257, 112-119 (1998)).

### Transfection assay:

Compounds were screened for functional potency in transient transfection assays in CV-1 cells for their ability to activate the PPAR subtypes (transactivation assay). A previously established chimeric receptor system was utilized to allow comparison of the relative transcriptional activity of the receptor subtypes on the same target gene and to prevent endogenous receptor activation from complicating the interpretation of results. See, for example, Lehmann, J. M.; Moore, L. B.; Smith-Oliver, T. A.; Wilkison, W. O.; Willson, T. M.; Kliewer, S. A., An antidiabetic thiazolidinedione is a high affinity ligand for peroxisome proliferator-activated receptor gamma (PPARgamma), J. Biol. Chem., 270, 12953-6 (1995*).* The ligand binding domains for murine and human PPAR alpha, PPAR gamma, and PPAR delta were each fused to the yeast transcription factor GAL4 DNA binding domain. CV-1 cells were transiently transfected with expression vectors for the respective PPAR chimera along with a reporter construct containing five copies of the GAL4 DNA binding site driving expression of secreted placental alkaline phosphatase (SPAP) and beta-galactosidase. After 16 h, the medium was exchanged to DME medium supplemented with 10% delipidated fetal calf serum and the test compound at the appropriate concentration. After an additional 24h, cell extracts were prepared and assayed for alkaline phosphatase and β-galactosidase activity. Alkaline phosphatase activity was corrected for transfection efficiency using the beta-galactosidase activity as an internal standard (see, for example, Kliewer, S. A., et. al. Cell 83, 813-819 (1995)). Rosiglitazone (BRL 49653) was used as a positive control in the hPPAR gamma assay. The positive control in the hPPAR alpha assays was 2-4-[2-(3-[4-fluorophenyl]-1-heptylureido)ethyl]-phenoxy-(2-methyl propionic acid (WO 97/36579). The positive control for PPAR delta assays was 2-{2-methyl-4-[({4-methyl-2-{trifluoromethyl)phenyl]-1,3-thiazol-5-yl}methyl)sulfanyl]phenoxy}acetic acid (WO 01/00603). The positive control was (5-{4-[2-(Methyl-pyridin-2-yl-amino)-ethoxy]-benzyl}-thiazolidine-2,4-dione *(*J.Med.Chem. 1994, 37(23), 3977), for PPAR gamma.

Activities in three hPPAR subtypes are reported in the table and are expressed in micromolar.

| **Example** | **EC50 µM HPPARα** | **EC50 µM HPPARδ** | **EC50 µM HPPARγ** |
|---|---|---|---|
| Example 1 | 0.014 | 5.447 | 0.007 |
| Example 2 | 0.018 | 0.820 | 0.044 |
| Example 3 | 0.100 | >10 | 0.212 |
| Example 4 | 0.066 | 1.087 | 0.072 |
| Example 5 | 0.301 | >10 | 0.444 |
| Example 7 | 0.002 | 2.060 | 0.008 |
| Example 8 | 0.051 | 1.069 | 0.059 |
| Example 10 | 2.895 | >10 | 0.554 |
| Example 11 | 0.050 | 2.166 | 0.029 |
| Example 12 | 1.054 | >10 | 0.432 |
| Example 13 | 0.009 | >10 | 0.011 |
| Example 14 | 0.177 | >10 | 0.130 |
| Example 15 | 0.075 | 2.439 | 0.024 |
| Example 16 | 0.163 | 10.000 | 1.090 |
| Example 17 | 0.070 | 10.000 | 0.376 |
| Example 18 | 0.004 | 4.021 | 0.009 |
| Example 20 | 0.076 | 1.063 | 0.020 |
| Example 21 | 0.010 | 1.122 | 0.005 |
| Example 22 | 0.020 | 1.552 | 0.024 |
| Example 23 | 0.067 | 4.33 | 0.779 |
| Example 25 | 0.002 | >25 | 0.008 |
| Example 26 | 0.003 | 1.00 | 0.016 |
| Example 27 | <0.001 | 0.206 | 0.031 |
| Example 28 | 0.589 | >25 | 1.466 |
| Example 29 | 0.029 | >25 | 0.014 |
| Example 30 | 0.046 | 3.118 | 0.229 |
| Example 31 | 0.093 | >25 | 0.026 |
| Example 32 | 0.015 | >25 | 0.023 |
| Example 33 | 0.004 | 1.639 | 0.028 |
| Example 34 | 0.734 | >25 | 0.309 |
| Example 35 | 0.024 | >25 | 0.054 |
| Example 36 | 0.176 | 5.17 | 0.037 |
| Example 37 | 0.006 | 0.449 | 0.021 |
| Example 38 | 0.010 | >25 | 0.072 |
| Example 39 | 1.06 | >25 | 1.044 |
| Example 40 | 0.293 | 5.585 | 0.187 |
| Example 41 | 0.504 | >25 | 0.151 |
| Example 42 | 0.293 | >25 | 1.664 |
| Example 43 | 0.257 | >25 | 0.138 |
| Example 44 | 1.30 | >25 | 0.006 |
| Example 45 | 0.836 | >25 | 0.049 |
| Example 46 | 0.294 | >25 | 0.353 |
| Example 47 | 0.044 | 1.149 | 0.059 |
| Example 48 | 0.066 | 2.048 | 0.303 |
| Example 49 | 0.257 | 1.173 | 0.052 |
| Example 50 | 0.204 | >25 | 1.702 |
| Example 51 | 0.300 | >25 | 0.140 |
| Example 52 | 0.042 | >25 | 0.478 |
| Example 53 | 0.383 | >25 | 0.269 |
| Example 54 | 0.571 | >25 | >25 |
| Example 55 | 0.444 | >25 | 0.748 |
| Example 56 | 0.894 | >25 | 0.287 |
| Example 57 | 0.657 | >8 | 0.118 |
| Example 59 | 0.094 | >25 | 0.028 |
| Example 60 | 0.192 | 5.928 | 1.380 |
| Example 61 | 0.115 | 2.42 | 0.147 |
| Example 62 | 0.032 | >25 | 0.069 |

## Claims

1. A compound of formula (I) and pharmaceutically acceptable salts, solvates and hydrolysable esters thereof 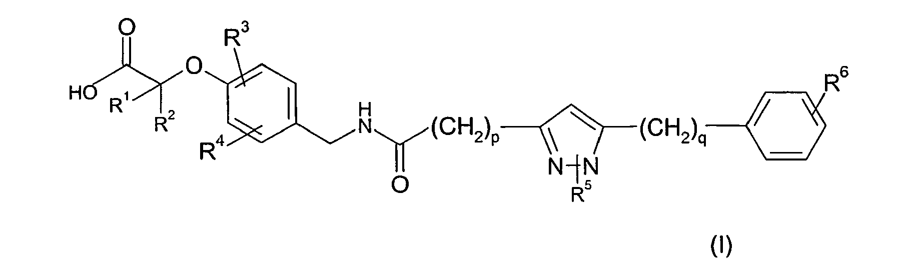 wherein:
p is O or 1;
q is O or 1;
R¹ and R² are independently H or C₁₋₃ alkyl;
R³ and R⁴ are independently H, C₁₋₆ alkyl, -OC₁₋₆ alkyl, halogen, OH, C₂₋₆ alkenyl or CF₃;
R⁵ is H, C₁₋₆ alkyl (optionally substituted by one or more halogens, -COphenyl, OC₁₋₆ alkyl, phenyl, morpholino) or C₂₋₆ alkenyl.
R⁶ is C₁₋₆ alkyl, halogen, -OCH₂ phenyl, phenyl (optionally substituted by C₁₋₃ alkiyl), morpholino, pyrrolidino, piperidino, thiophenyl, furanyl pyridinyl or -OC₂₋₆ alkenyl.

2. A compound according to claim 1 which is a selective hPPAR alpha/gamma agonist.

3. A compound according to claim 1 wherein p is 0.

4. A compound according to claim 1 wherein q is 0.

5. A compound according to claims 1 to 3 wherein p and q are 0.

6. A compound according to claims 1 - 5 wherein R¹ and R² are independently C₁₋₃ alkyl.

7. A compound according to claim 6 wherein R¹ and R² are both C₁₋₃alkyl.

8. A compound according to claim 7 wherein R¹ and R² are both methyl.

9. A compound according to claims 1 - 8 wherein R⁴ is H.

10. A compound according to claims 1 - 9 wherein R³ is -C₁₋₃ alkyl or -OC₁₋₃ alkyl

11. A compound according to claim 10 wherein R³ is methyl or -OCH₃.

12. A compound according to claims 1-11 wherein R³ is ortho to the depicted oxygen.

13. A compound according to claims 1 - 9 wherein R³ is methyl.

14. A compound according to claims 1-10 wherein R⁶ is C₁₋₆ alkyl.

15. A compound according to claims 1 - 11 wherein R⁶ is in the para position.

16. A compound according to claim 1 selected from:
2-[(4-{[({5-[4-(1,1-dimethylethyl)phenyl]-1-methyl-1*H*-pyrazol-3-yl}carbonyl)amino]methyl}-2-methylphenyl)oxy]-2-methylpropanoic add;
2-methyl-2-[(2-methyl-4-{[({1-methyl-3-[4-(1-methylethyl)phenyl]-1*H*-pyrazol-5-yl}carbonyl)amino]methyl}phenyl)oxy]propanoic acid;
2-{[4-{[({3-[4-(1,1-dimethylethyl)phenyl]-1-methyl-1*H*-pyrazol-5-yl}carbonyl)amino]methyl}-2-(2-propen-1-yl)phenyl]oxy}-2-methylpropanoic acid;
2-[(4-{[({3-[4-(1,1-dimethylethyl)phenyl]-1-methyl-1*H*-pyrazol-5-yl}carbonyl)amino]methyl}-2-propylphenyl)oxy]-2-methylpropanoic acid;
2-{[4-{[({3-[4-(1,1-dimethylethyl)phenyl]-1-ethyl-1*H*-pyrazol-5-yl}carbonyl)amino]methyl}-2-(methyloxy)phenyl]oxy}-2-methylpropanoic acid;
2-{[4-{[({5-[4-(1,1-dimethylethyl)phenyl]-1-ethyl-1*H*-pyrazol-3-yl}carbonyl)amino]methyl]-2-(methyloxy)phenyl]oxy}-2-methylpropanoic acid;
2-methyl-2-[(2-methyl-4-{[({1-methyl-5-[4-(2-methylpropyl)phenyl]-1H-pyrazol-3-yl}carbonyl)amino]methyl}phenyl)oxy]propanoic acid;
2-methyl-2-[(2-methyl-4-{[({1-methyl-3-[4-(2-methylpropyl)phenyl]-1*H*-pyrazol-5-yl}carbonyl)amino]methyl}phenyl)oxy]propanoic acid;
2-methyl-2-{[4-{[({1-methyl-5-[4-(-methylethyl)phenyl]-1*H*-pyrazol-3-yl}carbonyl)amino]methyl}-2-(methyloxy)phenyl]oxy}propanoic acid;
2-methyl-2-{[4-{[({1-methyl-3-[4-(1-methylethyl)phenyl]-1*H*-pyrazol-5-yl}carbonyl)amino]methyl}-2-(methyloxy)phenyl]oxy}propanoic acid;
2-[(4-{[({3-[4-(1,1-dimethylethyl)phenyl]-1-methyl-1*H*-pyrazol-5-yl}carbonyl)amino]methyl}-2-methylphenyl)oxy]-2-methylpropanoic acid;
2-{[4-{[({3-[4-(1,1-dimethylethyl)phenyl]-1-methyl-1*H*-pyrazol-5-yl}carbonyl)amino]methyl}-2-(methyloxy)phenyl]oxy}-2-methylpropanoic acid;
2-[(4-{[({5-[4-(1,1-dimethylethyl)phenyl]-1-ethyl-1 *H*-pyrazol-3-yl}carbonyl)amino]methyl}-2-methylphenyl)oxy]-2-methylpropanoic acid;
2-methyl-2-{[4-{[({1-methyl-5-[4-(2-methylpropyl)phenyl]-1*H*-pyrazol-3-yl}carbonyl)amino]methyl}-2-(methyloxy)phenyl]oxy}propanoic acid;
2-[(4-{[({3-[4-(1,1-dimethylethyl)phenyl]-1-ethyl-1*H*-pyrazol-5-yl}carbonyl)amino]methyl}-2-methylphenyl)oxy]-2-methyl propanoic acid;
2-[(4-{[({3-[4-(1,1-dimethylethyl)phenyl]-1-methyl-1*H*-pyrazol-5-yl}carbonyl)amino]methyl}phenyl)oxy]-2-methylpropanoic acid;
2-[(4-{[({5-[4-(1,1-dimethylethyl)phenyl]-1-methyl-1*H*-pyrazol-3-yl}carbonyl)amino]methyl}phenyl)oxy]-2-methylpropanoic acid;
2-methyl-2-[(2-methyl-4-{[({1-methyl-5-[4-(1-methylethyl)phenyl]-1*H*-pyrazol-3-yl}carbonyl)amino]methyl}phenyl)oxy]propanoic acid;
2-methyl-2-[(2-methyl-4-{[({1-methyl-5-[4-(4-morpholinyl)phenyl]-1*H*-pyrazol-3-yl}carbonyl)amino]methyl}phenyl)oxy]propanoic acid;
2-methyl-2-[(2-methyl-4-{[({1-methyl-5-[4-(1-pyrrolidinyl)phenyl]-1*H*-pyrazol-3-yl}carbonyl)amino]methyl}phenyl)oxy]propanoic acid;
2-methyl-2-[(2-methyl-4-{[({1-methyl-5-[4-(1-piperidinyl)phenyl]-1*H*-pyrazol-3-yl}carbonyl)amino]methyl}phenyl)oxy]propanoic acid;
2-({4-[({[5-(4-biphenylyl)-1-methyl-1*H*-pyrazol-3-yl]carbonyl}amino)methyl]-2-methylphenyl}oxy)-2-methylpropanoic acid;
2-methyl-2-[(2-methyl-4-{[({1-methyl-3-[3-(1-pyrrolidinyl)phenyl]-1*H*-pyrazol 5-yl}carbonyl)amino]methyl}phenyl)oxy]propanoic acid;
2-({4-[({[3-(4-biphenylyl)-1-methyl-1*H*-pyrazol-5-yl]carbonyl}amino)methyl]-2-methylphenyl}oxy)-2-methylpropanoic acid;
2-{4-[({[5-(4-tert-butylphenyl)-1-methyl-1*H*-pyrazol-3-yl]carbonyl}amino) methyl]-2,6-dimethylphenoxy}-2-methylpropanoic acid;
2-{4-[({[3-(4-*tert*-butylphenyl)-1-methyl-1*H*-pyrazol-5-yl]carbonyl}amino)methyl]-2,6-dimethylphenoxy}-2-methylpropanoic acid;
2-{2-chloro-4-[({[5-(4-isobutylphenyl)-1-methyl-1 H-pyrazol-3-yl]carbonyl}amino)methyl]-6-methylphenoxy}-2-methylpropanoic acid;
2-[4-({[(3-biphenyl-3-yl-1-methyl-1*H*-pyrazol-5-yl)carbonyl]amino}methyl)-2-methylphenoxy]-2-methylpropanoic acid;
2-({4-[({[5-(4-butylphenyl)-1-methyl-1*H*-pyrazol-3-yl]carbonyl}amino)methyl]-2-methylphenyl}oxy)-2-methylpropanoic acid;
2-({4-[({[5-(4-bromophenyl)-1-methyl-1*H*-pyrazol-3-yl]carbonyl}amino)methyl]-2-methylphenyl}oxy)-2-methylpropanoic acid;
2-methyl-2-{2-methyl-4-[({[1-methyl-5-(2'-methylbiphenyl-4-yl)-1*H*-pyrazol-3-yl]carbonyl}amino)methyl]phenoxy}propanoic acid;
2-methyl-2-[(2-methyl-4-{[({1-methyl-5-[4-(2-thienyl)phenyl]-1*H*-pyrazol-3-yl}carbonyl)amino]methyl}phenyl)oxy]propanoic acid;
2-[(4-{[({5-[4-(3-furanyl)phenyl]-1-methyl-1*H*-pyrazol-3-yl}carbonyl)amino]methyl}-2-methylphenyl)oxy]-2-methylpropanoic acid;
2-methyl-2-[(2-methyl-4-{[({1-methyl-5-[4-(4-pyridinyl)phenyl]-1*H*-pyrazol-3-yl}carbonyl)amino]methyl}phenyl)oxy]propanoic acid;
2-[(4-{[({5-[4-(2-furanyl)phenyl]-1-methyl-1*H*-pyrazol-3-yl}carbonyl)amino]methyl}-2-methylphenyl)oxy]-2-methylpropanoic acid;
2-methyl-2-{2-methyl-4-[({[1-methyl-3-(2'-methylbiphenyl-4-yl)-1*H*-pyrazol-5-yl]carbonyl}amino)methyl]phenoxy}propanoic acid;
2-{4-[({[5-(4-butylphenyl)-1-methyl-1*H*-pyrazol-3-yl]carbonyl}amino)methyl]-2,6-dimethylphenoxy}-2-methylpropanoic acid;
2-{[4-({2-{4-[({[5-(4-butylphenyl)-1-methyl-1*H*-pyrazol-3-yl]carbonyl}amino)methyl]-2-chloro-6-methylphenoxy}-2-methylpropanoic acid;
2-methyl-2-[(2-methyl-4-{[({1-methyl-3-[4-(4-morpholinyl)phenyl]-1*H*-pyrazol-5-yl}carbonyl)amino]methyl}phenyl)oxy]propanoic acid;
2-methyl-2-[(2-methyl-4-{[({1-methyl-5-[3-(1-piperidinyl)phenyl]-1*H*-pyrazol-3-yl}carbonyl)amino]methyl}phenyl)oxy]propanoic acid;
2-methyl-2-[(2-methyl-4-{[({1-methyl-5-[3-(1-pyrrolidinyl)phenyl]-1*H*-pyrazol-3-yl}carbonyl)amino]methyl}phenyl)oxy]propanoic acid;
2-methyl-2-{2-methyl-4-[({[1-methyl-3-(3-piperidin-1-ylphenyl)-1*H*-pyrazol-5-yl]carbonyl}amino)methyl]phenoxy}propanoic acid;
2-[(4-{[({3-[4-(1,1-dimethylethyl)phenyl]-1-methyl-1*H*-pyrazol-5-yl}acetyl)amino]methyl}-2-methylphenyl)oxy]-2-methylpropanoic acid;
2-[(4-{[({5-[4-(1,1-dimethylethyl)phenyl]-1-methyl-1*H*-pyrazol-3-yl}acetyl)amino]methyl}-2-methylphenyl)oxy]-2-methylpropanoic acid;
2-methyl-2-[(2-methyl-4-{[({1-methyl-3-[4-(1-piperidinyl)phenyl]-1*H*-pyrazol-5-yl}carbonyl)amino]methyl}phenyl)oxy]propanoic acid;
2-{[4-({[(3-{[4-(1,1-dimethylethyl)phenyl]methyl}-1-methyl-1*H*-pyrazol-5-yl)carbonyl]amino}methyl)-2-methylphenyl]oxy}-2-methylpropanoic acid;
2-({4-[({[3-[4-(1,1-dimethylethyl)phenyl]-1-(2-propen-1-yl)-1*H*-pyrazol-5-yl]carbonyl}amino)methyl]-2-methylphenyl}oxy)-2-methylpropanoic acid;
2-[(4-{[({3-[4-(1,1-dimethylethyl)phenyl]-1-[2-(methyloxy)ethyl]-1*H*-pyrazol-5-yl}carbonyl)amino]methyl}-2-methylphenyl)oxy]-2-methylpropanoic acid;
2-({4-[({[3-[4-(1,1-dimethylethyl)phenyl]-1-(2-oxo-2-phenylethyl)-1*H*-pyrazol-5-yl]carbonyl}amino)methyl]-2-methylphenyl}oxy)-2-methylpropanoic acid;
2-({4-[({[5-[4-(1,1-dimethylethyl)phenyl]-1-(phenylmethyl)-1*H*-pyrazol-3-yl]carbonyl}amino)methyl]-2-methylphenyl}oxy)-2-methylpropanoic acid;
2-({5-[({[3-[4-(1,1-dimethylethyl)phenyl]-1-(2-phenylethyl)-1*H*-pyrazol-5-yl]carbonyl}amino)methyl]-2-methylphenyl}oxy)-2-methylpropanoic acid;
2-[(4-{[({5-[4-(1,1-dimethylethyl)phenyl]-1-[2-(methyloxy)ethyl]-1*H*-pyrazol-3-yl}carbonyl)amino]methyl}-2-methylphenyl)oxy]-2-methylpropanoic acid;
2-[(4-{[({5-[4-(1,1-dimethylethyl)phenyl]-1*H*-pyrazol-3-yl}carbonyl)amino] methyl}-2-methylphenyl)oxy]-2-methylpropanoic acid;
2-[(4-{[({5-[4-(1,1-dimethylethyl)phenyl]-1-[2-(4-morpholinyl)ethyl]-1*H*-pyrazol-3-yl}carbonyl)amino]methyl}-2-methylphenyl)oxy]-2-methyl propanoic acid hydrochloride;
2-[(4-{[({3-[4-(1,1-dimethylethyl)phenyl]-1-[2-(4-morpholinyl)ethyl]-1*H*-pyrazol-5-yl}carbonyl)amino]methyl}-2-methylphenyl)oxy]-2-methylpropanoic acid;
2-methyl-[(2-methyl-4-{[({1-methyl-3-[4-(2-propen-1-yloxy)phenyl]-1*H*-pyrazol-5-yl}carbonyl)amino]methyl}phenyl)oxy]propanoic acid;
2-methyl-2-{[2-methyl-4-({[(1-methyl-3-{4-[(phenylmethyl)oxy]phenyl}-1*H*-pyrazol-5-yl)carbonyl]amino)methyl)phenyl]oxy}propanoic acid;
2-methyl-2-{[2-methyl-4-({[(1-methyl-5-(4-[(phenylmethyl)oxy]phenyl}-1*H*-pyrazol-3-yl)carbonyl]amino}methyl)phenyl]oxy}propanoic acid;
2-methyl-2-[{2-methyl-4-{[({1-methyl-5-[4-(2-propen-1-yloxy)phenyl]-1*H*-pyrazol-3-yl)carbonyl)amino]methyl}phenyl)oxy]propanoic acid;
2-({4-[({[3-[4-(1,1-dimethylethyl)phenyl]-1-(2-propen-1-yl)-1*H*-pyrazol-5-yl]carbonyl}amino)methyl]phenyl}oxy)-2-methylpropanoic acid;
2-({4-[({[3-[4-(1,1-dimethylethyl)phenyl]-1-(phenylmethyl)-1*H*-pyrazol-5-yl]carbonyl}amino)methyl]-2-methylphenyl}oxy)-2-methylpropanoic acid;
2-({4-[({[5-[4-(1,1-dimethylethyl)phenyl]-1-(2-propen-1-yl)-1*H*-pyrazol-3-yl]carbonyl}amino)methyl]-2-methylphenyl}oxy)-2-methylpropanoic acid.

17. A compound according to claim 16 which is 2-[(4-{[({5-[4-(1,1-dimethylethyl)phenyl]-1-methyl-1*H*-pyrazol-3-yl}carbonyl)amino]methyl}-2-methylphenyl)oxy]-2-methylpropanoic acid;

18. A compound of claim 1 which is a compound. of formula (la) and pharmaceutically acceptable salts, solvates and hydrolysable esters thereof: 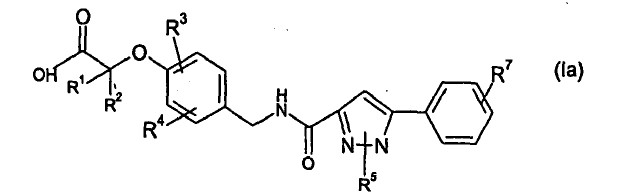 wherein
R¹ and R² are Independently H or C₁₋₃ alkyl;
R³ and R⁴ are independently H, C₁₋₆ alkyl, -OC₁₋₆ alkyl, halogen, OH, C₂₋₆ alkenyl, CF₃
R⁵ is H, C₁₋₆ alkyl or CF₃,
R⁷ is C₁₋₆ alkyl.

19. A compound according to claims 1 - 18 for use in therapy.

20. A compound according to claims 1 - 18 for use in treating a hPPAR mediated disease or condition.

21. A compound according to claims 1 - 18 for use in treating dyslipidemia, syndrome X, heart failure, hypercholesteremia, cardiovascular disease, type II diabetes mellitus, type I diabetes, insulin resistance, hyperlipidemia, obesity, anorexia bulimia and anorexia nervosa.

22. A pharmaceutical composition comprising a compound according to claims 1 - 18.

23. A pharmaceutical composition according to claim 22 further comprising a pharmaceutically acceptable carrier.

24. Use of a compound according to any of claims 1 - 18 for the manufacture of a medicament for the treatment of a hPPAR disease or condition.

25. Use according to claim 24 wherein the hPPAR mediated disease or condition is dyslipidemia, syndrome X, heart failure, hypercholesteremia, cardiovascular disease, type II diabetes mellitus, type I diabetes, insulin resistance, hyperlipidemia, obesity, anorexia bulimia and anorexia nervosa.

## Patentansprüche

1. Verbindung der Formel (I) und pharmazeutisch akzeptable Salze, Solvate und hydrolysierbare Ester davon: 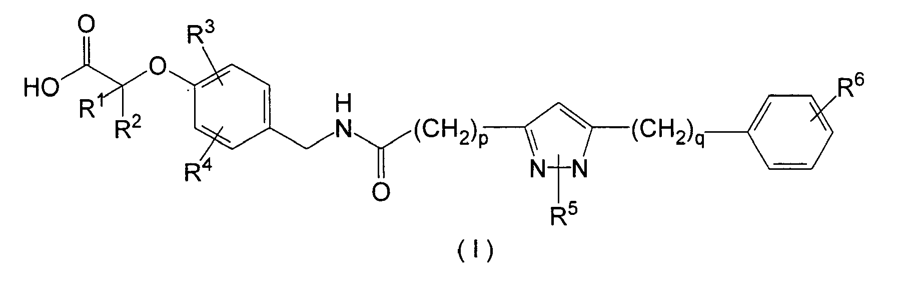 worin
p 0 oder 1 ist;
q 0 oder 1 ist;
R¹ und R² unabhängig H oder C₁₋₃-Alkyl sind;
R³ und R⁴ unabhängig H, C₁₋₆-Alkyl, -OC₁₋₆-Alkyl, Halogen, OH, C₂₋₆-Alkenyl oder CF₃ sind;
R⁵ H, C₁₋₆-Alkyl (gegebenenfalls substituiert mit einem oder mehreren Halogenen, -CO-Phenyl, OC₁₋₆-Alkyl, Phenyl oder Morpholino) oder C₂₋₆-Alkenyl ist;
R⁶ C₁₋₆-Alkyl, Halogen, -OCH₂-Phenyl, Phenyl (gegebenenfalls substituiert mit C₁₋₃-Alkyl), Morpholino, Pyrrolidino, Piperidino, Thiophenyl, Furanyl, Pyridinyl oder -OC₂₋₆-Alkenyl ist.

2. Verbindung gemäss Anspruch 1, die ein selektiver hPPAR-α/γ-Agonist ist.

3. Verbindung gemäss Anspruch 1, worin p 0 ist.

4. Verbindung gemäss Anspruch 1, worin q 0 ist.

5. Verbindung gemäss Ansprüchen 1 bis 3, worin p und q 0 sind.

6. Verbindung gemäss Ansprüchen 1 bis 5, worin R¹ und R² unabhängig C₁₋₃-Alkyl sind.

7. Verbindung gemäss Anspruch 6, worin R¹ und R² beide C₁₋₃-Alkyl sind.

8. Verbindung gemäss Anspruch 7, worin R¹ und R² beide Methyl sind.

9. Verbindung gemäss Ansprüchen 1 bis 8, worin R⁴ H ist.

10. Verbindung gemäss Ansprüchen 1 bis 9, worin R³ -C₁₋₃-Alkyl oder -OC₁₋₃-Alkyl ist.

11. Verbindung gemäss Anspruch 10, worin R³ Methyl oder -OCH₃ ist.

12. Verbindung gemäss Ansprüchen 1 bis 11, worin R³ ortho zu dem dargestellten Sauerstoff ist.

13. Verbindung gemäss Ansprüchen 1 bis 9, worin R³ Methyl ist.

14. Verbindung gemäss Ansprüchen 1 bis 10, worin R⁶ C₁₋₆-Alkyl ist.

15. Verbindung gemäss Ansprüchen 1 bis 11, worin R⁶ in para-Position ist.

16. Verbindung gemäss Anspruch 1, ausgewählt aus:
2-[(4-{[({5-[4-(1,1-Dimethylethyl)phenyl]-1-methyl-1H-pyrazol-3-yl}carbonyl)amino]methyl}-2-methylphenyl)oxy]-2-methylpropionsäure;
2-Methyl-2-[(2-methyl-4-{[({1-methyl-3-[4-(1-methylethyl)phenyl]-1H-pyrazol-5-yl}carbonyl)amino]-methyl}phenyl)oxy]propionsäure;
2-{[4-{[({3-[4-(1,1-Dimethylethyl)phenyl]-1-methyl-1H-pyrazol-5-yl}carbonyl)amino]methyl}-2-(2-propen-1-yl)phenyl]oxy}-2-methylpropionsäure;
2-[(4-{[({3-[4-(1,1-Dimethylethyl)phenyl]-1-methyl-1H-pyrazol-5-yl}carbonyl)amino]methyl}-2-propylphenyl)oxy]-2-methylpropionsäure;
2-{[4-{[({3-[4-(1,1-Dimethylethyl)phenyl]-1-ethyl-1H-pyrazol-5-yl}carbonyl)amino]methyl}-2-(methyloxy)-phenyl]oxy}-2-methylpropionsäure;
2-{[4-{[({5-[4-(1,1-Dimethylethyl)phenyl]-1-ethyl-1H-pyrazol-3-yl}carbonyl)amino]methyl}-2-(methyloxy)-phenyl]oxy}-2-methylpropionsäure;
2-Methyl-2-[(2-methyl-4-{[({1-methyl-5-[4-(2-methylpropyl)phenyl]-1H-pyrazol-3-yl}carbonyl)-amino]methyl}phenyl)oxy]propionsäure;
2-Methyl-2-[(2-methyl-4-{[({1-methyl-3-[4-(2-methylpropyl)phenyl]-1H-pyrazol-5-yl}carbonyl)amino]-methyl}phenyl)oxy]propionsäure;
2-Methyl-2-{[4-{[({1-methyl-5-[4-(1-methylethyl)-phenyl]-1H-pyrazol-3-yl}carbonyl)amino]methyl}-2-(methyloxy)phenyl]oxy}propionsäure;
2-Methyl-2-{(4-{[({1-methyl-3-[4-(1-methylethyl)-phenyl]-1H-pyrazol-5-yl}carbonyl)amino]methyl}-2-(methyloxy)phenyl)oxy}propionsäure;
2-[(4-{[({3-[4-(1,1-Dimethylethyl)phenyl]-1-methyl-1H-pyrazol-5-yl}carbonyl)amino]methyl}-2-methylphenyl)oxy]-2-methylpropionsäure;
2-{[4-{[({3-[4-(1,1-Dimethylethyl)phenyl]-1-methyl-1H-pyrazol-5-yl}carbonyl)amino]methyl}-2-(methyloxy)-phenyl]oxy}-2-methylpropionsäure;
2-[(4-{[({5-[4-(1,1-Dimethylethyl)phenyl]-1-ethyl-1H-pyrazol-3-yl}carbonyl)amino]methyl}-2-methylphenyl)-oxy]-2-methylpropionsäure;
2-Methyl-2-{[4-{[({1-methyl-5-[4-(2-methylpropyl)-phenyl]-1H-pyrazol-3-yl}carbonyl)amino]methyl}-2-(methyloxy)phenyl]oxy}propionsäure;
2-[(4-{[({3-[4-(1,1-Dimethylethyl)phenyl]-1-ethyl-1H-pyrazol-5-yl}carbonyl)amino]methyl}-2-methylphenyl)-oxy]-2-methylpropionsäure;
2-[(4-{[({3-[4-(1,1-Dimethylethyl)phenyl]-1-methyl-1H-pyrazol-5-yl}carbonyl)amino]methyl}phenyl)oxy]-2-methylpropionsäure;
2-[(4-{[({5-[4-(1,1-Dimethylethyl)phenyl]-1-methyl-1H-pyrazol-3-yl}carbonyl)amino]methyl}phenyl)oxy]-2-methylpropionsäure;
2-Methyl-2-[(2-methyl-4-{[({1-methyl-5-[4-(1-methylethyl)phenyl]-1H-pyrazol-3-yl}carbonyl)amino]-methyl}phenyl)oxy]propionsäure;
2-Methyl-2-[(2-methyl-4-{[({1-methyl-5-[4-(4-morpholinyl)phenyl]-1H-pyrazol-3-yl}carbonyl)amino]-methyl}phenyl)oxy]propionsäure;
2-Methyl-2-[(2-methyl-4-{[({1-methyl-5-[4-(1-pyrrolidinyl)phenyl]-1H-pyrazol-3-yl}carbonyl)amino]-methyl}phenyl)oxy]propionsäure;
2-Methyl-2-[(2-methyl-4-{[({1-methyl-5-[4-(1-piperidinyl)phenyl]-1H-pyrazol-3-yl}carbonyl)amino]-methyl}phenyl)oxy]propionsäure;
2-({-4-[({[5-(4-Biphenylyl)-1-methyl-1H-pyrazol-3-yl]carbonyl}amino)methyl]-2-methylphenyl}oxy)-2-methylpropionsäure;
2-Methyl-2-[(2-methyl-4-{[({1-methyl-3-[3-(1-pyrrolidinyl)phenyl]-1H-pyrazol-5-yl}carbonyl)amino]-methyl}phenyl)oxy]propionsäure;
2-({4-[({[3-(4-Biphenyl)-1-methyl-1H-pyrazol-5-yl]carbonyl}amino)methyl]-2-methylphenyl}oxy)-2-methylpropionsäure;
2-{4-[({[5-(4-tert-Butylphenyl)-1-methyl-1H-pyrazol-3-yl]carbonyl}amino)methyl]-2,6-dimethylphenoxy}-2-methylpropionsäure;
2-{4-[({[3-(4-tert-Butylphenyl)-1-methyl-1H-pyrazol-5-yl]carbonyl}amino)methyl]-2,6-dimethylphenoxy}-2-methylpropionsäure;
2-{2-Chlor-4-[({[5-(4-isobutylphenyl)-1-methyl-1H-pyrazol-3-yl]carbonyl}amino)methyl]-6-methylphenoxy}-2-methylpropionsäure;
2-[4-({[(3-Biphenyl-3-yl-1-methyl-1H-pyrazol-5-yl)carbonyl]amino}methyl)-2-methylphenoxy]-2-methylpropionsäure;
2-({4-[({[5-(4-Butylphenyl)-1-methyl-1H-pyrazol-3-yl]carbonyl}amino)methyl]-2-methylphenyl}oxy)-2-methylpropionsäure;
2-({4-[({[5-(4-Bromphenyl)-1-methyl-1H-pyrazol-3-yl]carbonyl}amino)methyl]-2-methylphenyl}oxy-2-methylpropionsäure;
2-Methyl-2-{2-methyl-4-[({[1-methyl-5-(2'-methylbiphenyl-4-yl)-1H-pyrazol-3-yl]carbonyl}amino)-methyl]phenoxy}propionsäure;
2-Methyl-2-[(2-methyl-4-{[({1-methyl-5-[4-(2-thienyl)phenyl]-1H-pyrazol-3-yl}carbonyl)amino]-methyl}phenyl)oxy]propionsäure;
2-[(4-{[({5-[4-(3-Furanyl)phenyl]-1-methyl-1H-pyrazol-3-yl}carbonyl)amino]methyl}-2-methylphenyl)-oxy]-2-methylpropionsäure;
2-Methyl-2-[(2-methyl-4-{[({1-methyl-5-[4-(4-pyridinyl)phenyl]-1H-pyrazol-3-yl}carbonyl)amino]-methyl}phenyl)oxy]propionsäure;
2-[(4-{[({5-[4-(2-Furanyl)phenyl]-1-methyl-1H-pyrazol-3-yl}carbonyl)amino]methyl}-2-methylphenyl)-oxy]-2-methylpropionsäure;
2-Methyl-2-{2-methyl-4-[({[1-methyl-3-(2'-methylbiphenyl-4-yl)-1H-pyrazol-5-yl]carbonyl}amino)-methyl]phenoxy}propionsäure;
2-{4-[({[5-(4-Butylphenyl)-1-methyl-1H-pyrazol-3-yl]carbonyl}amino)methyl]-2,6-dimethylphenoxy}-2-methylpropionsäure;
2-{[4-({2-{4-[({[5-(4-Butylphenyl)-1-methyl-1H-pyrazol-3-yl]carbonyl}amino)methyl]-2-chlor-6-methylphenoxy}-2-methylpropionsäure;
2-Methyl-2-[(2-methyl-4-{[({1-methyl-3-[4-(4-morpholinyl)phenyl]-1H-pyrazol-5-yl}carbonyl)amino]-methyl}phenyl)oxy]propionsäure;
2-Methyl-2-[(2-methyl-4-{[({1-methyl-5-[3-(1-piperidinyl)phenyl]-1H-pyrazol-3-yl}carbonyl)amino]-methyl}phenyl)oxy]propionsäure;
2-Methyl-2-[(2-methyl-4-{[({1-methyl-5-[3-(1-pyrrolidinyl)phenyl]-1H-pyrazol-3-yl}carbonyl)amino]-methyl}phenyl)oxy]propionsäure;
2-Methyl-2-{2-methyl-4-[({[1-methyl-3-(3-piperidin-1-ylphenyl)-1H-pyrazol-5-yl]carbonyl}amino)methyl]-phenoxy}propionsäure;
2-[(4-{[({3-[4-(1,1-Dimethylethyl)phenyl]-1-methyl-1H-pyrazol-5-yl}acetyl)amino]methyl}-2-methylphenyl)-oxy]-2-methylpropionsäure;
2-[(4-{[({5-[4-(1,1-Dimethylethyl)phenyl]-1-methyl-1H-pyrazol-3-yl}acetyl)amino]methyl}-2-methylphenyl)-oxy]-2-methylpropionsäure;
2-Methyl-2-[(2-methyl-4-{[({1-methyl-3-[4-(1-piperidinyl)phenyl]-1H-pyrazol-5-yl}carbonyl)amino]-methyl}phenyl)oxy]propionsäure;
2-{[4-({[(3-{[4-(1,1-Dimethylethyl)phenyl]methyl}-1-methyl-1H-pyrazol-5-yl)carbonyl]amino}methyl)-2-methylphenyl]oxy}-2-methylpropionsäure;
2-({4-[({[3-[4-(1,1-Dimethylethyl)phenyl]-1-(2-propen-1-yl)-1H-pyrazol-5-yl]carbonyl}amino)methyl]-2-methylphenyl}oxy)-2-methylpropionsäure;
2-[(4-{[({3-[4-(1,1-Dimethylethyl)phenyl]-1-[2-(methyloxy)ethyl]-1H-pyrazol-5-yl}carbonyl)amino]-methyl}-2-methylphenyl)oxy]-2-methylpropionsäure;
2-({4-[({[3-[4-(1,1-Dimethylethyl)phenyl]-1-(2-oxo-2-phenylethyl)-1H-pyrazol-5-yl]carbonyl}amino)methyl]-2-methylphenyl}oxy)-2-methylpropionsäure;
2-({4-[({[5-[4-(1,1-Dimethylethyl)phenyl]-1-(phenylmethyl)-1H-pyrazol-3-yl]carbonyl}amino)-methyl]-2-methylphenyl}oxy)-2-methylpropionsäure;
2-({5-[({[3-[4-(1,1-Dimethylethyl)phenyl]-1-(2-phenylethyl)-1H-pyrazol-5-yl]carbonyl}amino)methyl]-2-methylphenyl}oxy)-2-methylpropionsäure;
2-[(4-{[({5-[4-(1,1-Dimethylethyl)phenyl]-1-[2-(methyloxy)ethyl]-1H-pyrazol-3-yl}carbonyl)amino]-methyl}-2-methylphenyl)oxy]-2-methylpropionsäure;
2-[(4-{[({5-[4-(1,1-Dimethylethyl)phenyl]-1H-pyrazol-3-yl}carbonyl)amino]methyl}-2-methylphenyl)oxy]-2-methylpropionsäure;
2-[(4-{[({5-[4-(1,1-Dimethylethyl)phenyl]-1-[2-(4-morpholinyl)ethyl]-1H-pyrazol-3-yl}carbonyl)amino]-methyl}-2-methylphenyl)oxy]-2-methylpropionsäurehydrochlorid;
2-[(4-{[({3-[4-(1,1-Dimethylethyl)phenyl]-1-[2-(4-morpholinyl)ethyl]-1H-pyrazol-5-yl}carbonyl)amino]-methyl}-2-methylphenyl)oxy]-2-methylpropionsäure;
2-Methyl-2-[(2-methyl-4-{[({1-methyl-3-[4-(2-propen-1-yloxy)phenyl]-1H-pyrazol-5-yl}carbonyl)amino]-methyl}phenyl)oxy]propionsäure;
2-Methyl-2-{[2-methyl-4-({[(1-methyl-3-{4-[(phenylmethyl)oxy]phenyl}-1H-pyrazol-5-yl)carbonyl]-amino}methyl)phenyl]oxy}propionsäure;
2-Methyl-2-{[2-methyl-4-({[(1-methyl-5-{4-[(phenylmethyl)oxy]phenyl}-1H-pyrazol-3-yl)carbonyl]-amino}methyl)phenyl]oxy}propionsäure;
2-Methyl-2-[(2-methyl-4-{[({1-methyl-5-[4-(2-propen-1-yloxy)phenyl]-1H-pyrazol-3-yl}carbonyl)amino]-methyl}phenyl)oxy]propionsäure;
2-({4-[({[3-[4-(1,1-Dimethylethyl)phenyl]-1-(2-propen-1-yl)-1H-pyrazol-5-yl]carbonyl}amino)-methyl]phenyl}oxy)-2-methylpropionsäure;
2-({4-[({[3-[4-(1,1-Dimethylethyl)phenyl]-1-(phenylmethyl)-1H-pyrazol-5-yl]carbonyl}amino)-methyl]-2-methylphenyl}oxy)-2-methylpropionsäure;
2-({4-[({[5-[4-(1,1-Dimethylethyl)phenyl]-1-(2-propen-1-yl)-1H-pyrazol-3-yl]carbonyl}amino)methyl]-2-methylphenyl}oxy)-2-methylpropionsäure.

17. Verbindung gemäss Anspruch 16, die 2-[(4-{[({5-[4-(1,1-Dimethylethyl)phenyl]-1-methyl-1H-pyrazol-3-yl}carbonyl)amino]methyl}-2-methylphenyl)oxy]-2-methylpropionsäure ist.

18. Verbindung gemäss Anspruch 1, die eine Verbindung der Formel (Ia) ist, und pharmazeutisch akzeptable Salze, Solvate und hydrolysierbare Ester davon: 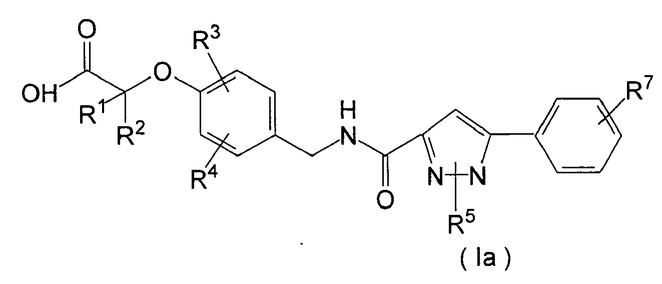 worin
R¹ und R² unabhängig H oder C₁₋₃-Alkyl sind;
R³ und R⁴ unabhängig H, C₁₋₆-Alkyl, -OC₁₋₆-Alkyl, Halogen, OH, C₂₋₆-Alkenyl oder CF₃ sind;
R⁵ H, C₁₋₆-Alkyl oder CF₃ ist;
R⁷ C₁₋₆-Alkyl ist.

19. Verbindung gemäss Ansprüchen 1 bis 18 für die therapeutische Verwendung.

20. Verbindung gemäss Ansprüchen 1 bis 18 für die Verwendung bei der Behandlung einer/eines durch hPPAR vermittelten Erkrankung oder Zustands.

21. Verbindung gemäss Ansprüchen 1 bis 18 für die Verwendung bei der Behandlung von Dyslipidämie, Syndrom X, Herzversagen, Hypercholesterinämie, einer kardiovaskulären Erkrankung, Typ II Diabetes mellitus, Typ I Diabetes, Insulinresistenz, Hyperlipidämie, Fettleibigkeit, Anorexia bulimia und Anorexia nervosa.

22. Pharmazeutische Zusammensetzung, umfassend eine Verbindung gemäss den Ansprüchen 1 bis 18.

23. Pharmazeutische Zusammensetzung gemäss Anspruch 22, ferner umfassend einen pharmazeutisch akzeptablen Träger.

24. Verwendung einer Verbindung gemäss einem der Ansprüche 1 bis 18 für die Herstellung eines Medikaments zur Behandlung einer/eines hPPAR-Erkrankung oder Zustands.

25. Verwendung gemäss Anspruch 24, worin die/der hPPARvermittelte Erkrankung oder Zustand Dyslipidämie, Syndrom X, Herzversagen, Hypercholesterinämie, eine kardiovaskuläre Erkrankung, Typ II Diabetes mellitus, Typ I Diabetes, Insulinresistenz, Hyperlipidämie, Fettleibigkeit, Anorexia bulimia und Anorexia nervosa ist.

## Revendications

1. Composé de formule (I) et sels, solvates et esters hydrolysables pharmaceutiquement acceptables de celui-ci 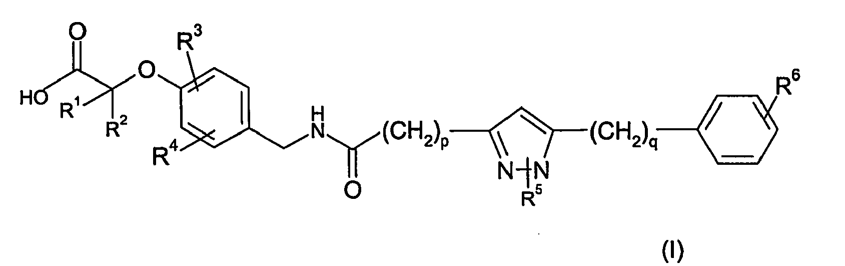 où
p vaut 0 ou 1 ;
q vaut 0 ou 1 ;
R¹ et R² représentent indépendant H ou un groupe alkyle en C₁ à C₃ ;
R³ et R⁴ représentent indépendamment H, un groupe alkyle en C₁ en C₆, O-alkyle en C₁ en C₆, halogène, OH, alcényle en C₂ en C₆ ou CF₃ ;
R⁵ représente H, un groupe alkyle en C₁ en C₆ (éventuellement substitué par un ou plusieurs atomes d'halogène, -CO-phényle, O-alkyle en C₁ en C₆, phényle, morpholino) ou alcényle en C₂ en C₆ ;
R⁶ représente un groupe alkyle en C₁ en C₆, halogène, -OCH₂-phényle, phényle (éventuellement substitué par un groupe alkyle en C₁ en C₃), morpholino, pyrrolidino, pipéridino, thiophényle, furanyle, pyridinyle ou -O-alcényle en C₂ en C₆.

2. Composé selon la revendication 1 qui est un agoniste sélectif du hPPAR alpha/gamma.

3. Composé selon la revendication 1, dans lequel p vaut 0.

4. Composé selon la revendication 1, dans lequel q vaut 0.

5. Composé selon les revendications 1 à 3, dans lequel p et q valent 0.

6. Composé selon les revendications 1 à 5, dans lequel R¹ et R² représentent indépendamment un groupe alkyle en C₁ en C₃.

7. Composé selon la revendication 6, dans lequel R¹ et R² représentent tous les deux un groupe alkyle en C₁ en C₃.

8. Composé selon la revendication 7, dans lequel R¹ et R² représentent tous les deux un groupe méthyle.

9. Composé selon les revendications 1 à 8 dans lequel R⁴ représente H.

10. Composé selon les revendications 1 à 9, dans lequel R³ représente un groupe alkyle en C₁ en C₃ ou -O-alkyle en C₁ en C₃.

11. Composé selon la revendication 10, dans lequel R³ représente un groupe méthyle ou -OCH₃.

12. Composé selon les revendications 1 à 11, dans lequel R³ est en ortho par rapport à l'atome d'oxygène illustré.

13. Composé selon les revendications 1 à 9, dans lequel R³ représente un groupe méthyle.

14. Composé selon les revendications 1 à 10, dans lequel R⁶ représente un groupe alkyle en C₁ en C₆.

15. Composé selon les revendications 1 à 11, dans lequel R⁶ est en position para.

16. Composé selon la revendication 1, choisi parmi :
l'acide 2-[(4-{[({5-[4-(1,1-diméthyléthyl)phényl]-1-méthyl-1*H*-pyrazol-3-yl}carbonyl)amino]méthyl}-2-méthylphényl)oxy]-2-méthylpropanoïque ;
l'acide 2-méthyl-2-[(2-méthyl-4-{[({1-méthyl-3-[4-(1-méthyléthyl)phényl]-1*H*-pyrazol-5-yl}carbonyl)amino]méthyl}phényl)oxy]propanoïque ;
l'acide 2-[(4-{[({3-[4-(1,1-diméthyléthyl)phényl]-1-méthyl-1*H*-pyrazol-5-yl}carbonyl)amino]méthyl}-2-(2-propén-1-yl)phényl)oxy]-2-méthylpropanoïque ;
l'acide 2- [(4-{ [( { 3- [4- (1, 1-diméthyléthyl) phényl] -1-méthyl-1*H*-pyrazol-5-yl}carbonyl)amino]méthyl}-2-propylphényl)-oxy]-2-méthylpropanoïque ;
l'acide 2-[(4-{[({3-[4-(1,1-diméthyléthyl)phényl]-1-éthyl-1*H*-pyrazol-5-yl}carbonyl)amino]méthyl}-2-(méthyloxy)-phényl)oxy]-2-méthylpropanoïque ;
l'acide 2-[(4-{[({5-[4-(1,1-diméthyléthyl)phényl]-1-méthyl-1*H*-pyrazol-3-yl}carbonyl)amino]méthyl}-2-(méthyloxy)-phényl)oxy]-2-méthylpropanoïque ;
l'acide 2-méthyl-2-[(2-méthyl-4-{[({1-méthyl-5-[4-(2-méthylpropyl)phényl]-1*H*-pyrazol-3-yl}carbonyl)amino]méthyl}phényl)oxy]propanoïque ;
l'acide 2-méthyl-2-[(2-méthyl-4-{[({1-méthyl-3-[4-(2-méthylpropyl)phényl]-1*H*-pyrazol-5-yl}carbonyl)amino]méthyl}-phényl)oxy]propanoïque ;
l'acide 2-méthyl-2-{[4-{[({1-méthyl-5-[4-(1-méthylëthyl)-phënyl]-1*H*-pyrazol-3-yl}carbonyl)amino]méthyl}-2-(mëthyloxy)phényl]oxy}propanoïque ;
l'acide 2-méthyl-2-{[4-([((1-méthyl-3- [4- (1-méthyléthyl)-phényl]-1*H*-pyrazol-5-yl)carbonyl)amino]méthyl)-2-(méthyloxy)phényl]oxy}propanoïque ;
l'acide 2-[(4-{[({3-[4-(1,1-diméthyléthyl)phényl]-1-méthyl-1*H*-pyrazol-5-yl}carbonyl)amino]méthyl}-2-méthylphényl)-oxy]-2-méthylpropanoïque ;
l'acide 2-{[4-{[({3-[4-(1,1-diméthyléthyl)phényl]-1-méthyl-1*H*-pyrazol-5-yl}carbonyl)amino]méthyl}-2-(méthyloxy)-phényl]oxy}-2-méthylpropanoïque ;
l'acide 2-[(4-{[({5-[4-(1,1-diméthyléthyl)phényl]-1-éthyl-1*H*-pyrazol-3-yl}carbonyl)amino]méthyl}-2-méthylphényl)-oxy]-2-méthylpropanoïque ;
l'acide 2-méthyl-2-{[4-{[({1-méthyl-5-[4-(2-méthyl-propyl)phényl]-1*H*-pyrazol-3-yl}carbonyl)amino]méthyl}-2-(méthyloxy)phényl]oxy}propanoïque ;
l'acide 2-[(4-{[({3-[4-(1,1-diméthyléthyl)phényl]-1-éthyl-1*H*-pyrazol-5-yl}carbonyl)amino]méthyl}-2-méthylphényl)-oxy]-2-méthylpropanoïque ;
l'acide 2-[(4-{[({3-[4-(1,1-diméthyléthyl)phényl]-1-méthyl-1*H*-pyrazol-5-yl}carbonyl)amino]méthyl}phényl)oxy]-2-méthylpropanoïque ;
l'acide 2-[(4-{[({5-[4-(1,1-diméthyléthyl)phényl]-1-méthyl-1*H*-pyrazol-3-yl}carbonyl)amino]méthyl}phényl)oxy]-2-méthylpropanoïque ;
l'acide 2-méthyl-2-[(2-méthyl-4-{[({1-méthyl-5-[4-(1-méthyléthyl)phényl]-1*H*-pyrazol-3-yl}carbonyl)amino]méthyl}phényl)oxy]propanoïque ;
l'acide 2-méthyl-2-[(2-méthyl-4-{[({1-méthyl-5-[4-(4-morpholinyl)phényl]-1*H*-pyrazol-3-yl}carbonyl)amino]méthyl}phényl)oxy]propanoïque ;
l'acide 2-méthyl-2-[(2-méthyl-4-{[({1-méthyl-5-[4-(1-pyrrolidinyl)phényl]-1*H*-pyrazol-3-yl}carbonyl)amino]méthyl}phényl)oxy]propanoïque ;
l'acide 2-méthyl-2-[(2-méthyl-4-{[({1-méthyl-5-[4-(1-pipéridinyl)phényl]-1*H*-pyrazol-3-yl}carbonyl)amino]méthyl}phényl)oxy]propanoïque ;
l'acide 2-({4-[({[5-(4-biphénylyl)-1-méthyl-1*H*-pyrazol-3-yl]carbonyl}amino)méthyl]-2-méthylphényl}oxy)-2-méthylpropanoïque ;
l'acide 2-méthyl-2-[(2-méthyl-4-{[({1-méthyl-3-[3-(1-pyrrolidinyl)phényl]-1*H*-pyrazol-5-yl}carbonyl)amino]méthyl}phényl)oxy]propanoïque ;
l'acide 2-({4-[({[3-(4-biphénylyl)-1-méthyl-1*H*-pyrazol-5-yl]carbonyl}amino)méthyl]-2-méthylphényl}oxy)-2-méthylpropanoïque ;
l'acide 2-{4-[({[5-(4-tert-butylphényl)-1-méthyl-1*H-*pyrazol-3-yl]carbonyl}amino)méthyl]-2,6-diméthylphénoxy}-2-méthylpropanoïque ;
l'acide 2-{4-[({[3-(4-tert-butylphényl)-1-méthyl-1*H-*pyrazol-5-yl]carbonyl}amino)méthyl]-2,6-diméthylphénoxy}-2-méthylpropanoïque ;
l'acide 2-{2-chloro-4-[({[5-(4-isobutylphényl)-1-méthyl-1*H*-pyrazol-3-yl]carbonyl}amino)méthyl]-6-méthylphénoxy}-2-méthylpropanoïque ;
l'acide 2-[4-({[(3-biphényl-3-yl-1-méthyl-1*H*-pyrazol-5-yl)carbonyl]amino}méthyl)-2-méthylphénoxy]-2-méthylpropanoïque ;
l'acide 2-({4-[({[5-(4-butylphényl)-1-méthyl-1*H*-pyrazol-3-yl]carbonyl}amino)méthyl]-2-méthylphényl}oxy)-2-méthylpropanoïque ;
l'acide 2-({4-[({[5-(4-bromophényl)-1-méthyl-1*H*-pyrazol-3-yl]carbonyl}amino)méthyl]-2-méthylphényl}oxy)-2-méthylpropanoïque ;
l'acide 2-méthyl-2-{2-méthyl-4-[({[1-méthyl-5-(2'-méthylbiphényl-4-yl)-1*H*-pyrazol-3-yl]carbonyl}amino)méthyl]phénoxy}propanoïque ;
l'acide 2-méthyl-2-[(2-méthyl-4-{[({1-méthyl-5-[4-(2-thiényl)phényl]-1*H*-pyrazol-3-yl}carbonyl)amino]méthyl}phényl)-oxy]propanoïque ;
l'acide 2-[(4-{[({5-[4-(3-furanyl)phényl]-1-méthyl-1*H-*pyrazol-3-yl}carbonyl)amino]méthyl}-2-méthylphényl)oxy]-2-méthylpropanoïque ;
l'acide 2-méthyl-2-[(2-méthyl-4-{[({1-méthyl-5-[4-(4-pyridinyl)phényl]-1*H*-pyrazol-3-yl}carbonyl)amino]méthyl}phényl)oxy]propanoïque ;
l'acide 2-[(4-{[({5-[4-(2-furanyl)phényl]-1-méthyl-1*H-*pyrazol-3-yl}carbonyl)amino]méthyl}-2-méthylphényl)oxy]-2-méthylpropanoïque ;
l'acide 2-méthyl-2-{2-méthyl-4-[({[1-méthyl-3-(2'-méthylbiphényl-4-yl)-1*H*-pyrazol-5-yl]carbonyl}amino)méthyl]phénoxy}propanoïque ;
l'acide 2-{4-[({[5-(4-butylphényl)-1-méthyl-1*H*-pyrazol-3-yl]carbonyl}amino)méthyl]-2,6-diméthylphénoxy}-2-méthylpropanoïque ;
l'acide 2-{[4-({2-[4-[({[5-(4-butylphényl)-1-méthyl-1*H-*pyrazol-3-yl]carbonyl}amino)méthyl]-2-chloro-6-méthylphénoxy}-2-méthylpropanoïque ;
l'acide 2-méthyl-2-[(2-méthyl-4-{[({1-méthyl-3-[4-(4-morpholinyl)phényl]-1*H*-pyrazol-5-yl}carbonyl)amino]méthyl}phényl)oxy]propanoïque ;
l'acide 2-méthyl-2-[(2-méthyl-4-{[({1-méthyl-5-[3-(1-pipéridinyl)phényl]-1*H*-pyrazol-3-yl}carbonyl)amino]méthyl}phényl)oxy]propanoïque ;
l'acide 2-méthyl-2-[(2-méthyl-4-{[({1-méthyl-5-[3-(1-pyrrolidinyl)phényl]-1*H*-pyrazol-3-yl}carbonyl)amino]méthyl}phényl)oxy]propanoïque ;
l'acide 2-méthyl-2-{2-méthyl-4-[({[1-méthyl-3-(3-pipéridin-1-ylphényl)-1*H*-pyrazol-5-yl]carbonyl}amino)méthyl]-phénoxy}propanoïque ;
l'acide 2-[(4-{[({3-[4-(1,1-diméthyléthyl)phényl]-1-méthyl-1*H*-pyrazol-5-yl}acétyl)amino]méthyl}-2-méthylphényl)-oxy]-2-méthylpropanoïque ;
l'acide 2-[(4-{[({5-[4-(1,1-diméthyléthyl)phényl]-1-méthyl-1*H*-pyrazol-3-yl}acétyl)amino]méthyl}-2-méthylphényl)-oxy]-2-méthylpropanoïque ;
l'acide 2-méthyl-2-[(2-méthyl-4-{[({1-méthyl-3-[4-(1-pipéridinyl)phényl]-1*H*-pyrazol-5-yl}carbonyl)amino]méthyl}phényl)oxy]propanoïque ;
l'acide 2-{[4-({[(3-{[4-(1,1-diméthyléthyl)phényl]-méthyl}-1-méthyl-1*H*-pyrazol-5-yl)carbonyl]amino}méthyl)-2-méthylphényl]oxy}-2-méthylpropanoïque ;
l'acide 2-({4-[({[3-(1,1-diméthyléthyl)phényl)-1-(2-propén-1-yl)-1*H*-pyrazol-5-yl]carbonyl}amino)méthyl]-2-méthylphényl}oxy)-2-méthylpropanoïque ;
l'acide 2-[(4-{[({3-[4-(1,1-diméthyléthyl)phényl]-1-[2-(méthyloxy)éthyl]-1*H*-pyrazol-5-yl}carbonyl)amino]méthyl}-2-méthylphényl)oxy]-2-méthylpropanoïque ;
l'acide 2-({4-[({[3-[4-(1,1-diméthyléthyl)phényl]-1-(2-oxo-2-phényléthyl)-1*H*-pyrazol-5-yl]carbonyl}amino)méthyl]-2-méthylphényl}oxy)-2-méthylpropanoïque ;
l'acide 2-({4-[({[5-[4-(1,1-diméthyléthyl)phényl]-1-(phénylméthyl)-1*H*-pyrazol-3-yl]carbonyl}amino)méthyl]-2-méthylphényl}oxy)-2-méthylpropanoïque ;
l'acide 2-({5-[({[3-[4-(1,1-diméthyléthyl)phényl]-1-(2-phényléthyl)-1*H*-pyrazol-5-yl]carbonyl}amino)méthyl]-2-méthylphényl}oxy)-2-méthylpropanoïque ;
l'acide 2-[(4-{[({5-[4-(1,1-diméthyléthyl)phényl]-1-[2-(méthyloxy)éthyl]-1*H*-pyrazol-3-yl}carbonyl)amino]méthyl}-2-méthylphényl)oxy]-2-méthylpropanoïque ;
l'acide 2- [(4-{ [( {5- [4- (1, 1-diméthyléthyl) phényl] -1*H-*pyrazol-3-yl}carbonyl)amino]méthyl}-2-méthylphényl)oxy]-2-méthylpropanoïque ;
le chlorhydrate de l'acide 2-[(4-{[({5-[4-(1,1-diméthyléthyl)phényl]-1-[2-(4-morpholinyl)éthyl]-1*H*-pyrazol-3-yl}carbonyl)amino]méthyl}-2-méthylphényl)oxy]-2-méthylpropanoïque ;
l'acide 2-[(4-{[({3-[4-(1,1-diméthyléthyl)phényl]-1-[2-(4-morpholinyl)éthyl]-1*H*-pyrazol-5-yl}carbonyl)amino]méthyl)-2-méthylphényl)oxy]-2-méthylpropanoïque ;
l'acide 2-méthyl-2- [(2-méthyl-4-[({1-méthyl-3- [4-(2-propén-1-yloxy)phényl]-1*H*-pyrazol-5-yl}carbonyl)amino]méthyl}phényl)oxy]propanoïque ;
l'acide 2-méthyl-2-{[2-méthyl-4-({[(1-méthyl-3-{4-[(phénylméthyl)oxy]phényl}-1*H*-pyrazol-5-yl)carbonyl]amino}méthyl)phényl}oxy)propanoïque ;
l'acide 2-méthyl-2-{[2-méthyl-4-({[(1-méthyl-5-{4-[(phénylméthyl)oxy]phényl}-1*H*-pyrazol-3-yl)carbonyl]amino}méthyl)phényl}oxy)propanoïque ;
l'acide 2-méthyl-2-[(2-méthyl-4-{[({1-méthyl-5-[4-(2-propén-1-yloxy)phényl]-1*H*-pyrazol-3-yl}carbonyl)amino]méthyl}phényl)oxy]propanoïque ;
l'acide 2-({4-[({[3-[4-(1,1-diméthyléthyl)phényl]-1-(2-propén-1-yl)-1*H*-pyrazol-5-yl]carbonyl}amino)méthyl]phényl}oxy)-2-méthylpropanoïque ;
l'acide 2-({4-[({[3-[4-(1,1-diméthyléthyl)phényl]-1-(phénylméthyl)-1*H*-pyrazol-5-yl]carbonyl}amino)méthyl]-2-méthylphényl}oxy)-2-méthylpropanoïque ;
l'acide 2-({4-[({[5-[4-(1,1-diméthyléthyl)phényl]-1-(2-propén-1-yl)-1*H*-pyrazol-3-yl]carbonyl}amino)méthyl]-2-méthylphényl}oxy)-2-méthylpropanoïque ;

17. Composé selon la revendication 16 qui est l'acide 2-[(4-{[({5-[4-(1,1-diméthyléthyl)phényl]-1-méthyl-1*H*-pyrazol-3-yl}carbonyl)amino]méthyl}-2-méthylphényl)oxy]-2-méthylpropanoïque.

18. Composé selon la revendication 1 qui est un composé de formule (Ia) et des sels, solvates et esters hydrolysables pharmaceutiquement acceptables de celui-ci : 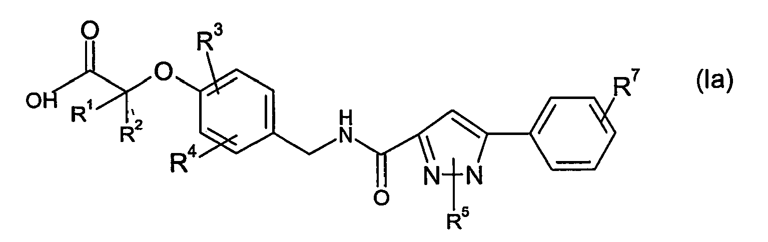 où
R¹ et R² représentent indépendamment H ou un groupe alkyle en C₁ à C₃ ;
R³ et R⁴ représentent indépendamment H, un groupe alkyle en C₁ en C₆, O-alkyle en C₁ en C₆, halogène, OH, alcényle en C₂ en C₆, CF₃ ;
R⁵ représente H, un groupe alkyle en C₁ en C₆ ou CF₃ ;
R⁷ représente un groupe alkyle en C₁ en C₆.

19. Composé selon les revendications 1 à 18 pour une utilisation en thérapie.

20. Composé selon les revendications 1 à 18 pour une utilisation dans le traitement d'une maladie ou d'une affection médiée par le hPPAR.

21. Composé selon les revendications 1 à 18 pour une utilisation dans le traitement d'une dyslipidémie, du syndrome X, d'une insuffisance cardiaque, d'une hypercholestérolémie, d'une maladie cardiovasculaire, d'un diabète de type II, d'un diabète de type I, d'une résistance à l'insuline, d'une hyperlipidémie, de l'obésité, de l'anorexie boulimique et de l'anorexie mentale.

22. Composition pharmaceutique comprenant un composé selon les revendications 1 à 18.

23. Composition pharmaceutique selon la revendication 22 comprenant en outre un support pharmaceutiquement acceptable.

24. Utilisation d'un composé selon l'une quelconque des revendications 1 à 18 pour la fabrication d'un médicament destiné au traitement d'une maladie ou d'une affection médiée par le hPPAR.

25. Utilisation selon la revendication 24, où la maladie ou l'affection médiée par le hPPAR est une dyslipidémie, le syndrome X, une insuffisance cardiaque, une hypercholestérolémie, une maladie cardiovasculaire, un diabète de type II, un diabète de type I, une résistance à l'insuline, une hyperlipidémie, l'obésité, l'anorexie boulimique et l'anorexie mentale.
